(19)

Europäisches Patentamt
European Patent Office
Office européen des brevets

(11) **EP 4 653 499 A2**

(12) **EUROPEAN PATENT APPLICATION**
published in accordance with Art. 153(4) EPC

(43) Date of publication:
26.11.2025 Bulletin 2025/48

(21) Application number: 23769952.5

(22) Date of filing: 17.05.2023

(51) International Patent Classification (IPC):
*C08L 83/07* (2006.01)    *A61K 9/02* (2006.01)
*A61P 15/18* (2006.01)

(52) Cooperative Patent Classification (CPC):
**B29C 48/32; A61L 27/165; A61L 27/54;**
**C08L 83/04;** B29K 2019/00; B29K 2105/0014;
B29K 2105/0088; B29K 2995/0097; B29L 2023/00;
B29L 2031/753; C08L 2314/08

(86) International application number:
**PCT/CN2023/094865**

(87) International publication number:
**WO 2023/174450 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 20.03.2023  CN 202310271571
20.03.2023  CN 202310273723
20.03.2023  CN 202310271569
20.03.2023  CN 202310271568

(71) Applicant: SHENYANG XINGHUA
PHARMACEUTICAL
TECHNOLOGY CO., LTD
Shenyang, Liaoning 110000 (CN)

(72) Inventors:
• **YANG, Xinggang**
**Shenyang, Liaoning 110000 (CN)**
• **PI, Linling**
**Shenyang, Liaoning 110000 (CN)**
• **YU, Hong**
**Shenyang, Liaoning 110000 (CN)**
• **SHEN, Rui**
**Shenyang, Liaoning 110000 (CN)**
• **SUN, Yuanhang**
**Shenyang, Liaoning 110000 (CN)**

(74) Representative: **Michalski Hüttermann & Partner
Patentanwälte mbB
Kaistraße 16A
40221 Düsseldorf (DE)**

(54) **SILICONE MATERIAL, SILICONE TUBE, IMPLANT, PHARMACEUTICAL COMPOSITION, AND TEST METHOD FOR AMOUNT OF DRUG RELEASED**

(57)    Disclosed are a silicone material, a silicone tube, an implant, a pharmaceutical composition, and a test method for the amount of a drug released. a raw material composition of the silicone material comprises the following components in parts by weight: 100 parts of R-vinyl silicone rubber; 20-80 parts of a reinforcing agent; 0.3-3.0 parts of hydrogen-containing silicone oil; and ≥ 0.000002 parts of a catalyst, preferably being 0.000002-0.00005 parts; the content of vinyl groups in the R-vinyl silicone rubber is 0.10-0.50 mol%; the content of Si-H groups in the hydrogen-containing silicone oil is 0.18-1.6 mol%; the molar ratio of the Si-H groups in the hydrogen-containing silicone oil to the vinyl groups in the R-vinyl silicone rubber is (0.5-4):1; and optionally, said material further comprises an inhibitor. A silicone tube prepared by the silicone material has an excellent mechanical property and good biocompatibility; and an implant made from the silicone tube, upon loading with an active drug, has a stable drug release curve.

**EP 4 653 499 A2**

Fig. 1

Methylacetylene polysiloxane

Fumed silica

Kneading via kneader + milling via miller

Hydrogen-containing silicone oil

Methylbutynol

Component A

Component B

Platinum catalyst

Let sit for 24-72 hours

Mixing and thinning pass-through via miller

Extrusion

**Description**

The present application claims the priority of Chinese patent application 202210273495X filed on March 18, 2022. The contents of the Chinese patent application are incorporated herein by reference in their entireties.

The present application claims the priority of Chinese patent application 2022102727087 filed on March 18, 2022. The contents of the Chinese patent application are incorporated herein by reference in their entireties.

The present application claims the priority of Chinese patent application 2022102727072 filed on March 18, 2022. The contents of the Chinese patent application are incorporated herein by reference in their entireties.

The present application claims the priority of Chinese patent application 2022102791223 filed on March 18, 2022. The contents of the Chinese patent application are incorporated herein by reference in their entireties.

The present application claims the priority of Chinese patent application 2023102715686 filed on March 20, 2023. The contents of the Chinese patent application are incorporated herein by reference in their entireties.

The present application claims the priority of Chinese patent application 2023102715690 filed on March 20, 2023. The contents of the Chinese patent application are incorporated herein by reference in their entireties.

The present application claims the priority of Chinese patent application 2023102737238 filed on March 20, 2023. The contents of the Chinese patent application are incorporated herein by reference in their entireties.

The present application claims the priority of Chinese patent application 2023102715718 filed on March 20, 2023. The contents of the Chinese patent application are incorporated herein by reference in their entireties.

TECHNICAL FIELD

The present disclosure relates to a silicone material, a silicone tube, an implant, a pharmaceutical composition, and a test method for the amount of a drug released.

BACKGROUND

Modern commonly used contraceptive methods include non-hormonal contraception (male/female condoms, male/female sterilization, etc.) and hormonal contraception (various oral contraceptives, subcutaneous implants, progestogen intrauterine devices, contraceptive injections, etc.). Among them, hormonal contraceptive methods have consistently demonstrated their reliable contraceptive effect.

Since the concept of "sustained-release and controlled-release preparations" was proposed, there has been significant development in contraceptive sustained-release and controlled-release preparations. These preparations effectively avoid the harms associated with surgical contraception and address issues such as low compliance with oral contraceptives. Sustained-release and controlled-release preparations that provide long-term contraception, especially subcutaneous contraceptive implants, have a very promising future in the field of birth control.

Levonorgestrel (LNG), also known as d-norgestrel, has been included in the pharmacopoeias of China, the United States, the United Kingdom, Japan, and other countries. Currently, there are many single-tablet preparations and levonorgestrel-based combination contraceptives available on the international and domestic markets, which are the earliest and most widely used over-the-counter medications in clinical practice.

Gestodene, a third-generation contraceptive, can achieve a contraceptive effect at a very small clinical dose without any estrogenic activity. It possesses both good anti-estrogenic activity and slight androgenic activity with bioavailability approaching 100%, which is currently the only progestogen that can exert its effect without undergoing metabolic transformation.

Subcutaneous implants primarily involve placing an active drug within a carrier and implanting it subcutaneously. The implant controls the release of the drug through the carrier, and the drug is absorbed into the bloodstream via local capillaries, allowing for stable release over several years and achieving a long-term contraceptive effect. This preparation avoids the first-pass effect in the gastrointestinal tract, thereby greatly enhancing the bioavailability of the drug. Subcutaneous implants are suitable for individuals seeking long-term contraception who are not ideal candidates for IUD placement. After implantation, if there is a desire for pregnancy, removing the implant can immediately restore fertility.

Organosilicon materials are among the best biocompatible synthetic materials available today. As early as the 1960s, medical textbooks recognized organosilicon as an important implant material, and its practical applications are very extensive. Dow Corning Corporation in the United States has provided a large number of literature reports demonstrating the safety of silicone gel breast implants in humans. As research on organosilicon materials has progressed, their applications in the medical field have become increasingly widespread. Researchers have conducted extensive animal experiments, implanting silicon materials into animals for up to three years without any adverse reactions. The safety of these materials implanted into humans has also been progressively validated.

Currently, addition-cure silicone rubber is commonly used in the production of medical products, particularly silicone rubber products intended for long-term implantation in the human body. Addition-cure silicone rubber is primarily used in applications involving contact with blood and various implanted scenarios within the body. The existing silicone rubber is generally produced through a simple extrusion process, such as the preparation process disclosed in Chinese patent CN1727409A. The silicone rubber tubes produced by this addition method have poor mechanical properties, making it difficult to meet the requirements for clinical use.

## CONTENT OF THE PRESENT INVENTION

The technical problem to be solved by the present disclosure is to overcome the defects of poor mechanical properties of silicone rubber tubes produced by the addition method in the prior art, which are insufficient to meet the requirements for clinical use. The present disclosure therefore provides a silicone material, a preparation method therefor, a silicone tube, and an implant containing the same. The silicone tube made from the silicone material in the present disclosure exhibits excellent mechanical properties and good biocompatibility. The implant prepared using the silicone tube has a stable drug release profile. When the loaded drug is a contraceptive (e.g., gestodene, levonorgestrel, estradiol), the prepared implant can produce a significant contraceptive effect in rats.

**Silicone material**

The present disclosure provides a raw material composition of the silicone material comprising the following components in parts by weight:

> R-vinyl silicone rubber: 100 parts;
> a reinforcing agent: 20-80 parts;
> hydrogen-containing silicone oil: 0.3-3.0 parts;
> a catalyst: $\geq$ 0.000002 parts, preferably 0.000002-0.00005 parts;
> wherein
> in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
> the content of vinyl groups in the R-vinyl silicone rubber is 0.10-0.50 mol%;
> the content of Si-H groups in the hydrogen-containing silicone oil is 0.18-1.6 mol%;
> the molar ratio of Si-H groups in the hydrogen-containing silicone oil to vinyl groups in the R-vinyl silicone rubber is (0.5-4): 1;
> optionally, the raw material composition further comprises an inhibitor, and the inhibitor is an inhibitor capable of inhibiting the addition reaction between the R-vinyl silicone rubber and the hydrogen-containing silicone oil.

In the present disclosure, in the R-vinyl silicone rubber, R may be a substituted or unsubstituted $C_1$-$C_5$ linear alkane, such as methyl.
When the R is methyl, the R-vinyl silicone rubber is methyl vinyl silicone rubber.
Herein, the methyl vinyl silicone rubber may be a conventional methyl vinyl silicone rubber in the art, such as methyl vinyl silicone rubber with a relative molecular weight of 100000-800000 g/mol.
In the present disclosure, the content of vinyl groups in the R-vinyl silicone rubber is preferably 0.10-0.23 mol%, such as 0.17 mol% or 0.23 mol%, more preferably 0.17-0.23 mol%.
In the present disclosure, the reinforcing agent may be a conventional reinforcing agent in the art that can improve the hardness of the R-vinyl silicone rubber, such as one or more of white carbon black, diatomite, quartz powder, silica powder, calcium carbonate, aluminum hydroxide, magnesium oxide, titanium white powder, magnesium silicate, carbon black, zinc oxide, iron oxide, titanium dioxide, zirconium silicate, and calcium carbonate, such as white carbon black.
Herein, the white carbon black may be conventional white carbon black in the art, such as fumed white carbon black, precipitated white carbon black, gel white carbon black, or surface-treated white carbon black, preferably fumed white carbon black. The fumed white carbon black may be fumed white carbon black purchased from Dalian Shengsen Nano Silicon Carbon Materials Co., Ltd.
Herein, the calcium carbonate may be conventional calcium carbonate in the art, such as precipitated calcium carbonate.
In the present disclosure, the amount of the reinforcing agent is preferably 30-80 parts, such as 30 parts, 35 parts, 40 parts, 45 parts, 50 parts, or 60 parts.
In the present disclosure, the hydrogen-containing silicone oil may be conventional hydrogen-containing silicone oil in the art, such as hydrogen-containing silicone oil purchased from Guangdong Silicon Ye New Material Technology Co., Ltd.
In the present disclosure, the amount of the hydrogen-containing silicone oil is preferably 0.4-2.8 parts, such as 0.42 parts, 0.67 parts, 0.84 parts, 1.01 parts, 1.26 parts, 1.36 parts, 1.51 parts, 1.68 parts, or 2.52 parts.

In the present disclosure, the content of Si-H groups in the hydrogen-containing silicone oil is preferably 0.36-1.6 mol%, such as 0.36 mol%, 0.5 mol%, 0.75 mol%, 1.0 mol%, or 1.6 mol%.

In the present disclosure, the molar ratio of Si-H groups in the hydrogen-containing silicone oil to vinyl groups in the methyl vinyl silicone rubber is preferably (0.8-4.0):1, such as (0.8-3.0):1 or (0.8-2.0):1, or such as 0.8:1, 1.0:1, 1.2:1, 1.5:1, 1.8:1, 2.0:1, or 3.0:1, preferably (1.2-1.8):1 or (1.2-1.5):1.

In the present disclosure, the inhibitor may be a conventional inhibitor in the art capable of inhibiting the addition reaction between R-vinyl silicone rubber (e.g., methyl vinyl silicone rubber) and hydrogen-containing silicone oil at room temperature, such as an acetylenic alcohol compound, a nitrogen-containing compound, or an organic peroxide, or such as methylbutynol, or such as 2-methyl-3-butyn-2-ol.

In the present disclosure, the amount of the inhibitor is preferably 0.03-2.0 parts, such as 0.3-1.0 parts, or such as 0.3 parts, 0.5 parts, 0.7 parts, or 0.9 parts.

In the present disclosure, the catalyst may be a conventional catalyst in the art capable of catalyzing the addition reaction between methyl vinyl silicone rubber and hydrogen-containing silicone oil, such as a rhodium catalyst, a palladium catalyst, or a platinum catalyst, preferably a platinum catalyst.

Herein, the concentration of platinum in the platinum catalyst may be 3000 ppm, meaning that the mass concentration of platinum in the platinum catalyst is 3000 parts per million.

In the present disclosure, the amount of the catalyst is preferably 0.000005-0.00005 parts, such as 0.000005 parts, 0.00001 parts, 0.00002 parts, or 0.00003 parts.

In the present disclosure, the vulcanization principle of the two-component addition-cure silicone rubber is described as follows: Two-component addition-cure silicone rubber vulcanized at room temperature, such as using vinyl polydimethylsiloxane as the base polymer and hydrogen-containing silicone oil as the cross-linking agent, undergoes a hydrosilylation reaction between vinyl groups and hydrogen groups under the catalysis of a catalyst (e.g., a platinum catalyst) to form a cross-linked network structure, which can control drug release.

Due to the minimal intermolecular forces between polyorganosilane molecules, the raw rubber, when vulcanized alone, exhibits poor mechanical properties and has no practical value. To improve its mechanical properties, fillers are generally used for reinforcement. White carbon black is the most commonly used reinforcing filler and can greatly increase the hardness of silicone materials. Among them, fumed white carbon black treated with silazane is hydrophobic on the surface, which facilitates dispersion in silicone materials and enhances the reinforcing effect.

After mixing the raw rubber with fillers, cross-linking agents, and catalysts, a reaction can occur at room temperature. However, the mixing and processing of the rubber require a certain amount of time. If the reactants are prematurely cured during operation, the desired shape and properties cannot be achieved. This is especially true for addition-cure silicone rubber. Therefore, it is generally required that the catalytic reaction remains almost inactive before vulcanization (when mixed at room temperature) and reacts rapidly upon reaching the vulcanization temperature. The method of inhibiting the reaction is usually the addition of an inhibitor. The inhibitor can form a certain complex with the platinum catalyst. Effective inhibitors can be stored with the rubber for a considerable amount of time and can only be vulcanized when heated to a certain vulcanization temperature. The commonly used inhibitors with good compatibility are acetylenic alcohol compounds, nitrogen-containing compounds, organic peroxides, etc.

In one preferred embodiment of the present disclosure, the raw material composition of the silicone material comprises the following components in parts by weight:

methyl vinyl silicone rubber: 100 parts;
a reinforcing agent: 20-80 parts;
hydrogen-containing silicone oil: 0.3-3.0 parts;
a catalyst: 0.000002-0.00005 parts;
an inhibitor: 0.03-2.0 parts;
wherein
the content of vinyl groups in the methyl vinyl silicone rubber is 0.10-0.50 mol%;
the content of Si-H groups in the hydrogen-containing silicone oil is 0.18-1.6 mol%;
the molar ratio of Si-H groups in the hydrogen-containing silicone oil to vinyl groups in the methyl vinyl silicone rubber is (0.5-4): 1.

In one preferred embodiment of the present disclosure, the raw material composition of the silicone material comprises the following components in parts by weight:

methyl vinyl silicone rubber: 100 parts;
a reinforcing agent: 30-60 parts;
hydrogen-containing silicone oil: 0.4-2.8 parts;
a catalyst: 0.000002-0.00005 parts;

an inhibitor: 0.3-1.0 parts;
wherein
the content of vinyl groups in the methyl vinyl silicone rubber is 0.17-0.23 mol%;
the content of Si-H groups in the hydrogen-containing silicone oil is 0.18-1.6 mol%;
the molar ratio of Si-H groups in the hydrogen-containing silicone oil to vinyl groups in the methyl vinyl silicone rubber is (0.8-2): 1.

In one preferred embodiment of the present disclosure, the raw material composition of the silicone material comprises the following components in parts by weight:

methyl vinyl silicone rubber: 100 parts;
a reinforcing agent: 30-45 parts;
hydrogen-containing silicone oil: 0.42-2.52 parts;
a catalyst: 0.000002-0.00005 parts;
an inhibitor: 0.3-0.9 parts;
wherein
the content of vinyl groups in the methyl vinyl silicone rubber is 0.17-0.23 mol%;
the content of Si-H groups in the hydrogen-containing silicone oil is 0.5-1.0 mol%;
the molar ratio of Si-H groups in the hydrogen-containing silicone oil to vinyl groups in the methyl vinyl silicone rubber is (1.2-1.8):1.

In some preferred embodiments of the present disclosure, the raw material composition of the silicone material is any one of the following numbers in parts by weight:

| Material name | Amount | | | |
|---|---|---|---|---|
| | 1-1 | 1-2 | 1-3 | 1-4 |
| Methyl vinyl silicone rubber | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% |
| Fumed white carbon black | 30 PHR | 30 PHR | 30 PHR | 30 PHR |
| Hydrogen-containing silicone oil | 1.01 PHR | 1.01 PHR | 1.01 PHR | 1.01 PHR |
| Molar ratio of Si-H groups in hydrogen-containing silicone oil to vinyl groups in R-vinyl silicone rubber | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR |
| Platinum catalyst (3000 ppm) | 0.000005 PHR | 0.00001 PHR | 0.00002 PHR | 0.00003 PHR |

In some preferred embodiments of the present disclosure, the raw material composition of the silicone material is any one of the following numbers in parts by weight:

| Material name | Amount | | | |
|---|---|---|---|---|
| | 2-1 | 2-2 | 2-3 | 2-4 |
| Methyl vinyl silicone rubber | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% |
| Fumed white carbon black | 30 PHR | 30 PHR | 30 PHR | 30 PHR |
| Hydrogen-containing silicone oil | 1.01 PHR | 1.01 PHR | 1.01 PHR | 1.01 PHR |

(continued)

| Material name | Amount | | | |
|---|---|---|---|---|
| | 2-1 | 2-2 | 2-3 | 2-4 |
| Molar ratio of Si-H groups in hydrogen-containing silicone oil to vinyl groups in methyl vinyl silicone rubber | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% |
| 2-Methyl-3-butyn-2-ol | 0.3 PHR | 0.5 PHR | 0.7 PHR | 0.9 PHR |
| Platinum catalyst (3000 ppm) | 0.00001 PHR | 0.00001 PHR | 0.00001 PHR | 0.00001 PHR |

In some preferred embodiments of the present disclosure, the raw material composition of the silicone material is any one of the following numbers in parts by weight:

| Material name | Amount | |
|---|---|---|
| | 3-1 | 3-2 |
| Methyl vinyl silicone rubber | 100 PHR | 100 PHR |
| Content of vinyl groups in methyl vinyl silicone rubber | 0.17 mol% | 0.23 mol% |
| Fumed white carbon black | 30 PHR | 30 PHR |
| Hydrogen-containing silicone oil | 1.01 PHR | 1.01 PHR |
| Molar ratio of Si-H groups in hydrogen-containing silicone oil to vinyl groups in methyl vinyl silicone rubber | 1.2:1 | 1.2:1 |
| Content of hydrogen in hydrogen-containing silicone oil | 0.75 mol% | 0.75 mol% |
| 2-Methyl-3-butyn-2-ol | 0.7 PHR | 0.7 PHR |
| Platinum catalyst (3000 ppm) | 0.00001 PHR | 0.00001 PHR |

In some preferred embodiments of the present disclosure, the raw material composition of the silicone material is any one of the following numbers in parts by weight:

| Material name | Amount | | | | | |
|---|---|---|---|---|---|---|
| | 4-1 | 4-2 | 4-3 | 4-4 | 4-5 | 4-6 |
| Methyl vinyl silicone rubber | 100 PHR | 100 PHR | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% | 0.17 mol% | 0.17 mol% |
| Fumed white carbon black | 20 PHR | 30 PHR | 40 PHR | 45 PHR | 50 PHR | 60 PHR |
| Hydrogen-containing silicone oil | 1.01 PHR | 1.01 PHR | 1.01 PHR | 1.01 PHR | 1.01 PHR | 1.01 PHR |
| Molar ratio of Si-H groups in hydrogen-containing silicone oil to vinyl groups in methyl vinyl silicone rubber | 1.2:1 | 1.2:1 | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR | 0.7 PHR | 0.7 PHR |

(continued)

| Material name | Amount | | | | | |
|---|---|---|---|---|---|---|
| | 4-1 | 4-2 | 4-3 | 4-4 | 4-5 | 4-6 |
| Platinum catalyst (3000 ppm) | 0.00001 PHR | 0.00001 PHR | 0.00001 PHR | 0.00001 PHR | 0.00001 PHR | 0.00001 PHR |

In some preferred embodiments of the present disclosure, the raw material composition of the silicone material is any one of the following numbers in parts by weight:

| Material name | Amount | | | | | |
|---|---|---|---|---|---|---|
| | 5-1 | 5-2 | 5-3 | 5-4 | 5-5 | 5-6 |
| Methyl vinyl silicone rubber | 100 PHR | 100 PHR | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% | 0.17 mol% | 0.17 mol% |
| Fumed white carbon black | 40 PHR | 40 PHR | 40 PHR | 40 PHR | 40 PHR | 40 PHR |
| Hydrogen-containing silicone oil | 1.01 PHR | 1.01 PHR | 1.01 PHR | 1.01 PHR | 1.01 PHR | 1.01 PHR |
| Molar ratio of Si-H groups in hydrogen-containing silicone oil to vinyl groups in methyl vinyl silicone rubber | 1.2:1 | 1.2:1 | 1.2:1 | 1.2:1 | 1.2:1 | 1.2:1 |
| Content of hydrogen in hydrogen-containing silicone oil | 0.18 mol% | 0.36 mol% | 0.5 mol% | 0.75 mol% | 1.0 mol% | 1.6 mol% |
| 2-Methyl-3-butyn-2-ol | 0.7 PHR | 0.7 PHR | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR | 0.00001 PHR | 0.00001 PHR |

In some preferred embodiments of the present disclosure, the raw material composition of the silicone material is any one of the following numbers in parts by weight:

| Material name | Amount | | | |
|---|---|---|---|---|
| | 6-1 | 6-2 | 6-3 | 6-4 |
| Methyl vinyl silicone rubber | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% |
| Fumed white carbon black | 40 PHR | 40 PHR | 40 PHR | 40 PHR |
| Hydrogen-containing silicone oil | 0.84 PHR | 1.01 PHR | 1.26 PHR | 1.51 PHR |
| Molar ratio of Si-H groups in hydrogen-containing silicone oil to vinyl groups in methyl vinyl silicone rubber | 1:1 | 1.2:1 | 1.5:1 | 1.8:1 |
| Content of hydrogen in hydrogen-containing silicone oil | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR |

In some preferred embodiments of the present disclosure, the raw material composition of the silicone material is any one of the following numbers in parts by weight:

| Material name | Amount | | |
|---|---|---|---|
| | 7-1 | 7-2 | 8-2 |
| Methyl vinyl silicone rubber | 100 PHR | 100 PHR | 100 PHR |
| Content of vinyl groups in methyl vinyl silicone rubber | 0.17 mol% | 0.23 mol% | 0.17 mol% |
| Fumed white carbon black | 40 PHR | 40 PHR | 40 PHR |
| Hydrogen-containing silicone oil | 1.01 PHR | 1.01 PHR | 1.26 PHR |
| Molar ratio of Si-H groups in hydrogen-containing silicone oil to vinyl groups in methyl vinyl silicone rubber | 1.2:1 | 1.2:1 | 1.5:1 |
| Content of hydrogen in hydrogen-containing silicone oil | 0.75 mol% | 0.75 mol% | 0.75 mol% |
| 2-Methyl-3-butyn-2-ol | 0.7 PHR | 0.7 PHR | 0.7 PHR |
| Platinum catalyst (3000 ppm) | 0.00001 PHR | 0.00001 PHR | 0.00001 PHR |

In some preferred embodiments of the present disclosure, the raw material composition of the silicone material is any one of the following numbers in parts by weight:

| No. | Content of vinyl groups in methyl vinyl silicone rubber | Methyl vinyl silicone rubber (PHR) | Fumed white carbon black (PHR) | Hydrogen-containing silicone oil (PHR) | Molar ratio of Si-H groups in hydrogen-containing silicone oil to vinyl groups in methyl vinyl silicone rubber | Content of hydrogen in hydrogen-containing silicone oil | Platinum catalyst (PHR) | 2-Methyl-3-butyn-2-ol (PHR) |
|---|---|---|---|---|---|---|---|---|
| 9-1 | 0.17 mol% | 100 | 30 | 1.01 | 1.2:1 | 0.75 mol% | $10^{-5}$ | 0.70 |
| 9-2 | 0.23 mol% | 100 | 30 | 1.36 | 1.2:1 | 0.75 mol% | $10^{-5}$ | 0.70 |

In some preferred embodiments of the present disclosure, the raw material composition of the silicone material is any one of the following numbers in parts by weight:

| No. | Content of vinyl groups in vinyl polysiloxane | Methyl vinyl silicone rubber (PHR) | Fumed white carbon black (PHR) | Hydrogen-containing silicone oil (PHR) | Molar ratio of Si-H groups in hydrogen-containing silicone oil to vinyl groups in methyl vinyl silicone rubber | Content of hydrogen in hydrogen-containing silicone oil | Platinum catalyst (PHR) | 2-Methyl-3-butyn-2-ol (PHR) |
|---|---|---|---|---|---|---|---|---|
| 10-1 | 0.17 mol% | 100 | 30 | 1.01 | 1.2:1 | 0.75 mol% | $10^{-5}$ | 0.70 |
| 10-2 | 0.17 mol% | 100 | 35 | 1.01 | 1.2:1 | 0.75 mol% | $10^{-5}$ | 0.70 |
| 10-3 | 0.17 mol% | 100 | 40 | 1.01 | 1.2:1 | 0.75 mol% | $10^{-5}$ | 0.70 |

In some preferred embodiments of the present disclosure, the raw material composition of the silicone material is any one of the following numbers in parts by weight:

| No. | Molar ratio of Si-H groups in hydrogen-containing silicone oil to vinyl groups in methyl vinyl silicone rubber | Methyl vinyl silicone rubber (PHR) | Content of vinyl groups in vinyl polysiloxane | Fumed white carbon black (PHR) | Hydrogen-containing silicone oil (PHR) | Content of hydrogen in hydrogen-containing silicone oil | Platinum catalyst (PHR) | 2-Methyl-3-butyn-2-ol (PHR) |
|------|------|------|------|------|------|------|------|------|
| 11-1 | 0.5:1 | 100 | 0.17 mol% | 35 | 0.42 | 0.75 mol% | $10^{-5}$ | 0.70 |
| 11-2 | 0.8:1 | 100 | 0.17 mol% | 35 | 0.67 | 0.75 mol% | $10^{-5}$ | 0.70 |
| 11-3 | 1:1 | 100 | 0.17 mol% | 35 | 0.84 | 0.75 mol% | $10^{-5}$ | 0.70 |
| 11-4 | 1.2:1 | 100 | 0.17 mol% | 35 | 1.01 | 0.75 mol% | $10^{-5}$ | 0.70 |
| 11-5 | 1.5:1 | 100 | 0.17 mol% | 35 | 1.26 | 0.75 mol% | $10^{-5}$ | 0.70 |
| 11-6 | 2:1 | 100 | 0.17 mol% | 35 | 1.68 | 0.75 mol% | $10^{-5}$ | 0.70 |
| 11-7 | 3:1 | 100 | 0.17 mol% | 35 | 2.52 | 0.75 mol% | $10^{-5}$ | 0.70 |

In some preferred embodiments of the present disclosure, the raw material composition of the silicone material is any one of the following numbers in parts by weight:

| Material name | 12-1 | 12-2 | 13-1 | 13-3 |
|------|------|------|------|------|
| Methyl vinyl silicone rubber | 100 PHR | 100 PHR | 100 PHR | 100 PHR |
| Content of vinyl groups in vinyl polysiloxane | 0.17 mol% | 0.23 mol% | 0.17 mol% | 0.17 mol% |
| Fumed white carbon black | 35 PHR | 35 PHR | 35 PHR | 35 PHR |
| Hydrogen-containing silicone oil | 1.01 PHR | 1.36 PHR | 0.84 PHR | 1.26 PHR |
| Molar ratio of Si-H groups in hydrogen-containing silicone oil to vinyl groups in methyl vinyl silicone rubber | 1.2:1 | 1.2:1 | 1.0:1 | 1.5:1 |
| Content of hydrogen in hydrogen-containing silicone oil | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR |

The present disclosure further provides a preparation method for the silicone material, comprising the following step: molding a mixture of the raw material composition of the silicone material through a catalytic addition process, wherein

(1) the raw material composition of the silicone material comprises the following components in parts by weight:

R-vinyl silicone rubber: 100 parts;
a reinforcing agent: 20-80 parts;
hydrogen-containing silicone oil: 0.3-3.0 parts;
a catalyst: $\geq$ 0.000002 parts, preferably 0.000002-0.00005 parts;
wherein
in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl groups in the R-vinyl silicone rubber is 0.10-0.50 mol%;
the content of Si-H groups in the hydrogen-containing silicone oil is 0.18-1.6 mol%;

the molar ratio of Si-H groups in the hydrogen-containing silicone oil to vinyl groups in the R-vinyl silicone rubber is (0.5-4): 1;
optionally, the raw material composition further comprises an inhibitor, and the inhibitor is an inhibitor capable of inhibiting the addition reaction between the R-vinyl silicone rubber and the hydrogen-containing silicone oil;

(2) The catalytic addition process sequentially comprises a first heat treatment and a second heat treatment; the temperature of the first heat treatment is 250-360°C, and the temperature of the second heat treatment is 120-300°C, such as 120-280°C.

In some preferred embodiments of the present disclosure, the components and their contents in the raw material composition of the silicone material are as previously described.

In the present disclosure, the catalytic addition process may be an extrusion process.

In the present disclosure, the temperature of the first heat treatment may be 250-330°C, such as 270°C, 280°C, 300°C, or 330°C.

In the present disclosure, the duration of the first heat treatment is related to the processing length in the catalytic addition process, such as the extrusion length in an extrusion process. For example, when the processing length of the first heat treatment is 0.8 meters, the duration of the first heat treatment may be approximately 5 seconds.

In the present disclosure, the temperature of the second heat treatment may be 150-300°C, such as 150-280°C, such as 150°C, 180°C, 210°C, 260°C, or 280°C.

In the present disclosure, the duration of the second heat treatment is related to the processing length in the catalytic addition process, such as the extrusion length in an extrusion process. For example, when the extrusion length of the second heat treatment is 2.5 meters, the duration of the second heat treatment may be approximately 2 minutes.

In the present disclosure, a third heat treatment may be performed after the second heat treatment.

Herein, the temperature of the third heat treatment may be 100-280°C, such as 180°C.

Herein, the duration of the third heat treatment is preferably 0-72 hours, such as 0 hours, 24 hours, 48 hours, or 72 hours.

In the present disclosure, preferably, the temperature of the first heat treatment is 270°C, and the temperature of the second heat treatment is 180°C.

In the present disclosure, the catalytic addition process may be carried out through extrusion molding in a tubular mold. When extruded in a tubular mold, the silicone material forms a tubular shape.

In the present disclosure, the mixture of the raw material composition of the silicone material may be prepared by the following method:

method 1: when the raw material composition of the silicone material does not comprise an inhibitor, the preparation method of the mixture comprises the following steps:

S1: mixing the R-vinyl silicone rubber and the reinforcing agent uniformly to obtain a mixture A;
S2: mixing the mixture A and the hydrogen-containing silicone oil in the presence of the catalyst to obtain a mixture B;

method 2: when the raw material composition of the silicone material further comprises an inhibitor, the preparation method for the silicone material comprises the following steps:

S1: mixing the R-vinyl silicone rubber and the reinforcing agent uniformly to obtain a mixture A;
S2: dividing the mixture A into a component A1 and a component A2, mixing the component A1 with the catalyst to obtain a component B1, and mixing the component A2 with the hydrogen-containing silicone oil and the inhibitor to obtain a component B2;
S3: mixing the component B1 and the component B2 to obtain a mixture B.

Herein, in the method 1 and method 2, the reinforcing agent may be pretreated using conventional methods in the art, such as by drying at 100-210°C (e.g., 180-210°C, or e.g., 200°C) for 1-24 hours (e.g., 24 hours) for later use.

Herein, in the method 1 and method 2, the R-vinyl silicone rubber may be pretreated using conventional methods in the art, such as by drying at 30-60°C (e.g., 40°C) for 1-24 hours (e.g., 24 hours) for later use.

Herein, in the method 1 and method 2, the step of mixing in S1 may be as follows:
wrapping the R-vinyl silicone rubber with the reinforcing agent, then extruding and passing through an open mill, and sheeting.

Herein, in the method 1 and method 2, the mixture A may be left in a desiccator at room temperature for 24-72 hours.

Herein, in the method 1 and method 2, the step of mixing in S1 may be as follows:
Kneading the R-vinyl silicone rubber and the reinforcing agent in a kneader at 30°C for 30 minutes, then removing the mixture; performing triangular wrapping and passing through an open mill at a roll spacing of 1-10 mm (e.g., 1 mm) five times, rolling, sheeting, then being left for 24 hours to obtain the mixture A.

Herein, in the method 1, prior to the catalytic addition process, the mixture B may undergo the following post-treatment: performing triangular wrapping and passing through an open mill 4-6 times, then uniformly cutting and sheeting.

Herein, in the method 2, the step of mixing the component B1 and the component B2 may be as follows: adding the component B1 and the component B2 to an open mill in a 1:1 ratio, then performing triangular wrapping and passing through the open mill 4-6 times, and uniformly cutting and sheeting.

The present disclosure further provides a preparation method for the silicone material, comprising the following methods:

method 1: when the raw material composition of the silicone material does not comprise an inhibitor, the preparation method for the silicone material comprises the following steps:

S1: mixing the R-vinyl silicone rubber and the reinforcing agent uniformly to obtain a mixture A;
S2: mixing the mixture A and the hydrogen-containing silicone oil in the presence of the catalyst to obtain a mixture B, then performing catalytic addition;

method 2: when the raw material composition of the silicone material further comprises an inhibitor, the preparation method for the silicone material comprises the following steps:

S1: mixing the R-vinyl silicone rubber and the reinforcing agent uniformly to obtain a mixture A;
S2: dividing the mixture A into a component A1 and a component A2, mixing the component A1 with the catalyst to obtain a component B1, and mixing the component A2 with the hydrogen-containing silicone oil and the inhibitor to obtain a component B2;
S3: mixing the component B1 and the component B2 to obtain a mixture B, then performing catalytic addition.

In some preferred embodiments of the present disclosure, the components and their contents in the raw material composition of the silicone material are as previously described.

In some preferred embodiments of the present disclosure, the conditions in the method 1 and method 2 are as previously described.

The present disclosure further provides a silicone material, which is prepared by the methods described above.

When the catalytic addition process is carried out through extrusion molding in a tubular mold, the silicone material forms a tubular shape.

The present disclosure further provides a silicone tube, which is produced by the following method;

extruding the silicone material into a tubular shape;
alternatively, in the preparation method for the silicone material as described above, performing the catalytic addition process in a tubular mold to obtain the silicone tube.

The present disclosure further provides a use of the silicone material or the silicone tube as a release rate-modulating medium in sustained-release and controlled-release preparations.

**Pharmaceutical composition**

In the prior art, subcutaneous implants are generally prepared by directly filling the bulk drug into the silicone tube. However, after pulverization, the bulk drug particles are relatively small and prone to static electricity. During the filling process, the bulk drug powder tends to become airborne, causing significant adherence of the powder to the outer surface of the silicone tube. This not only leads to waste of the bulk drug but also poses health risks to the personnel involved. Furthermore, it can easily lead to the burst release of the drug, compromising the safety of drug users. Additionally, the spontaneous aggregation of the bulk drug can affect the drug release stability of the implant.

Furthermore, when the structure (e.g., wall thickness, length, or diameter), composition (components and content), and preparation method (process parameters) of the silicone tube remain unchanged, the subcutaneous implants in the prior art are unable to adjust the drug release dose.

Therefore, there is an urgent need to provide a pharmaceutical composition that can effectively address the above issues and a preparation method therefor. The pharmaceutical composition can prevent the bulk drug powder from becoming airborne during the filling process, reduce static electricity of the bulk drug powder, and facilitate drug filling. It can also prevent spontaneous aggregation of the drug. The subcutaneous implant prepared from the pharmaceutical composition exhibits high drug release stability. By incorporating different types of insoluble acids and bases to adjust the pH value, subcutaneous implants with different daily release doses and high drug release stability can be obtained.

To overcome the defects in the prior art, where the core (bulk drug) powder of the subcutaneous implant becomes airborne, has strong static electricity, adheres significantly to the outer tube wall of the implant, causes instability in drug release due to spontaneous aggregation, making it difficult to precisely fill small doses of the drug, and the implant further prepared has poor drug release stability, the present disclosure provides a pharmaceutical composition, a preparation method therefor, and a use thereof, as well as an implant containing the same. The pharmaceutical composition of the present disclosure exhibits low static electricity, thereby facilitating the filling process, and effectively preventing airborne powder and spontaneous aggregation. This ensures high utilization of the bulk drug and enhances the drug release stability of the subcutaneous implant prepared from the pharmaceutical composition.

The present disclosure provides a pharmaceutical composition, comprising a bulk drug and an insoluble excipient; the insoluble excipient comprises a silicon material.

In the present disclosure, the density of the insoluble excipient may be 1-10000 g/L, such as 1000 g/L.

The particle size of the insoluble excipient may be 1-200 $\mu$m.

The pore size of the silicon material may be less than 1 $\mu$m, such as 0 nm, 5 nm, 10 nm, 18 nm, 50 nm, or 100 nm. It can be understood by those skilled in the art that when the pore size of the silicon material is 0 nm, the silicon material is a non-porous silicon material.

In the silicon material, the content of silicon dioxide may be more than 50%, preferably 80%, 90%, 95%, 99%, or 99.8%.

In the present disclosure, the insoluble excipient may include one or more of white carbon black, AL-1FP mesoporous silica, XDP3050 mesoporous silica, and XDP3150 mesoporous silica.

Herein, the white carbon black is preferably fumed white carbon black, precipitated white carbon black, gel white carbon black, or surface-treated white carbon black.

Preferably, the insoluble excipient is one or more of white carbon black, AL-1FP mesoporous silica, XDP3050 mesoporous silica, and XDP3150 mesoporous silica.

More preferably, the insoluble excipient is one or more of white carbon black, AL-1FP mesoporous silica, and XDP3050 mesoporous silica.

In the present disclosure, the insoluble excipient may further comprise an insoluble weak acid and/or an insoluble weak base.

Herein, the insoluble weak acid preferably includes one or more of boric acid, fumaric acid, molybdic acid, silicic acid, tungstic acid, and germanic acid, more preferably including boric acid and/or fumaric acid.

Herein, the insoluble weak base preferably includes one or more of magnesium hydroxide, aluminum hydroxide, zinc hydroxide, ferrous hydroxide, and magnesium oxide, more preferably including one or more of magnesium hydroxide, aluminum hydroxide, and zinc hydroxide.

In the present disclosure, the insoluble excipient may be one of the following a to z:

    a. white carbon black;
    b. white carbon black and magnesium hydroxide;
    c. white carbon black and aluminum hydroxide;
    d. white carbon black and zinc hydroxide;
    e. white carbon black and fumaric acid;
    f. white carbon black and boric acid;
    g. AL-1FP mesoporous silica;
    h. AL-1FP mesoporous silica and XDP3050 mesoporous silica;
    i. AL-1FP mesoporous silica and XDP3150 mesoporous silica;
    j. AL-1FP mesoporous silica and magnesium hydroxide;
    k. AL-1FP mesoporous silica and aluminum hydroxide;
    l. AL-1FP mesoporous silica and zinc hydroxide;
    m. AL-1FP mesoporous silica and fumaric acid;
    n. AL-1FP mesoporous silica and boric acid;
    o. XDP3050 mesoporous silica;
    p. XDP3050 mesoporous silica and magnesium hydroxide;
    q. XDP3050 mesoporous silica and aluminum hydroxide;
    r. XDP3050 mesoporous silica and zinc hydroxide;
    s. XDP3050 mesoporous silica and fumaric acid;
    t. XDP3050 mesoporous silica and boric acid;
    u. XDP3150 mesoporous silica;

v. XDP3150 mesoporous silica and magnesium hydroxide;

w. XDP3150 mesoporous silica and aluminum hydroxide;

x. XDP3150 mesoporous silica and zinc hydroxide;

y. XDP3150 mesoporous silica and fumaric acid;

z. XDP3150 mesoporous silica and boric acid.

In the present disclosure, the insoluble excipient may also be white carbon black combined with one or more of molybdic acid, silicic acid, tungstic acid, and germanic acid; for example, white carbon black and molybdic acid, white carbon black and silicic acid, white carbon black and tungstic acid, or white carbon black and germanic acid.

Alternatively, the insoluble excipient may also be white carbon black combined with ferrous hydroxide and/or magnesium oxide; for example, white carbon black and ferrous hydroxide, or white carbon black and magnesium oxide.

Alternatively, the insoluble excipient may also be AL-1FP mesoporous silica combined with one or more of molybdic acid, silicic acid, tungstic acid, and germanic acid; for example, AL-1FP mesoporous silica and molybdic acid, AL-1FP mesoporous silica and silicic acid, AL-1FP mesoporous silica and tungstic acid, or AL-1FP mesoporous silica and germanic acid.

Alternatively, the insoluble excipient may also be AL-1FP mesoporous silica combined with ferrous hydroxide and/or magnesium oxide; for example, AL-1FP mesoporous silica and ferrous hydroxide, or AL-1FP mesoporous silica and magnesium oxide.

Alternatively, the insoluble excipient may also be XDP3050 mesoporous silica combined with one or more of molybdic acid, silicic acid, tungstic acid, and germanic acid; for example, XDP3050 mesoporous silica and molybdic acid, XDP3050 mesoporous silica and silicic acid, XDP3050 mesoporous silica and tungstic acid, or XDP3050 mesoporous silica and germanic acid.

Alternatively, the insoluble excipient may also be XDP3050 mesoporous silica combined with ferrous hydroxide and/or magnesium oxide; for example, XDP3050 mesoporous silica and ferrous hydroxide, or XDP3050 mesoporous silica and magnesium oxide.

Alternatively, the insoluble excipient may also be XDP3150 mesoporous silica combined with one or more of molybdic acid, silicic acid, tungstic acid, and germanic acid; for example, XDP3150 mesoporous silica and molybdic acid, XDP3150 mesoporous silica and silicic acid, XDP3150 mesoporous silica and tungstic acid, or XDP3150 mesoporous silica and germanic acid.

Alternatively, the insoluble excipient may also be XDP3150 mesoporous silica combined with ferrous hydroxide and/or magnesium oxide; for example, XDP3150 mesoporous silica and ferrous hydroxide, or XDP3150 mesoporous silica and magnesium oxide.

In the present disclosure, the content of the bulk drug may be 10%-99.9%, such as 50%-99.5%, or such as 95%; the percentage represents the mass percentage of the bulk drug in the pharmaceutical composition.

The content of the insoluble excipient may be 0.1%-90%, such as 0.1%-50%, or such as 0.5%-5%; the percentage represents the mass percentage of the insoluble excipient in the pharmaceutical composition.

In the present disclosure, when the insoluble excipient is two or three of white carbon black, AL-1FP mesoporous silica, and XDP3150 mesoporous silica, then the mass ratio may be any ratio. For example, when the insoluble excipient is two of white carbon black, AL-1FP mesoporous silica, and XDP3050 mesoporous silica, then the mass ratio is (0.001-1000):1.

For another example, when the insoluble excipient is XDP3150 mesoporous silica and fumaric acid, then the mass ratio may be (0.01-100):1, preferably 9:1, 1.5:1, or 0.43:1.

For another example, when the insoluble excipient is fumed white carbon black and boric acid, then the mass ratio may be (0.01-100): 1, preferably 9:1, 1.5:1, or 0.43:1.

For another example, when the insoluble excipient is XDP3050 mesoporous silica and magnesium hydroxide, then the mass ratio may be (0.01-100): 1, preferably 9:1, 1.5:1, or 0.43:1.

For another example, when the insoluble excipient is AL-1FP mesoporous silica and zinc hydroxide, then the mass ratio may be (0.01-100):1, preferably 9:1, 1.5:1, or 0.43:1.

In the present disclosure, the bulk drug may be a small molecule drug with a solubility of less than 100 mg/mL.

In the present disclosure, the molecular weight of the bulk drug may be less than 1000 Da.

In the present disclosure, the active pharmaceutical ingredient of the bulk drug may include an active pharmaceutical ingredient acting on the reproductive system, an active pharmaceutical ingredient acting on the urinary system, an active pharmaceutical ingredient acting on chronic diseases related to metabolism and nutrition, an active pharmaceutical ingredient for treating chronic diseases related to connective tissue and rheumatism, an active pharmaceutical ingredient for treating hyperlipidemia, tumors, neuropsychiatric disorders, chronic dental diseases (dental caries, periodontal disease), simple obesity, chronic low back pain, or leukemia, an active pharmaceutical ingredient acting on the circulatory system, an active pharmaceutical ingredient acting on the respiratory system, an active pharmaceutical ingredient acting on the digestive system, an active pharmaceutical ingredient acting on the blood system, or an active pharmaceutical ingredient acting on the endocrine system.

Herein, the active pharmaceutical ingredient acting on the reproductive system may include a contraceptive active pharmaceutical ingredient or steroidal estrogen.

The contraceptive active pharmaceutical ingredient may be conventional in the art, preferably including levonorgestrel, gestodene, or gestrinone.

The steroidal estrogen is preferably estradiol.

Herein, the active pharmaceutical ingredient acting on the urinary system preferably includes an active pharmaceutical ingredient for treating chronic nephritis, chronic renal failure, or chronic prostatitis.

The active pharmaceutical ingredient for treating chronic prostatitis is preferably a non-steroidal anti-inflammatory drug, more preferably ibuprofen. Generally, the drug for treating chronic prostatitis may include: antibiotics (e.g., fluoroquinolones (e.g., norfloxacin, enoxacin, ofloxacin, ciprofloxacin), macrolides (e.g., erythromycin, roxithromycin, azithromycin, acetylspiramycin), tetracyclines (tetracycline), $\alpha$-receptor blockers (e.g., doxazosin, terazosin), non-steroidal anti-inflammatory drugs (e.g., ibuprofen, diclofenac, loxoprofen, flurbiprofen axetil, ketorolac, celecoxib), analgesics (e.g., acetaminophen), opioids (e.g., buprenorphine, fentanyl, nalbuphine, pentazocine), anti-neuropathic pain drugs (e.g., 5-hydroxytryptamine, duloxetine, venlafaxine), antiepileptics (e.g., pregabalin), and M-receptor blockers (e.g., solifenacin, tolterodine).

The drug for treating chronic nephritis may generally include: angiotensin-converting enzyme inhibitors (e.g., benazepril, ramipril, fosinopril, perindopril, cilazapril, enalapril), angiotensin II receptor blockers (e.g., irbesartan, valsartan, losartan), calcium channel blockers (e.g., amlodipine, felodipine, nifedipine), $\beta$-receptor blockers (e.g., atenolol, bisoprolol, carvedilol, propranolol), diuretics (e.g., furosemide, spironolactone, hydrochlorothiazide, bendroflumethiazide, bumetanide), immunosuppressants (e.g., prednisone, leflunomide, methylprednisolone, cyclophosphamide, dexamethasone), and statin lipid-lowering drugs (e.g., fluvastatin, simvastatin, pravastatin).

The drug for treating chronic renal failure may generally include: angiotensin-converting enzyme inhibitors (e.g., benazepril, ramipril, fosinopril, perindopril, cilazapril, enalapril), angiotensin II receptor blockers (e.g., irbesartan, valsartan, losartan), diuretics (e.g., furosemide, spironolactone, hydrochlorothiazide, bendroflumethiazide, bumetanide), iron supplements (e.g., ferrous fumarate), compound amino acids, $\alpha$-keto acids, and recombinant human erythropoietin.

Herein, the active pharmaceutical ingredient acting on chronic diseases related to metabolism and nutrition preferably includes an active pharmaceutical ingredient for anti-diabetes, treating nutritional deficiency diseases, anti-gout, or anti-osteoporosis.

The active pharmaceutical ingredient for anti-gout is preferably an anti-gout drug, such as colchicine, indomethacin, diclofenac, ibuprofen, rofecoxib, prednisone, hydrocortisone, prednisolone, aspirin, diflunisal, para-aminosalicylic acid, salsalate, or benorilate, more preferably ibuprofen. Generally, the anti-gout drug may also be a uricosuric agent (e.g., benzbromarone, probenecid) or a uric acid synthesis inhibitor (e.g., allopurinol).

The drug for anti-diabetes may generally be an anti-type 1 diabetes drug (e.g., insulin) or an anti-type 2 diabetes drug. The anti-type 2 diabetes drug may be a sulfonylurea (e.g., glimepiride, gliquidone), a glinide (e.g., repaglinide, nateglinide), a metformin (e.g., metformin), an $\alpha$-glucosidase inhibitor (e.g., acarbose, voglibose), a DPP-4 inhibitor (e.g., sitagliptin), a GLP-1 receptor agonist (e.g., liraglutide, exenatide), or a SGLT-2 inhibitor (e.g., dapagliflozin, empagliflozin).

The drug for treating nutritional deficiency diseases may generally be a drug targeting gastrointestinal diseases (e.g., omeprazole, mosapride) or a nutritional supplement (e.g., vitamin E).

The drug for anti-osteoporosis may generally be a bisphosphonate (e.g., alendronate sodium, ibandronate sodium, pamidronate sodium, aminobisphosphonate, clodronate disodium, zoledronate sodium, risedronate sodium), a calcitonin (e.g., salmon calcitonin), an estrogen (e.g., estradiol, estradiol benzoate, estradiol acetate, estradiol valerate), a selective estrogen receptor modulator (SERM) (e.g., raloxifene, benzothiophene), a RANKL inhibitor, a parathyroid hormone drug, a glutamine monofluorophosphate drug, a strontium salt (e.g., strontium ranelate), active vitamin D and an analogue thereof (e.g., calcitriol, alfacalcidol), or vitamin K (e.g., menatetrenone).

Herein, the active pharmaceutical ingredient for treating chronic diseases related to connective tissue and rheumatism preferably includes an active pharmaceutical ingredient for treating rheumatoid arthritis, systemic lupus erythematosus, ankylosing spondylitis, Sjögren's syndrome, vasculitis, idiopathic inflammatory myopathies, systemic sclerosis, or osteoarthritis.

The active pharmaceutical ingredient for treating rheumatoid arthritis is preferably a non-steroidal anti-inflammatory drug, such as aspirin, meloxicam, celecoxib, ibuprofen, nimesulide, or nabumetone, more preferably meloxicam or ibuprofen. Generally, the drug for treating rheumatoid arthritis may be a glucocorticoid (e.g., hydrocortisone, dexamethasone, prednisone) or a slow-acting antirheumatic drug (e.g., methotrexate, cyclophosphamide, azathioprine, cyclosporine, leflunomide).

The active pharmaceutical ingredient for treating osteoarthritis is preferably an analgesic, such as ibuprofen, voltaren, meloxicam, celecoxib, loxoprofen sodium, or nimesulide, more preferably ibuprofen or meloxicam. Generally, the drug for treating osteoarthritis may be a cartilage-nourishing drug or an opioid (e.g., oxycodone, tramadol).

The drug for treating systemic lupus erythematosus may generally be a non-steroidal anti-inflammatory drug (e.g., naproxen, fenbid), an antimalarial drug, a glucocorticoid (e.g., prednisone acetate, methylprednisolone), an immunosuppressant (e.g., methotrexate, cyclophosphamide, azathioprine, methotrexate, cyclosporine, mycophenolate mofetil, tacrolimus), or a biologic agent (e.g., rituximab, belimumab).

The drug for treating ankylosing spondylitis may generally be an non-steroidal drug (e.g., diclofenac sodium, etoricoxib, celecoxib, nabumetone, imrecoxib), a disease-modifying antirheumatic drug (e.g., sulfasalazine, methotrexate, hydroxychloroquine), a glucocorticoid (e.g., prednisone, prednisolone, diprospan), or a biological TNF-$\alpha$ antagonist (e.g., etanercept).

The drug for treating Sjögren's syndrome may generally be a systemic therapy drug (e.g., levamisole, transfer factor coenzyme Q10, thymosin), a glucocorticoid (e.g., prednisone), or an immunosuppressant (e.g., hydroxychloroquine, azathioprine, iguratimod).

The drug for treating vasculitis may generally be a glucocorticoid (e.g., prednisone, methylprednisolone, dexamethasone) or an immunosuppressant (e.g., cyclophosphamide, cyclosporine, azathioprine).

The drug for treating idiopathic inflammatory myopathies may generally be a glucocorticoid (e.g., dexamethasone, prednisone, hydrocortisone, methylprednisolone) or an immunosuppressant (e.g., cyclophosphamide, azathioprine, methotrexate, cyclosporine, tacrolimus, mycophenolate mofetil, mycophenolate).

The drug for treating systemic sclerosis may generally be an antifibrotic drug (e.g., colchicine), a vasoactive drug (e.g., nifedipine, Adalat), or several other commonly used drugs (e.g., naproxen, nifedipine, bosentan, sildenafil, epoprostenol, nintedanib, tocilizumab).

Herein, the active pharmaceutical ingredient for treating neuropsychiatric disorders may generally be a drug for treating schizophrenia, an antidepressant drug, a drug for treating opioid abuse, or a drug for treating anxiety disorders, preferably a drug for treating schizophrenia, more preferably a phenothiazine (e.g., chlorpromazine), a thioxanthene (e.g., chlorprothixene), a butyrophenone (e.g., haloperidol), a dibenzodiazepine (e.g., olanzapine, clozapine), a benzisoxazole (e.g., risperidone, paliperidone), a benzisothiazole (e.g., ziprasidone), a dibenzothiazepine (e.g., quetiapine), or a quinolone (e.g., aripiprazole), further preferably a benzisoxazole (e.g., paliperidone).

The antidepressant drug may generally be a tricyclic antidepressant (e.g., imipramine, clomipramine, amitriptyline), a monoamine oxidase inhibitor (e.g., moclobemide), a selective serotonin reuptake inhibitor (e.g., sertraline), a serotonin-norepinephrine reuptake inhibitor (e.g., venlafaxine), a serotonin antagonist and reuptake inhibitor (e.g., trazodone), a norepinephrine-dopamine reuptake inhibitor (e.g., bupropion), a norepinephrine inhibitor (e.g., reboxetine), and an $\alpha$2-adrenergic receptor blocker (e.g., mirtazapine).

The drug for treating opioid abuse may generally be buprenorphine or methadone.

The drug for treating anxiety disorders may generally be a benzodiazepine (e.g., diazepam), a 5-HT1A receptor partial agonist (e.g., buspirone), a $\beta$-adrenergic receptor blocker (e.g., propranolol), or a valproate.

Herein, the drug for treating hyperlipidemia may generally be a statin (e.g., simvastatin, atorvastatin, pravastatin), a fibrate (e.g., fenofibrate, bezafibrate, gemfibrozil), or a niacin (e.g., nicotinic acid).

Herein, the antitumor drug may generally be an anti-breast cancer drug (e.g., azacitidine, docetaxel, buserelin, tamoxifen, mitoxantrone, adriamycin, paclitaxel, capecitabine, goserelin, cyclophosphamide, megestrol, cetuximab, or leuprorelin), an anti-prostate cancer drug (e.g., degarelix, leuprorelin, histrelin, flutamide, estramustine, cyproterone), an anti-ovarian cancer drug (e.g., carboplatin, topotecan, methotrexate), an anti-rectal cancer drug (e.g., panitumumab), an anti-colon cancer drug (e.g., bevacizumab, oxaliplatin), an anti-liver cancer drug (e.g., sorafenib), an anti-lung cancer drug (e.g., erlotinib, gefitinib, decitabine), an anti-kidney cancer drug (e.g., pazopanib, everolimus, temsirolimus), an anti-gastric cancer drug (e.g., fluorouracil, mitomycin, cisplatin, adriamycin, etoposide), an anti-pancreatic cancer drug (e.g., nimotuzumab), an anti-esophageal cancer drug (e.g., docetaxel, paclitaxel, cisplatin, gemcitabine, tegafur, irinotecan, oxaliplatin, gefitinib, trastuzumab, anlotinib), an anti-skin cancer drug (e.g., fluorouracil), an anti-lymphoma drug (e.g., vincristine, bexarotene, dacarbazine, etoposide), an anti-myeloma drug (e.g., bortezomib), an anti-cervical cancer drug (e.g., bleomycin), or an anti-bladder cancer drug (e.g., epirubicin, BCG vaccine).

Herein, the drug for treating chronic dental diseases (e.g., dental caries, periodontal disease) may generally be categorized into nitroimidazoles (e.g., metronidazole, tinidazole, ornidazole), penicillins, and other commonly used drugs (e.g., minocycline, chlorhexidine diacetate).

Herein, the drug for treating simple obesity may generally be sibutramine or fenfluramine hydrochloride.

Herein, the active pharmaceutical ingredient for treating chronic low back pain is preferably a non-steroidal analgesic, such as ibuprofen, celecoxib, tramadol, oxycodone, meloxicam, loxoprofen, acetaminophen-codeine, or voltaren, more preferably ibuprofen or meloxicam;

the drug for treating leukemia is generally a drug that interferes with nucleic acid biosynthesis (e.g., cytarabine, methotrexate, 6-mercaptopurine), a drug that directly affects the structure and function of DNA in cancer cells (e.g., busulfan, mitomycin, chlorambucil, melphalan, cyclophosphamide), a drug that interferes with transcription process and prevents RNA synthesis (e.g., daunorubicin, doxorubicin, adriamycin, aclacinomycin), a drug that inhibits protein synthesis and function (e.g., vindesine, vincristine, L-asparaginase, homoharringtonine), or other commonly used drugs (e.g., interferon, fludarabine, arsenic trioxide, etoposide, carmustine).

Herein, the active pharmaceutical ingredient acting on the circulatory system preferably includes an active pharmaceutical ingredient for treating chronic heart failure, coronary heart disease, congenital heart disease, chronic infective endocarditis, or chronic pericarditis;

the active pharmaceutical ingredient for treating coronary heart disease may generally be a drug that improves angina symptoms (e.g., puerarin, isosorbide mononitrate), a drug that inhibits platelet aggregation (e.g., aspirin, clopidogrel bisulfate, ticagrelor), a drug that lowers lipids and stabilizes plaques (e.g., atorvastatin, rosuvastatin, pravastatin), a drug that inhibits sympathetic nerve activity (e.g., metoprolol, bisoprolol fumarate), or a drug that improves myocardial remodeling (e.g., angiotensin-converting enzyme inhibitors and angiotensin II receptor antagonists), preferably a drug that improves angina symptoms, more preferably puerarin; the active pharmaceutical ingredient for treating chronic heart failure may generally be a cardiotonic drug (e.g., digitalis, digoxin, cedilanid), a vasodilator (e.g., sodium nitroprusside, nitroglycerin), an angiotensin-converting enzyme inhibitor (e.g., enalapril, lisinopril), or a diuretic (e.g., furosemide, hydrochlorothiazide, spironolactone).

The active pharmaceutical ingredient for treating congenital heart disease may generally be digitalis, furosemide, spironolactone, phentolamine, quinidine, digoxin, hydrochlorothiazide, or coenzyme Q10.

The active pharmaceutical ingredient for treating chronic infective endocarditis may generally be an antibiotic (e.g., vancomycin, cephalosporin, penicillin, aminoglycoside).

The active pharmaceutical ingredient for treating chronic pericarditis may generally be digitalis.

Herein, the active pharmaceutical ingredient acting on the respiratory system may generally include an active pharmaceutical ingredient for treating chronic obstructive pulmonary emphysema, asthma, chronic pulmonary heart disease, chronic respiratory failure, silicosis, or pulmonary fibrosis.

The active pharmaceutical ingredient for treating chronic obstructive pulmonary emphysema may generally be a bronchodilator (including β-receptor agonists and anticholinergics), an inhaled corticosteroid (e.g., budesonide, fluticasone), a theophylline-based antiasthmatic (e.g., theophylline), an expectorant (e.g., carbocisteine, fudosteine), and, if required, a glucocorticoid or an antibiotic (e.g., penicillins, glycosides, and cephalosporins), as appropriate.

The active pharmaceutical ingredient for treating asthma may generally be a common inhaled medication (e.g., beclomethasone, budesonide, fluticasone, mometasone), a β2-agonist (e.g., salbutamol), a sustained-release theophylline, a leukotriene modifier (which can be used in combination), an anticholinergic (e.g., isopropyl scopolamine), or an antihistamine (e.g., astemizole, ketotifen).

The active pharmaceutical ingredient for treating chronic pulmonary heart disease may generally be an antibiotic (e.g., amoxicillin, ceftizoxime, cefuroxime, levofloxacin), a corticosteroid anti-inflammatory bronchodilator (e.g., selective β2-receptor agonists, theophyllines), a medication that eliminates nonspecific airway inflammation (e.g., prednisone), an inhaled medication (e.g., becotide), or a respiratory stimulant (e.g., lobeline, doxapram, duxil).

The active pharmaceutical ingredient for treating chronic respiratory failure may generally be a bronchospasm relieving and expectorant drug (e.g., salbutamol, acetylcysteine).

The active pharmaceutical ingredient for treating silicosis may generally be tetrandrine, acetylcysteine, aluminum preparations, polyvinylpyridine oxide, or salvia miltiorrhiza.

The active pharmaceutical ingredient for treating pulmonary fibrosis may generally include pirfenidone, nintedanib, glucocorticoids (e.g., methylprednisolone, prednisone), immunosuppressants (e.g., azathioprine, methotrexate), colchicine, interferon, ACEI, or statins.

Herein, the active pharmaceutical ingredient acting on the digestive system may generally include an active pharmaceutical ingredient for treating chronic gastritis, peptic ulcers, intestinal tuberculosis, chronic enteritis, chronic diarrhea, chronic hepatitis, liver cirrhosis, chronic pancreatitis, or chronic cholecystitis.

The active pharmaceutical ingredient for treating chronic gastritis may generally be an analgesic (e.g., atropine, propantheline bromide), a proton pump inhibitor (PPI) for increased gastric acidity (e.g., lansoprazole, omeprazole), a H2-receptor antagonist for mild symptoms (e.g., cimetidine, ranitidine, aluminum amine hydroxide), a digestive aid (supplemented with pancreatin), or a medication for bile reflux (e.g., metoclopramide, domperidone, cholestyramine, sucralfate, which can bind with bile acids).

The active pharmaceutical ingredient for treating peptic ulcers may generally be levofloxacin, tinidazole, or omeprazole.

The active pharmaceutical ingredient for treating intestinal tuberculosis may generally be rifampin.

The active pharmaceutical ingredient for treating chronic enteritis may generally be an anti-inflammatory analgesic, a probiotic, or an antispasmodic analgesic (e.g., atropine, propantheline bromide).

The active pharmaceutical ingredient for treating chronic diarrhea may generally be an antidiarrheal (e.g., montmorillonite powder, diphenoxylate, loperamide), an intestinal microbial preparation (e.g., lactobacillus, bifidobacterium), or an antispasmodic analgesic (e.g., pinaverium bromide).

The active pharmaceutical ingredient for treating chronic hepatitis may generally be a hepatoprotective drug (e.g., silymarin preparations, schisandra preparations), an antifibrotic drug (e.g., oral preparations of Chinese patent medicines), an antiviral drug (e.g., standard interferon and pegylated interferon), an oral nucleoside antiviral drug (e.g.,

lamivudine, adefovir dipivoxil, telbivudine, entecavir), or an immunosuppressant (e.g., azathioprine).

The active pharmaceutical ingredient for treating liver cirrhosis may generally be a drug for treating hepatitis B (e.g., nucleoside analogs), a drug for treating autoimmune hepatitis (e.g., glucocorticoids), an anti-inflammatory drug, a hepatoprotective drug, an antifibrotic drug (e.g., reduced glutathione, polyene phosphatidylcholine, magnesium isoglycyrrhizinate), a drug for treating spontaneous bacterial peritonitis (e.g., antibiotics), or a drug for treating portal hypertension (e.g., carvedilol).

The active pharmaceutical ingredient for treating chronic pancreatitis may generally be an analgesic (e.g., buprenorphine and fentanyl) or a pancreatic enzyme therapy drug (e.g., pancreatin).

The active pharmaceutical ingredient for treating chronic cholecystitis may generally be an antibacterial and anti-inflammatory drug (e.g., levofloxacin, ciprofloxacin, amoxicillin), an antispasmodic analgesic, or a choleretic (e.g., ursodeoxycholic acid).

Herein, the active pharmaceutical ingredient acting on the blood system may generally include an active pharmaceutical ingredient for treating chronic anemia, chronic myeloid leukemia, or chronic lymphocytic leukemia.

The drug for treating chronic anemia may generally include: trace elements (e.g., folic acid, vitamin B12), bone marrow stimulants (e.g., strychnine nitrate, securinine, tropane (hyoscyamine)), adenosylcobalamin, glucocorticoids (e.g., prednisone, meprednisone, betamethasone, beclomethasone propionate, prednisolone, hydrocortisone, dexamethasone, prednisone), iron supplements (e.g., ferrous fumarate, ferrous gluconate, ferrous succinate, ferrous lactate, ferric saccharate, low molecular weight iron dextran, ferric carboxymaltose, iron isomaltoside, ferric gluconate, iron oxide nanoparticles, iron sorbitol), or erythropoiesis-stimulating agents (e.g., recombinant human erythropoietin $\alpha$, darbepoetin $\alpha$).

The drug for treating chronic myeloid leukemia may generally include: tyrosine kinase inhibitors (e.g., imatinib, nilotinib, bosutinib, ponatinib) or homoharringtonine.

The drug for treating chronic lymphocytic leukemia may generally include: chemotherapy drugs (e.g., nimustine, fludarabine, chlorambucil, bendamustine), targeted drugs (e.g., idelalisib, venetoclax, ibrutinib, imatinib, dasatinib), or monoclonal antibodies (e.g., ofatumumab, rituximab, obinutuzumab, alemtuzumab).

Herein, the active pharmaceutical ingredient acting on the endocrine system may generally include an active pharmaceutical ingredient for treating chronic lymphocytic thyroiditis, hyperthyroidism, or hypothyroidism.

The drug for treating chronic lymphocytic thyroiditis may generally include: thyroid hormones (e.g., levothyroxine, thyroxine) or glucocorticoids (e.g., prednisone, meprednisone, betamethasone, beclomethasone propionate, prednisolone, hydrocortisone, dexamethasone, prednisone).

The drug for treating hyperthyroidism may generally include: thiouracils (e.g., propylthiouracil, methylthiouracil), imidazoles (e.g., methimazole, carbimazole), iodine or iodides (e.g., Lugol's solution), radioactive iodine (e.g., iodine-131), or $\beta$-blockers (e.g., metoprolol, atenolol, bisoprolol, carvedilol, propranolol).

The drug for treating hypothyroidism may generally include: thyroid hormones (e.g., levothyroxine, levothyroxine sodium, thyroxine).

Preferably, the bulk drug is a levonorgestrel bulk drug, a gestodene bulk drug, an ibuprofen bulk drug, a paliperidone bulk drug, a meloxicam bulk drug, or a puerarin bulk drug.

In the present disclosure, when the bulk drug is a gestodene bulk drug, the gestodene bulk drug is generally in powder form. The particle size of the gestodene bulk drug is preferably 1-180 $\mu$m, more preferably 2.81 $\mu$m.

The levonorgestrel bulk drug may be D(-)-17$\alpha$-ethynyl-17$\beta$-hydroxy-18-methyl-estr-4-en-3-one, which is conventional in the art. Herein, the levonorgestrel bulk drug is generally in powder form. The particle size of the levonorgestrel bulk drug is preferably 1-180 $\mu$m, such as 2.12 $\mu$m.

When the bulk drug is an ibuprofen bulk drug, the ibuprofen bulk drug is in powder form. The particle size of the ibuprofen bulk drug is preferably 1-200 $\mu$m, such as 80 $\mu$m.

When the bulk drug is a paliperidone bulk drug, the paliperidone bulk drug is in powder form. The particle size of the paliperidone bulk drug is preferably 1-200 $\mu$m, such as 1 $\mu$m, 10 $\mu$m, 50 $\mu$m, 80 $\mu$m, 120 $\mu$m, 150 $\mu$m, or 180 $\mu$m.

When the bulk drug is a meloxicam bulk drug, the meloxicam bulk drug is in powder form. The particle size of the meloxicam bulk drug is preferably 1-200 $\mu$m, such as 1 $\mu$m, 10 $\mu$m, 50 $\mu$m, 80 $\mu$m, 120 $\mu$m, 150 $\mu$m, or 180 $\mu$m.

When the bulk drug is a puerarin bulk drug, the puerarin bulk drug is in powder form. The particle size of the puerarin bulk drug is preferably 1-200 $\mu$m, such as 1 $\mu$m, 10 $\mu$m, 50 $\mu$m, 80 $\mu$m, 120 $\mu$m, 150 $\mu$m, or 180 $\mu$m.

In the present disclosure, the pharmaceutical composition is generally in powder form.

The present disclosure further provides a preparation method for the pharmaceutical composition as described above, comprising the following step: mixing the bulk drug with the insoluble excipient.

Herein, the method and conditions for the mixing may be conventional in the art.

The present disclosure further provides a use of the pharmaceutical composition as described above in the preparation of an implant.

Herein, the pharmaceutical composition is a medicinal core of the implant.

The diameter of the medicinal core is preferably 1.5-4.0 mm, such as 1.6 mm or 2.0 mm.

The length of the medicinal core is preferably 1.0-4.0 cm, such as 1.5 cm, 2.2 cm, or 3.9 cm.

**Implant**

The present disclosure further provides an implant, comprising a medicinal core and the silicone tube as described above, and the medicinal core comprises an active pharmaceutical ingredient.

Herein, the active pharmaceutical ingredient may be a low-solubility small molecule drug, such as an active pharmaceutical ingredient with a solubility of ≤ 100 mg/mL (using water as the solvent) and a molecular weight of less than 1000 Da; or such as one or more of levonorgestrel, gestodene, gestrinone, estradiol, ibuprofen, paliperidone, meloxicam, and puerarin.

The active pharmaceutical ingredient may be an active pharmaceutical ingredient with a solubility of ≤ 60 mg/mL (using water as the solvent) and a molecular weight of less than 1000 Da, such as levonorgestrel, gestodene, gestrinone, estradiol, ibuprofen, paliperidone, meloxicam, or puerarin.

The active pharmaceutical ingredient may be an active pharmaceutical ingredient with a solubility of ≤ 50 mg/mL (using water as the solvent) and a molecular weight of less than 1000 Da, such as levonorgestrel, gestodene, gestrinone, estradiol, paliperidone, meloxicam, or puerarin.

The active pharmaceutical ingredient may be an active pharmaceutical ingredient with a solubility of ≤ 10 mg/mL (using water as the solvent) and a molecular weight of less than 1000 Da, such as levonorgestrel, gestodene, gestrinone, estradiol, meloxicam, or puerarin.

The active pharmaceutical ingredient may be an active pharmaceutical ingredient with a solubility of ≤ 5 mg/mL (using water as the solvent) and a molecular weight of less than 1000 Da, such as meloxicam or puerarin.

In the present disclosure, the medicinal core may be a powder-type medicinal core.

Herein, in the powder-type medicinal core, the particle size of the active pharmaceutical ingredient may be 2-180 μm.

When the medicinal core is a powder-type medicinal core, the implant may be prepared by the following method: cutting the silicone tube into segments, filling each segment with the powder-type medicinal core, and sealing both ends with silicone to obtain the implant.

In the present disclosure, the medicinal core may be the pharmaceutical composition as described above.

In the present disclosure, the outer diameter of the silicone tube is preferably 2.0-5.0 mm, such as 2.4 mm or 2.6 mm.

In the present disclosure, the length of the silicone tube is preferably 1.5-4.5 cm, such as 1.9 cm or 4.4 cm.

In the present disclosure, the wall thickness of the silicone tube is preferably 0.2-0.5 mm, such as 0.2 mm, 0.3 mm, 0.4 mm, or 0.5 mm.

In the present disclosure, the drug release area of the silicone tube is preferably 0.4-15.0 cm$^2$, such as 0.69 cm$^2$, 1.38 cm$^2$, 2.07 cm$^2$, 2.76 cm$^2$, or 3.45 cm$^2$.

In the present disclosure, the diameter of the medicinal core is preferably 1.5-4.0 mm, such as 1.6 mm or 2.0 mm.

In the present disclosure, the length of the medicinal core is preferably 1.0-5.0 cm, such as 1.0-4.0 cm, or such as 1.5 cm or 3.9 cm, or such as 1 cm, 2 cm, 3 cm, 4 cm, or 5 cm.

In some preferred embodiments of the present disclosure, the specifications of the implant are as shown in the table below, and the active pharmaceutical ingredient in the medicinal core is preferably levonorgestrel;

| Component | Diameter | Length | Wall thickness | Daily release amount |
|---|---|---|---|---|
| Silicone tube | 2.4 mm | 4.4 cm | 0.4 mm | 40-50 μg/day (1 set of 6 tubes) |
| Medicinal core | 1.6 mm | 3.9 cm | | |

In some preferred embodiments of the present disclosure, the specifications of the implant are as shown in the table below, and the active pharmaceutical ingredient in the medicinal core is preferably gestodene;

| Component | Diameter | Length | Wall thickness | Daily release amount |
|---|---|---|---|---|
| Silicone tube | 2.6 mm | 1.9 cm | 0.3 mm | 10-30 μg/day (1 tube) |
| Medicinal core | 2.0 mm | 1.5 cm | | |

In some preferred embodiments of the present disclosure, the specifications of the implant are as shown in the table below, and the active pharmaceutical ingredient in the medicinal core is preferably estradiol;

| No. | Silicone tube outer diameter/mm | Silicone tube wall thickness/mm | Drug release area/cm$^2$ |
|-----|----------------------------------|----------------------------------|---------------------------|
| 1 | 2.2 | 0.2 | 0.69 |
| 2 | 2.2 | 0.2 | 1.38 |
| 3 | 2.2 | 0.2 | 2.07 |
| 4 | 2.2 | 0.2 | 2.76 |
| 5 | 2.2 | 0.2 | 3.45 |

The preparation process of the implant of the present disclosure may involve extruding the silicone material into a tubular shape or cutting the silicone tube into segments, filling with the drug, sealing both ends with an adhesive, and then packaging and sterilizing to obtain the implant of the present disclosure.

The present disclosure further provides an implant, comprising the pharmaceutical composition and the silicone tube as described above.

The preparation method for the implant may involve filling the silicone tube with the pharmaceutical composition.

In the present disclosure, the silicone tube may be conventional in the art. Preferably, the raw material composition of the silicone material is as described above.

**Test method for amount of drug released from implant**

To overcome the difficulty in accurately evaluating the amount of a drug released from implants based on their physical parameters in the prior art, the present disclosure provides a implant and a test method for the amount of a drug released. The implant of the present disclosure can achieve stable drug release, with a precisely controlled release amount, and the side surface area of the drug-loaded segment can be adjusted to obtain an implant with a desired release amount.

The present disclosure further provides an implant, comprising a medicinal core and a silicone tube;

the medicinal core comprises an active pharmaceutical ingredient, and the particle size $D_{50}$ of the active pharmaceutical ingredient is 180 $\mu$m or less;
the raw material composition of the silicone tube comprises the following components in parts by weight:

R-vinyl silicone rubber: 100 parts;
hydrogen-containing silicone oil: 0.3-3.0 parts;
a reinforcing agent: 20-80 parts;
a catalyst: $\geq$ 0.000002 parts, preferably $2\times10^{-6}$-$5\times10^{-5}$ parts;
wherein
in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl groups in the R-vinyl silicone rubber is 0.05-0.50 mol%;
the content of Si-H groups in the hydrogen-containing silicone oil is 0.18-1.6 mol%;
the molar ratio of Si-H groups in the hydrogen-containing silicone oil to vinyl groups in the R-vinyl silicone rubber is (0.5-4.0): 1;
the preparation method for the silicone tube comprises the following step: subjecting the raw material mixture of the silicone tube to extrusion molding.

In the present disclosure, the active pharmaceutical ingredient may be as described above.

Preferably, the active pharmaceutical ingredient is levonorgestrel, gestodene, ibuprofen, paliperidone, meloxicam, puerarin, or estradiol.

In the present disclosure, the medicinal core may be a powder-type medicinal core.

In the present disclosure, the particle size $D_{50}$ of the active pharmaceutical ingredient is preferably 1-180 $\mu$m, such as 2.12 $\mu$m, 2.81 $\mu$m, 43.24 $\mu$m, 80.3 $\mu$m, or 100 $\mu$m. It is known to those skilled in the art that the particle size $D_{50}$ is generally obtained through pulverization.

In the present disclosure, in the medicinal core, the mass of the active pharmaceutical ingredient (i.e., the drug loading) may be conventional in the art.

When the active pharmaceutical ingredient is gestodene, the drug loading is preferably 10-100 mg, such as 12.5 mg, 22.8 mg, 29.6mg, 42mg, 60mg, or 84mg.

When the active pharmaceutical ingredient is levonorgestrel, the drug loading is preferably 15-300 mg, such as 15 mg, 36 mg, 72 mg, 75 mg, 100 mg, 150 mg, 180 mg, 216 mg, or 252 mg.

When the active pharmaceutical ingredient is estradiol, the drug loading is preferably 10-300 mg, such as 75 mg or 100 mg.

When the active pharmaceutical ingredient is ibuprofen, the drug loading is 10-2000 mg, such as 40 mg or 100 mg.

When the active pharmaceutical ingredient is meloxicam, the drug loading is 10-2000 mg, such as 40 mg or 100 mg.

When the active pharmaceutical ingredient is paliperidone, the drug loading is 10-2000 mg, such as 40 mg or 100 mg.

When the active pharmaceutical ingredient is puerarin, the drug loading is 10-2000 mg, such as 40 mg or 100 mg.

In the present disclosure, the medicinal core preferably further comprises an excipient, and the excipient comprises a silicon material and an insoluble pH adjuster.

Herein, the silicon material is, for example, white carbon black and/or mesoporous silica. The white carbon black is, for example, fumed white carbon black. The mesoporous silica is, for example, one or more of AL-1FP mesoporous silica, XDP3050 mesoporous silica, and XDP3150 mesoporous silica.

Herein, the pore size of the silicon material may be less than 1 μm, such as 0 nm, 5 nm, 10 nm, 18 nm, 50 nm, or 100 nm. It can be understood by those skilled in the art that when the pore size of the silicon material is 0 nm, the silicon material is a non-porous silicon material.

In the silicon material, the content of silicon dioxide may be more than 50%, preferably 80%, 90%, 95%, 99%, or 99.8%.

Herein, the pH adjuster includes an insoluble weak acid and/or an insoluble weak base.

Herein, the insoluble weak acid preferably includes one or more of boric acid, fumaric acid, molybdic acid, silicic acid, tungstic acid, and germanic acid, more preferably including boric acid and/or fumaric acid.

Herein, the insoluble weak base preferably includes one or more of magnesium hydroxide, aluminum hydroxide, zinc hydroxide, ferrous hydroxide, and magnesium oxide, more preferably including one or more of magnesium hydroxide, aluminum hydroxide, and zinc hydroxide.

Herein, the mass ratio of the excipient to the active pharmaceutical ingredient is preferably 1:1.

In the present disclosure, the raw material composition of the silicone tube may be the same as the raw material composition of the silicon material.

In the present disclosure, the extrusion molding process may be the same as the molding process in the preparation method for the silicon material.

In the present disclosure, the implant preferably further undergoes a depowdering treatment. The depowdering treatment is generally used to remove the drug powder adhered to the surface of the silicone tube, as the levonorgestrel or gestodene implant without depowdering treatment has the drug powder electrostatically adhered to the surface of the silicone tube, resulting in a significant burst release effect in the release of the implant. To make the initial release amount close to the steady-state release amount and reduce the risk of burst release when the implant is introduced into the body, a depowdering pre-treatment is required.

Herein, the depowdering treatment may be conventional in the art, for example, comprising the following steps: mixing the implant with anhydrous ethanol and sonicating for 1 minute, repeating three times, then adding 100 mL of distilled water and leaving it overnight, and discarding the soaking solution the next day.

In the present disclosure, the wall thickness of the silicone tube may be conventional in the art, preferably 0.1-0.5 mm, such as 0.3 mm or 0.4 mm.

In the present disclosure, the inner diameter of the silicone tube may be conventional in the art, preferably 1-5 mm, such as 1.4 mm, 1.6 mm, 1.8 mm, or 2.0 mm.

In the present disclosure, the outer diameter of the silicone tube may be conventional in the art, preferably 1-6 mm, such as 2.6 mm.

In the present disclosure, after the implant is prepared, if it is necessary to change the daily drug release amount, the side surface area of the medicinal core can be adjusted, as the side surface area of the medicinal core is proportional to the drug release amount. For example, if it is necessary to increase the daily drug release amount, the side surface area of the medicinal core can be increased. If the medicinal core is cylindrical, when the diameter of the core is fixed, the length of the core may be 0.5-25 cm, such as 0.8 cm, 1.4 cm, 2.2 cm, 2.7 cm, 3.3 cm, 4.3 cm, 9 cm, 15 cm, 18 cm, or 21 cm; when the length of the core is fixed, the diameter of the core may be 0.5-10 mm, such as 0.5 mm, 0.8 mm, 1.4 mm, 1.6 mm, 2.0 mm, 2.7 mm, 3.3 mm, 4.3 mm, 6.8 mm, 9.0 mm, or 10.0 mm. Alternatively, the side surface area of the medicinal core can be adjusted by simultaneously adjusting both the length and diameter of the medicinal core, thereby adjusting the drug release amount.

The present disclosure further provides a test method for the drug release amount of the implant, comprising the following step: simulating with different side surface areas of the medicinal core and daily drug release amounts of the implant to obtain a mathematical model formula: $y = kx + b$;

y is the daily drug release amount, in $\mu$g/d; x is the side surface area of the medicinal core, in cm$^2$; x = $\pi$DL, where D is the diameter of the medicinal core, and L is the length of the medicinal core.

In the present disclosure, it is known to those skilled in the art that the daily drug release amount may have the meaning conventional understood in the art, generally referring to the *in vitro* simulation of the *in vivo* daily drug release amount.

From the mathematical model formula, it can be derived that the drug release side surface area is only related to the diameter (D) and length (L) of the medicinal core. When the length of the medicinal core is fixed, the side surface area (i.e., the drug release area) can be adjusted by adjusting the diameter of the medicinal core. When the diameter of the medicinal core is fixed, the side surface area can be adjusted by adjusting the length of the medicinal core. Additionally, the side surface area can also be adjusted by simultaneously adjusting both the length and diameter of the medicinal core.

In the present disclosure, when the active pharmaceutical ingredient is gestodene, preferably, the value of k is 4 to 16, and the value of b is -4 to 4. Preferably, R$^2$ is greater than 0.99. Herein, the value of k is, for example, 6.257, 7.520, 8.7363, 8.8140, 9.057, 10.229, or 11.591.

Herein, the value of b is preferably 0 to 1, such as 0.0802, 0.1767, 0.498, 0.561, 0.587, 0.708, or 0.802.

Herein, the R$^2$ is, for example, 0.9947, 0.998, 0.9982, 0.9987, 0.9998, or 0.9999.

In one specific embodiment of the present disclosure, when the active pharmaceutical ingredient is gestodene, the mathematical model formula is y = 11.591x + 0.561, with R$^2$ = 0.9947.

In one specific embodiment of the present disclosure, when the active pharmaceutical ingredient is gestodene, the mathematical model formula is y = 6.257x + 0.498, with R$^2$ = 0.998.

In one specific embodiment of the present disclosure, when the active pharmaceutical ingredient is gestodene, the mathematical model formula is y = 7.520x + 0.587, with R$^2$ = 0.9987.

In one specific embodiment of the present disclosure, when the active pharmaceutical ingredient is gestodene, the mathematical model formula is y = 10.229x + 0.802, with R$^2$ = 0.9982.

In one specific embodiment of the present disclosure, when the medicinal core is gestodene, the mathematical model formula is y = 9.057x + 0.708, with R$^2$ = 0.9987.

In one specific embodiment of the present disclosure, when the active pharmaceutical ingredient is gestodene, the mathematical model formula is y = 8.7363x + 0.0802, with R$^2$ = 0.9999.

In one specific embodiment of the present disclosure, when the medicinal core is gestodene, the mathematical model formula is y = 8.8140x + 0.1767, with R$^2$ = 0.9998.

In the present disclosure, when the active pharmaceutical ingredient is levonorgestrel, preferably, the value of k is 1 to 10, and the value of b is -6 to 6; preferably, R$^2$ is greater than 0.99.

Herein, the value of k is, for example, 3.412, 4.2459, 5.0172, 5.6294, 5.6865, 5.8036, 6.4689, 6.4978, or 6.6085.

Herein, the value of b is preferably -2 to 1, such as -1.7641, -1.919, -0.2767, -0.2482, -0.9893, -0.3064, 0.3281, 0.3967, or 0.5.

Herein, the R$^2$ is, for example, 0.9951, 0.997, 0.9973, 0.9957, 0.9978, 0.998, or 0.9979.

In one specific embodiment of the present disclosure, when the active pharmaceutical ingredient is levonorgestrel, the mathematical model formula is y = 4.2459x + 0.3967, with R$^2$ = 0.9957.

In one specific embodiment of the present disclosure, when the active pharmaceutical ingredient is levonorgestrel, the mathematical model formula is y = 6.4978x - 1.9190, with R$^2$ = 0.9979.

In one specific embodiment of the present disclosure, when the active pharmaceutical ingredient is levonorgestrel, the mathematical model formula is y = 6.4689x - 0.2767, with R$^2$ = 0.9973.

In one specific embodiment of the present disclosure, when the active pharmaceutical ingredient is levonorgestrel, the mathematical model formula is y = 5.8036x - 0.2482, with R$^2$ = 0.9973.

In one specific embodiment of the present disclosure, when the active pharmaceutical ingredient is levonorgestrel, the mathematical model formula is y = 3.4120x - 0.9893, with R$^2$ = 0.9978.

In one specific embodiment of the present disclosure, when the active pharmaceutical ingredient is levonorgestrel, the mathematical model formula is y = 6.6085x + 0.3064, with R$^2$ = 0.997.

In one specific embodiment of the present disclosure, when the active pharmaceutical ingredient is levonorgestrel, the mathematical model formula is y = 5.0172x + 0.3281, with R$^2$ = 0.998.

In one specific embodiment of the present disclosure, when the active pharmaceutical ingredient is levonorgestrel, the mathematical model formula is y = 5.6865x - 1.7641, with R$^2$ = 0.9951.

In one specific embodiment of the present disclosure, when the active pharmaceutical ingredient is levonorgestrel, the mathematical model formula is y = 5.6294x + 0.5000, with R$^2$ = 0.9970.

In the present disclosure, based on the established mathematical model formulas, the drug release amount of the implant with different medicinal core side surface areas can be quantitatively prepared and tested.

In the present disclosure, it is known to those skilled in the art that the k value and b value in the mathematical model formula are calculated based on at least three or more sets of data on implants with different medicinal core side surface areas and daily drug release amounts.

In the present disclosure, it is known to those skilled in the art that in order to obtain the drug release amount of the implant to be tested, the medicinal core side surface area of the implant to be tested is generally substituted into the mathematical model to obtain the y value, which is the drug release amount of the implant to be tested. According to the mathematical model formula, it can be understood that the daily drug release amount of the implant can be adjusted by altering the side surface area of the medicinal core. Combined with the definition of the side surface area of the medicinal core, it can be understood that once the length and diameter of the medicinal core are determined, the side surface area of the medicinal core is determined. Therefore, the desired daily drug release amount can be obtained by altering the length, the diameter, or both the length and diameter of the medicinal core.

In the present disclosure, the daily drug release amount may have the meaning conventional understood in the art, generally referring to the daily release amount when the release medium is water or an aqueous medium. Research has shown that the drug release amount of the implant in water or an aqueous medium is consistent with the drug release amount *in vivo.*

In the present disclosure, the content of vinyl groups in the R-vinyl silicone rubber refers to the molar percentage, which indicates the number of moles of vinyl groups per hundred moles of the R-vinyl silicone rubber.

In the present disclosure, the content of hydrogen in the hydrogen-containing silicone oil refers to the molar percentage, which indicates the number of moles of hydrogen per hundred moles of the hydrogen-containing silicone oil.

In the present disclosure, PHR refers to the parts by mass of each specific component per 100 parts by mass of R-vinyl silicone rubber (such as methyl vinyl silicone rubber).

In the present disclosure, the term "room temperature" refers to 25°C ± 5°C.

In the present disclosure, the terms "first", "second", "third", etc. are used to describe various heat treatments, and these heat treatments should not be limited by the terms. These terms are only used to distinguish one heat treatment from another.

Based on common knowledge in the art, the above preferred conditions can be arbitrarily combined to obtain the preferred examples of the present disclosure.

The reagents and raw materials used in the present disclosure are all commercially available.

The positive and progressive effects of the present disclosure are as follows:

(1) The silicone tube made from the silicone material in the present disclosure exhibits excellent mechanical properties and good biocompatibility. The implant prepared using the silicone tube has a stable drug release profile upon loading with an active drug. When the loaded drug is a contraceptive (e.g., gestodene, levonorgestrel), the prepared implant can produce a significant contraceptive effect in rats.

(2) The positive and progressive effect of the present disclosure is that: the pharmaceutical composition of the present disclosure exhibits low static electricity, thereby facilitating the filling process, and can prevent airborne powder and spontaneous aggregation. It also allows for adjustable loading doses and drug release doses. As a result, high utilization of the bulk drug and high drug release stability of the implant further prepared from the pharmaceutical composition are achieved.

(3) In the present disclosure, the prepared implant can accurately control the daily drug release amount of the active pharmaceutical ingredient with a stable release by specially selecting the particle size of the active pharmaceutical ingredient in the medicinal core and optimizing the preparation process for the silicone tube. Simultaneously, in the implant of the present disclosure, there is a linear positive correlation between the drug release side surface area of the medicinal core and the daily drug release amount. By altering the side surface area of the medicinal core in the implant, the desired implant with different daily drug release amounts can be obtained.

BRIEF DESCRIPTION OF THE DRAWINGS

Fig. 1 shows a process flow diagram for the preparation of silicone tube.
Fig. 2 shows a process flow diagram for the extrusion of silicone tube.
Fig. 3 shows an image of the GEST contraceptive implant.
Fig. 4 shows the relationship between daily drug release amount and release time *in vitro* for the gestodene implants ZJ001, ZJ002, and ZJ003.
Fig. 5 shows the relationship between daily drug release amount and release time *in vitro* for the gestodene implants ZJ001, ZJ002, and ZJ003, based on Fig. 4, with the data simplified to show the daily drug release amount in a 5-day cycle.
Fig. 6 shows pathological images of muscle tissue after the implantation of gestodene implants, where A, C, and E represent the pathological images on days 3, 10, and 30 post-implantation, respectively (HE, ×2), and B, D, and F represent the pathological images on days 3, 10, and 30 post-implantation, respectively (HE, ×20).

Fig. 7 shows vaginal smears of the estrous cycle in rats, with Panel A representing the proestrus stage, Panel B representing the estrus stage, Panel C representing the metestrus stage, and Panel D representing the diestrus stage.

Fig. 8 shows images of vaginal plugs in rats. In Panel A, a milky white vaginal plug is observed at the vaginal opening of rat numbered 1, which had a normal estrous cycle in the GEST implant experimental dose group I, 13 days post-implantation when vaginal secretions were collected. In Panel B, a vaginal plug is observed at the vaginal opening of rat numbered 4, which had a normal estrous cycle in the GEST implant experimental dose group I, 20 days post-implantation when vaginal secretions were collected.

Fig. 9 shows the over-cured silicone tube produced at a front drying tunnel temperature of 360°C.

Fig. 10 shows tissue sections from SD rats post-implantation of levonorgestrel implants, including: a tissue section on day 3 post-implantation (A, HE, ×2), a local view of the tissue section on day 3 post-implantation (B, HE, ×20), a tissue section on day 10 post-implantation (C, HE, ×2), and a local view of the tissue section on day 10 post-implantation (D, HE, ×20).

Fig. 11 shows the daily dose release curves of LNG implants for the depowdered group and the untreated group.

Fig. 12 shows the cumulative dose release curves of LNG implants for the depowdered group and the untreated group.

Fig. 13 shows the linear relationship between l/T and ln(K).

Fig. 14 shows the measurement results of LH in rats from the levonorgestrel dose group I.

Fig. 15 shows the relationship between the drug surface area of silicone tube and the release amount of estradiol.

Fig. 16 shows the relationship curve between daily drug release amount and release time *in vitro* for the implants in Example 22.

Fig. 17 shows the relationship curve between different release times and daily drug release amounts for gestodene implants in Example 27 and the control experiment with 0.23% vinyl content of raw rubber.

Fig. 18 shows the relationship curve between different release times and daily drug release amounts for gestodene implants in Example 27 and the control experiment with a molar ratio of 1.5:1.

Fig. 19 shows the relationship curve between different release times and daily drug release amounts for gestodene implants in Example 27 and the control experiment with a wall thickness of 0.5 mm.

Fig. 20 shows the relationship curve between different release times and daily drug release amounts for gestodene implants in Example 27 and the control experiment with a drug particle size of 80.3 $\mu$m.

Fig. 21 shows the relationship curve between different release times and daily drug release amounts for gestodene implants in Example 27 and the control experiment with a drug loading of 29.6 mg.

Fig. 22 shows the relationship curve between different release times and daily drug release amounts for levonorgestrel implants in Example 28 and the control experiment with 0.23% vinyl content of raw rubber.

Fig. 23 shows the relationship curve between different release times and daily drug release amounts for levonorgestrel implants in Example 28 and the control experiments with molar ratios of 1.2:1 and 1.5:1.

Fig. 24 shows the relationship curve between different release times and daily drug release amounts for levonorgestrel implants with different white carbon black contents in Example 28.

Fig. 25 shows the relationship curve between different release times and cumulative release amounts of active pharmaceutical ingredients for levonorgestrel implants in Example 28 and those with a particle size of 43.24 $\mu$m.

Fig. 26 shows the relationship curve between different release times and daily drug release amounts for levonorgestrel implants in Example 28 and those with different wall thicknesses.

Fig. 27 shows the relationship curve between different release times and daily drug release amounts for levonorgestrel implants in Example 28 and those with different drug loadings.

## DETAILED DESCRIPTION OF THE PREFERRED EMBODIMENT

The present disclosure is further illustrated below by means of examples, but the present disclosure is not thereby limited to the scope of the described examples. The experimental methods for which specific conditions are not indicated in the following examples are selected according to conventional methods and conditions, or according to product instructions.

In the following examples and comparative examples:

The content of vinyl groups in methyl vinyl silicone rubber refers to the molar percentage. The molar percentage of vinyl groups refers to the number of moles of vinyl groups per hundred moles of methyl vinyl silicone rubber.

The content of hydrogen in hydrogen-containing silicone oil refers to the molar percentage. The molar percentage of hydrogen refers to the number of moles of hydrogen per hundred moles of hydrogen-containing silicone oil.

## I. Long-acting gestodene contraceptive implants

**(I) Content determination and *in vitro* release testing methods for gestodene contraceptive implants**

(1) Chromatographic conditions

Chromatographic column: Diamonsil® $C_{18}$ column (250 mm × 4.60 mm, 5 μm);
Mobile phase: methanol-water (80:20, v/v);
Column temperature: 30°C;
Detection wavelength: 239 nm;
Flow rate: 1 mL·min$^{-1}$;
Injection volume: 20 μL.

*(2) In vitro* release testing method

The filled implant was placed in an ethanol solution and subjected to ultrasonication for 30 minutes. After ultrasonication, the implant was dried by blowing and then placed in a stoppered conical flask containing the release medium. The stoppered conical flask was placed in a shaker set at 37°C and shaken at 100 rpm for 24 hours. After 24 hours, it was taken out and dried by blowing.

Both ends of the dried gestodene implant were adhered to the bottom and wall of the stoppered conical flask using silicone rubber sealant, ensuring that the silicone tube filled with gestodene bulk drug powder was suspended in the stoppered conical flask without touching the wall, thus maintaining a stable contact area between the drug-loaded segment and the release medium during shaking. After adhesion, it was left to stand for 24 hours to allow the sealant to fully cure. Once cured, the required amount of release medium was accurately added to the stoppered conical flask, which was then placed in a shaker set at 37°C and shaken at 100 rpm. Samples were taken and the medium was exchanged every 24 hours, with the samples being retained for testing.

**(II) Preparation of two-component addition-cure silicone tubes**

1. Instruments and reagents

1.1 Instruments

| | |
|---|---|
| Small Kneader | Laizhou Shenhong Machinery Co., Ltd. |
| Small Open Mill | Dongguan Yizong Machinery Equipment Co., Ltd. |
| Φ16 Silicone Rubber Extruder | Zhejiang Baina Rubber & Plastic Equipment Co., Ltd. |
| 3020N Outer Diameter Measurement and Control Instrument | Shanghai Pinzhong Testing Equipment Co., Ltd. |
| Electron Microscope | Shenzhen Zhongwei Kechuang Technology Co., Ltd. |
| XPE Analytical Balance | Mettler-Toledo Instruments Co., Ltd., Switzerland |
| SMD200-2 Electronic Analytical Balance | Ohaus International Trade Co., Ltd. |
| 101-2AB Electric Blast Drying Oven | Tianjin Taist Instrument Co., Ltd. |
| Universal Mechanical Tester | Dongguan Nanyue Laboratory Equipment Co., Ltd. |
| Shore A Durometer | Deqing Shengtaixin Electronic Technology Co., Ltd. |

1.2 Reagents

| | |
|---|---|
| Methyl vinyl silicone rubber (Relative molecular weight: 100,000-800,000 g/mol) | Dongjue Silicone Group Co., Ltd. |

(continued)

| Hydrogen-containing silicone oil (Hydrogen content: 0.18-1.6 mol%) | Guangdong Silicon Ye New Material Technology Co., Ltd. |
|---|---|
| Fumed white carbon black | Dalian Shengsen Nano Silicon Carbon Materials Co., Ltd. |
| Platinum catalyst | Guangdong Silicon Ye New Material Technology Co., Ltd. |
| Methylbutynol | Jiuding Chemical Technology Co., Ltd. |
| XDP3050 mesoporous silica | W. R. Grace & Co., USA |
| AL-1FP mesoporous silica | W. R. Grace & Co., USA |
| Gestodene bulk drug | Hebei Mokai Technology Development Co., Ltd. |

2. Experimental methods

2.1 Optimization of preparation process of silicone tube

2.1.1 Preparation

| Material name | Amount |
|---|---|
| Methyl vinyl silicone rubber | 100 PHR |
| Content of vinyl groups in vinyl polysiloxane | 0.17 mol% |
| Fumed white carbon black | 40 PHR |
| Hydrogen-containing silicone oil | 1.01 PHR |
| Molar ratio of Si-H groups in hydrogen-containing silicone oil to vinyl groups in methyl vinyl silicone rubber | 1.2:1 |
| Content of hydrogen in hydrogen-containing silicone oil | 0.75 mol% |
| 2-Methyl-3-butyn-2-ol | 0.7 PHR |
| Platinum catalyst (3000 ppm) | 0.00001 PHR |
| Note: PHR refers to the parts by mass of a given component per 100 parts by mass of the polymer compound (methyl vinyl silicone rubber); | |

ppm refers to parts per million, indicating the concentration of platinum in the platinum catalyst; 3000 ppm indicates that the mass concentration of platinum in the platinum catalyst is 3000 parts per million; PHR indicates the proportion of the platinum catalyst in the overall silicone tube preparation, and taking the above preparation as an example, it specifically means that 0.000002 to 0.00005 parts of platinum catalyst are added to 100 parts of methyl vinyl silicone rubber, with the concentration of platinum in the platinum catalyst being 3000 parts per million.

2.1.2 Preparation process

(1) Pre-treatment of raw rubber: Raw rubber (methyl vinyl silicone rubber) and fumed white carbon black were added to a kneader in a specific proportion, ensuring that the fumed white carbon black was fully wrapped by the raw rubber. The mixture was then processed by extruding and passing through an open mill to mix the raw rubber and the fumed white carbon black thoroughly, and pressed into sheets. The sheet was unloaded, wrapped thoroughly in cling film, and sealed in a ziplock bag. The sealed mixture was stored at room temperature in a desiccator for 24-72 hours for later use.

(2) Preparation of matrix components: The pretreated raw rubber mixed with white carbon black was divided equally into two portions according to mass, designated as component A and component B. Component A was added with the prescribed amount of cross-linking agent (hydrogen-containing silicone oil) and inhibitor (methylbutynol), and processed by mixing thoroughly and passing through an open mill. The mixture was then stored at room temperature in a desiccator for 24-72 hours for later use.

(3) Preparation of catalyst components: Component B was added with the prescribed amount of platinum catalyst, and processed by mixing thoroughly and passing through an open mill. The mixture was then stored at room temperature in a desiccator for 24-72 hours for later use.

(4) Components A and B, which had been stored for the same duration, were mixed and processed by passing through an open mill continuously. The resultant sheet was then stored for 12 hours.

(5) The stored rubber was cut into thin strips for later use.

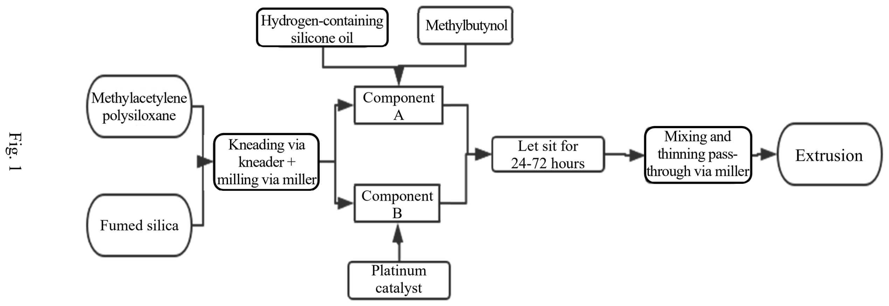

The flowchart for the above steps (1) to (5) can be seen in Fig. 1.

After the two-component silicone rubber material was prepared, it was pressed into sheets and extruded to prepare a silicone tube. During the extrusion process, the silicone rubber material was extruded by a screw and formed into a silicone tube through a mold. The extruded silicone tube passed through the high-temperature front drying tunnel, quickly vulcanized, and initially molded. It then entered the rear drying tunnel to continue vulcanization, allowing the addition reaction to proceed almost completely. Finally, the silicone tube was placed in an oven at a specified temperature for vulcanization to ensure complete addition reaction.

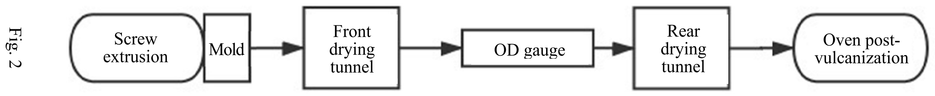

During the extrusion process, the front drying tunnel, the rear drying tunnel, and the oven provided the vulcanization conditions for the addition reaction between the raw rubber and hydrogen-containing silicone oil, resulting in the curing and molding of the silicone material. The extrusion speed, mold, and rear drying tunnel stretching speed (extrusion speed: 3.5 rpm, rear drying tunnel stretching speed: 0.1 m/s, mandrel outer diameter: 2.50 mm, die inner diameter: 3.90 mm) were adjusted to regulate the outer diameter and wall thickness of the prepared silicone tube, allowing for the production of silicone tubes with different specifications. The extrusion process is shown in Fig. 2.

2.1.3 Performance testing of silicone tubes

Tensile strength (Ts) refers to the maximum tensile force the silicone tube can withstand during breakage, indicating the maximum resistance of the sample to external damage.

Elongation at break (Eb) refers to the deformation condition of the mold sample when it breaks, indicating the range of deformation the sample can endure before breaking.

Tear strength (T) refers to the strength required to tear the silicone tube, indicating the ability of the sample to resist tearing.

Hardness (H) refers to the ability of the silicone tube to resist extrusion by external forces.

2.1.3.1 Tensile stress-strain performance testing

The tensile stress-strain performance was measured according to GB/T 828-2009. The prepared silicone tube samples were symmetrically placed on the upper and lower grippers of a servo system tensile testing machine, ensuring that the tensile force was evenly distributed across the cross-section. The gripper distance was set at 50 mm. A device to measure elongation was prepared, and the tensile testing machine was started with a sample tensile rate of 200 mm/min. The maximum tensile stress during the stretching process, the length of the sample when stretched to break, and the force required to tear the sample were recorded. The elongation at break, tensile strength, and tear strength of the samples were calculated according to the following formulas:

$$\text{Elongation at break (\%): } E_b = 100 \, (L_b - L_0) / L_0$$

wherein $L_b$ is the gauge length at break (mm), and $L_0$ is the initial gauge length (mm).

$$\text{Tensile strength (MPa): } Ts = Fm/Wt$$

wherein Fm is the recorded maximum force, W is the width of the narrow part of the cutter (mm), and t is the thickness of the length part of the sample.

$$\text{Tear strength (KN/m): } T = F/t$$

wherein F is the maximum force required to tear the sample, and t is the thickness of the sample (mm).

2.1.3.2 Hardness testing

The hardness test of the product was conducted according to GB/T 531.1-2008. After thoroughly mixing the prepared matrix component polymer with the catalyst component polymer, the mixture was cured and molded at room temperature, forming a sample with a thickness of 6 mm, which was then cut into 24 × 24 mm square test pieces. The test pieces were placed on a solid, flat surface. The durometer was held steadily, and the presser foot was gently pressed on the test piece, ensuring full contact. The data was read within 1 second. The measurement was repeated three times at different positions on the test piece, and the average value was recorded.

The preferred physical and mechanical performance indicators of the silicone tube products are shown in the table below.

| Item | Index |
| --- | --- |
| Hardness (Shore A) | 50-70 |
| Tensile strength (MPa) | ≥ 7.5 |
| Elongation at break (%) | ≥ 200 |
| Tear strength (KN/m) | ≥ 20 |

2.1.4 Optimization of preparation process of silicone tube

This experiment fixed the preparation to evaluate the front drying tunnel temperature (first vulcanization temperature), rear drying tunnel temperature (second vulcanization temperature), and oven vulcanization time (third vulcanization time) in the extrusion process, using the elongation at break, tensile strength, tear strength, and hardness as mechanical properties.

2.1.4.1 Investigation of front drying tunnel temperature (first vulcanization temperature)

After being extruded through a single-screw extruder, the silicone rubber passes through a high-temperature oven, and the inhibitor in the silicone tube decomposes into gas upon exposed to high temperatures. Subsequently, the catalyst begins to function, initiating the catalytic addition reaction and causing the silicone tube to be quickly cured and molded. The oven temperature significantly affects both the appearance and mechanical properties of the extruded silicone tube. If the temperature is too low, vulcanization is insufficient, resulting in an incompletely formed outer wall that is difficult to stretch for subsequent processes. Conversely, if the temperature is too high, the silicone tube is prone to over-vulcanization, leading to degraded mechanical properties. As the silicone tube passes through the high-temperature oven, its outer diameter rapidly shrinks, and then returns to its original size after exiting the oven. This fluctuation can potentially affect the outer diameter and wall thickness of the silicone tube.

In this experiment, the front drying tunnel temperatures were set at 270°C, 300°C, 330°C, and 360°C. The mechanical properties of elongation at break, tensile strength, tear strength, and hardness were used to evaluate the high-temperature oven temperature (for the investigation of front drying tunnel temperature, the extrusion time in the front drying tunnel was very short, approximately 5 seconds; the rear drying tunnel temperature was set at 180°C, with a reaction time of approximately 2 minutes; the oven vulcanization temperature was set at 180°C with a duration of 48 hours; other process conditions followed the details provided in section 2.1.2 preparation process; the preparation followed the details provided in section 2.1.1 preparation). The prepared silicone tube was measured for its outer diameter and wall thickness to investigate whether changes in the front drying tunnel temperature affected the size of the silicone tube.

| No. | Front drying tunnel temperature |
| --- | --- |
| Example 1-1 | 270°C |
| Example 1-2 | 300°C |
| Example 1-3 | 330°C |
| Example 1-4 | 360°C |

2.1.4.2 Investigation of rear drying tunnel temperature (second vulcanization temperature)

After the silicone tube was extruded and rapidly vulcanized in the front drying tunnel, it underwent initial curing and molding, followed by low-temperature vulcanization in the 2.5 m long rear drying tunnel. This allowed the silicone tube to be further cured under specific temperature and time conditions and basically molded.

In this experiment, the rear drying tunnel temperatures were set at 120°C, 150°C, 180°C, and 210°C. The mechanical properties of elongation at break, tensile strength, tear strength, and hardness were used to evaluate the rear drying tunnel temperature (For the investigation of rear drying tunnel temperature, the front drying tunnel temperature was set at 270°C, with a very short extrusion time of approximately 5 seconds; the reaction time in the rear drying tunnel was also short, approximately 2 minutes; the oven vulcanization temperature was set at 180°C with a duration of 48 hours; other process conditions followed the details provided in section 2.1.2 preparation process; the preparation followed the details provided in section 2.1.1 preparation). The prepared silicone tube was measured for its outer diameter and wall thickness to investigate whether changes in the rear drying tunnel temperature affected the size of the silicone tube.

| No. | Rear drying tunnel temperature |
|---|---|
| Example 2-1 | 120°C |
| Example 2-2 | 150°C |
| Example 2-3 | 180°C |
| Example 2-4 | 210°C |

2.1.4.3 Investigation of oven vulcanization time (third vulcanization time)

To ensure complete vulcanization of the silicone tube, an oven vulcanization (third vulcanization) treatment was conducted on the silicone tube. Insufficient third vulcanization time would result in incomplete vulcanization of the silicone tube, whereas excessive vulcanization time could lead to over-vulcanization of the silicone tube. In this experiment, the third vulcanization times were set at 0 hours, 24 hours, 48 hours, and 72 hours. The mechanical properties of elongation at break, tensile strength, tear strength, and hardness were used to evaluate the oven vulcanization time (For the investigation of oven vulcanization time, the front drying tunnel temperature was set at 270°C, with an extrusion time of approximately 5 seconds; the rear drying tunnel temperature was set at 180°C, with a reaction time of approximately 20 seconds; the oven vulcanization temperature was set at 180°C; other process conditions followed the details provided in section 2.1.2 preparation process; the preparation followed the details provided in section 2.1.1 preparation).

| No. | Oven vulcanization time |
|---|---|
| Example 3-1 | 0 hours |
| Example 3-2 | 24 hours |
| Example 3-3 | 48 hours |
| Example 3-4 | 72 hours |

2.1.5 Investigation of amount of catalyst

In this experiment, the amounts of raw rubber, hydrogen-containing silicone oil, inhibitor, and reinforcing agent were kept constant at 100 PHR, 1.01 PHR, 0.7 PHR, and 30 PHR, respectively. The amount of catalyst was varied at 0.000005 PHR, 0.00001 PHR, 0.00002 PHR, and 0.00003 PHR. The mechanical properties of the silicone tube, including elongation at break, tensile strength, tear strength, and hardness, were tested under these different amounts of catalyst.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | | | |
|---|---|---|---|---|
| | Example 4-1 | Example 4-2 | Example 4-3 | Example 4-4 |
| Methyl vinyl silicone rubber | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% |
| Fumed white carbon black | 30 PHR | 30 PHR | 30 PHR | 30 PHR |
| Hydrogen-containing silicone oil | 1.01 PHR | 1.01 PHR | 1.01 PHR | 1.01 PHR |
| Molar ratio of Si-H groups in hydrogen-containing silicone oil to vinyl groups in methyl vinyl silicone rubber | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR |
| Platinum catalyst (3000 ppm) | 0.000005 PHR | 0.00001 PHR | 0.00002 PHR | 0.00003 PHR |

2.1.6 Investigation of amount of inhibitor

Under the catalysis of platinum, the addition reaction between raw rubber and hydrogen-containing silicone oil can be carried out at room temperature. As the addition reaction progresses rapidly, the silicone material is quickly cured. To prevent the silicone rubber from curing within the extruder chamber during the extrusion process, the inhibitor methylbutynol can be added to the silicone rubber to halt the reaction at room temperature.

In this experiment, the amounts of raw rubber, hydrogen-containing silicone oil, catalyst, and reinforcing agent were kept constant at 100 PHR, 1 PHR, 0.00001 PHR, and 30 PHR, respectively. The amount of inhibitor was varied at 0.3 PHR, 0.5 PHR, 0.7 PHR, and 0.9 PHR. The mechanical properties of the silicone tube, including elongation at break, tensile strength, tear strength, and hardness, were tested under these different amounts of inhibitor.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | | | |
|---|---|---|---|---|
| | Example 5-1 | Example 5-2 | Example 5-3 | Example 5-4 |
| Methyl vinyl silicone rubber | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% |
| Fumed white carbon black | 30 PHR | 30 PHR | 30 PHR | 30 PHR |
| Hydrogen-containing silicone oil | 1.01 PHR | 1.01 PHR | 1.01 PHR | 1.01 PHR |
| Molar ratio of Si-H groups in hydrogen-containing silicone oil to vinyl groups in methyl vinyl silicone rubber | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% |
| 2-Methyl-3-butyn-2-ol | 0.3 PHR | 0.5 PHR | 0.7 PHR | 0.9 PHR |
| Platinum catalyst (3000 ppm) | 0.00001 PHR | 0.00001 PHR | 0.00001 PHR | 0.00001 PHR |

2.1.7 Investigation of vinyl content in raw rubber

Based on the principle of the addition reaction between methyl vinyl raw rubber and hydrogen-containing silicone oil, it can be seen that the vinyl content in the raw rubber significantly affects the cross-linking degree of the silicone tube. Under the catalysis of platinum, an addition reaction occurs between raw rubber and hydrogen-containing silicone oil.

Vinyl polysiloxane forms the cross-linking links in the resulting network structure, and the length of its molecular chain determines the cross-linking density after curing.

In this experiment, the amounts of vinyl polysiloxane, hydrogen-containing silicone oil, catalyst, inhibitor, and fumed white carbon black were kept constant at 100 PHR, 1 PHR, 0.00001 PHR, 0.7 PHR, and 30 PHR, respectively. The vinyl content of vinyl polysiloxane was varied at 0.05%, 0.07%, 0.17%, and 0.23%. The mechanical properties of the silicone tube, including elongation at break, tensile strength, tear strength, and hardness, were tested under these different vinyl contents.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | | | |
|---|---|---|---|---|
| | Example 6-1 | Example 6-2 | Example 6-3 | Example 6-4 |
| Methyl vinyl silicone rubber | 100 PHR | 100 PHR | 100 PHR | 100 PHR |
| Content of vinyl groups in methyl vinyl silicone rubber | 0.05 mol% | 0.07 mol% | 0.17 mol% | 0.23 mol% |
| Fumed white carbon black | 30 PHR | 30 PHR | 30 PHR | 30 PHR |
| Hydrogen-containing silicone oil | 1.01 PHR | 1.01 PHR | 1.01 PHR | 1.01 PHR |
| Molar ratio of Si-H groups in hydrogen-containing silicone oil to vinyl groups in methyl vinyl silicone rubber | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR |

2.1.8 Investigation of amount of fumed white carbon black

The hardness of the raw rubber is too low, which is not conducive to molding and processing. Adding fumed white carbon black to the raw rubber can reinforce the hardness of the prepared silicone tube and improve the hardness of the raw rubber.

In this experiment, the amounts of raw rubber with a vinyl content of 0.17%, hydrogen-containing silicone oil, catalyst, and inhibitor were kept constant at 100 PHR, 1 PHR, 0.00001 PHR, and 0.7 PHR, respectively. The amount of fumed white carbon black was varied at 20, 30, 40, 45, 50, and 60 PHR. The mechanical properties of the silicone tube, including elongation at break, tensile strength, tear strength, and hardness, were tested under these different amounts of fumed white carbon black.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | | | | | |
|---|---|---|---|---|---|---|
| | Example 7-1 | Example 7-2 | Example 7-3 | Example 7-4 | Example 7-5 | Example 7-6 |
| Methyl vinyl silicone rubber | 100 PHR | 100 PHR | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% | 0.17 mol% | 0.17 mol% |
| Fumed white carbon black | 20 PHR | 30 PHR | 40 PHR | 45 PHR | 50 PHR | 60 PHR |
| Hydrogen-containing silicone oil | 1.01 PHR | 1.01 PHR | 1.01 PHR | 1.01 PHR | 1.01 PHR | 1.01 PHR |

(continued)

| Material name | Amount | | | | | |
|---|---|---|---|---|---|---|
| | Example 7-1 | Example 7-2 | Example 7-3 | Example 7-4 | Example 7-5 | Example 7-6 |
| Molar ratio of Si-H groups in hydrogen-containing silicone oil to vinyl groups in methyl vinyl silicone rubber | 1.2:1 | 1.2:1 | 1.2:1 | 1.2:1 | 1.2:1 | 1.2:1 |
| Content of hydrogen in hydrogen-containing silicone oil | 0.75 mol% | 0.75 mol% | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR | 0.00001 PHR | 0.00001 PHR |

2.1.9 Investigation of hydrogen-containing silicone oil

Hydrogen-containing silicone oil acts as a cross-linking agent and undergoes a cross-linking reaction with the raw rubber. The amount added can be calculated according to the following formula:

$$W_{\text{cross-linking}}/W_1 = (A \times V_i\%) / (H\% \times 27)$$

wherein $W_{\text{cross-linking}}$ is the amount of cross-linking agent added; A is the molar ratio of Si-H to Si-Vi (preferably 1.0-1.5), that is, when the molar ratio of vinyl to hydrogen in the hydrogen-containing silicone oil reaches 1:1, the value of A is 1; Vi% is the weight percentage of vinyl in the base rubber; H% is the weight percentage of hydrogen in the cross-linking agent; $W_1$ is the weight of the base rubber. In practical applications, the optimal hydrogen content and the amount of hydrogen-containing silicone oil added cannot be determined solely by the formula. The optimal hydrogen content and the amount of hydrogen-containing silicone oil added should be obtained through comparative testing.

2.1.9.1 Investigation of hydrogen content in hydrogen-containing silicone oil

In this experiment, the amounts of raw rubber with a vinyl content of 0.17%, catalyst, inhibitor, and fumed white carbon black were kept constant at 100 PHR, 0.00001 PHR, 0.7 PHR, and 40 PHR, respectively. The molar ratio of Si-H groups in the hydrogen-containing silicone oil to vinyl groups in the methyl vinyl silicone rubber was maintained at 1.2:1. Hydrogen-containing silicone oils with hydrogen contents of 0.18%, 0.36%, 0.5%, 0.75%, 1.0%, and 1.6% were used to prepare the silicone tube. The mechanical properties of the silicone tube, including elongation at break, tensile strength, tear strength, and hardness, were tested under these different hydrogen contents.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | | | | | |
|---|---|---|---|---|---|---|
| | Example 8-1 | Example 8-2 | Example 8-3 | Example 8-4 | Example 8-5 | Example 8-6 |
| Methyl vinyl silicone rubber | 100 PHR | 100 PHR | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% | 0.17 mol% | 0.17 mol% |
| Fumed white carbon black | 40 PHR | 40 PHR | 40 PHR | 40 PHR | 40 PHR | 40 PHR |
| Hydrogen-containing silicone oil | 1.01 PHR | 1.01 PHR | 1.01 PHR | 1.01 PHR | 1.01 PHR | 1.01 PHR |

(continued)

| Material name | Amount | | | | | |
|---|---|---|---|---|---|---|
| | Example 8-1 | Example 8-2 | Example 8-3 | Example 8-4 | Example 8-5 | Example 8-6 |
| Molar ratio of Si-H groups in hydrogen-containing silicone oil to vinyl groups in methyl vinyl silicone rubber | 1.2:1 | 1.2:1 | 1.2:1 | 1.2:1 | 1.2:1 | 1.2:1 |
| Content of hydrogen in hydrogen-containing silicone oil | 0.18 mol% | 0.36 mol% | 0.5 mol% | 0.75 mol% | 1.0 mol% | 1.6 mol% |
| 2-Methyl-3-butyn-2-ol | 0.7 PHR | 0.7 PHR | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR | 0.00001 PHR | 0.00001 PHR |

2.1.9.2 Investigation of amount of hydrogen-containing silicone oil

In this experiment, the amounts of raw rubber with a vinyl content of 0.17%, catalyst, inhibitor, and reinforcing agent were kept constant at 100 PHR, 0.00001 PHR, 0.7 PHR, and 40 PHR, respectively. Hydrogen-containing silicone oil with a hydrogen content of 0.75% was used for the experiment, wherein the molar ratios of Si-H groups in the hydrogen-containing silicone oil to vinyl groups in the methyl vinyl silicone rubber were 1:1, 1.2:1, 1.5:1, and 1.8:1. The mechanical properties of the silicone tube, including elongation at break, tensile strength, tear strength, and hardness, were tested under these different amounts of hydrogen-containing silicone oil.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | | | |
|---|---|---|---|---|
| | Example 9-1 | Example 9-2 | Example 9-3 | Example 9-4 |
| Methyl vinyl silicone rubber | 100 PHR | 100 PHR | 100 PHR | 100 PHR |
| Content of vinyl groups in methyl vinyl silicone rubber | 0.17 mol% | 0.17 mol% | 0.17 mol% | 0.17 mol% |
| Fumed white carbon black | 40 PHR | 40 PHR | 40 PHR | 40 PHR |
| Hydrogen-containing silicone oil | 0.84 PHR | 1.01 PHR | 1.26 PHR | 1.51 PHR |
| Molar ratio of Si-H groups in hydrogen-containing silicone oil to vinyl groups in methyl vinyl silicone rubber | 1:1 | 1.2:1 | 1.5:1 | 1.8:1 |
| Content of hydrogen in hydrogen-containing silicone oil | 0.75 mol% | 0.75 mol% | 0.75 mol% | 0.75 mol% |
| 2-Methyl-3-butyn-2-ol | 0.7 PHR | 0.7 PHR | 0.7 PHR | 0.7 PHR |
| Platinum catalyst (3000 ppm) | 0.00001 PHR | 0.00001 PHR | 0.00001 PHR | 0.00001 PHR |

3. Results and discussion

3.1 Optimization of preparation process of silicone tube

3.1.1 Investigation of front drying tunnel temperature

Using the aforementioned preparation, the extrusion speed was fixed at 3.5 rpm, and the stretching speed was set at 0.1 m/s. The front drying tunnel temperature was varied at 270°C, 300°C, 330°C, and 360°C. Three different molds were used for the preparation of silicone tube, and the outer diameter and wall thickness of the silicone tube were measured. The results are as follows.

The effect of the front drying tunnel temperature on the outer diameter and wall thickness of the silicone tube is shown in the table below.

| Front drying tunnel temperature (°C) | Silicone tube outer diameter (mm) | | | Silicone tube wall thickness (mm) | | |
|---|---|---|---|---|---|---|
| | 3# mold | 4# mold | 5# mold | 3# mold | 4# mold | 5# mold |
| 270 | 2.25 | 2.41 | 2.12 | 0.46 | 0.39 | 0.28 |
| 300 | 2.23 | 2.39 | 2.17 | 0.44 | 0.38 | 0.27 |
| 330 | 2.25 | 2.42 | 2.14 | 0.45 | 0.36 | 0.25 |
| 360 | 2.27 | 2.40 | 2.15 | 0.44 | 0.38 | 0.28 |
| Note: Mold size (mandrel diameter/die inner diameter): 3# mold (2.0113.50); 4# mold (2.50/3.90); 5# mold (3.02/4.32). | | | | | | |

The mechanical properties of the silicone tube prepared at different front drying tunnel temperatures were tested, and the results are shown in the table below.

| Mechanical properties | Example 1-1 | Example 1-2 | Example 1-3 | Example 1-4 |
|---|---|---|---|---|
| Elongation at break/Eb (%) | 900.49 | 803.56 | 557.47 | 362.06 |
| Tensile strength/Ts (MPa) | 8.49 | 7.94 | 6.31 | 4.25 |
| Tear strength/T (KN/m) | 44.28 | 37.22 | 33.27 | 22.94 |
| Hardness/H (Shore A) | 58 | 63 | 64 | 66 |

The measurement results of the outer diameter and wall thickness of the silicone tube indicate that the outer diameter and wall thickness of the silicone tube prepared with different molds showed almost no change with increasing front drying tunnel temperature. The finding suggests that the front drying tunnel temperature has no effect on the final outer diameter and wall thickness of the silicone tube. Therefore, in the subsequent preparation of silicone tube, the effect of the front drying tunnel temperature on the size of the silicone tube can be excluded.

The mechanical results show that as the pre-vulcanization temperature increases, the elongation at break, tensile strength, and tear strength of the silicone tube continuously decrease, while the hardness continuously increases. Among them, the mechanical properties of the silicone tubes prepared at 270°C and 300°C both meet the requirements, with the overall mechanical properties of the silicone tube prepared at 270°C being superior. Additionally, it was observed during the experiment that higher front drying tunnel temperatures led to a higher occurrence of bubbles in the prepared silicone tube. Considering all factors, the front drying tunnel temperature is preferably 270°C.

After being extruded by the extruder, the silicone rubber first passes through the front drying tunnel for rapid heating, causing the inhibitor to decompose into gas. The catalyst plays a catalytic role, so that the cross-linking reaction proceeds rapidly, leading to the rapid curing and molding of the outer wall of the silicone tube. If the front drying tunnel temperature is too low, the cross-linking reaction is slow and incomplete, and the silicone tube can not be cured quickly. As a result, the silicone rubber is easily deformed during stretching, making the extrusion process infeasible. If the front drying tunnel temperature is too high, it can cause local over-vulcanization of the silicone tube, making the silicone tube excessively hard and brittle, drastically reducing its mechanical properties and rendering it unusable.

3.1.2 Investigation of rear drying tunnel temperature

Using the aforementioned preparation, the extrusion speed was fixed at 3.5 rpm, and the stretching speed was set at 0.1 m/s. The rear drying tunnel temperature was varied at 120°C, 150°C, 180°C, and 210°C. Three different molds were used for the preparation of silicone tube, and the outer diameter and wall thickness of the silicone tube were measured. The results are as follows.

The effect of the rear drying tunnel temperature on the outer diameter and wall thickness of the silicone tube is shown in the table below.

| Rear drying tunnel temperature(°C) | Silicone tube outer diameter (mm) | | | Silicone tube wall thickness (mm) | | |
|---|---|---|---|---|---|---|
| | 3# mold | 4# mold | 5# mold | 3# mold | 4# mold | 5# mold |
| 120 | 2.25 | 2.41 | 2.12 | 0.46 | 0.39 | 0.28 |
| 150 | 2.23 | 2.39 | 2.17 | 0.44 | 0.38 | 0.27 |
| 180 | 2.25 | 2.42 | 2.14 | 0.45 | 0.37 | 0.25 |
| 210 | 2.27 | 2.40 | 2.15 | 0.44 | 0.38 | 0.28 |

Note: Mold size (mandrel diameter/die inner diameter): 3# mold (2.01/3.50); 4# mold (2.50/3.90); 5# mold (3.02/4.32).

The mechanical properties of the silicone tube prepared at different rear drying tunnel temperatures were tested, and the results are shown in the table below.

| Mechanical properties | Example 2-1 | Example 2-2 | Example 2-3 | Example 2-4 |
|---|---|---|---|---|
| Elongation at break/Eb (%) | 315.05 | 561.16 | 900.49 | 937.83 |
| Tensile strength/Ts (MPa) | 3.39 | 5.11 | 8.49 | 8.17 |
| Tear strength/T (KN/m) | 12.25 | 27.46 | 44.28 | 45.12 |
| Hardness/H (Shore A) | 48 | 54 | 59 | 58 |

The measurement results of the outer diameter and wall thickness of the silicone tube indicate that the outer diameter and wall thickness of the silicone tube prepared with different molds showed almost no change with increasing rear drying tunnel temperature. It can be concluded that the rear drying tunnel temperature has no effect on the final outer diameter and wall thickness of the silicone tube. Therefore, it can be judged that the temperature change during the preparation process has no effect on the molding size of the silicone tube.

The mechanical results show that as the rear drying tunnel temperature increases, the elongation at break, tensile strength, tear strength, and hardness of the silicone tube all increase. When the rear drying tunnel temperature reaches 180°C, the mechanical properties are similar to those at 210°C. This indicates that a rear drying tunnel temperature of 180°C is sufficient to meet the mechanical performance requirements. Additionally, considering the presence of a conveyor belt in the rear drying tunnel, it is not suitable to set an excessively high temperature. Therefore, the rear drying tunnel temperature is preferably 180°C.

The primary function of the rear drying tunnel is to provide conditions for the subsequent addition reaction of the silicone rubber, allowing further vulcanization to occur. Therefore, an excessive temperature in the rear drying tunnel can lead to over-vulcanization, whereas an appropriate temperature can make the cross-linking reaction complete and the mechanical properties better.

3.1.3 Investigation of oven vulcanization time

The oven vulcanization temperature was set at 180°C. The silicone tube was subjected to oven vulcanization for 0 hours, 24 hours, 48 hours, and 72 hours, respectively, and the mechanical properties were measured.

| Mechanical properties | Example 3-1 | Example 3-2 | Example 3-3 | Example 3-4 |
|---|---|---|---|---|
| Elongation at break/Eb (%) | 900.49 | 608.81 | 565.13 | 603.54 |
| Tensile strength/Ts (MPa) | 8.49 | 8.58 | 9.79 | 8.76 |
| Tear strength/T (KN/m) | 44.28 | 46.57 | 54.77 | 43.48 |
| Hardness/H (Shore A) | 58 | 61 | 62 | 62 |

The results indicate that after oven vulcanization, the elongation at break of the silicone tube decreased, but still met the requirements. Both tensile strength and tear strength initially increased and then decreased, while the hardness gradually increased. The silicone tube subjected to oven vulcanization for 48 hours exhibited the best tensile strength and tear strength, with elongation at break and hardness also meeting the standards. Therefore, the preferred condition for oven vulcanization is 180°C for 48 hours.

The mechanical properties of the silicone tube after oven vulcanization were improved compared to the unvulcanized silicone tube, proving that the cross-linking reaction was incomplete when the silicone tube was extruded from the rear drying tunnel. Although the silicone tube was essentially cured after exiting the rear drying tunnel, the internal cross-linking reaction was incomplete. The silicone tube needs to be subjected to oven vulcanization in order to ensure complete vulcanization. Therefore, oven vulcanization is the final step to ensure the complete vulcanization of the silicone tube. However, as the oven vulcanization time continues to increase, the silicone tube may experience over-vulcanization, leading to reduced toughness and increased brittleness. Therefore, the oven vulcanization time must be appropriate, neither too short nor too long.

3.1.4 Investigation of amount of catalyst

Silicone tubes were prepared by varying the amount of catalyst to 0.000005, 0.00001, 0.00002, and 0.00003 PHR. The results of mechanical properties are as follows.

| Mechanical properties | Example 4-1 | Example 4-2 | Example 4-3 | Example 4-4 |
|---|---|---|---|---|
| Elongation at break/Eb (%) | 766.80 | 1045.50 | 1035.34 | 1018.65 |
| Tensile strength/Ts (MPa) | 6.74 | 8.50 | 8.58 | 8.09 |
| Tear strength/T (KN/m) | 37.31 | 45.65 | 45.87 | 43.01 |
| Hardness/H (Shore A) | 43 | 46 | 45 | 46 |

It can be seen from the results that when the amount of catalyst increased from 0.000005 PHR to 0.00001 PHR, all the mechanical properties increased. However, as the amount of catalyst continued to increase, there was no significant difference in the mechanical properties. Therefore, the amount of catalyst is preferably 0.00001 PHR.

When the amount of catalyst is too low, the catalyst is unevenly dispersed in the rubber, resulting in some areas lacking sufficient catalyst to initiate the cross-linking reaction, leading to poor mechanical properties of the prepared silicone tube. When the amount of catalyst is excessive, there is no significant improvement in the mechanical properties. Excessive catalyst cannot enhance the mechanical performance of the silicone tube. Additionally, since platinum is a precious metal, it is important to avoid waste during usage.

3.1.5 Investigation of amount of inhibitor

Silicone tubes were prepared by fixing the preparation composition and amount, fixing the process, and varying the amount of inhibitor to 0.3 PHR, 0.5 PHR, 0.7 PHR, and 0.9 PHR. The results of mechanical properties are as follows.

| Mechanical properties | Example 5-1 | Example 5-2 | Example 5-3 | Example 5-4 |
|---|---|---|---|---|
| Elongation at break/Eb (%) | 588.11 | 868.23 | 1045.50 | 918.83 |
| Tensile strength/Ts (MPa) | 6.68 | 7.22 | 8.50 | 8.07 |
| Tear strength/T (KN/m) | 37.22 | 40.82 | 45.65 | 43.44 |
| Hardness/H (Shore A) | 51 | 48 | 46 | 46 |

It can be seen from the results that as the amount of inhibitor increased, the elongation at break, tensile strength, and tear strength initially increased and then decreased. The optimal elongation at break, tensile strength, and tear strength were observed at an amount of inhibitor of 0.7 PHR. Therefore, the amount of inhibitor is preferably 0.7 PHR.

When the amount of inhibitor is too low, partial vulcanization can occur at room temperature. If the silicone tube undergoes additional vulcanization, it may result in over-vulcanization, leading to too low mechanical properties. Therefore, as the amount of inhibitor increases, the elongation at break, tensile strength, and tear strength of the silicone tube all increase. When the amount of inhibitor is increased to 0.9 PHR, the mechanical properties are equivalent to those of the silicone tube at 0.7 PHR, indicating that 0.7 PHR is the optimal amount for effective inhibition while increasing the amount to 0.9 PHR results in excessive inhibitor.

3.1.6 Investigation of vinyl content in raw rubber

Silicone tubes were prepared by fixing the preparation composition and amount, fixing the process, and varying the vinyl content of the raw rubber to 0.05%, 0.07%, 0.17%, and 0.23%. The results of mechanical properties are as follows.

| Mechanical properties | Example 6-1 | Example 6-2 | Example 6-3 | Example 6-4 |
|---|---|---|---|---|
| Elongation at break/Eb (%) | 640.38 | 757.47 | 1045.50 | 559.67 |
| Tensile strength/Ts (MPa) | 1.10 | 2.20 | 8.50 | 7.41 |
| Tear strength/T (KN/m) | 6.27 | 10.36 | 45.65 | 38.64 |
| Hardness/H (Shore A) | 40 | 41 | 46 | 59 |

The results indicate that as the vinyl content of the raw rubber increased, the elongation at break, tensile strength, and tear strength of the extruded silicone tube initially increased and then decreased. The peak values were observed when the vinyl content of the raw rubber was 0.17%. Hardness increased with the increase of vinyl content. Therefore, the vinyl content of the raw rubber is preferably 0.17%.

The vinyl content of 0.05% and 0.07% is too low, resulting in too low cross-linking density. As the vinyl content increases, the cross-linking density continues to increase, improving the mechanical properties. However, when the vinyl content increases to 0.23%, the cross-linking density becomes too high, resulting in increased hardness but reduced toughness, which leads to lower elongation at break, tensile strength, and tear strength. The silicone tube with excessively high vinyl content becomes harder and more brittle, making it prone to tearing. Although the hardness of the silicone tube with 0.23% vinyl content meets the requirements, its elongation at break, tensile strength, and tear strength are inferior to those of the silicone tube with 0.17% vinyl content.

3.1.7 Investigation of amount of fumed white carbon black

Silicone tubes were prepared by fixing the preparation composition and amount, fixing the process, and varying the amount of fumed white carbon black to 20 PHR, 30 PHR, 40 PHR, 45 PHR, and 50 PHR. When the amount of white carbon black was 20 PHR (Example 7-1), the silicone tube was too soft, so this dose was discarded. The mechanical properties for the remaining samples are as follows.

| Mechanical properties | Example 7-2 | Example 7-3 | Example 7-4 | Example 7-5 | Example 7-6 |
|---|---|---|---|---|---|
| Amount of fumed white carbon black | 30 PHR | 40 PHR | 45 PHR | 50 PHR | 60 PHR |
| Elongation at break/Eb (%) | 1045.50 | 900.49 | 874.92 | 677.36 | 582.27 |
| Tensile strength/Ts (MPa) | 8.50 | 8.49 | 8.49 | 9.44 | 8.76 |
| Tear strength/T (KN/m) | 45.65 | 45.02 | 42.40 | 43.08 | 41.26 |
| Hardness/H (Shore A) | 46 | 58 | 69 | 80 | 80 |

The results indicate that as the amount of fumed white carbon black increased, the hardness of the extruded silicone tube significantly increased, the tensile strength and tear strength showed no significant changes, and the elongation at break showed a decreasing trend, but all values were within the qualified range. According to the requirements, the hardness of the silicone tube should be between 50 and 70, and the amount of fumed white carbon black is preferably 40 PHR.

When the amount of fumed white carbon black is insufficient, the hardness of the silicone tube is too low, resulting in poor moldability during extrusion and a rough surface. The greater the amount of fumed white carbon black added, the higher the hardness reinforcement of the silicone tube, and the stronger the ability of the silicone tube to resist extrusion by external forces. The purpose of adding fumed white carbon black is to reinforce the hardness of the silicone material. Therefore, when other mechanical properties are relatively good, the focus is primarily on the hardness data. When the amount of fumed white carbon black is 40 PHR, the hardness meets the standard, and the elongation at break, tensile strength, and tear strength are all favorable. However, if too much fumed white carbon black is added, the hardness of the silicone material becomes excessively high, leading to insufficient power of the extruder during extrusion, causing unstable discharge speed. Moreover, excessive hardness can also shift the position of the extrusion mold, resulting in uneven wall thickness of the extruded silicone tube.

3.1.8 Investigation of hydrogen-containing silicone oil

3.1.8.1 Investigation of hydrogen content in hydrogen-containing silicone oil

Silicone tubes were prepared by fixing the preparation composition and amount, fixing the process, fixing the molar ratio of silicon-hydrogen (Si-H) groups in the hydrogen-containing silicone oil to vinyl groups in the base polymer at 1.2, and varying the hydrogen content of the hydrogen-containing silicone oil to 0.18%, 0.36%, 0.5%, 0.75%, 1%, and 1.6%. The results of mechanical properties are as follows.

| Mechanical properties | Example 8-1 | Example 8-2 | Example 8-3 | Example 8-4 | Example 8-5 | Example 8-6 |
|---|---|---|---|---|---|---|
| Content of hydrogen in hydrogen-containing silicone oil | 0.18 mol% | 0.36 mol% | 0.5 mol% | 0.75 mol% | 1.0 mol% | 1.6 mol% |
| Elongation at break/ Eb (%) | 293.09 | 343.43 | 549.78 | 899.72 | 900.49 | 922.72 |
| Tensile strength/ Ts (MPa) | 5.38 | 6.67 | 7.50 | 8.49 | 8.31 | 6.65 |
| Tear strength/T (KN/m) | 10.97 | 13.61 | 15.31 | 48.64 | 34.34 | 36.34 |
| Hardness/H (Shore A) | 54 | 57 | 57 | 58 | 59 | 61 |

The results indicate that the hydrogen-containing silicone oil significantly affects the mechanical properties of the silicone tube. As the hydrogen content of the hydrogen-containing silicone oil increased, the hardness of the prepared silicone tube increased continuously, while the elongation at break, tensile strength, and tear strength initially increased and then decreased. The peak values were observed at a hydrogen content of 0.75%. Therefore, the hydrogen content of the hydrogen-containing silicone oil is preferably 0.75%.

When the molar ratio of Si-H groups in the hydrogen-containing silicone oil to vinyl groups in the methyl vinyl silicone rubber is fixed, the lower the hydrogen content, the more the amount of the hydrogen-containing silicone oil will be. Since hydrogen-containing silicone oil is a transparent oily substance, the more the amount added, the more viscous the material will be, resulting in lower strength of the prepared silicone tube. As the hydrogen content of the hydrogen-containing silicone oil increases, the amount of the hydrogen-containing silicone oil decreases, leading to more concentrated Si-H groups. As a result, the local cross-linking reaction occurs rapidly, and the cross-linking density continues to increase. When the silicone rubber is stretched, the flexibility of the cross-linking chains is reduced, preventing effective orderly alignment. Only a small portion of the cross-linking chains can bear the external force, thereby reducing the elasticity of the silicone tube, along with the tensile strength and tear strength.

3.1.8.2 Investigation of amount of hydrogen-containing silicone oil

Silicone tubes were prepared by fixing the preparation composition and amount, fixing the process, fixing the hydrogen content of the hydrogen-containing silicone oil at 0.75%, and varying the molar ratio of Si-H groups to vinyl groups in the base polymer to 1:1, 1.2:1, 1.5:1, and 1.8:1. The results of mechanical properties are as follows.

| Mechanical properties | Example 9-1 | Example 9-2 | Example 9-3 | Example 9-4 |
|---|---|---|---|---|
| Elongation at break/Eb (%) | 630.54 | 900.49 | 835.05 | 676.59 |
| Tensile strength/Ts (MPa) | 7.71 | 8.49 | 6.56 | 6.04 |
| Tear strength/T (KN/m) | 38.81 | 44.28 | 32.36 | 22.07 |
| Hardness/H (Shore A) | 55 | 57 | 61 | 58 |

It can be seen from the results that when the hydrogen content of the hydrogen-containing silicone oil was fixed, the hardness, elongation at break, tensile strength, and tear strength initially increased and then decreased with the increase in the amount of hydrogen-containing silicone oil. The peak values were observed at a molar ratio of 1.2:1. Therefore, the molar ratio of Si-H groups in the hydrogen-containing silicone oil to vinyl groups in the methyl vinyl silicone rubber is preferably 1.2:1.

Increasing the amount of hydrogen-containing silicone oil makes the addition reaction more complete, resulting in more cross-linking points and increased tensile strength. However, when the amount of hydrogen-containing silicone oil is too high, the cross-linking density becomes excessive, reducing toughness and making the resulting silicone tube hard and brittle. When the molar ratio increases to 1.8:1, there is an excess of hydrogen-containing silicone oil. The excess hydrogen-containing silicone oil prevents some of the cross-linking reactions from proceeding, reducing the overall degree of cross-linking, which is reflected in the decreased mechanical properties observed.

3.2 Preparation of optimal silicone tube

The preparation is shown in the table below.

| Material name | Amount |
|---|---|
| Methyl vinyl silicone rubber | 100 PHR |
| Content of vinyl groups in vinyl polysiloxane | 0.17% |
| Fumed white carbon black | 40 PHR |
| Hydrogen-containing silicone oil | 1.01 PHR |
| Molar ratio of Si-H groups in hydrogen-containing silicone oil to vinyl groups in methyl vinyl silicone rubber | 1.2:1 |
| Content of hydrogen in hydrogen-containing silicone oil | 0.75% |
| 2-Methyl-3-butyn-2-ol | 0.7 PHR |
| Platinum catalyst (3000 ppm) | 0.00001 PHR |

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".
The composition of the medicinal core is as follows: the medicinal core is 42 mg of gestodene bulk drug powder with a particle size of 2.81 $\mu$m after micronization.
The mechanical properties of the silicone tube are shown in the table below.

| No. | Eb (%) | Ts (MPa) | T (KN/m) | H (Shore A) |
|---|---|---|---|---|
| GJ001 | 595.78 | 9.81 | 54.88 | 61 |
| GJ002 | 519.94 | 9.46 | 53.05 | 62 |
| GJ003 | 579.80 | 10.08 | 56.38 | 62 |
| Note: Eb - elongation at break; Ts - tensile strength; T - tear strength; H - hardness. | | | | |

Three batches of silicone tubes were prepared in parallel according to the optimal preparation and process. The elongation at break and hardness of all batches met the standards, and the tensile strength and tear strength were significantly higher than the standards. The mechanical properties of the three batches were similar, indicating good reproducibility of the preparation and process.

**(III) Preparation and *in vitro* release study of gestodene implants**

Silicone tube, due to its ability to provide long-term stable control of drug release, is used as a carrier in implants. After evaluating the process of the silicone tube based on mechanical properties, it is necessary to investigate its drug release control capabilities.

1. Instruments and reagents

1.1 Instruments

TS-100B Thermostatic Shaker, Shanghai Jiecheng Experimental Instrument Co., Ltd.
High Performance Liquid Chromatograph, Shimadzu Corporation, Japan
KQ-250DE CNC Ultrasonic Cleaner, Kunshan Ultrasonic Instrument Co., Ltd.
AG245 Ultra-micro Electronic Analytical Balance, Mettler Toledo, Switzerland

1.2 Reagents

Gestodene (Purity of 98%, Batch No.: 20190327), Hebei Mokai Technology Development Co., Ltd.
KN-300N silicone adhesive, Kanglibang Polymer New Materials Co., Ltd.

2. Experimental methods

2.1 Preparation of gestodene implants

Silicone tubes were prepared according to the preparations and processes described in the following examples. The silicone tube was used as a drug release carrier to control drug release. The homemade silicone tube was disinfected by soaking in 75% ethanol for 30 minutes and then dried by blowing. One end of the silicone tube was sealed with silicone sealant for later use. The drug (42 mg of gestodene bulk drug powder with a particle size of 2.81 $\mu$m after micronization) was loaded into the tube using a filling funnel. The other end was then sealed with silicone sealant and cured for 24 hours to prepare a gestodene implant. The gestodene implant had a silicone tube wall thickness of 0.3 mm and a drug release area of 94.2 mm$^2$.

2.2 Selection of release medium

The prepared implant needs to be implanted in the subcutaneous tissue of the human upper arm, where the pH is close to neutral. Therefore, the release medium can be either water or normal saline. A supersaturated solution of the gestodene bulk drug in water and normal saline, both before and after micronization, was prepared. The solution was placed in a shaker and shaken for 72 hours. After shaking, the mixture was taken out and centrifuged, and the supernatant was filtered. The equilibrium solubility of the gestodene bulk drug in water and normal saline, both before and after micronization, was determined using high performance liquid chromatography. Based on the results, the appropriate release medium was selected.

2.3 *In vitro* release and investigation of influencing factors

2.3.1 Investigation of oven-vulcanized silicone tube

In the experiment of preparing silicone tubes, the silicone tube that underwent 48 hours of oven vulcanization exhibited better mechanical properties, indicating that the oven-vulcanized silicone tube was fully vulcanized. In order to further determine whether the oven-vulcanized silicone tube has an effect on the *in vitro* drug release, both unvulcanized and oven-vulcanized silicone tubes (corresponding to the silicone tubes in Example 3-1 and Example 3-3) were filled with implants with the same specification, the same drug-loaded segment length, and the same drug loading for *in vitro* release experiments to examine the effect of oven vulcanization on the *in vitro* drug release data.

2.3.2 Investigation of silicone tube prepared with different vinyl contents

In the addition reaction of silicone tube, the cross-linking degree of the silicone tube is greatly influenced not only by the amount of hydrogen-containing silicone oil added but also by the vinyl content of the raw rubber. To investigate the effect of vinyl content on *in vitro* drug release, silicone tubes were prepared using raw rubber with a vinyl content of 0.17% and 0.23%. The effect of silicone tubes prepared with different vinyl contents on the *in vitro* release of gestodene was examined.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | |
|---|---|---|
| | Example 10-1 | Example 10-2 |
| Methyl vinyl silicone rubber | 100 PHR | 100 PHR |
| Content of vinyl groups in methyl vinyl silicone rubber | 0.17 mol% | 0.23 mol% |
| Fumed white carbon black | 40 PHR | 40 PHR |
| Hydrogen-containing silicone oil | 1.01 PHR | 1.01 PHR |
| Molar ratio of Si-H groups in hydrogen-containing silicone oil to vinyl groups in methyl vinyl silicone rubber | 1.2:1 | 1.2:1 |
| Content of hydrogen in hydrogen-containing silicone oil | 0.75 mol% | 0.75 mol% |
| 2-Methyl-3-butyn-2-ol | 0.7 PHR | 0.7 PHR |
| Platinum catalyst (3000 ppm) | 0.00001 PHR | 0.00001 PHR |

2.3.3 Investigation of silicone tubes prepared with different molar ratios of Si-H groups in hydrogen-containing silicone oil to vinyl groups in methyl vinyl silicone rubber

The implant was placed in a release medium, and the release medium entered the inside of the silicone tube through the pores of the silicone tube, dissolving the drug and allowing its release driven by the concentration gradient. Silicone tubes were prepared using raw rubber with a vinyl content of 0.17% and hydrogen-containing silicone oil with a hydrogen content of 0.75%, with molar ratios of 1:1.2 and 1:1.5, respectively. All other factors were the same. The effect of the amount of hydrogen-containing silicone oil on the daily drug release amount of the implant was examined.

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

| Material name | Amount | |
|---|---|---|
| | Example 11-1 | Example 11-2 |
| Methyl vinyl silicone rubber | 100 PHR | 100 PHR |
| Content of vinyl groups in methyl vinyl silicone rubber | 0.17 mol% | 0.17 mol% |
| Fumed white carbon black | 40 PHR | 40 PHR |
| Hydrogen-containing silicone oil | 1.01 PHR | 1.26 PHR |
| Molar ratio of Si-H groups in hydrogen-containing silicone oil to vinyl groups in methyl vinyl silicone rubber | 1.2:1 | 1.5:1 |
| Content of hydrogen in hydrogen-containing silicone oil | 0.75 mol% | 0.75 mol% |
| 2-Methyl-3-butyn-2-ol | 0.7 PHR | 0.7 PHR |
| Platinum catalyst (3000 ppm) | 0.00001 PHR | 0.00001 PHR |

3. Results and discussion

3.1 Preparation of gestodene contraceptive implants

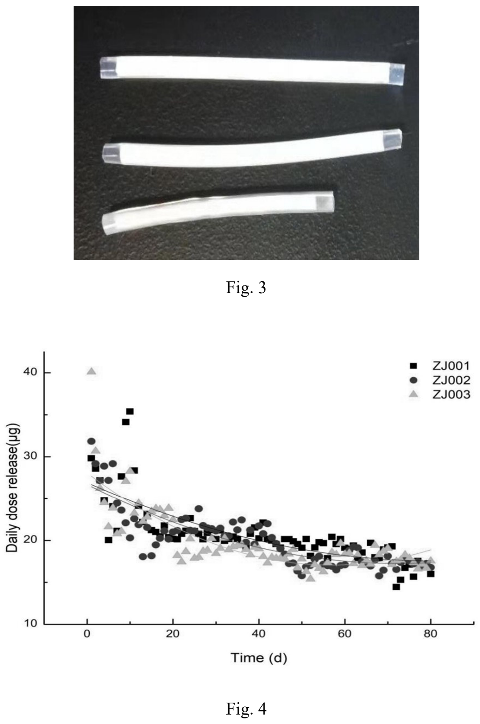

When preparing the implant, special attention should be paid to the static electricity of the bulk drug powder to avoid adhesion to the outer wall of the silicone tube. Additionally, when sealing the implant, care should be taken to prevent irregular shapes of the sealant, which could affect the drug release area of the drug-loaded segment. The implants of various specifications were finally prepared, exhibiting good appearance with uniform sealing and evenly filled drug segments. The GEST (gestodene) contraceptive implants are shown in Fig. 3.

3.2 Selection of *in vitro* release medium

The equilibrium solubility results of GEST in different solvents are shown in the table below.

| Drug particle size ($\mu$m) | Solvent | Solubility ($\mu$g/mL) |
|---|---|---|
| 2.81 | water | 4.11 $\pm$ 0.52 |
| | Normal saline (NS) | 2.71 $\pm$ 0.24 |
| 80.30 | water | 5.64 $\pm$ 0.62 |
| | Normal saline (NS) | 5.30 $\pm$ 0.58 |

The results indicate that the solubility of gestodene bulk drug with both particle sizes in water was greater than that in normal saline. Additionally, the solubility of the unmicronized bulk drug was greater than that of the micronized bulk drug. Therefore, water was selected as the release medium for the *in vitro* release experiments.

Theoretically, the smaller the particle size of the bulk drug, the larger the contact area with the solvent, the higher the solubility should be. However, the experimental data showed that the solubility of the micronized bulk drug was instead lower. During the experiment, it was observed that the micronized bulk drug had significant static electricity, causing agglomeration. This likely resulted in a smaller contact area with the medium compared to the bulk drug with a large particle size, thus reducing the solubility.

3.3 Investigation of factors influencing *in vitro* release

3.3.1 Investigation of oven-vulcanized silicone tube

The effect of the oven vulcanization process on *in vitro* release is shown in the table below.

| Example No. | Oven vulcanization process | Average release rate ($\mu$g/d) | RSD (%) |
|---|---|---|---|
| Example 3-3 | Oven vulcanization for 48 hours | 10.57 $\pm$ 0.76 | 7.19 |
| Example 3-1 | No oven vulcanization | 12.46 $\pm$ 0.92 | 7.38 |

The effect of the oven vulcanization process on *in vitro* daily dose release is shown in the table below.

| Time (d) | Daily dose release ($\mu$g) | |
|---|---|---|
| | Oven vulcanization for 48 hours | No oven vulcanization |
| 1 | 13.91 | 14.54 |
| 2 | 13.28 | 14.53 |
| 3 | 10.03 | 14.21 |
| 4 | 8.53 | 11.21 |
| 5 | 8.42 | 12.11 |
| 6 | 13.07 | 13.94 |
| 7 | 10.23 | 11.11 |
| 8 | 10.84 | 12.49 |

(continued)

| Time (d) | Daily dose release (μg) | |
| --- | --- | --- |
| | Oven vulcanization for 48 hours | No oven vulcanization |
| 9 | 10.43 | 13.30 |
| 10 | 11.06 | 13.02 |
| 11 | 10.93 | 12.93 |
| 12 | 11.43 | 12.47 |
| 13 | 11.11 | 12.10 |
| 14 | 10.49 | 12.02 |
| 15 | 11.10 | 12.79 |
| 16 | 11.81 | 14.25 |
| 17 | 9.61 | 13.96 |
| 18 | 9.89 | 13.05 |
| 19 | 9.70 | 12.11 |
| 20 | 11.32 | 12.07 |
| 21 | 9.82 | 13.96 |
| 22 | 9.53 | 12.30 |
| 23 | 9.29 | 11.90 |
| 24 | 10.32 | 12.19 |
| 25 | 10.05 | 12.43 |
| 26 | 10.53 | 13.22 |
| 27 | 11.31 | 10.95 |
| 28 | 11.54 | 12.04 |
| 29 | 10.89 | 10.68 |
| 30 | 10.69 | 11.74 |

The results indicate that the implants filled with oven-vulcanized silicone tubes had a slightly lower average daily drug release amount compared to those filled with unvulcanized silicone tubes. However, the difference was minimal. The implants prepared from oven-vulcanized silicone tubes exhibited more stable *in vitro* release profiles compared to those prepared from unvulcanized silicone tubes. Additionally, considering that the oven-vulcanized silicone tubes demonstrated better mechanical properties, and taking the release data and mechanical properties into consideration, it is preferable to use silicone tubes that have been oven-vulcanized at 180°C for 48 hours for drug filling.

The specific process conditions were as follows: the front vulcanization temperature was set at 270°C, with a vulcanization time of approximately 5 seconds; the rear vulcanization temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

3.3.2 Investigation of silicone tubes prepared with different vinyl contents

The effect of vinyl content on *in vitro* release is shown in the table below.

| Silicone tube preparation | Vinyl content (%) | Average release rate (μg/d) | RSD (%) |
| --- | --- | --- | --- |
| Example 10-1 | 0.17 | 10.57 ± 0.76 | 6.89 |
| Example 10-2 | 0.23 | 15.33 ± 1.38 | 10.23 |

The effect of vinyl content on *in vitro* daily dose release is shown in the table below.

| Time (d) | Daily dose release ($\mu$g) | |
|---|---|---|
| | Example 10-1 | Example 10-2 |
| 1 | 13.91 | 32.38 |
| 2 | 13.28 | 31.03 |
| 3 | 10.03 | 23.60 |
| 4 | 8.53 | 18.21 |
| 5 | 8.42 | 17.67 |
| 6 | 13.07 | 17.67 |
| 7 | 10.23 | 16.71 |
| 8 | 10.84 | 17.07 |
| 9 | 10.43 | 16.91 |
| 10 | 11.06 | 16.50 |
| 11 | 10.93 | 15.76 |
| 12 | 11.43 | 15.72 |
| 13 | 11.11 | 14.80 |
| 14 | 10.49 | 13.67 |
| 15 | 11.10 | 12.86 |
| 16 | 11.81 | 17.07 |
| 17 | 9.61 | 16.91 |
| 18 | 9.89 | 16.50 |
| 19 | 9.70 | 15.76 |
| 20 | 11.32 | 15.72 |
| 21 | 9.82 | 14.80 |
| 22 | 9.53 | 13.67 |
| 23 | 9.29 | 12.86 |
| 24 | 10.32 | 17.07 |
| 25 | 10.05 | 16.91 |
| 26 | 10.53 | 16.50 |
| 27 | 11.31 | 15.76 |
| 28 | 11.54 | 15.72 |
| 29 | 10.89 | 14.80 |
| 30 | 10.69 | 13.67 |

The results indicate that silicone tubes prepared from raw rubber with different vinyl contents (0.17% and 0.23%) significantly affect the *in vitro* average drug release of the implant. The initial burst release of raw rubber with 0.23% vinyl content is greater than that of raw rubber with 0.17% vinyl content. The silicone tubes prepared from raw rubber with 0.17% vinyl content exhibit more stable *in vitro* release. Additionally, considering the comprehensive mechanical properties, the performance of silicone tubes with 0.17% vinyl content is superior.

3.3.3 Investigation of silicone tubes with different amounts of hydrogen-containing silicone oil

The effect of the amount of hydrogen-containing silicone oil on *in vitro* release is shown in the table below.

| Silicone tube preparation | Molar ratio | Average release rate (µg/d) | RSD (%) |
|---|---|---|---|
| Example 11-2 | 1.5:1 | 10.57 ± 0.76 | 7.16 |
| Example 11-1 | 1.2:1 | 8.27 ± 0.53 | 6.37 |
| Note: The molar ratio refers to the molar ratio of Si-H groups in the hydrogen-containing silicone oil to vinyl groups in the methyl vinyl silicone rubber. | | | |

The effect of the amount of hydrogen-containing silicone oil on *in vitro* daily dose release is shown in the table below.

| Time (d) | Daily dose release (µg) | |
|---|---|---|
| | Example 11-2 | Example 11-1 |
| 1 | 13.91 | 13.23 |
| 2 | 13.28 | 12.12 |
| 3 | 10.03 | 13.12 |
| 4 | 8.53 | 13.96 |
| 5 | 8.42 | 10.19 |
| 6 | 13.07 | 17.71 |
| 7 | 10.23 | 8.56 |
| 8 | 10.84 | 7.79 |
| 9 | 10.43 | 8.98 |
| 10 | 11.06 | 8.95 |
| 11 | 10.93 | 7.36 |
| 12 | 11.43 | 8.28 |
| 13 | 11.11 | 8.50 |
| 14 | 10.49 | 8.03 |
| 15 | 11.10 | 8.39 |
| 16 | 11.81 | 8.56 |
| 17 | 9.61 | 7.79 |
| 18 | 9.89 | 8.98 |
| 19 | 9.70 | 8.95 |
| 20 | 11.32 | 7.36 |
| 21 | 9.82 | 8.28 |
| 22 | 9.53 | 8.50 |
| 23 | 9.29 | 8.03 |
| 24 | 10.32 | 8.39 |
| 25 | 10.05 | 8.56 |
| 26 | 10.53 | 7.79 |
| 27 | 11.31 | 8.98 |
| 28 | 11.54 | 8.95 |
| 29 | 10.89 | 7.36 |
| 30 | 10.69 | 8.28 |

The results indicate that the contraceptive implants filled with silicone tubes prepared from raw rubber with a vinyl content of 0.17% and hydrogen-containing silicone oil with a hydrogen content of 0.75%, using a preparation with a molar ratio of 1:1.2 between vinyl groups in the methyl vinyl silicone rubber and Si-H groups in the hydrogen-containing silicone oil, have a slightly lower daily drug release than those filled with silicone tubes prepared using a preparation with a molar ratio of 1:1.5 between vinyl groups in the methyl vinyl silicone rubber and Si-H groups in the hydrogen-containing silicone oil. However, the drug release curve is relatively more stable. Since the implants must first achieve stable release, it can be concluded that the molar ratio of 1:1.2 between vinyl groups in the methyl vinyl silicone rubber and Si-H groups in the hydrogen-containing silicone oil provides better controlled release of the drug.

3.4 Long-term *in vitro* release experiment of gestodene implants

Three batches of gestodene contraceptive implants (with a silicone tube wall thickness of 0.3 mm and a drug release area of 94.2 mm$^2$; the gestodene bulk drug was micronized to a particle size of 2-4 $\mu$m, e.g., 2.81 $\mu$m, with 42 mg of micronized gestodene bulk drug filled through a filling funnel) labeled as ZJ001, ZJ002, and ZJ003, were subjected to long-term *in vitro* release experiments at a temperature of 37°C and a shaking speed of 100 rpm. The purpose was to investigate the long-term release stability of the preparation and its capability to achieve long-term controlled release. The results are as follows.

| Material name | Amount | | |
|---|---|---|---|
| | ZJ001 | ZJ002 | ZJ003 |
| Methyl vinyl silicone rubber | 100 PHR | 100 PHR | 100 PHR |
| Content of vinyl groups in methyl vinyl silicone rubber | 0.17 mol% | 0.17 mol% | 0.17 mol% |
| Fumed white carbon black | 40 PHR | 40 PHR | 40 PHR |
| Molar ratio of Si-H groups in hydrogen-containing silicone oil to vinyl groups in methyl vinyl silicone rubber | 1.2:1 | 1.2:1 | 1.2:1 |
| Content of hydrogen in hydrogen-containing silicone oil | 0.75 mol% | 0.75 mol% | 0.75 mol% |
| 2-Methyl-3-butyn-2-ol | 0.7 PHR | 0.7 PHR | 0.7 PHR |
| Platinum catalyst (3000 ppm) | 0.00001 PHR | 0.00001 PHR | 0.00001 PHR |

The long-term release data for implants ZJ001, ZJ002, and ZJ003 are shown in the table below.

| Time (d) | Daily dose release ($\mu$g) | | |
|---|---|---|---|
| | ZJ001 | ZJ002 | ZJ003 |
| 1 | 28.64 | 31.85 | 40.11 |
| 2 | 24.22 | 29.17 | 30.68 |
| 3 | 27.72 | 27.15 | 26.25 |
| 4 | 23.65 | 28.89 | 24.51 |
| 5 | 18.48 | 27.19 | 21.63 |
| 6 | 18.84 | 29.17 | 23.86 |
| 7 | 19.37 | 24.44 | 20.79 |
| 8 | 18.97 | 23.60 | 21.08 |
| 9 | 17.83 | 22.09 | 27.09 |
| 10 | 17.93 | 20.30 | 28.28 |
| 11 | 16.35 | 22.59 | 23.25 |
| 12 | 18.66 | 21.87 | 24.43 |
| 13 | 18.60 | 18.05 | 22.26 |

(continued)

| Time (d) | Daily dose release ($\mu$g) | | |
|---|---|---|---|
| | ZJ001 | ZJ002 | ZJ003 |
| 14 | 18.91 | 21.55 | 22.78 |
| 15 | 16.97 | 18.16 | 23.46 |
| 16 | 16.56 | 19.46 | 23.79 |
| 17 | 20.47 | 20.24 | 23.67 |
| 18 | 21.78 | 21.12 | 22.94 |
| 19 | 20.38 | 20.16 | 23.84 |
| 20 | 20.19 | 21.00 | 22.46 |
| 21 | 20.12 | 21.18 | 18.22 |
| 22 | 20.78 | 22.48 | 17.45 |
| 23 | 21.16 | 22.64 | 18.66 |
| 24 | 22.65 | 21.19 | 20.18 |
| 25 | 21.12 | 21.12 | 17.88 |
| 26 | 20.78 | 23.78 | 17.99 |
| 27 | 20.13 | 21.76 | 18.70 |
| 28 | 20.89 | 21.48 | 18.83 |
| 29 | 21.23 | 21.39 | 20.23 |
| 30 | 20.17 | 21.13 | 18.45 |
| 31 | 21.18 | 21.45 | 19.07 |
| 32 | 19.95 | 20.53 | 20.28 |
| 33 | 20.16 | 20.54 | 18.88 |
| 34 | 20.46 | 22.19 | 19.25 |
| 35 | 20.14 | 21.25 | 19.37 |
| 36 | 19.56 | 22.43 | 19.47 |
| 37 | 19.27 | 19.77 | 18.93 |
| 38 | 20.78 | 21.23 | 18.29 |
| 39 | 21.54 | 19.56 | 19.18 |
| 40 | 20.23 | 21.72 | 19.00 |
| 41 | 22.12 | 20.79 | 18.08 |
| 42 | 20.18 | 21.99 | 17.31 |
| 43 | 19.99 | 20.34 | 18.00 |
| 44 | 19.57 | 19.63 | 17.89 |
| 45 | 19.15 | 19.05 | 17.86 |
| 46 | 20.14 | 18.37 | 17.81 |
| 47 | 20.15 | 17.23 | 18.38 |
| 48 | 19.89 | 17.31 | 18.92 |
| 49 | 19.56 | 16.35 | 16.74 |
| 50 | 19.13 | 15.80 | 17.80 |
| 51 | 20.17 | 16.86 | 16.29 |

(continued)

| Time (d) | Daily dose release (μg) | | |
| --- | --- | --- | --- |
| | ZJ001 | ZJ002 | ZJ003 |
| 52 | 18.16 | 17.01 | 15.40 |
| 53 | 19.16 | 17.75 | 17.10 |
| 54 | 19.79 | 18.11 | 16.68 |
| 55 | 19.67 | 17.17 | 16.25 |
| 56 | 17.88 | 16.98 | 17.35 |
| 57 | 20.42 | 16.91 | 18.57 |
| 58 | 19.56 | 16.49 | 18.32 |
| 59 | 20.16 | 16.99 | 19.66 |
| 60 | 19.12 | 17.08 | 18.35 |
| 61 | 19.00 | 18.80 | 18.48 |
| 62 | 19.38 | 19.24 | 17.24 |
| 63 | 17.90 | 19.11 | 18.26 |
| 64 | 18.56 | 17.45 | 17.00 |
| 65 | 17.20 | 17.42 | 17.60 |
| 66 | 19.70 | 16.95 | 17.18 |
| 67 | 18.00 | 17.66 | 19.41 |
| 68 | 17.89 | 15.78 | 18.31 |
| 69 | 18.92 | 16.45 | 18.72 |
| 70 | 19.17 | 18.13 | 19.05 |
| 71 | 17.17 | 17.24 | 17.13 |
| 72 | 14.45 | 16.54 | 17.50 |
| 73 | 15.30 | 17.39 | 17.67 |
| 74 | 16.74 | 17.83 | 18.28 |
| 75 | 17.11 | 17.53 | 17.88 |
| 76 | 15.67 | 17.19 | 17.35 |
| 77 | 17.56 | 17.45 | 16.61 |
| 78 | 17.15 | 16.86 | 17.18 |
| 79 | 16.78 | 17.26 | 16.69 |
| 80 | 16.00 | 16.77 | 17.56 |

Based on the 80-day *in vitro* drug release data from the table, a graph depicting the relationship between the daily *in vitro* drug release amount and release time for three batches of gestodene subcutaneous contraceptive implants with the same specifications was plotted, as shown in Fig. 4.

It can be basically seen from Fig. 4 that the fluctuation range of drug release among the three batches of preparations with the same specifications is not significantly different, and the overall trend is consistent. The curves show large fluctuations in the early stage, gradually stabilizing in the later stage, with most of the daily *in vitro* drug release eventually stabilized within the range of 17-20 μg. A review of the literature revealed that a daily release of 10-20 μg of gestodene is sufficient to produce a contraceptive effect in humans. The three batches of preparations were almost stable at a release amount of 17-20 μg from day 11 to day 80, which was in line with the experimental expectations.

The above data was then sorted and simplified with a 5-day cycle to observe the long-term drug release of the three batches of preparations. The relationship between the drug release amount and release time at various periods after implantation is shown in the table below.

| Time | ZI001 | | ZJ002 | | ZJ003 | |
|---|---|---|---|---|---|---|
| (d) | Daily dose release ($\mu$g) | RSD % | Daily dose release ($\mu$g) | RSD % | Daily dose release ($\mu$g) | RSD % |
| 1-5 | 26.09 $\pm$ 3.86 | 14.81 | 28.85 $\pm$ 1.92 | 6.66 | 28.64 $\pm$ 7.20 | 25.16 |
| 6-10 | 28.48 $\pm$ 6.20 | 21.78 | 23.92 $\pm$ 3.33 | 13.92 | 24.22 $\pm$ 3.41 | 14.08 |
| 11-15 | 23.81 $\pm$ 2.77 | 11.63 | 20.45 $\pm$ 2.17 | 10.60 | 23.24 $\pm$ 0.81 | 3.49 |
| 16-20 | 20.76 $\pm$ 0.64 | 3.09 | 20.40 $\pm$ 0.68 | 3.33 | 23.34 $\pm$ 0.61 | 2.62 |
| 21-25 | 21.17 $\pm$ 0.93 | 4.38 | 21.72 $\pm$ 0.77 | 3.53 | 18.48 $\pm$ 1.05 | 5.69 |
| 26-30 | 20.64 $\pm$ 0.48 | 2.31 | 21.91 $\pm$ 1.07 | 4.89 | 18.84 $\pm$ 0.84 | 4.47 |
| 31-35 | 20.38 $\pm$ 0.48 | 2.38 | 21.19 $\pm$ 0.69 | 3.27 | 19.37 $\pm$ 0.54 | 2.79 |
| 36-40 | 20.28 $\pm$ 0.92 | 4.53 | 20.94 $\pm$ 1.24 | 5.94 | 18.97 $\pm$ 0.44 | 2.30 |
| 41-45 | 20.20 $\pm$ 1.14 | 5.66 | 20.36 $\pm$ 1.13 | 5.55 | 17.83 $\pm$ 0.30 | 1.70 |
| 46-50 | 19.77 $\pm$ 0.43 | 2.19 | 17.01 $\pm$ 0.98 | 5.79 | 17.93 $\pm$ 0.81 | 4.53 |
| 51-55 | 19.39 $\pm$ 0.78 | 4.01 | 17.38 $\pm$ 0.53 | 3.03 | 16.35 $\pm$ 0.63 | 3.86 |
| 56-60 | 19.43 $\pm$ 1.00 | 5.17 | 16.89 $\pm$ 0.23 | 1.39 | 18.45 $\pm$ 0.82 | 4.47 |
| 61-65 | 18.41 $\pm$ 0.87 | 4.74 | 18.40 $\pm$ 0.90 | 4.90 | 17.72 $\pm$ 0.63 | 3.61 |
| 66-70 | 18.74 $\pm$ 0.78 | 4.15 | 17.00 $\pm$ 0.94 | 5.52 | 18.53 $\pm$ 0.86 | 4.65 |
| 71-75 | 16.16 $\pm$ 1.21 | 7.52 | 17.31 $\pm$ 0.48 | 2.76 | 17.69 $\pm$ 0.43 | 2.42 |
| 76-80 | 16.63 $\pm$ 0.79 | 4.73 | 17.11 $\pm$ 0.28 | 1.66 | 17.08 $\pm$ 0.41 | 2.42 |

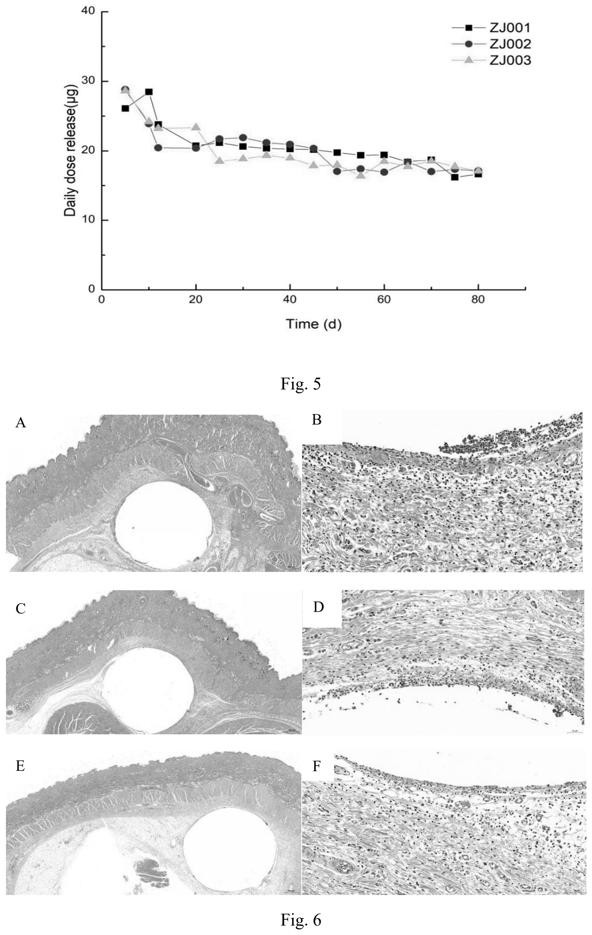

Based on the data in the above table and the curve changes in Fig. 5, the following observations can be made.

(1) The drug release from the implants is unstable and fluctuates significantly during the first ten days. The unstable drug release and high release amount in the early stage may be due to some residual drugs gradually dissolved on the surface of the silicone tube.

(2) The subsequent release from the implants shows better stability. Overall, the release amount exhibits a decreasing trend in the early stage, followed by minor fluctuations, and then stabilizes, with the release amount consistently maintained within the range of 17 $\mu$g to 20 $\mu$g. The release amount meets the requirements, indicating that the gestodene contraceptive implants can achieve controlled release for at least three months.

(3) The average drug release amounts for the three batches of implants are 19.70 $\mu$g, 19.15 $\mu$g, and 18.84 $\mu$g, respectively. The RSD value is 2.26%. The results demonstrate good reproducibility across the three batches of preparations, with basically consistent long-term *in vitro* release data.

**(IV) *In vivo* experiment of gestodene contraceptive implants in rats**

The prepared silicone tubes must meet mechanical standards to ensure stable control of drug release and exhibit good *in vivo* biocompatibility, without causing irritation to body tissues. This section primarily investigates the irritation potential of silicone tubes on rat skin by injecting silicone tube extracts into rats. It also examines the tissue irritation of silicone tubes by implanting silicone tubes into rats for a long period of time and performing tissue section analysis of surrounding tissues. The study involves implanting contraceptive implants of different specifications into rats to observe the trend in body weight changes before and after implantation, assessing whether the contraceptive implants affect normal growth. Vaginal smears after implantation are used to monitor estrous cycle changes, the presence of vaginal plugs, and luteinizing hormone (LH) levels in rats post-implantation to evaluate the contraceptive efficacy of the implants.

1. Instruments and reagents

1.1 Instruments

XS105 Electronic Analytical Balance, Mettler-Toledo Instrument Co., Ltd.

SMD200-2 Electronic Analytical Balance, Ohaus International Trade Co., Ltd.
TG16MW Desktop High-Speed Centrifuge, Hunan Herexi Instrument and Equipment Co., Ltd.
KQ-250DE CNC Ultrasonic Cleaner, Kunshan Ultrasonic Instrument Co., Ltd.
Rat LH Assay Kit, Shanghai Tongwei Biotechnology Co., Ltd.
Electron Microscope, Shenzhen Zhongwei Kechuang Technology Co., Ltd.
Autoclave, Shanghai Shenan Medical Instrument Factory

1.2 Reagents

Gestodene (Purity of 98%, Batch No.: 20190327), Hebei Mokai Technology Development Co., Ltd.
Crystal violet, Tianjin Damao Chemical Reagent Factory
Normal saline, Shandong Yuwang Chemical Reagent Co., Ltd.
4% paraformaldehyde solution, Shanghai Yantuo Biotechnology Co., Ltd.

1.3 Experimental animals

12 healthy male SD rats (weighing 180-200 g) and 57 healthy female SD rats (weighing 180-200 g) were purchased from Benxi Changsheng Biotechnology Co., Ltd., License No. SCXK (Liao) 2020-0001.

2. Experimental methods

2.1 Local irritation test of silicone tubes in rats

The prepared silicone tubes (from preparations ZJ001, ZJ002, and ZJ003) were cut into pieces and soaked in 10 mL of sterile, pyrogen-free purified water. The mixture was heated and extracted at 70°C for 24 hours. The extract was then sterilized by autoclaving for later use. Six experimental rats were randomly divided into two groups: one experimental group and one saline control group. The back of the rats was shaved for the test. The experimental group received an intradermal injection of 0.2 mL of the extract on one side of the rat's back, while the control group received an intradermal injection of 0.2 mL of normal saline on one side of the rat's back. Skin reactions at the injection sites were observed after 24 hours, 48 hours, and 72 hours.

2.2 Histopathological examination after implantation of silicone tubes in rats

The prepared silicone tubes were sealed, and after the sealant was completely cured, they were sterilized for later use. Experimental rats were randomly divided into three groups, with three rats in each group. Each rat had a sterilized homemade silicone tube implanted in the back. The rats were sacrificed 3 days, 10 days, and 30 days post-implantation, respectively. A 2 cm section of tissue around the implanted silicone tube was excised, and the skin tissue was fixed with 4% paraformaldehyde for histopathological examination. The surrounding tissue was observed for inflammation reactions such as redness and swelling. The tissue was then subjected to histological sectioning to examine for any abnormal subcutaneous and muscular lesions at the implantation site.

2.3 *In vivo* pharmacodynamic experiment of gestodene contraceptive implants in rats

Using the optimal preparation and process (corresponding to the silicone tubes and medicinal cores of the aforementioned preparations ZJ001, ZJ002, and ZJ003), silicone tubes with an outer diameter of 2.38 mm and a wall thickness of 0.3 mm, along with gestodene implants of five specifications (drug-loaded segment length/drug loading) were prepared for later use. The specifications are as follows: 1) 0.2 cm/2.3 mg, 2) 0.5 cm/5.4 mg, 3) 1 cm/10.5 mg, 4) 2 cm/20.7 mg, and 5) 4 cm/41.1 mg.
A total of 42 female rats were selected and examined via vaginal smears to check for a normal estrous cycle. The 42 female SD rats, weighing approximately 180-200 g, were randomly divided into experimental dose groups I, II, III, IV, and V (corresponding to the implants with specifications of 1) 0.2 cm/2.3 mg, 2) 0.5 cm/5.4 mg, 3) 1 cm/10.5 mg, 4) 2 cm/20.7 mg, and 5) 4 cm/41.1 mg, respectively), one blank group (K group), and one positive control group with levonorgestrel silicone rods (Y group) (levonorgestrel silicone rod I, purchased from Liaoning Ludan Pharmaceutical Co., Ltd.).
The rats were intraperitoneally injected with an appropriate amount of anesthetic. Once the rats were anesthetized, the hair on their neck and back was shaved, and the area was disinfected with 2% iodine tincture. A small incision of approximately 0.5 cm was cut on the local skin of the rat's back using surgical scissors. An implantation needle specifically designed for the implant was inserted subcutaneously to the designated mark on the needle. The sterilized implant was placed inside the implantation needle. The needle was then advanced further into the skin until the implant was fully inserted. The implantation needle was withdrawn, leaving the implant under the skin. The incision was sutured, and the wound was disinfected with 2% iodine tincture. Twenty-four hours after implantation, the female rats were housed with male rats in a ratio of 3:1.

### 2.3.1 Observation of rat condition

The rats were weighed and recorded before the implantation surgery. Thirty days after the implantation of gestodene contraceptive implants, the rats were weighed again to observe whether the rats grew normally after the implant was inserted. The mental state, activity, and urination and defecation of the rats were also observed daily.

### 2.3.2 Vaginal smear test to observe estrous cycle in rats

The estrous cycle of rats averages 4-5 days and is generally divided into four stages, namely proestrus, estrus, metestrus, and diestrus. Proestrus and estrus are the phases during which mating and ovulation can lead to fertilization. Metestrus is the regression and degradation phase of the reproductive tract. Diestrus is the relatively quiescent and slow growth phase. The vaginal smear method is commonly used to determine the course of the estrous cycle in rats. When the contraceptive implant exerts its contraceptive effect, the rats should not exhibit the estrus phase.

Vaginal smear method: The rats were taken out and held securely in the palm. A fine cotton swab was moistened with normal saline and gently inserted into the vagina of the female rats for approximately 0.5 cm. The swab was rotated slowly before being withdrawn, and the vaginal contents on the cotton swab were evenly smeared onto a slide. The slide was numbered and naturally dried before being stained with crystal violet. Once air-dried, the vaginal smears were examined under an electron microscope to determine the estrous cycle of the rats. After the implantation surgery, the rats were subjected to vaginal smear tests 3 days post-implantation, with vaginal secretions collected daily before 9:00 a.m. The estrous cycle changes in the experimental rats were observed through the vaginal smears.

### 2.3.3 Observation of vaginal plugs to determine mating and pregnancy in rats

When the contraceptive implant exerts its contraceptive effect, the female rats should not have an estrus phase and therefore will not mate with the male rats. Thus, the presence of vaginal plugs can be used to determine the contraceptive efficacy. After mating, semen left at the vaginal opening forms a vaginal plug, usually milky white and sometimes slightly yellow. Since mating typically takes place at night, vaginal secretion collection and vaginal plug observation need to be completed before 9:00 a.m. to accurately observe the mating status of the rats. After the implantation surgery, the presence of vaginal plugs was observed in the rats three days post-implantation before conducting the vaginal smear test.

### 2.3.4 Measurement of luteinizing hormone (LH) levels in rats using enzyme-linked immunosorbent assay

Gestodene primarily acts on the hypothalamus and pituitary gland, significantly reducing the levels of follicle-stimulating hormone (FSH) and luteinizing hormone (LH) in the body, preventing ovulation, exhibiting marked anti-estrogenic activity, and thickening cervical mucus to impede sperm penetration. It shows strong progestogenic activity on endometrial transformation, which can make the endometrium thinner. The endometrial epithelial cells appear as low columnar cells with poor secretory function, thus making it unfavorable for embryo implantation. To confirm the contraceptive effect, the LH levels in rats implanted with the contraceptive implant are expected to be significantly lower than those in the blank control group.

Each day, after collecting vaginal secretions, blood samples were taken from the orbital sinus of the female rats cohabited with the male rats post-implantation surgery. Blood was collected in an EP tube pre-rinsed with an anticoagulant, centrifuged for approximately 10 minutes (5000 rpm), and the supernatant was carefully collected and stored in a freezer at - 80°C. The LH levels in the rats were measured using the enzyme-linked immunosorbent assay to evaluate the efficacy of the contraceptive implant.

### 3. Experimental results and discussion

### 3.1 *In vivo* tissue irritation test of silicone tubes in rats

The experimental group received an intradermal injection of 0.2 mL of the extract on one side of the rat's back, while the control group received an intradermal injection of 0.2 mL of normal saline on one side of the rat's back. Skin reactions at the injection sites were observed after 24 hours, 48 hours, and 72 hours. Dissection of the injection sites revealed no signs of redness, swelling, or necrosis, indicating a negative outcome.

3.2 Histopathological examination after implantation of silicone tubes in rats

Muscle tissues from the implantation site were collected on days 3, 10, and 30 post-implantation, and their pathological images (HE, ×2 or HE, ×20) were observed, as shown in Fig. 6.

The results showed that on day 3 post-implantation (refer to Panel A and Panel B of Fig. 6), acute inflammatory response was observed, with a slight thickening of the epidermis, a high content of collagen fibers in the dermis, and a large cystic structure formed in the local subcutaneous tissue. Connective tissue proliferation was observed around the cystic structure, accompanied by significant lymphocyte infiltration. On day 10 post-implantation (refer to Panel C and Panel D of Fig. 6), the acute inflammatory response gradually diminished, with a slight thickening of the epidermis, a high content of collagen fibers in the dermis, and a large cystic structure formed in the local subcutaneous tissue. Connective tissue proliferation was observed around the cystic structure, accompanied by diffuse lymphocyte infiltration. On day 30 post-implantation (refer to Panel E and Panel F of Fig. 6), the epidermis was structurally intact, and the squamous epithelial cells were normal in structure and tightly arranged, with a high content of collagen fibers in the dermis and a large cystic structure formed in the local subcutaneous tissue. Mild connective tissue proliferation was observed around the cystic structure, accompanied by diffuse lymphocyte and macrophage infiltration.

The implantation sites consistently exhibited large cystic cavities, indicating that the implantation of the implant caused some damage to the subcutaneous tissue, resulting in cystic cavity formation. Mild connective tissue proliferation occurred around these cystic cavities, gradually forming a capsule at the interface between the silicone tube and the tissue. Upon dissection of the silicone tube three days post-implantation, it was observed that the silicone tube could be easily removed. When it was dissected ten days post-implantation, a capsule formed around the silicone tube, making it less movable, indicating that with prolonged implantation, a capsule forms around the silicone tube, preventing it from shifting within the body. Furthermore, the inflammatory response was most pronounced at three days post-implantation, and gradually decreased with prolonged implantation, suggesting that the inflammatory response induced by the silicone tube implantation is capable of subsiding over time.

3.3 *In vivo* pharmacodynamic experiment of gestodene contraceptive implants in rats

3.3.1 Body weight changes in rats before and after implantation

Following the implantation surgery, the rats were observed for their feeding status and activity levels. Normal urination and defecation were noted, and no signs of lethargy were observed.

The body weights of the rats before and after implantation are shown in the table below.

| Group | Weight before implantation (g) | Weight on day 30 post-implantation (g) |
|---|---|---|
| K | 188.00 ± 6.29 | 223.50 ± 6.92 |
| Y | 190.00 ± 5.41 | 212.50 ± 6.61 |
| Experiment I | 180.00 ± 2.50 | 204.00 ± 7.83 |
| Experiment II | 182.11 ± 4.50 | 210.23 ± 6.51 |
| Experiment III | 190.00 ± 7.02 | 221.50 ± 4.36 |
| Experiment IV | 182.25 ± 5.56 | 213.25 ± 5.50 |
| Experiment V | 183.55 ± 4.56 | 220.25 ± 3.86 |

From the data on the body weight changes observed over a month, it can be concluded that the implantation surgery and the silicone tube did not affect the growth of the rats. The trend in body weight changes in the experimental group was similar to that of the blank group. Overall, the gestodene implant did not have any effect on the growth of the rats.

3.3.2 Observation of estrous cycle in rats via vaginal smears

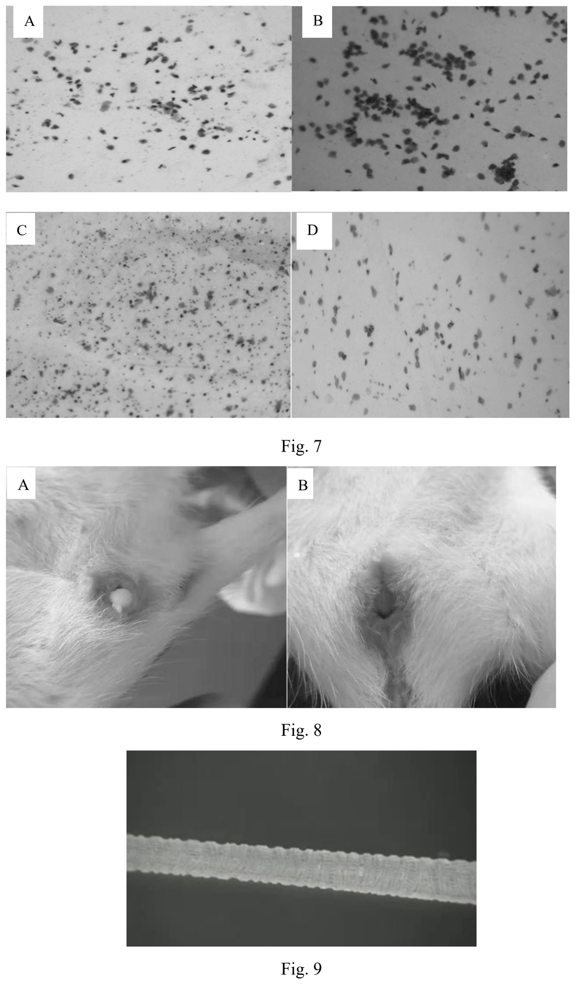

For the blank control group, vaginal secretions from female rats were collected daily at 24-hour intervals. These secretions were stained with crystal violet for smear observation to monitor the estrous cycle, which was compared to that of the experimental group. Over a continuous one-month period, it was observed that the rats generally experienced estrus approximately every seven days. The vaginal smears of the estrous cycle in rats are shown in Fig. 7. In rats implanted with commercially available levonorgestrel implants, the vaginal smears showed no signs of estrus, indicating a contraceptive effect.

The experimental group was divided into five dose groups. Based on the vaginal smear observation over one month, in experimental dose groups II, III, IV, and V, a large amount of mucus from the vaginal secretions was observed starting from day 4 post-implantation. The vaginal smears revealed a large number of leukocytes but no keratinocytes, indicating the absence of a normal estrous cycle in these rats. However, in experimental group I, rats numbered 1 and 4 still displayed complete estrous cycle changes, suggesting contraceptive failure in these two rats. This indicates that the dose administered to these rats may be relatively ineffective for contraception, resulting in a low contraceptive rate.

3.3.3 Observation of vaginal plugs to determine mating and pregnancy in rats

During the observation of vaginal plugs, it was noted that in the experimental dose group I, rat numbered 1, which displayed a normal estrous cycle in the vaginal smear, was observed to have a milky white vaginal plug at the vaginal opening when vaginal secretions were collected 13 days post-implantation, as shown in Panel A of Fig. 8. Similarly, rat numbered 4 was found to have a vaginal plug 20 days post-implantation, as shown in Panel B of Fig. 8. The results showed that the two female rats numbered 1 and 4 in the experimental dose group I had mated with male rats, indicating the occurrence of estrous cycles in both mice and thus a failure in contraception. No vaginal plugs were observed in the remaining experimental dose groups or in the commercial positive control group, demonstrating that the rats did not mate with male rats and confirming a contraceptive effect.

3.3.4 Measurement of plasma LH concentration in rats using enzyme-linked immunosorbent assay

LH levels in rats were measured using the enzyme-linked immunosorbent assay. The LH levels were determined in six rats in each group 28 days after the implantation of the gestodene contraceptive implants. The results are as follows.
The LH levels in rats are shown in the table below.

| Group | Replicates | LH (IU/L) |
|---|---|---|
| K | 6 | 73.71 $\pm$ 4.53 |
| I | 6 | 65.01 $\pm$ 6.71* |
| II | 6 | 50.85 $\pm$ 3.36* |
| III | 6 | 47.41 $\pm$ 3.39* |
| IV | 6 | 42.31 $\pm$ 2.43* |
| V | 6 | 37.46 $\pm$ 1.99* |
| Y | 6 | 41.83 $\pm$ 7.05* |
| Note: * means P < 0.01 compared to the blank group. | | |

The results showed that the LH levels in the positive control group and each experimental dose group were significantly different from the blank group (P < 0.01). Since the above table represents the mean values obtained from six rats in each experimental group, and considering the contraceptive failure of two rats in the experimental dose group I based on vaginal smear and vaginal plug results, the LH levels of each of the six rats in the experimental dose group I are listed separately. The results are as follows.
The LH levels of each rat in the experimental dose group I are shown in the table below.

| Group | 1 | 2 | 3 | 4 | 5 | 6 |
|---|---|---|---|---|---|---|
| I | 77.04 $\pm$ 10.96 | 55.10 $\pm$ 1.80* | 55.599 $\pm$ 7.62* | 72.54 $\pm$ 12.79 | 63.53 $\pm$ 7.04* | 64.06 $\pm$ 9.40* |
| Note: * means P < 0.01 compared to the blank group. | | | | | | |

The results showed that the LH levels of the two rats numbered 1 and 4 in the experimental dose group I were not significantly different from those of the blank group, whereas the other four rats had significantly different LH levels compared to the blank group (P < 0.01).

From the LH level data in rats, it can be observed that the experimental dose groups II, III, IV, and V as well as the positive control group showed significantly reduced LH levels in rats compared to the blank group. The higher the dose, the lower the LH levels, indicating a better hormone inhibitory effect. It was found that the LH levels of rats numbered 1 and 4 in the experimental dose group I were higher and more variable compared to the other numbered rats, suggesting that the implantation and the drug release from the implant in these two rats was unstable and did not achieve the contraceptive effect. Due to the short length of the drug-loaded segment (0.2 cm) in the implant of the experimental dose group I, the preparation process of the implant was challenging, leading to unstable drug filling and thus contraceptive failure in these two rats. In contrast, the other four rats in the same group achieved the contraceptive effect.

4. Summary

(1) The local irritation test of silicone tube extracts in rats demonstrated that the silicone tube was safe and non-irritating to the local skin of rats. The implantation test of the silicone tube demonstrated that the prepared silicone tube was non-irritating to rat tissues and had good histocompatibility, with no inflammation or irritation observed in the surrounding tissues post-implantation.

(2) Through the implantation tests, the normal growth of rats post-implantation was evidenced by the body weight changes before and after implantation. The estrous cycle of rats was observed through stained vaginal secretion smears. The mating and pregnancy status of rats were observed through vaginal plugs. The contraceptive effect of the gestodene implant was evaluated through three tests exploring the changes in LH levels in rats post-implantation. The results showed that the effective dose groups exhibited a good contraceptive effect, with no estrous cycles observed through the vaginal smears and no vaginal plugs observed. The LH levels were significantly reduced compared to the normal group.

(3) The pharmacodynamic experiment in rats showed that the gestodene implants with a drug-loaded segment length of 0.2 cm and a drug loading of 2.3 mg had a poor contraceptive effect, with a contraceptive rate of less than 70%. In contrast, the implants with a drug-loaded segment length greater than 0.5 cm and a drug loading greater than 5.4 mg had an excellent contraceptive effect.

## II. Long-acting levonorgestrel implants

### (I) Content determination and *in vitro* release testing methods for levonorgestrel contraceptive implants

(1) Chromatographic conditions

Chromatographic column: Diamonsil® $C_{18}$ column (4.6 mm $\times$ 250 mm, 5 $\mu$m);
Mobile phase: methanol-water (80:20, v/v);
Column temperature: 30°C;
Detection wavelength: 240 nm;
Flow rate: 1.0 mL·min$^{-1}$;
Injection volume: 20 $\mu$L.

(2) *In vitro* release rate determination method

The release experiment was conducted using the horizontal shaking method. A set of six implants was fixed to the wall of a 125 mL stoppered conical flask using an adhesive, ensuring an appropriate spacing between each rob (to prevent the implants from floating on the liquid surface during release, which could lead to inaccurate release results). Exactly 100 mL of distilled water was measured and injected into the conical flask. The flask was then placed in a thermostatic air shaker set at 37°C and shaken at a frequency of 100 rpm. The medium was replaced every 24 hours with an equal volume of fresh medium. The samples were filtered through a 0.22 $\mu$m microporous filter membrane and detected under the chromatographic conditions specified in section (1), with an injection volume of 20 $\mu$L.

### (II) Preparation and evaluation of addition-cure silicone tubes

Addition-cure silicone rubber products, synthesized through a hydrosilylation reaction between methyl vinyl silicone rubber and hydrogen-containing silicone oil under the catalysis of a platinum catalyst, with the addition of a suitable reinforcing agent, exhibit excellent physiological inertness. These products are non-toxic and odorless, have good biocompatibility, resist biological aging, possess good permeability, show no adverse reactions when implanted in the body, and undergo minimal changes in physical properties over long-term implantation. These attributes make them suitable for applications involving contact with blood and various implanted scenarios within the body.

This section focuses on the process of cross-linking vinyl-terminated linear polymer methyl vinyl silicone rubber into a network-like polymer elastomer through the addition of a reinforcing agent (silica) and under the catalysis of a platinum catalyst. The elastomer is extruded into a tube through an extruder and then subjected to oven vulcanization to obtain an addition-cure silicone tube.

1. Instruments and materials

The instruments, reagents, and materials used are the same as those described in "I. Long-acting gestodene contraceptive implants; (II) Preparation of two-component addition-cure silicone tubes".

2. Preparation and determination of addition-cure silicone tubes

2.1 Preparation of addition-cure silicone tubes by high-temperature vulcanization

The preparation process of the silicone tube is the same as that described in "I. Long-acting gestodene contraceptive implants; (II) Preparation of two-component addition-cure silicone tubes".

2.2 Determination of physical and mechanical properties of addition-cure silicone tubes

It is conducted in the same manner as described in section 2.1.3 Performance testing of silicone tubes under "I. Long-acting gestodene contraceptive implants; (II) Preparation of two-component addition-cure silicone tubes".

3. Methods and results

3.1 Investigation of silicone tube preparations

3.1.1 Investigation of vinyl content in polydimethylsiloxane

To investigate the effect of different vinyl contents in raw rubber (polydimethylsiloxane) on the physical and mechanical properties of silicone tubes, extrusion vulcanization experiments of silicone tubes were conducted using the preparations shown in the table below.

| No. | Content of vinyl groups in methyl vinyl silicone rubber | Methyl vinyl silicone rubber (PHR) | Fumed white carbon black (PHR) | Hydrogen-containing silicone oil (PHR) | Molar ratio of Si-H groups in hydrogen-containing silicone oil to vinyl groups in methyl vinyl silicone rubber | Content of hydrogen in hydrogen-containing silicone oil | Platinum catalyst (PHR) | 2-Methyl-3-butyn-2-ol (PHR) |
|---|---|---|---|---|---|---|---|---|
| Example 12-1 | 0.05 mol% | 100 | 30 | 0.12 | 1.2:1 | 0.75 mol% | $10^{-5}$ | 0.70 |
| Example 12-2 | 0.17 mol% | 100 | 30 | 1.01 | 1.2:1 | 0.75 mol% | $10^{-5}$ | 0.70 |
| Example 12-3 | 0.23 mol% | 100 | 30 | 1.36 | 1.2:1 | 0.75 mol% | $10^{-5}$ | 0.70 |

PHR refers to the parts by mass of a given component per 100 parts by mass of the polymer compound.

Methyl vinyl silicone rubber undergoes an addition reaction at the terminal vinyl groups with the active hydrogen atoms of hydrogen-containing silicone oil, resulting in a crosslinked polymer elastomer. The vinyl groups, acting as active groups, form the cross-linking points of the polymer network. The vinyl content affects the physical and mechanical properties of medical silicone tubes.

As shown in the mechanical properties in the table below, the hardness of the silicone tube increases with the increase of vinyl content. However, the elongation at break, tensile strength, and tear strength of the silicone tube initially increase and then decrease, displaying a peak-shaped variation. At a vinyl content of 0.05%, the cross-linking points formed by the addition reaction are relatively few, resulting in low tear strength and tensile strength, and the mechanical properties are too poor for subsequent use. At a vinyl content of 0.23%, the excessive cross-linking points lead to a decrease in elongation at break and a slight reduction in tensile strength and tear strength. This is because the high cross-linking density results in excessive cross-linking points and shorter cross-linking chains, increasing rigidity. At this time, the hardness of the silicone tube increases, and the elongation at break significantly decreases. The internal cross-linking chains cannot be arranged in an orderly and effective manner, leading to an increase in ineffective cross-linking networks. Consequently, the number of cross-linking chains capable of withstanding external forces decreases, reducing the tensile strength, tear strength, and other mechanical properties of the silicone tube. At a vinyl content of 0.17%, the internal cross-linking chains are distributed in an orderly and reasonable manner, resulting in excellent physical and mechanical properties of the silicone tube. Therefore, methyl vinyl silicone rubber with a vinyl content of 0.17% is the preferred methyl vinyl silicone rubber.

| Mechanical properties | Example 12-1 | Example 12-2 | Example 12-3 |
|---|---|---|---|
| Elongation at break/Eb (%) | 640.38 | 1045.50 | 559.67 |
| Tensile strength/Ts (MPa) | 1.10 | 8.50 | 7.41 |
| Tear strength/T (KN/m) | 6.27 | 45.65 | 38.64 |
| Hardness/H (Shore A) | 40 | 46 | 59 |

3.1.2 Screening of white carbon black proportion

Compared to other rubber raw materials, methyl vinyl silicone rubber has a single-chain structure as the main chain. The Si-O bonds on the molecular chain are longer than CO and C-C σ bonds, and it contains isolated double bonds without other large or polar groups, leading to extremely high flexibility of the molecular chain, which macroscopically translates to poor mechanical properties. To enhance the mechanical properties of methyl vinyl silicone rubber, various fillers are typically added depending on its application. White carbon black, due to its similar main elements and chemical bonds to silicone rubber, is the most compatible inorganic filler with silicone rubber and is a commonly used reinforcing agent in silicone rubber products.

Experiments were conducted using the preparations shown in the table below to investigate the effect of the amount of white carbon black on the mechanical properties of the silicone tube.

| No. | Content of vinyl groups in vinyl polysiloxane | Methyl vinyl silicone rubber (PHR) | Fumed white carbon black (PHR) | Hydrogen-containing silicone oil (PHR) | Molar ratio of Si-H groups in hydrogen-containing silicone oil to vinyl groups in methyl vinyl silicone rubber | Content of hydrogen in hydrogen-containing silicone oil | Platinum catalyst (PHR) | 2-Methyl-3-butyn-2-ol (PHR) |
|---|---|---|---|---|---|---|---|---|
| Example 13-1 | 0.17 mol% | 100 | 30 | 1.01 | 1.2:1 | 0.75 mol% | $10^{-5}$ | 0.70 |
| Example 13-2 | 0.17 mol% | 100 | 35 | 1.01 | 1.2:1 | 0.75 mol% | $10^{-5}$ | 0.70 |

(continued)

| No. | Content of vinyl groups in vinyl polysiloxane | Methyl vinyl silicone rubber (PHR) | Fumed white carbon black (PHR) | Hydrogen-containing silicone oil (PHR) | Molar ratio of Si-H groups in hydrogen-containing silicone oil to vinyl groups in methyl vinyl silicone rubber | Content of hydrogen in hydrogen-containing silicone oil | Platinum catalyst (PHR) | 2-Methyl-3-butyn-2-ol (PHR) |
|---|---|---|---|---|---|---|---|---|
| Example 13-3 | 0.17 mol% | 100 | 40 | 1.01 | 1.2:1 | 0.75 mol% | 10⁻⁵ | 0.70 |

PHR refers to the parts by mass of a given component per 100 parts by mass of the polymer compound.

The effect of the amount of white carbon black on the physical and mechanical properties of the silicone tube is shown in the table below. Within the range of 30 PHR to 40 PHR, as the amount of white carbon black increases, the hardness of the silicone tube gradually increases, the deformation of the extruded tube decreases, the wall thickness becomes more uniform, and the other physical and mechanical properties such as the tensile strength, tear strength, and elongation at break of the tube exhibit excellent performance. However, when the amount of white carbon black increases to 40 PHR, the increased hardness of the rubber results in slightly insufficient extrusion power of the extruder and large fluctuations in the extrusion rate, leading to an uneven appearance of the extruded tube.

The improvement in the mechanical properties of vulcanized rubber by white carbon black is attributed to the hydroxyl groups on its surface interacting with macromolecules and forming a spatial network structure with macromolecules. This structure allows the silicone rubber to absorb the impact of external forces through the sliding of molecular chains and extensive physical adsorption when deformed by external forces, buffering friction or hysteresis deformation caused by external forces and at the same time ensuring even stress distribution. Thus, adding an appropriate amount of white carbon black can enhance the mechanical properties of silicone rubber without significantly reducing its tensile elasticity. Therefore, it is preferred to add white carbon black at 35% of the mass of the raw rubber for the preparation of silicone tubes.

| Mechanical properties | Example 13-1 | Example 13-2 | Example 13-3 |
|---|---|---|---|
| Elongation at break/Eb (%) | 1045.50 | 979.84 | 900.49 |
| Tensile strength/Ts (MPa) | 8.50 | 8.49 | 8.49 |
| Tear strength/T (KN/m) | 45.65 | 45.02 | 44.28 |
| Hardness/H (Shore A) | 46 | 50 | 58 |

3.1.3 Investigation of amount of hydrogen-containing silicone oil

Using raw rubber with a vinyl content of 0.17% and hydrogen-containing silicone oil with a hydrogen content of 0.75%, the amount of hydrogen-containing silicone oil was calculated based on the vinyl content. The calculation formula is as follows:

$$W = \frac{A \times \omega(Vi)}{\omega(H) \times 27} \times W_1$$

wherein W is the amount of hydrogen-containing silicone oil added; A is the molar ratio of Si-H to Si-Vi, that is, when the molar ratio of vinyl to hydrogen in the hydrogen-containing silicone oil reaches 1:1, the value of A is 1; $\omega(Vi)$ is the mass percentage of vinyl in the raw rubber; $\omega(H)$ is the mass percentage of hydrogen in the hydrogen-containing silicone oil; 27 is the molar mass of vinyl; $W_1$ is the mass of the raw rubber. The variation in the amount of hydrogen-containing silicone oil was expressed by the value of A. Experiments were conducted using the preparations shown in the table below to investigate the effect of the amount of hydrogen-containing silicone oil on the mechanical properties of the silicone tube.

| No. | Value of A | Methyl vinyl silicone rubber (PHR) | Fumed white carbon black (PHR) | Hydrogen-containing silicone oil (PHR) | Platinum catalyst (PHR) | 2-Methyl-3-butyn-2-ol (PHR) |
|---|---|---|---|---|---|---|
| Example 14-1 | 0.5 | 100 | 35 | 0.42 | $10^{-5}$ | 0.70 |
| Example 14-2 | 0.8 | 100 | 35 | 0.67 | $10^{-5}$ | 0.70 |
| Example 14-3 | 1 | 100 | 35 | 0.84 | $10^{-5}$ | 0.70 |
| Example 14-4 | 1.2 | 100 | 35 | 1.01 | $10^{-5}$ | 0.70 |
| Example 14-5 | 1.5 | 100 | 35 | 1.26 | $10^{-5}$ | 0.70 |
| Example 14-6 | 2 | 100 | 35 | 1.68 | $10^{-5}$ | 0.70 |
| Example 14-7 | 3 | 100 | 35 | 2.52 | $10^{-5}$ | 0.70 |
| [0723] Note: PHR refers to the parts by mass of a given component per 100 parts by mass of the polymer compound; | | | | | | |

In the table above, the vinyl molar percentage of raw rubber is 0.17%, and the hydrogen molar percentage of hydrogen-containing silicone oil is 0.75%.

The experimental results, as shown in the table below, indicate that with the increase in the amount of hydrogen-containing silicone oil, the physical and mechanical properties of the silicone tube exhibit a peak-shaped variation. This is also related to the density of cross-linking points within the silicone tube. According to the reaction mechanism, the hydrogen in the hydrogen-containing silicone oil and the vinyl groups are involved in the reaction at a molar ratio of 1:1. When the value of A is less than 1, the quantity of active hydrogen involved in the addition reaction is insufficient, resulting in fewer cross-linking points formed by the addition reaction. As the value of A increases, the tensile strength, tear strength, and hardness of the silicone tube gradually increase, reaching a peak when the value of A is 1.2, that is, when the molar ratio of vinyl to hydrogen in the hydrogen-containing silicone oil is 1:1.2, the optimal physical and mechanical properties of the silicone tube are achieved. A slight excess of hydrogen-containing silicone oil allows the addition reaction to occur fully, achieving the optimal mechanical properties of the extruded tube. However, as the amount of hydrogen-containing silicone oil continues to increase, the cross-linking density of the silicone tube becomes larger and the cross-linking points become disordered, leading to a decline in physical and mechanical properties. Therefore, the value of A for calculating the amount of hydrogen-containing silicone oil when feeding is preferably 1.2.

| Mechanical properties | Example 14-1 | Example 14-2 | Example 14-3 | Example 14-4 | Example 14-5 | Example 14-6 | Example 14-7 |
|---|---|---|---|---|---|---|---|
| Elongation at break/ Eb (%) | 402.19 | 670.82 | 900.49 | 970.54 | 835.05 | 650.80 | 433.19 |
| Tensile strength/ Ts (MPa) | 6.10 | 6.58 | 7.71 | 8.49 | 6.56 | 5.97 | 4.02 |
| Tear strength/T (KN/m) | 31.67 | 36.40 | 38.81 | 44.28 | 38.36 | 30.80 | 17.48 |
| Hardness/H (Shore A) | 31 | 39 | 48 | 50 | 47 | 43 | 40 |

3.2 Investigation of preparation process of silicone tube

3.2.1 Investigation of vulcanization temperature and time

| Material name | Amount |
|---|---|
| Methyl vinyl silicone rubber | 100 PHR |
| Content of vinyl groups in vinyl polysiloxane | 0.17% |
| Fumed white carbon black | 35 PHR |
| Hydrogen-containing silicone oil | 1.01 PHR |
| Molar ratio of Si-H groups in hydrogen-containing silicone oil to vinyl groups in methyl vinyl silicone rubber | 1.2:1 |
| Content of hydrogen in hydrogen-containing silicone oil | 0.75% |
| 2-Methyl-3-butyn-2-ol | 0.7 PHR |
| Platinum catalyst (3000 ppm) | 0.00001 PHR |

After extrusion from the extruder, the silicone rubber undergoes high-temperature vulcanization through a short front drying tunnel (0.8 m) followed by vulcanization through a long rear drying tunnel (2.5 m) while being conveyed by a conveyor belt. This constitutes the first stage of vulcanization, the main purpose of which is to achieve molding of the silicone tube. The purpose of high-temperature vulcanization in the short drying tunnel is to quickly set the shape of the silicone tube, thereby minimizing deformation during subsequent vulcanization on the conveyor belt. The temperature in the short drying tunnel should not be too high to avoid over-vulcanization of the silicone tube (as shown in Fig. 9, with the state of over-vulcanization corresponding to a front drying tunnel temperature of 360°C), which can cause macromolecular breakdown within the silicone tube, resulting in cracks under tensile stress and a drastic decline in physical and mechanical properties of the silicone tube. To ensure molding of the silicone tube through continued vulcanization, the vulcanization temperature in the rear drying tunnel should not be too low. However, a rear drying tunnel temperature above 200°C can affect the service life of the conveyor belt. Therefore, the temperature for the silicone tube passing through the rear drying tunnel under the traction of the conveyor belt was set at 180°C. At this temperature, the silicone tube was well molded with minimal deformation and no over-vulcanization.

After the first stage of heating and molding, due to the short reaction time, the cross-linking of the silicone tube was not complete, resulting in insufficient cross-linking density. The silicone tube was subjected to secondary vulcanization in an oven at 180°C for further cross-linking reaction. Samples were taken at 0 hours, 12 hours, 24 hours, 48 hours, and 72 hours to measure their physical and mechanical properties and to observe the changes in the physical and mechanical properties of the silicone tube after oven vulcanization.

| No. | Oven heat treatment time |
|---|---|
| Example 15-1 | 0 hours |
| Example 15-2 | 12 hours |
| Example 15-3 | 24 hours |
| Example 15-4 | 48 hours |
| Example 15-5 | 72 hours |

The secondary vulcanization time and the changes in the physical and mechanical properties of the silicone tube are shown in the table below. When the secondary vulcanization time ranged from 0 to 48 hours, the cross-linking reaction within the silicone tube continued to occur, increasing the number of cross-linking points. Consequently, the elongation at break of the silicone tube decreased, while the tensile strength and tear strength significantly increased compared to the silicone tube after primary vulcanization. When the secondary vulcanization time ranged from 48 to 72 hours, the cross-linking reaction within the silicone tube was essentially completed, resulting in a slight enhancement of tear strength and tensile strength. However, with the increase of the vulcanization time, the silicone tube began to undergo over-vulcanization, leading to thermal cracking of internal cross-linking bonds and chain segments, and causing a decrease in tensile strength and tear strength. By comprehensively comparing the physical and mechanical properties of the silicone tube after secondary vulcanization, the preferred conditions for secondary vulcanization were determined to be 180°C for 48 hours.

| Mechanical properties | Example 15-1 | Example 15-2 | Example 15-3 | Example 15-4 | Example 15-5 |
|---|---|---|---|---|---|
| Elongation at break/Eb (%) | 900.49 | 718.40 | 608.81 | 565.13 | 603.54 |
| Tensile strength/Ts (MPa) | 8.49 | 8.65 | 8.58 | 9.79 | 8.76 |
| Tear strength/T (KN/m) | 44.28 | 49.06 | 46.57 | 54.77 | 43.48 |
| Hardness/H (Shore A) | 40 | 52 | 57 | 63 | 64 |

4. Biological performance testing of addition-cure silicone tubes

Implant materials used in the body need to be safe, reliable, and non-toxic in terms of biological performance to ensure implantation safety. The material was subjected to the following biological tests in accordance with "GBT16175-2008 - Biological Evaluation Test Methods for Medical Silicone Materials" to examine its biocompatibility and compliance with *in vivo* implantation requirements.

4.1 Acute toxicity test

The prepared clean silicone tubes (corresponding to the silicone tubes in Example 14-4) were cut into a length of 5 cm and immersed in 100 mL of redistilled water. The mixture was heated and extracted at 70°C for 24 hours, followed by sterilization in an autoclave at 121°C for 30 minutes for later use.
Ten healthy SD rats weighing approximately 200 g were randomly divided into an experimental group and a control group. One group received a tail vein injection of the above extract at a dose of 5 mL/kg, while the other group received a tail vein injection of the same dose of normal saline. The rats were observed within 24 hours, 48 hours, and 72 hours. The results showed no adverse reactions or deaths.

4.2 Hemolysis test

The hemolysis test was conducted according to General Rule 1148 of the Chinese Pharmacopoeia (2020 edition). 1 mL of blood from healthy rabbits was collected and placed in a conical flask containing glass beads, shaken for 10 minutes to remove fibrinogen, and converted into defibrinated blood. Approximately 10 times the volume of 0.9% sodium chloride solution was added, shaken well, and centrifuged at 1000 rpm for 15 minutes. The supernatant was removed, and the precipitated red blood cells were washed three times with 0.9% sodium chloride solution using the same method until the supernatant showed no redness. The obtained red blood cells were then made into a 2% suspension with 0.9% sodium chloride solution for later use.
Five clean glass test tubes were prepared and labeled as follows: Tubes 1 and 2 for the test sample, Tube 3 for the negative control, Tube 4 for the positive control, and Tube 5 for the test sample control. 2% red blood cell suspension, 0.9% sodium chloride solution, purified water, and the extract from "4.1 Acute toxicity test" were sequentially added as shown in the table below, mixed well, and immediately placed in a thermostatic shaker at 37°C $\pm$ 0.5°C for incubation. The hemolysis and agglutination reactions were observed after 3 hours.
The hemolysis test results are shown in the table below.

| Tube No. | 1 | 2 | 3 | 4 | 5 |
|---|---|---|---|---|---|
| 2% red blood cell suspension (mL) | 2.5 | 2.5 | 2.5 | 2.5 | / |
| 0.9% sodium chloride solution (mL) | 2.5 | 2.5 | 2.5 | / | 4.7 |
| Purified water (mL) | / | / | / | 2.5 | / |
| Extract (mL) | 0.3 | 0.3 | / | / | 0.3 |

The experiment revealed no significant difference in appearance among Tubes 1, 2, 3, and 5, with the supernatant being clear. Tube 4, the positive control, showed hemolysis, indicating that the material tested negative for hemolysis.

4.3 Irritation test

Ten healthy SD rats weighing approximately 200 g were randomly divided into an experimental group and a control group. Four hours before the start of the experiment, the hair on both sides of the rat's back (an area of approximately 3 × 3 cm) was removed.

An appropriate amount of the extract from "4.1 Acute toxicity test" was dropwise added to a 2.5 × 2.5 cm absorbent gauze pad, which was then applied to the depilated skin of the rats and fixed with adhesive tape. The rats in the control group were applied with a saline-soaked gauze pad similarly manipulated as described above, and the gauze pad was fixed. After 4 hours, the gauze pads were removed and no skin reaction was observed.

Ten healthy SD rats weighing approximately 200 g were randomly divided into an experimental group and a saline control group. The experimental group received an intradermal injection of the extract at five points on one side of the rat's back, with 0.2 mL per point. The opposite side of the back was injected with an equal amount of normal saline. The saline control group was injected with an equal amount of normal saline on both sides of the rat's back following the above procedure. The injection sites were observed within 24 hours, 48 hours, and 72 hours after injection. No redness, swelling, necrosis, or other adverse reactions were observed, indicating that the material tested negative for irritation.

4.4 Short-term implantation test

Several clean silicone tubes with an outer diameter of 2.4 mm, an inner diameter of 1.6 mm, and a length of 35 mm (corresponding to the silicone tubes in Example 14-4) were prepared. Both ends were sealed with adhesive. The silicone tubes were then rinsed with distilled water, sterilized at 121°C for 30 minutes, and the sterilized tubes were dried for later use. Twelve healthy SD rats weighing approximately 200 g were selected. After anesthetizing the rats, the sterilized silicone tubes were implanted into the skin of the rat's back using an implantation needle, and the wounds were sutured. The rats were then normally fed. On day 3 and day 10 after implantation, five SD rats were randomly selected and euthanized, and the tissues around the silicone tubes were removed and prepared into tissue sections for histological examination.

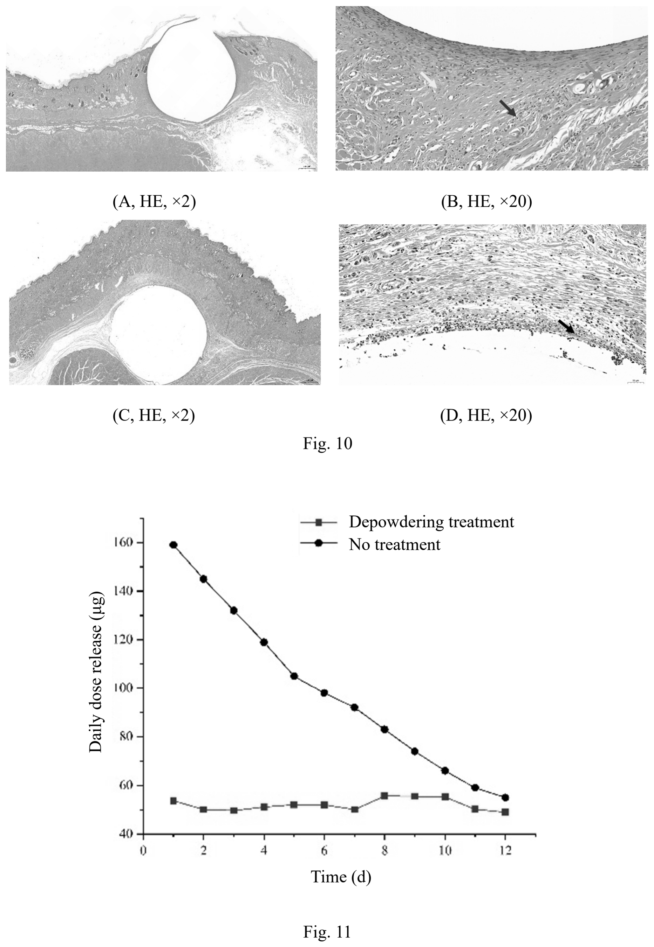

During the implantation period, the rats exhibited normal diet, daily routine, and body weight gain. No abnormal lesions were observed in the subcutaneous and muscle tissues of the implantation site. The experimental results of tissue sections are shown in Fig. 10. Panel A of Fig. 10 shows a full view of the tissue section on day 3 after implantation, where slight epidermal thickening can be observed. Panel B of Fig. 10 shows a local view of the tissue section on day 3 after implantation, where connective tissue proliferation (leftmost arrow) can be seen around the implantation site, accompanied by scattered lymphocyte infiltration (middle arrow), and multinucleated giant cells (rightmost arrow) can be seen. Based on the grading standard for inflammatory cell response, the degree of tissue inflammatory cell response on day 3 after implantation was classified as Grade III. Panel C of Fig. 10 shows a full view of the tissue section on day 10 after implantation, where slight epidermal thickening can still be observed. Panel D of Fig. 10 shows a local view of the tissue section on day 10 after implantation, where connective tissue proliferation (right arrow) can be seen around the implantation site, accompanied by diffuse lymphocytes (left arrow). Based on the grading standard for inflammatory cell response, the degree of tissue inflammatory cell response on day 10 after implantation was classified as Grade II. During the implantation period, the rats exhibited normal diet, daily routine, and body weight gain. No abnormal lesions were observed in the subcutaneous and muscle tissues of the implantation site.

As the implantation time increased, the degree of inflammatory response at the implantation site gradually decreased. The histological results showed that the degree of inflammatory cell response at the implantation site was less than or equal to Grade IV in the first week and less than or equal to Grade II in the fourth week, meeting the tissue response criteria of the "GBT16175-2008 - Biological Evaluation Test Methods for Medical Silicone Materials" for implantation tests. Thus, the silicone tube material passed the implantation test.

The grading standard for inflammatory cell response is shown in the table below.

| Grade | Inflammatory cell response |
|---|---|
| I | No or very few lymphocytes observed around the sample |
| II | A small number of lymphocytes observed around the sample |
| III | A small number of neutrophils, lymphocyte infiltration, and giant cell response observed around the sample |
| IV | Inflammatory response mainly characterized by neutrophil infiltration, with phagocytes observed around the sample |

The biocompatibility of the silicone tube was evaluated through acute toxicity test, hemolysis test, irritation test, and implantation test. As a result, the silicone tube has good biocompatibility, laying a foundation for the preparation of future implants.

**(III) Preparation and evaluation of long-acting levonorgestrel implants**

Silicone rubber, known for its good permeability and biological inertness, serves as a carrier for long-acting contraceptive implants. These implants can slowly and constantly release the contained drugs, maintaining long-term contraceptive efficacy while avoiding the first-pass effect of the liver.

This section focuses on the preparation and *in vitro* release testing of long-acting levonorgestrel implants. The factors affecting the *in vitro* release rate of the levonorgestrel implants were investigated. A long-term release experiment was conducted to compare the homemade preparations with commercially available implants, and the conditions for accelerated experiments were also examined.

1. Instruments and drugs

1.1 Instruments

| LC-10ATVP High Performance Liquid Chromatograph | Shimadzu Corporation, Japan |
|---|---|
| AG245 Ultra-micro Electronic Analytical Balance | Mettler Toledo, Switzerland |
| KQ-250DE CNC Ultrasonic Cleaner | Kunshan Ultrasonic Instrument Co., Ltd. |
| TS-100B Thermostatic Shaker | Shanghai Jiecheng Experimental Instrument Co., Ltd. |

1.2 Drugs and reagents

| LNG (Batch No.: 20190327, Purity > 98%) | Hebei Mokai Technology Development Co., Ltd. |
|---|---|
| Anhydrous ethanol (analytical grade) | Shandong Yuwang Chemical Reagent Co., Ltd. |
| Chromatographic methanol | Shandong Yuwang Chemical Reagent Co., Ltd. |
| Levonorgestrel silicone rod (I) | Liaoning Ludan Pharmaceutical Co., Ltd. |
| KN-300N adhesive | Kanglibang Polymer New Materials Co., Ltd. |

2. Preparation and *in vitro* release testing of long-acting levonorgestrel implants

2.1 Preparation of long-acting levonorgestrel implants

2.1.1 Treatment of silicone tubes

Addition-cure silicone tubes (outer diameter of 2.4 mm, inner diameter of 1.6 mm) with qualified appearance and performance were cut into the same length of 35 mm. The tubes were placed into a 100 mL beaker, with an appropriate amount of detergent and distilled water added. The mixture was subjected to ultrasonication for 30 minutes. After ultrasonication, the tubes were rinsed three times with distilled water, then washed with 75% ethanol, and naturally air-dried for later use.

| Material name | Amount |
|---|---|
| Methyl vinyl silicone rubber | 100 PHR |
| Content of vinyl groups in vinyl polysiloxane | 0.17% |
| Fumed white carbon black | 35 PHR |
| Hydrogen-containing silicone oil | 1.01 PHR |
| Molar ratio of Si-H groups in hydrogen-containing silicone oil to vinyl groups in methyl vinyl silicone rubber | 1.2:1 |

(continued)

| Material name | Amount |
|---|---|
| Content of hydrogen in hydrogen-containing silicone oil | 0.75% |
| 2-Methyl-3-butyn-2-ol | 0.7 PHR |
| Platinum catalyst (3000 ppm) | 0.00001 PHR |

The specific process conditions were as follows: the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; other process conditions followed the details provided in section 2.1.2 Preparation process under "(II) Preparation of two-component addition-cure silicone tubes".

Medicinal core preparation: levonorgestrel powder with a particle size of 2.12 $\mu$m after micronization.

2.1.2 Preparation of implants

One end of the silicone tube was folded, and the tube was precisely filled with 36 mg of LNG (levonorgestrel) powder through the other end using a tool (a medicinal core length of 30 mm). Both ends of the silicone tube were then sealed with adhesive. After the adhesive was cured, the silicone tube was kneaded and shaken to ensure even distribution of the drug within the tube. The drug powder adhered to the surface of the silicone tube was then washed off. The above steps were repeated six times to obtain a set of levonorgestrel implants (the wall thickness of the levonorgestrel implants used herein was (2.4 - 1.6) / 2 = 0.4 mm, and the drug release area was calculated according to the formula $S = \pi d \times L$, where d is the inner diameter of the silicone tube and L is the length of the drug-loaded segment).

2.2 *In vitro* release testing of long-acting levonorgestrel implants

The *in vitro* release testing of long-acting levonorgestrel implants was conducted according to the method described in the section *"In vitro* release rate determination method" of the first part.

The *in vitro* release rate of implants is a key indicator for quality control, which determines the contraceptive efficacy and provides a basis for subsequent clinical medication.

3. Experimental methods and results

3.1 Study on factors affecting *in vitro* release rate of levonorgestrel implants

3.1.1 Effect of silicone tube preparation on *in vitro* release rate of implants

The silicone tube, serving as the functional membrane of the implant, was cross-linked by vinyl-terminated methyl vinyl silicone rubber and hydrogen-containing silicone oil in the presence of additives. Since the drug needs to diffuse outward through the silicone tube, the properties of the tube itself also affect drug release. Clean silicone tubes with qualified physical and mechanical properties were selected to prepare implants of the same specifications. After depowdering pre-treatment, the implants were placed in a shaker for release testing, with continuous sampling for 35 days to investigate the effect of the silicone tube preparation on the *in vitro* release rate of the implants.

3.1.1.1 Effect of vinyl content on *in vitro* release rate of implants

Silicone tubes were extruded using raw rubber with a vinyl content of 0.17% and 0.23%. The implants were prepared according to the section "2.1 Preparation of long-acting levonorgestrel implants" of this part for *in vitro* release testing.

| Material name | Example 16-1 | Example 16-2 |
|---|---|---|
| Methyl vinyl silicone rubber | 100 PHR | 100 PHR |
| Content of vinyl groups in vinyl polysiloxane | 0.17 mol% | 0.23 mol% |
| Fumed white carbon black | 35 PHR | 35 PHR |

(continued)

| Material name | Example 16-1 | Example 16-2 |
|---|---|---|
| Hydrogen-containing silicone oil | 1.01 PHR | 1.36 PHR |
| Molar ratio of Si-H groups in hydrogen-containing silicone oil to vinyl groups in methyl vinyl silicone rubber | 1.2:1 | 1.2:1 |
| Content of hydrogen in hydrogen-containing silicone oil | 0.75 mol% | 0.75 mol% |
| 2-Methyl-3-butyn-2-ol | 0.7 PHR | 0.7 PHR |
| Platinum catalyst (3000 ppm) | 0.00001 PHR | 0.00001 PHR |

The daily dose release of the silicone tubes corresponding to Examples 16-1 and 16-2 is shown in the table below.

| Time/d | Daily dose release ($\mu$g) | |
|---|---|---|
| | Example 16-1 | Example 16-2 |
| 1 | 50.66 | 41.89 |
| 2 | 52.11 | 42.26 |
| 3 | 47.13 | 39.39 |
| 4 | 48.04 | 38.42 |
| 5 | 51.12 | 40.72 |
| 6 | 54.71 | 36.72 |
| 7 | 51.08 | 39.68 |
| 8 | 53.61 | 40.83 |
| 9 | 56.56 | 36.16 |
| 10 | 54.27 | 42.52 |
| 11 | 52.16 | 40.91 |
| 12 | 50.02 | 36.44 |
| 13 | 49.94 | 39.85 |
| 14 | 52.10 | 37.69 |
| 15 | 50.98 | 35.06 |
| 16 | 55.15 | 41.43 |
| 17 | 51.43 | 37.20 |
| 18 | 53.34 | 38.06 |
| 19 | 55.35 | 34.55 |
| 20 | 55.46 | 36.45 |
| 21 | 54.23 | 39.15 |
| 22 | 52.23 | 40.05 |
| 23 | 53.11 | 37.95 |
| 24 | 48.66 | 35.80 |
| 25 | 50.40 | 36.75 |
| 26 | 49.64 | 42.07 |
| 27 | 52.03 | 40.61 |
| 28 | 49.80 | 39.84 |
| 29 | 55.35 | 37.20 |

(continued)

| Time/d | Daily dose release (μg) | |
|---|---|---|
| | Example 16-1 | Example 16-2 |
| 30 | 54.46 | 38.06 |

The experimental results, as shown in the table above, indicated that both groups of implants exhibited stable release profiles. The implants prepared with raw rubber with a vinyl content of 0.23% had a lower daily release compared to those prepared with raw rubber with a vinyl content of 0.17%.

During the release process, the small drug molecules moved outward through the silicone tube wall. The drug dissolved in the membrane diffused to the interface between the membrane and the release medium, and eventually distributed and dissolved into the receptor. The density of cross-linking points within the silicone tube influenced the relaxation process of macromolecular chain segments, thereby controlling the diffusion behavior of the drug through the cross-linking network. That is, the cross-linking network of macromolecules within the silicone tube had a "sieving effect" on drug diffusion, where a higher cross-linking density, with more cross-linking points, resulted in a shorter molecular chain length and more difficult chain segment movement. This reduced the ability of the drug to penetrate the inter-chain gaps, decreased the solubility of the drug in the membrane, and lowered the drug release rate.

3.1.1.2 Effect of amount of hydrogen-containing silicone oil on *in vitro* release rate of implants

*In vitro* release testing was conducted on silicone tubes prepared by adding different amounts of hydrogen-containing silicone oil (value of A of 1, 1.2, and 1.5 for the addition of hydrogen-containing silicone oil). The preparation is shown in the table below.

| Material name | Example 17-1 | Example 17-2 | Example 17-3 |
|---|---|---|---|
| Methyl vinyl silicone rubber | 100 PHR | 100 PHR | 100 PHR |
| Content of vinyl groups in vinyl polysiloxane | 0.17 mol% | 0.17 mol% | 0.17 mol% |
| Fumed white carbon black | 35 PHR | 35 PHR | 35 PHR |
| Hydrogen-containing silicone oil | 0.84 PHR | 1.01 PHR | 1.26 PHR |
| Molar ratio of Si-H groups in hydrogen-containing silicone oil to vinyl groups in methyl vinyl silicone rubber | 1.0:1 | 1.2:1 | 1.5:1 |
| Content of hydrogen in hydrogen-containing silicone oil | 0.75 mol% | 0.75 mol% | 0.75 mol% |
| 2-Methyl-3-butyn-2-ol | 0.7 PHR | 0.7 PHR | 0.7 PHR |
| Platinum catalyst (3000 ppm) | 0.00001 PHR | 0.00001 PHR | 0.00001 PHR |

The daily dose release of the silicone tubes corresponding to Examples 17-1, 17-2 and 17-3 is shown in the table below.

| Time/d | Daily dose release (μg) | | |
|---|---|---|---|
| | Example 17-1 | Example 17-2 | Example 17-3 |
| 1 | 61.71 | 55.13 | 50.66 |
| 2 | 61.60 | 57.02 | 52.11 |
| 3 | 59.05 | 58.74 | 46.13 |
| 4 | 63.20 | 57.72 | 48.04 |
| 5 | 61.01 | 59.12 | 51.12 |
| 6 | 59.05 | 57.36 | 54.71 |
| 7 | 57.96 | 53.56 | 51.08 |
| 8 | 63.23 | 57.39 | 53.61 |

(continued)

| Time/d | Daily dose release (µg) | | |
| --- | --- | --- | --- |
| | Example 17-1 | Example 17-2 | Example 17-3 |
| 9 | 62.19 | 54.30 | 56.56 |
| 10 | 55.77 | 55.56 | 54.27 |
| 11 | 56.16 | 55.69 | 52.16 |
| 12 | 60.11 | 55.51 | 50.02 |
| 13 | 59.91 | 60.45 | 49.94 |
| 14 | 62.65 | 53.57 | 52.10 |
| 15 | 55.97 | 54.39 | 50.98 |
| 16 | 62.90 | 55.55 | 55.15 |
| 17 | 62.25 | 53.35 | 51.44 |
| 18 | 61.28 | 51.78 | 53.34 |
| 19 | 57.05 | 54.43 | 56.35 |
| 20 | 62.64 | 52.56 | 55.46 |
| 21 | 57.99 | 56.39 | 54.23 |
| 22 | 59.87 | 54.13 | 52.23 |
| 23 | 60.21 | 54.38 | 53.11 |
| 24 | 61.07 | 57.56 | 48.66 |
| 25 | 59.96 | 58.39 | 50.40 |
| 26 | 58.82 | 53.30 | 49.64 |
| 27 | 58.05 | 55.43 | 52.03 |
| 28 | 55.52 | 54.70 | 49.80 |
| 29 | 61.16 | 51.07 | 55.35 |
| 30 | 59.20 | 49.35 | 54.46 |

The amount of hydrogen-containing silicone oil did not significantly affect the release rate of LNG. When the value of A was 1.5, the release rate of the implant was slightly lower than when the value of A was 1 and 1.2. This was primarily due to the varying degrees of cross-linking within the silicone tube, which resulted in different diffusion rates of the drug in the silicone tube.

3.1.1.3 Effect of amount of white carbon black on *in vitro* release rate of implants

The release rate was investigated for silicone tubes with different amounts of white carbon black. The preparation is shown in the table below.

| Material name | Example 18-1 | Example 18-2 | Example 18-3 |
| --- | --- | --- | --- |
| Methyl vinyl silicone rubber | 100 PHR | 100 PHR | 100 PHR |
| Content of vinyl groups in vinyl polysiloxane | 0.17 mol% | 0.17 mol% | 0.17 mol% |
| Fumed white carbon black | 30 PHR | 35 PHR | 40 PHR |
| Hydrogen-containing silicone oil | 1.01 PHR | 1.01 PHR | 1.01 PHR |
| Molar ratio of Si-H groups in hydrogen-containing silicone oil to vinyl groups in methyl vinyl silicone rubber | 1.2:1 | 1.2:1 | 1.2:1 |
| Content of hydrogen in hydrogen-containing silicone oil | 0.75 mol% | 0.75 mol% | 0.75 mol% |

(continued)

| Material name | Example 18-1 | Example 18-2 | Example 18-3 |
|---|---|---|---|
| 2-Methyl-3-butyn-2-ol | 0.7 PHR | 0.7 PHR | 0.7 PHR |
| Platinum catalyst (3000 ppm) | 0.00001 PHR | 0.00001 PHR | 0.00001 PHR |

The daily dose release of the silicone tubes corresponding to Examples 18-1, 18-2 and 18-3 is shown in the table below.

| Time/d | Daily dose release (μg) | | |
|---|---|---|---|
| | Example 18-1 | Example 18-2 | Example 18-3 |
| 1 | 58.08 | 50.66 | 47.56 |
| 2 | 53.49 | 52.11 | 48.56 |
| 3 | 52.02 | 46.12 | 45.32 |
| 4 | 60.81 | 48.04 | 43.12 |
| 5 | 55.14 | 51.12 | 44.02 |
| 6 | 58.11 | 54.71 | 41.94 |
| 7 | 56.44 | 51.08 | 39.66 |
| 8 | 56.84 | 53.61 | 43.40 |
| 9 | 59.66 | 56.56 | 42.64 |
| 10 | 51.98 | 54.27 | 44.03 |
| 11 | 56.97 | 52.16 | 41.80 |
| 12 | 56.77 | 50.02 | 45.35 |
| 13 | 54.99 | 49.94 | 43.80 |
| 14 | 57.02 | 52.10 | 42.35 |
| 15 | 57.81 | 50.98 | 47.46 |
| 16 | 61.34 | 55.15 | 44.23 |
| 17 | 60.68 | 51.43 | 46.34 |
| 18 | 59.53 | 53.34 | 49.23 |
| 19 | 57.91 | 56.35 | 44.34 |
| 20 | 58.18 | 55.46 | 45.64 |
| 21 | 59.40 | 54.23 | 45.03 |
| 22 | 56.98 | 52.24 | 42.80 |
| 23 | 60.26 | 53.11 | 40.35 |
| 24 | 61.86 | 48.66 | 39.80 |
| 25 | 59.10 | 50.40 | 45.11 |
| 26 | 64.89 | 49.64 | 43.66 |
| 27 | 63.40 | 52.03 | 44.70 |
| 28 | 59.20 | 49.80 | 42.65 |
| 29 | 57.99 | 55.35 | 46.66 |
| 30 | 57.79 | 54.46 | 43.48 |

The amount of white carbon black slightly affected the release of the implant. As the amount of white carbon black increased, the release rate of the implant slightly decreased.

In summary, the preparation preparation of the silicone tube affected the release of the implant, primarily due to the varying degrees of cross-linking within the silicone tube, which altered the solubility of small drug molecules in the silicone tube. However, the drug was able to release stably in these silicone tubes with different preparations. The difference in the diffusion rate of the drug in the silicone tubes was not significant. There was neither an excessively low diffusion rate causing the drug to not release nor an excessively high release rate causing burst release. Therefore, the silicone tube with the best mechanical properties was preferred (preparation: raw rubber with a vinyl content of 0.17% at 100 PHR, white carbon black at 35 PHR, hydrogen-containing silicone oil with value of A of 1.2, catalyst at $10^{-5}$ PHR, and inhibitor at 0.7 PHR) to prepare the implant, which better ensured the long-term stable release of the implant *in vivo.*

3.1.2 Effect of depowdering on *in vitro* release rate of implants

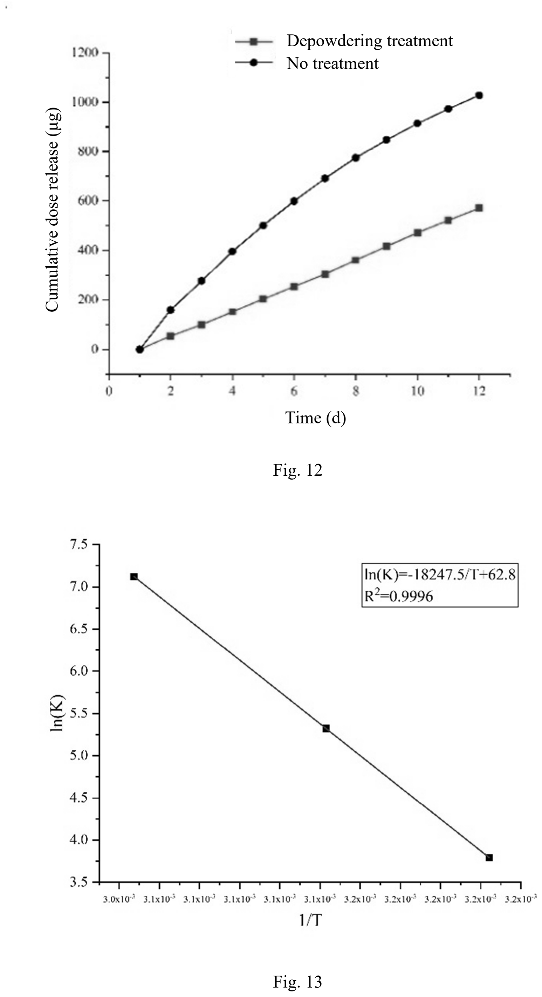

Two groups of levonorgestrel implants were prepared according to the section "2.1 Preparation of long-acting levonorgestrel implants" of this part. One group was directly placed into a shaker for *in vitro* release testing without any treatment. The other group was added to a stoppered conical flask with 50 mL of anhydrous ethanol and ultrasonicated for 1 minute. This process was repeated three times, after which 100 mL of distilled water was added and left overnight. The next day, the soaking solution was discarded, and the implant was placed in a shaker for *in vitro* release testing. Daily sampling at regular intervals and replacement of the release medium were performed for 12 consecutive days to observe the *in vitro* release rate of the implant. The experimental results are shown in the table below and Figs. 11 and 12.

As shown in Figs. 11 and 12, the implant without depowdering pre-treatment had high and unstable daily release during the first 12 days, showing a very significant downward trend. The relative burst release of the implant was expressed by comparing the release on the first day to the average daily release (Day 1 release/Mean release). It was found that the relative burst release of the depowdered implants was close to 1, that is, the depowdered levonorgestrel implants had a very smooth daily release from the initial release. The cumulative drug release (Qc) of the depowdered implants was linearly related to the release time (t) ($R^2 = 0.996$), and the release rate conformed to zero-order release kinetics, with no significant fluctuations in the daily release curve.

The presence of drug powder electrostatically adsorbed by the silicone tube on the surface of the levonorgestrel implants without depowdering treatment caused a very noticeable burst release effect of the release of the implants. In order to bring the initial release close to the steady-state release, reduce the observation time of *in vitro* release rate, and lower the risk of burst release after implanting the implants *in vivo,* appropriate treatment of the implants was required. After the depowdering treatment, the initial release of the implants was close to steady-state release.

The release data of LNG implants are shown in the table below.

| Implant type | Day 1 release ($\mu g$) | Mean release ($\mu g$) | Day 1 release/Mean release | $Q_C$/t Equation | $R^2$ |
|---|---|---|---|---|---|
| Depowdering treatment | 51.56 | 52.24 | 0.99 | Qc = 29.63t + 23.35 | 0.997 |
| Without treatment | 159.23 | 98.92 | 1.61 | Qc = 91.83t - 0.24 | 0.977 |
| Note: $Q_C$ represents cumulative drug release. | | | | | |

3.2 Comparison of levonorgestrel implants with commercially available preparations

The commercially available levonorgestrel silicone rod I contains six drug-loaded silicone rods, each containing 36 mg of LNG powder, with a total drug loading of 216 mg. The silicone tube has an outer diameter of 2.4 mm, a wall thickness of 0.4 mm, and a medicinal core length of 30 mm. Based on the results of the investigation of the influencing factors of the *in vitro* release testing, 2.4 mm $\times$ 1.6 mm $\times$ 35 mm $\times$ 6 silicone tubes were selected to prepare six levonorgestrel implants with a medicinal core length of 30 mm and a drug loading of 216 mg. Both the commercially available preparations (levonorgestrel silicone rod I, purchased from Liaoning Ludan Pharmaceutical Co., Ltd.) and levonorgestrel implants were subjected to depowdering treatment and then to a 100-day *in vitro* release test.

During the 100-day release period, the daily release profile of the implants is shown in the table below. The daily release of both the homemade preparations (silicone tubes corresponding to Example 18-2) and the commercially available preparations was stable, with no significant burst release or reduction in release observed.

The *in vitro* release data of the homemade and commercially available LNG preparations are shown in the table below.

| / | Mean ($\mu$g) | RSD (%) | $Q_C$/t equation | $R^2$ | Relationship between daily drug release and medicinal core side area |
|---|---|---|---|---|---|
| Homemade preparation | 49.02 | 7.19 | Qc = 52.54t - 5.59 | 0.997 | y = 5.467x - 0.4617 |
| Commercially available preparation | 45.76 | 6.70 | Qc = 42.50t - 0.61 | 0.998 | y = 5.022x - 0.3547 |
| Note: $Q_C$ represents cumulative drug release. | | | | | |

As shown in the table below, the *in vitro* release rate of the homemade preparation was not significantly different from that of the commercially available preparation. During the *in vitro* release experiment, the RSD value of the daily drug release for the homemade preparation was 7.19%, while the RSD value of the daily drug release for the commercially available preparation was 6.70%, indicating that the release stability of both preparations is relatively similar, and both exhibit zero-order release behavior.

| Time (d) | Homemade preparation Daily dose release ($\mu$g) | Commercially available preparation Daily dose release ($\mu$g) |
|---|---|---|
| 1 | 50.66 | 48.38 |
| 2 | 52.11 | 53.63 |
| 3 | 46.12 | 50.59 |
| 4 | 48.04 | 49.66 |
| 5 | 51.12 | 43.15 |
| 6 | 54.71 | 47.86 |
| 7 | 51.08 | 47.38 |
| 8 | 53.61 | 45.93 |
| 9 | 56.56 | 46.10 |
| 10 | 54.27 | 46.83 |
| 11 | 52.16 | 46.82 |
| 12 | 50.02 | 45.23 |
| 13 | 49.94 | 50.59 |
| 14 | 52.10 | 49.66 |
| 15 | 50.98 | 43.15 |
| 16 | 55.15 | 50.59 |
| 17 | 51.43 | 49.66 |
| 18 | 53.34 | 43.15 |
| 19 | 55.35 | 47.86 |
| 20 | 55.46 | 47.38 |
| 21 | 54.23 | 47.38 |
| 22 | 52.23 | 45.93 |
| 23 | 53.11 | 46.10 |
| 24 | 48.66 | 46.83 |
| 25 | 50.40 | 46.82 |
| 26 | 49.64 | 45.23 |
| 27 | 52.03 | 50.59 |
| 28 | 49.80 | 49.66 |

(continued)

| Time (d) | Homemade preparation | Commercially available preparation |
|---|---|---|
| | Daily dose release (μg) | Daily dose release (μg) |
| 29 | 55.35 | 52.94 |
| 30 | 54.46 | 50.77 |
| 31 | 50.23 | 45.93 |
| 32 | 45.34 | 46.58 |
| 33 | 48.11 | 49.69 |
| 34 | 46.66 | 47.77 |
| 35 | 50.48 | 49.23 |
| 36 | 50.40 | 45.29 |
| 37 | 49.64 | 43.68 |
| 38 | 49.65 | 45.20 |
| 39 | 53.61 | 47.99 |
| 40 | 48.11 | 49.79 |
| 41 | 46.66 | 48.29 |
| 42 | 43.70 | 49.81 |
| 43 | 49.65 | 45.50 |
| 44 | 47.43 | 45.94 |
| 45 | 49.10 | 45.12 |
| 46 | 47.98 | 44.29 |
| 47 | 55.15 | 42.22 |
| 48 | 47.43 | 41.64 |
| 49 | 53.25 | 44.36 |
| 50 | 49.64 | 45.08 |
| 51 | 47.03 | 44.25 |
| 52 | 51.80 | 44.45 |
| 53 | 50.35 | 45.34 |
| 54 | 52.46 | 46.31 |
| 55 | 54.23 | 41.67 |
| 56 | 53.34 | 42.92 |
| 57 | 48.11 | 44.55 |
| 58 | 46.66 | 43.73 |
| 59 | 47.70 | 42.71 |
| 60 | 49.65 | 41.47 |
| 61 | 47.43 | 40.46 |
| 62 | 49.55 | 45.83 |
| 63 | 47.86 | 42.49 |
| 64 | 49.10 | 40.61 |
| 65 | 47.98 | 46.50 |
| 66 | 50.15 | 43.39 |

(continued)

| Time (d) | Homemade preparation | Commercially available preparation |
|---|---|---|
| | Daily dose release ($\mu$g) | Daily dose release ($\mu$g) |
| 67 | 47.43 | 46.15 |
| 68 | 46.34 | 48.24 |
| 69 | 50.35 | 45.70 |
| 70 | 50.46 | 51.26 |
| 71 | 47.23 | 50.60 |
| 72 | 45.23 | 44.37 |
| 73 | 46.11 | 48.42 |
| 74 | 45.66 | 48.49 |
| 75 | 43.40 | 41.74 |
| 76 | 42.64 | 46.81 |
| 77 | 50.03 | 38.63 |
| 78 | 47.80 | 41.25 |
| 79 | 47.35 | 45.34 |
| 80 | 45.80 | 46.31 |
| 81 | 44.35 | 41.67 |
| 82 | 44.46 | 42.92 |
| 83 | 47.23 | 44.55 |
| 84 | 46.34 | 45.06 |
| 85 | 47.23 | 44.86 |
| 86 | 48.34 | 41.70 |
| 87 | 45.64 | 40.19 |
| 88 | 45.03 | 40.59 |
| 89 | 42.80 | 44.47 |
| 90 | 46.35 | 48.81 |
| 91 | 46.80 | 45.84 |
| 92 | 45.11 | 45.22 |
| 93 | 43.66 | 45.76 |
| 94 | 44.70 | 45.48 |
| 95 | 42.65 | 45.70 |
| 96 | 46.66 | 40.76 |
| 97 | 43.48 | 44.74 |
| 98 | 43.35 | 43.62 |
| 99 | 45.46 | 43.89 |
| 100 | 44.03 | 40.66 |

4. *In vitro* accelerated experiment

The levonorgestrel implant needs to maintain an effective drug concentration in the body for several years. Therefore, any rapid release or unexpected changes in the release of the preparation in the body can lead to severe adverse reactions. For this long-acting implant, screening preparations through real-time drug release requires a long period of time, which is relatively inconvenient. Therefore, finding an appropriate method for accelerated drug release is of great importance the preparation optimization and quality control of preparations. Elevating temperature is widely used in *in vitro* accelerated experiments of preparations. However, the accelerated experiment needs to be able to accurately predict real-time release. Thus, it is also necessary to determine whether there is a correlation between the accelerated release and the normal release.

### 4.1 Experimental methods

A clean silicone tube (corresponding to the preparation in Example 18-2; the front drying tunnel temperature was set at 270°C, with a vulcanization time of approximately 5 seconds; the rear drying tunnel temperature was set at 180°C, with a vulcanization time of approximately 2 minutes; the oven vulcanization temperature was set at 180°C, with a vulcanization time of 48 hours; medicinal core preparation: levonorgestrel powder with a particle size of 2.12 $\mu$m after micronization) with an outer diameter of 2.4 mm, an inner diameter of 1.6 mm, and a length of 35 mm (the length of the silicone tube was 35 mm, the length of the pure medicinal core was 30 mm, and the extra 5 mm was the length of the end-sealing adhesive at both ends) was used to prepare the implant. Using the drug release at 37°C as a reference, the release of LNG at 45°C and 55°C was investigated. According to the normal release conditions, using 100 mL of water as the medium, release samples were taken and the medium was replaced every 1-3 hours, with three sets of experiments conducted in parallel at each temperature.

### 4.2 Experimental results

The release data for *in vitro* release of LNG implants at different temperatures are shown in the table below.

| Release temperature (°C) | $Q_C$/t equation | K | $R^2$ |
|---|---|---|---|
| 37 | $Q_C = 52.54t - 5.59$ | 52.54 | 0.996 |
| 45 | $Q = 221.54t + 2.27$ | 221.54 | 0.997 |
| 55 | $Q = 1271.09t + 4.35$ | 1271.09 | 0.991 |
| Note: $Q_C$ represents cumulative drug release. | | | |

The increase in the release rate of LNG with increasing temperature is related to the Arrhenius equation, which is shown below:

$$K = A \times e^{-Ea/RT} \quad \text{(Equation 3-1)}$$

wherein K is the zero-order release rate, A is a constant, Ea is the activation energy, R is the gas constant, and T is the absolute temperature. Taking the natural logarithm in Equation 3-1 yields the following equation:

$$ln(K) = -\frac{Ea}{R} \times \frac{1}{T} + \ln(A) \quad \text{(Equation 3-2)}$$

wherein K is calculated based on the drug release amount at different temperatures; the plot of ln(K) versus 1/T yields a straight line, with the slope of the line being -Ea/2.303R. Based on the straight line plotted by applying the Arrhenius equation at multiple temperatures set in the accelerated temperature experiment, the predicted release rate at 37°C was read out and compared with the actual release rate obtained from the 37°C release experiment. The comparison was used to examine whether the release rate from the accelerated temperature experiment could predict the actual release rate. Therefore, the equation ln(K) = -18841.7 + 64.507 ($R^2$ = 0.999) was obtained. The linear relationship between ln(K) and 1/T is shown in Fig. 13. Using this equation, the predicted release rate at 37°C, $K_{37°C}$ = 51.94 $\mu$g, was calculated, which was close to the actual release amount of the implant at 37°C. This indicated a good correlation between the accelerated release and the normal release.

From the data in the above table, it can be seen that the release rate of LNG at 45°C was approximately 4.5 times that at 37°C, and the release rate of LNG at 55°C was approximately 28 times that at 37°C. Specifically, the 24-hour release amount at 37°C was approximately equal to the 5.2-hour release amount at 45°C, and the 24-hour release amount at 37°C was approximately equal to the 51-minute release amount at 55°C, which greatly reduced the time required for the *in vitro* release test. Therefore, elevating the release temperature can be used to increase the *in vitro* release amount of LNG and reduce the experimental time for the *in vitro* release test.

## (IV) *In vivo* pharmacodynamic study of long-acting levonorgestrel implants

This section focuses on the pharmacodynamic study of levonorgestrel implants in rats. Subcutaneous implants of different specifications were implanted in rats. The estrous cycle changes in SD rats post-implantation were observed through vaginal smears, the LH (luteinizing hormone) levels in the rats were measured using the enzyme-linked immunosorbent assay, and the presence or absence of mating behavior in the rats was monitored to study the contraceptive effect of subcutaneous implantation of levonorgestrel.

### 1. Instruments and reagents

#### 1.1 Instruments

| | |
|---|---|
| KQ-250DE CNC Ultrasonic Cleaner | Kunshan Ultrasonic Instrument Co., Ltd. |
| Rat LH Assay Kit | Shanghai Tongwei Biotechnology Co., Ltd. |
| Electron Microscope | Shenzhen Zhongwei Kechuang Technology Co., Ltd. |
| Levonorgestrel silicone rod (I) | Liaoning Ludan Pharmaceutical Co., Ltd. |
| Autoclave | Shanghai Shenan Medical Instrument Factory |
| XS105 Electronic Analytical Balance | Mettler-Toledo Instruments Co., Ltd. |
| SMD200-2 Electronic Analytical Balance | Ohaus International Trade Co., Ltd. |
| TG16MW Desktop High-Speed Centrifuge | Hunan Herexi Instrument and Equipment Co., Ltd. |

#### 1.2 Reagents

| | |
|---|---|
| LNG (Batch No.: 20190327, Purity > 98%) | Hebei Mokai Technology Development Co., Ltd. |
| Levonorgestrel silicone rod (I) | Liaoning Ludan Pharmaceutical Co., Ltd. |
| Heparin sodium | Shandong Yuwang Pharmaceutical Co., Ltd. |
| 4% paraformaldehyde solution | Shanghai Yantuo Biotechnology Co., Ltd. |
| Normal saline | Shandong Yuwang Chemical Reagent Co., Ltd. |

#### 1.3 Experimental animals

SD rats (License No. SCXK (Liao) 2020-0001), Liaoning Changsheng Biotechnology Co., Ltd.

### 2. Experimental methods

#### 2.1 Administration method

2.4 mm × 1.6 mm silicone tubes were used to prepare five specifications of levonorgestrel implants (preparation corresponding to Example 18-2, the front drying tunnel temperature was set at 270°C, with a vulcanization time of approximately 5 seconds; the rear drying tunnel temperature was set at 180°C, with a vulcanization time of approximately 20 seconds; the oven vulcanization temperature was set at 180°C, with a vulcanization time of 48 hours; medicinal core preparation: levonorgestrel powder with a particle size of 2.12 μm after micronization) (medicinal core length/drug loading) for later use. The five specifications were as follows: I (3 mm/3.6 mg), II (10 mm/12 mg), III (15 mm/18 mg), IV (20 mm/24 mg), V (30 mm/36 mg).

The specific preparations are shown in the table below.

| Material name | Amount |
|---|---|
| Methyl vinyl silicone rubber | 100 PHR |
| Content of vinyl groups in vinyl polysiloxane | 0.17% |
| Fumed white carbon black | 35 PHR |
| Molar ratio of Si-H groups in hydrogen-containing silicone oil to vinyl groups in methyl vinyl silicone rubber | 1.2:1 |
| Content of hydrogen in hydrogen-containing silicone oil | 0.75% |
| 2-Methyl-3-butyn-2-ol | 0.7 PHR |
| Platinum catalyst (3000 ppm) | 0.00001 PHR |

Fourteen healthy male SD rats weighing approximately 200 g were randomly divided into 7 groups for later use. Forty-two healthy female SD rats weighing approximately 200 g were randomly divided into 7 dose groups, namely the negative control group, the positive control group, and 5 dosing groups. The positive control group used a levonorgestrel silicone rod I with a medicinal core length of 30 mm (from Liaoning Ludan Pharmaceutical Co., Ltd.). The negative control group used a silicone tube with an outer diameter of 2.40 mm and a wall thickness of 0.5 mm, which was cut into 15 mm segments and sealed at both ends for later use. All preparations and silicone tubes were sterilized in an autoclave (121°C, 30 minutes) for later use.

The implantation method for the dosing groups followed the method described in "2.3 *In vivo* pharmacodynamic experiment of gestodene contraceptive implants in rats" under "I. Long-acting gestodene contraceptive implants; (IV) *In vivo* experiment of gestodene contraceptive implants in rats". Twenty-four hours after the implantation surgery, the female rats in both the negative control group and the dosing groups were housed with the male rats, with 6 female rats and 2 male rats per cage.

2.2 Observation of rat vulva and preparation of vaginal smears

To monitor the estrus, mating, and pregnancy of the rats, the characteristics of the vulva were observed and recorded daily at 9:00 a.m. for 30 consecutive days post-administration. Vaginal smears of the rats were prepared to observe and record the characteristics of the vaginal exfoliated epithelial cells.

2.2.1 Preparation method of rat vaginal smears

0.1 g of crystal violet powder was weighed and dissolved in 100 mL of distilled water to prepare a 0.1% gentian violet solution for later use. Clean anti-detachment slides were labeled for later use. Cotton swabs were moistened with sterile saline for later use. A female rat was taken out of the cage and placed on the cage cover. The tail of the rat was held and gently lifted to expose the vaginal opening. The vaginal opening was gently wiped clean of urine with a cotton swab. The pre-moistened cotton swab was then inserted into the vagina of the rat for approximately 0.5 cm and gently rotated 1-2 times. The cotton swab was then evenly smeared onto the anti-detachment slide for smearing. After the anti-detachment slide smeared with rat vaginal exfoliated cells was air-dried, 50 μL of anhydrous ethanol was dropwise added to fix the slide for 10 minutes. 50 μL of gentian violet solution was then dropwise added to the slide and left for 1 minute. The slide was immersed in distilled water for 1 minute, then rinsed with distilled water for 10 seconds, and naturally air-dried. After the slide was air-dried, it was mounted with neutral balsam and then observed under an electron microscope to examine the rat vaginal smears.

2.2.2 Observation of estrous cycle in rats via vaginal smears

The identification method for the estrous cycle in rats is shown in the table below.

| / | Vaginal appearance characteristics | Cellular characteristics of vaginal smears |
|---|---|---|
| Proestrus | Vaginal opening not open or slightly open, mucosa pale pink, vulvar folds slightly red and swollen, relatively moist, small amount of clear secretion | Presence of nucleated epithelial cells and anucleate keratinocytes, with round nucleated epithelial cells being predominant |
| Estrus | Vaginal opening widely open; mucosa reddish, vulvar folds obviously swollen, relatively moist, small amount of viscous secretion | Anucleate epithelial keratinocytes in the full field of view |
| Metestrus | Vaginal opening loose or closed, mucosa pale pink, vulvar swelling gradually subsides, not very moist, small amount of serous or coagulated white secretion around | Mainly anucleate epithelial keratinocytes and leukocytes |
| Diestrus | Vaginal opening tightly closed, vaginal mucosa pale, vulvar swelling completely subsided, dry, no secretion | Large number of leukocytes, small number of anucleate epithelial keratinocytes |

2.3 Measurement of plasma LH concentration in rats using enzyme-linked immunosorbent assay

2.3.1 Blood sample collection

Starting from the third day post-administration, 100 μL of blood was collected from the orbital sinus of the rats daily at 9:30 a.m. for 28 consecutive days. The blood was placed in an EP tube pre-treated with heparin sodium, mixed thoroughly, and centrifuged in a centrifuge for 10 minutes at 5000 rpm. The supernatant was then transferred to a new EP tube and stored at -80°C for measurement.

2.3.2 Measurement of plasma LH concentration in rats using enzyme-linked immunosorbent assay

After the implantation of levonorgestrel implants in rats, the pharmacodynamic characteristics of the levonorgestrel implants were investigated by observing the plasma LH levels in rats. Serum samples were taken and the LH (luteinizing hormone) content in rat serum was detected strictly following the instructions of the enzyme-linked immunosorbent assay kit.

A standard curve was plotted using the standard concentration and OD value, and the LH concentration in the serum samples was calculated based on the standard curve.

2.3.3 Statistical analysis

Data were analyzed using SPSS 21.0 software, and experimental results were expressed as mean $\pm$ standard deviation (mean $\pm$ SD). For comparisons among multiple groups, one-way analysis of variance (One-Way ANOVA) was used to evaluate the overall variance differences. If the variances were homogeneous, LSD was used for multiple comparisons. If the variances were heterogeneous, Dunnett T3 was used for multiple comparisons. For comparisons between two groups, an unpaired t-test was used. $P < 0.05$ was considered to indicate a significant difference.

3. Results and discussion

3.1 Monitoring results of estrus and mating in rats

The estrus and mating status of the rats were determined by observing the daily vulva condition and vaginal smears of the rats in each group. During the experiment, the rats maintained normal diet, daily routine, and body weight gain. It was found that, after caging together, white gelatinous vaginal plugs were examined in the vagina of female rats in the negative control group. The vaginal plugs were formed by a mixture of semen of male rats, vaginal secretions of female rats, and vaginal epithelial cells that hardened quickly upon exposure to air, which was an important indicator of successful mating. Additionally, the vaginal smears of each female rat in the negative control group showed a complete estrous cycle. The progesterone levels in the negative control group were normal, and the rats exhibited normal estrous and mating behaviors.

In the dosing groups (II, III, IV, and V) and the positive control group, after the implantation of the preparations, the vulvar examination of the rats over a one-month period showed that the vaginal openings were tightly closed, the vulva was dry without secretions, no redness or swelling was observed, and no vaginal plugs were found. Vaginal smears revealed the presence of a large number of leukocytes, which is a typical feature of the diestrus phase, demonstrating that the rats remained in the diestrus phase without entering the normal estrous phase. However, in the dosing group I, female rats numbered 2 and 4 displayed a complete estrous cycle based on vulvar and vaginal smear examinations, indicating that the levonorgestrel implants at this dose did not provide contraceptive efficacy after implantation in these two rats.

3.2 Changes in LH levels in rats

The experimental results are shown in the table below. After the implantation of levonorgestrel implants in rats, there was a significant reduction in the LH levels in the rats. That is, the LH levels in the positive control group and different dosing groups were significantly different from those in the negative control group ($P < 0.05$). The experiment also revealed that the LH levels in rats were influenced by the dose of LNG; the higher the dose of LNG, the lower the LH levels in rats.

During the estrous cycle of rats, the secretion of LH is regulated by GnRH (gonadotropin-releasing hormone), and LH has a noticeable peak in the proestrus phase of rats. In the dosing groups and the positive control group, levonorgestrel primarily acted on the hypothalamus and pituitary gland, inhibiting the release of GnRH through a negative feedback mechanism, thereby reducing the secretion of LH, which resulted in the significant reduction or disappearance of the LH peak, affecting the growth and maturation of follicles and preventing ovulation, thus achieving the contraceptive effect. This is consistent with the observation results of vaginal smears. After the implants exerted the pharmacological effect in the rats, the rats remained in the diestrus phase. The LH peak present in the proestrus phase was inhibited by the negative feedback regulation and did not occur, preventing ovulation. Consequently, the male and female rats did not mate, thus preventing pregnancy in rats. In contrast, the LH levels in the negative control group were not inhibited, and the LH peak before estrus still existed, allowing the rats to undergo normal estrus, mating, and pregnancy.

| Group | Replicates | LH (IU/L) |
|---|---|---|
| Blank group | 6 | 78.99 $\pm$ 4.24 |
| I | 6 | 66.76 $\pm$ 7.48* |
| II | 6 | 57.66 $\pm$ 2.13* |
| III | 6 | 50.76 $\pm$ 5.23* |
| IV | 6 | 46.68 $\pm$ 3.39* |
| V | 6 | 36.61 $\pm$ 2.43* |
| Positive control group | 6 | 39.91 $\pm$ 4.75* |
| Note: * means $P < 0.05$ compared to the blank group. | | |

Additionally, the dose of the dosing group V was consistent with the dose of the positive control group. It was found that the LH levels in the rats of these two groups were also similar (Group V: LH = 36.61 $\pm$ 2.43 IU/L; positive control group: LH = 39.91 $\pm$ 4.75 IU/L). Comparing the LH levels in the rats of these two groups showed no significant difference ($P > 0.05$), indicating that the efficacy of the implant prepared in this study was equivalent to that of the commercially available levonorgestrel silicone rod I.

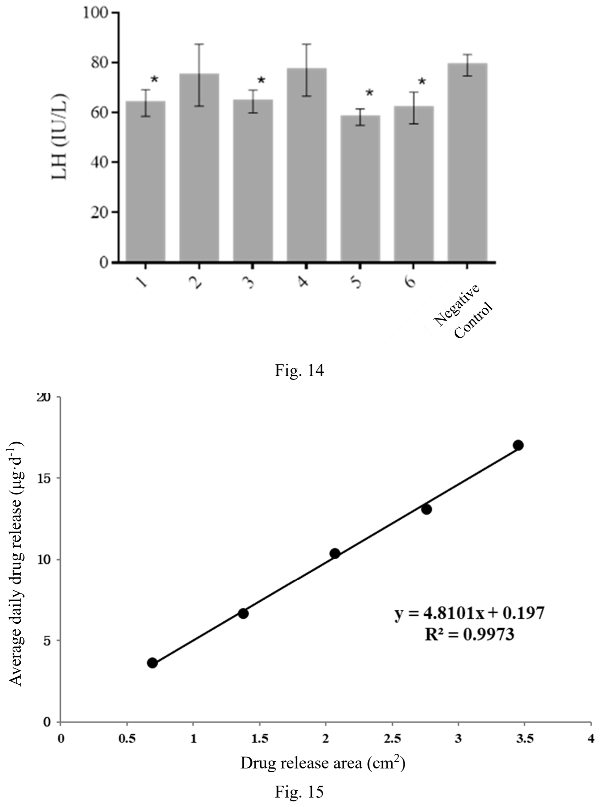

Moreover, in the vaginal smear examination of the rats in dosing group I, it was found that two rats exhibited estrous cycles. The LH measurement results of the rats in this group are shown in Fig. 14. It was found that the LH levels of the two rats numbered 2 and 4 were close to the LH levels of the rats in the negative control group, whereas the LH levels of the other rats were significantly different from those of the rats in the negative control group ($P < 0.05$). Combined with the vaginal smear examination results, it indicated that the levonorgestrel implants in these two rats did not produce a pharmacological effect. It is speculated that the reason is that the implant of this specification (3 mm/3.6 mg), due to the short length of the medicinal core, may have experienced infiltration of the adhesive into the drug powder in some implants during preparation, making drug release difficult and insufficient. Consequently, the implant did not achieve the contraceptive effect after implantation in the rats.

4. Summary

(1) The experiment assessed the presence of the estrous cycle and mating behavior in rats by observing the vulva and vaginal smears of rats in different groups. The efficacy of the levonorgestrel implant was evaluated by measuring the LH levels of rats in different groups. The results showed that the LH levels in groups I, II, III, IV, V, and the positive control group were significantly lower than those in the negative control group (P < 0.05), and the LH levels in the rats decreased with the increase in the dose of the implant. No estrous cycles or mating behaviors were observed in groups II, III, IV, V, and the positive control group after the levonorgestrel implant was implanted *in vivo*.

(2) It was found that the LH levels in the rats with the same dose of implant as the positive control group were close to those in the positive control group, with no significant difference (P > 0.05), indicating that the efficacy of the implant prepared in this study was equivalent to that of the commercially available levonorgestrel silicone rod.

(3) During the experiment, the rats maintained normal daily routine, diet, and body weight gain, indicating that the implant had no adverse effects on the rats.

## III. Long-acting estradiol implants

### (I) Content determination and *in vitro* release testing methods for long-acting estradiol implants

(1) Content determination method

One estradiol implant was taken, and the silicone tube was cut open. The medicinal core was cut into several small segments and placed in a 100 mL volumetric flask. 10 mL of methanol was added to soak the segments, and the mixture was allowed to swell for 3 hours. The mixture was then diluted to the mark with anhydrous ethanol, shaken well, and filtered. 0.2 mL of the subsequent filtrate was accurately measured and placed in a 50 mL volumetric flask, diluted to the mark with the mobile phase, and shaken well. 20 μL of the sample was then injected into HPLC, and the drug content was calculated based on the standard curve. The chromatographic conditions are as follows:

Chromatographic column: Diamonsil® $C_{18}$ column (250 mm × 4 mm, 5 μm)
Mobile phase: acetonitrile-water (55:45, v/v)
Column temperature: 25°C
Detection wavelength: 202 nm
Flow rate: 0.8 mL·min$^{-1}$
Injection volume: 20 μL

(2) Release rate determination method

The release rate determination was conducted using the horizontal shaking method. One estradiol implant was taken and fixed in a 100 mL stoppered conical flask using an adhesive, ensuring that the robs were arranged in an interlaced manner. Exactly 100 mL of distilled water was measured as the release medium. The stoppered conical flask was placed in a thermostatic shaker set at 37°C and shaken at a frequency of 100 rpm, ensuring that the implants remained submerged below the liquid surface. Samples were taken every 24 hours, and the release medium was replaced with an equal volume of fresh medium. The sample solution was filtered through a 0.22 μm microporous filter membrane and injected according to the section "(1) Content determination method". The drug release amount was calculated based on the standard curve.

### (II) Preparation and *in vitro* release study of reservoir-type estradiol implants

1. Instruments and reagents

1.1 Instruments

The instruments used are the same as those described in "I. Long-acting gestodene contraceptive implants; (III) Preparation and *in vitro* release study of gestodene implants".

1.2 Materials

| | |
|---|---|
| Estradiol (Purity of 99%, Batch No.: 190327) | Hebei Mokai Technology Development Co., Ltd. |
| Petroleum ether (boiling range of 60 to 90°C) | Shandong Yuwang Chemical Reagent Co., Ltd. |

All other materials are the same as those described in "I. Long-acting gestodene contraceptive implants; (III) Preparation and *in vitro* release study of gestodene implants".

2. Experimental methods

2.1 Preparation of reservoir-type estradiol implants

The preparation and preparation method for the silicone tube are as follows.

| Material name | Amount |
|---|---|
| Methyl vinyl silicone rubber | 100 PHR |
| Content of vinyl groups in vinyl polysiloxane | 0.17 mol% |
| Fumed white carbon black | 30 PHR |
| Hydrogen-containing silicone oil | 1.01 PHR |
| Molar ratio of Si-H groups in hydrogen-containing silicone oil to vinyl groups in methyl vinyl silicone rubber | 1.2:1 |
| Content of hydrogen in hydrogen-containing silicone oil | 0.75 mol% |
| 2-Methyl-3-butyn-2-ol | 0.7 PHR |
| Platinum catalyst (3000 ppm) | 0.00001 PHR |

Using extrusion technology, different types of addition-cure controlled-release silicone tubes were prepared. The mechanical properties such as elongation at break, tensile strength, and tear strength were used as evaluation indicators to investigate the preparation and process factors of the silicone tube. The finally determined preparation for the silicone tube was as follows: 100 PHR of methyl vinyl silicone rubber, a vinyl content of 0.17 mol% in vinyl polysiloxane, 30 PHR of fumed white carbon black, 1.01 PHR of hydrogen-containing silicone oil, a molar ratio of Si-H groups in the hydrogen-containing silicone oil to vinyl groups in the methyl vinyl silicone rubber of 1.2:1, a hydrogen content of 0.75 mol% in the hydrogen-containing silicone oil, 0.7 PHR of 2-methyl-3-butyn-2-ol, and 0.00001 PHR of platinum catalyst (3000 ppm). The process conditions were as follows: front drying tunnel vulcanization temperature of 300°C (vulcanization time of approximately 5 seconds), rear drying tunnel vulcanization temperature of 280°C (vulcanization time of approximately 2 minutes), and oven temperature of 180°C (for 48 hours). Silicone tubes with a wall thickness of 0.2/0.4/0.6 mm were prepared.

One end of the silicone tube was sealed with adhesive, and after curing for 24 hours, the drug was filled through a filling funnel. Upon completion of filling, the other end was similarly sealed with KN-300N adhesive and cured for 24 hours. After the curing process was completed, the tube was checked for any drug leakage. Once confirmed that there was no leakage, the tube was repeatedly washed with anhydrous ethanol for 30 seconds, and the preparation was completed, resulting in a reservoir-type estradiol implant with a particle size $D_{50}$ of 9.5 $\mu$m, a wall thickness of 0.2 mm, an outer diameter of 2.2 to 2.3 mm, and a release area of 2.7 $\pm$ 0.5 cm$^2$.

2.2 Release conditions

Purified water was selected as the release medium, and a thermostatic shaker set at 37°C was used as the release apparatus.

2.3 *In vitro* release and investigation of influencing factors

2.3.1 Vulcanization temperature

During the preparation of silicone tubes, the silicone tube prepared under the vulcanization conditions of a front drying tunnel temperature of 300°C and a rear drying tunnel temperature of 280°C has better mechanical properties, resulting in more complete vulcanization. On this basis, with other conditions unchanged, silicone tubes with different vulcanization temperatures were respectively prepared into implants for *in vitro* release experiments to investigate the effect of vulcanization temperature on the drug release behavior *in vitro*.

| No. | Front drying tunnel temperature (°C) | Rear drying tunnel temperature (°C) | Oven vulcanization temperature (°C) |
|---|---|---|---|
| Example 19-1 (300-280) | 300 | 280 | 180 |
| Example 19-2 (300-260) | 300 | 260 | 180 |
| Example 19-3 (280-280) | 280 | 280 | 180 |

2.3.2 Silicone tube wall thickness

The effect of the wall thickness of the silicone tube on the drug release behavior *in vitro* was investigated. Implants with a consistent length and outer diameter were prepared using the silicone tubes with a wall thickness of 0.2 mm, 0.4 mm, and 0.6 mm, as obtained previously. The *in vitro* release data of these implants were measured.

| No. | Silicone tube outer diameter/mm | Silicone tube wall thickness/mm | Drug-loaded segment length/cm |
|---|---|---|---|
| Example 20-1 (0.2) | 2.2 | 0.2 | 4 |
| Example 20-2 (0.4) | 2.2 | 0.4 | 4 |
| Example 20-3 (0.6) | 2.2 | 0.6 | 4 |

2.3.3 Drug release area

The silicone implant controls drug release through a cylindrical silicone tube, and the drug release area can be expressed by the following formula:

$$S = \pi d \times L$$

wherein d is the inner diameter of the silicone tube, and L is the length of the drug-loaded segment.

As indicated by the formula, the drug release area can be altered by changing the length of the drug-loaded segment and the inner diameter of the silicone tube. Considering that altering the length of the drug-loaded segment is simpler and more convenient, in this experiment, the silicone tube with a fixed outer diameter of 2.4 mm and a fixed wall thickness at 0.2 mm was tightly filled with estradiol implants with a drug-loaded segment length of 1, 2, 3, 4, and 5 cm, respectively, for *in vitro* release, and the variation pattern of the *in vitro* release amount with the drug-loaded segment length was investigated.

| No. | Silicone tube outer diameter/mm | Silicone tube wall thickness/mm | Drug release area/cm$^2$ |
|---|---|---|---|
| Example 21-1 | 2.2 | 0.2 | 0.69 |
| Example 21-2 | 2.2 | 0.2 | 1.38 |
| Example 21-3 | 2.2 | 0.2 | 2.07 |
| Example 21-4 | 2.2 | 0.2 | 2.76 |
| Example 21-5 | 2.2 | 0.2 | 3.45 |

2.4 Long-term *in vitro* release experiment of reservoir-type estradiol implants

After determining the wall thickness and preparation of the silicone tube, vulcanization temperature, drug loading, and drug release area in the implant, three batches of optimal preparations were prepared to investigate their long-term stable release performance and observe the trend of daily release.

The preparation and preparation of the silicone tube were the same as in section 2.1. The other preparation conditions were as follows: vulcanization conditions of a front drying tunnel temperature of 300°C (vulcanization time of approximately 5 seconds), a rear drying tunnel temperature of 280°C (vulcanization time of approximately 2 minutes), and an oven temperature of 180°C (for 48 hours), resulting in a silicone tube with a wall thickness of 0.2 mm. The particle size

$D_{50}$ of estradiol was 9.5 $\mu$m.

3. Results and discussion

3.1 Preparation of reservoir-type estradiol implants

Different specifications of reservoir-type implants were prepared, exhibiting good appearance with uniform sealing and evenly filled drug segments.

3.2 Investigation of factors influencing *in vitro* release

3.2.1 Vulcanization conditions

| Time (d) | Daily release dose ($\mu$g) | | |
|---|---|---|---|
| | 300-280 | 280-280 | 300-260 |
| 1 | 25.3 | 23.6 | 28.3 |
| 2 | 22.3 | 21.2 | 17.8 |
| 3 | 18.2 | 18.9 | 20.9 |
| 4 | 15.5 | 16.5 | 22.6 |
| 5 | 17.5 | 16.1 | 18.6 |
| 6 | 16.2 | 15.8 | 19.3 |
| 7 | 16.8 | 15.4 | 15.7 |
| 8 | 15.6 | 14.7 | 17.2 |
| 9 | 15.5 | 12.2 | 16.5 |
| 10 | 14.5 | 16.1 | 14.6 |
| 11 | 14.2 | 14.2 | 13.8 |
| 12 | 14.3 | 12.9 | 15.9 |
| 13 | 12.6 | 13.1 | 16.2 |
| 14 | 12.5 | 13.2 | 14.5 |
| 15 | 14.3 | 13.5 | 11.9 |
| 16 | 13.5 | 11.7 | 12.3 |
| 17 | 12.6 | 12.3 | 13.7 |
| 18 | 12.7 | 15.6 | 12.4 |
| 19 | 13.7 | 14.4 | 13.1 |
| 20 | 13.1 | 12.2 | 14.2 |
| 21 | 15.6 | 12.7 | 12.3 |
| 22 | 13.5 | 14.4 | 12.7 |
| 23 | 13.4 | 11.2 | 11.8 |
| 24 | 12.1 | 12.4 | 12.3 |
| 25 | 11.7 | 12.9 | 14.5 |
| 26 | 13.4 | 11.9 | 12.8 |
| 27 | 11.6 | 12.7 | 11.5 |
| 28 | 12.3 | 11.9 | 11.2 |

(continued)

| Time (d) | Daily release dose ($\mu$g) | | |
|---|---|---|---|
| | 300-280 | 280-280 | 300-260 |
| 29 | 13.1 | 12.3 | 12.7 |
| 30 | 13.3 | 14.4 | 12.5 |
| 31 | 12.8 | 13.6 | 14.6 |
| 32 | 11.9 | 14.7 | 12.9 |
| 33 | 14.6 | 11.6 | 13.7 |
| 34 | 15.2 | 12.3 | 11.8 |
| 35 | 14.3 | 10.6 | 12.1 |
| 36 | 11.3 | 10 | 14.6 |
| 37 | 10.8 | 11.4 | 12 |
| 38 | 12.1 | 12.6 | 10.6 |
| 39 | 15.4 | 11.9 | 13.1 |
| 40 | 13.5 | 10.5 | 11.2 |
| 41 | 11.5 | 12.3 | 10.2 |
| 42 | 11.9 | 10.7 | 10.4 |
| 43 | 11.7 | 8.9 | 11.7 |
| 44 | 10.2 | 12.3 | 13.7 |
| 45 | 12.9 | 11.7 | 12.3 |
| 46 | 13 | 13.1 | 11.5 |
| 47 | 11.5 | 12.7 | 10.5 |
| 48 | 12.5 | 11.6 | 12.7 |
| 49 | 10.4 | 11.7 | 15.4 |
| 50 | 11.6 | 10.8 | 11.2 |
| 51 | 10.2 | 12.1 | 10.9 |
| 52 | 9.6 | 14.2 | 12.1 |
| 53 | 11.4 | 13.6 | 12.8 |
| 54 | 11 | 12.5 | 12.3 |
| 55 | 11.6 | 11.7 | 10.5 |
| 56 | 12.3 | 10.9 | 11.2 |
| 57 | 11.5 | 10.5 | 11.6 |
| 58 | 11.2 | 11.2 | 12.4 |
| 59 | 10.3 | 10.3 | 13.6 |
| 60 | 12.7 | 9.7 | 12.1 |
| 61 | 13.2 | 11.2 | 11.8 |
| 62 | 11.3 | 12 | 11.5 |
| 63 | 10.8 | 11.6 | 10.9 |
| 64 | 10.6 | 10.5 | 9.7 |
| 65 | 13.5 | 10.8 | 11.4 |
| 66 | 11 | 11.9 | 11.5 |

(continued)

| Time (d) | Daily release dose (μg) | | |
|---|---|---|---|
| | 300-280 | 280-280 | 300-260 |
| 67 | 11.6 | 10.5 | 12.1 |
| 68 | 11.9 | 11.7 | 13.5 |
| 69 | 11.6 | 10.8 | 11.3 |
| 70 | 11.2 | 12.2 | 10.7 |
| 71 | 13.6 | 11.7 | 11.2 |
| 72 | 12.3 | 12.9 | 11.6 |
| 73 | 11.9 | 12.6 | 12.1 |
| 74 | 10.6 | 12.1 | 11.6 |
| 75 | 12.9 | 11.3 | 11.9 |
| 76 | 11.1 | 10.5 | 12.3 |
| 77 | 10.8 | 10.1 | 13.1 |
| 78 | 10.4 | 9.6 | 12.5 |
| 79 | 10.7 | 9.9 | 11.3 |
| 80 | 13.2 | 11.7 | 11.8 |
| 81 | 11.5 | 12.2 | 11.2 |
| 82 | 10.3 | 11.7 | 11.7 |
| 83 | 10.7 | 10.6 | 11.8 |
| 84 | 12.5 | 10.2 | 11.6 |
| 85 | 10.9 | 11.3 | 10.6 |
| 86 | 11.4 | 12.6 | 11.2 |
| 87 | 11.3 | 11.4 | 12 |
| 88 | 11.6 | 10.7 | 11.7 |
| 89 | 10.5 | 11.1 | 10.6 |
| 90 | 11.2 | 10.6 | 10.3 |
| Average daily release dose (μg) | 12.75 | 12.44 | 12.95 |

The results indicated that changing the vulcanization temperature of the silicone tubes had no significant effect on the average daily drug release of the implants, with only minor fluctuations observed. Therefore, silicone tubes with better mechanical properties were chosen for the preparation of the implants, specifically those prepared under the vulcanization conditions of a front drying tunnel temperature of 300°C and a rear drying tunnel temperature of 280°C.

3.3.2 Silicone tube wall thickness

| Time (d) | Daily release dose (μg) | | |
|---|---|---|---|
| | 0.2 mm | 0.4 mm | 0.6 mm |
| 1 | 26.2 | 19.9 | 16.2 |
| 2 | 24.8 | 17.1 | 14.1 |
| 3 | 19.3 | 15.4 | 16 |
| 4 | 19.4 | 16.3 | 13.2 |

(continued)

| Time (d) | Daily release dose (µg) | | |
|---|---|---|---|
| | 0.2 mm | 0.4 mm | 0.6 mm |
| 5 | 18.2 | 15.4 | 12.6 |
| 6 | 17.5 | 14.9 | 13.3 |
| 7 | 15.8 | 13.2 | 10.9 |
| 8 | 15.4 | 11.2 | 10.1 |
| 9 | 16.2 | 11.1 | 11.3 |
| 10 | 14.2 | 10.7 | 9.9 |
| 11 | 15.4 | 10.5 | 9.1 |
| 12 | 14.6 | 9.7 | 10.4 |
| 13 | 14.8 | 9.8 | 9.3 |
| 14 | 13.8 | 10.3 | 8.2 |
| 15 | 13.9 | 10.4 | 8.4 |
| 16 | 14.3 | 10 | 7.3 |
| 17 | 12.4 | 10.7 | 8.1 |
| 18 | 13.1 | 9.9 | 9.7 |
| 19 | 12.2 | 10.3 | 8.3 |
| 20 | 12.7 | 9.1 | 9.6 |
| 21 | 11.3 | 8.9 | 10.7 |
| 22 | 12.1 | 9.7 | 8.3 |
| 23 | 13.4 | 9.4 | 9.7 |
| 24 | 13.2 | 10.7 | 7.2 |
| 25 | 12.1 | 9.6 | 8.1 |
| 26 | 11.9 | 9.7 | 7.7 |
| 27 | 12.2 | 8.9 | 8.8 |
| 28 | 12.3 | 6.2 | 7.5 |
| 29 | 11.9 | 10.1 | 8.3 |
| 30 | 13.1 | 9.7 | 7.2 |
| 31 | 12.9 | 9.5 | 8.5 |
| 32 | 14.1 | 8.6 | 8.2 |
| 33 | 11.8 | 10.7 | 8.8 |
| 34 | 12.4 | 10.5 | 7.5 |
| 35 | 11.6 | 9.9 | 7.9 |
| 36 | 13.8 | 9.5 | 8.1 |
| 37 | 14.2 | 9.4 | 8.6 |
| 38 | 11.7 | 10.3 | 7.4 |
| 39 | 11.2 | 11 | 10.1 |
| 40 | 12.6 | 10.4 | 9.2 |
| 41 | 13.1 | 10.8 | 7.6 |
| 42 | 13.5 | 9.6 | 8.4 |

(continued)

| Time (d) | Daily release dose (µg) | | |
|---|---|---|---|
| | 0.2 mm | 0.4 mm | 0.6 mm |
| 43 | 12.8 | 8.6 | 7.5 |
| 44 | 12.2 | 9.4 | 7.7 |
| 45 | 11.7 | 10.5 | 6.9 |
| 46 | 11.3 | 10.3 | 7.2 |
| 47 | 11.9 | 9.7 | 6.8 |
| 48 | 11.1 | 12.1 | 7.3 |
| 49 | 12.7 | 10.3 | 7.6 |
| 50 | 12.4 | 9.5 | 8.5 |
| 51 | 13 | 9.6 | 8.1 |
| 52 | 11.8 | 8.8 | 7.9 |
| 53 | 12.5 | 9.2 | 7.7 |
| 54 | 12.1 | 10.9 | 6.8 |
| 55 | 12.4 | 10.2 | 7.3 |
| 56 | 11.9 | 11.5 | 7.5 |
| 57 | 13.7 | 10.6 | 8.9 |
| 58 | 12.9 | 10.2 | 7.6 |
| 59 | 12.6 | 10.4 | 6.3 |
| 60 | 11.8 | 9.8 | 6.7 |
| 61 | 10.4 | 9.6 | 7 |
| 62 | 11.5 | 10.6 | 7.2 |
| 63 | 11.2 | 8.5 | 7.4 |
| 64 | 12.2 | 9.4 | 6.6 |
| 65 | 11 | 9.2 | 6.2 |
| 66 | 11.8 | 10.1 | 6.4 |
| 67 | 11.5 | 10.3 | 6.1 |
| 68 | 10.8 | 9.5 | 5.9 |
| 69 | 12.5 | 9.8 | 6.3 |
| 70 | 12.7 | 10.4 | 6.9 |
| 71 | 11.9 | 11.4 | 7.3 |
| 72 | 11.6 | 10.5 | 5.5 |
| 73 | 12.3 | 10.8 | 7.6 |
| 74 | 13.1 | 9.7 | 7.4 |
| 75 | 12.8 | 9.2 | 6.7 |
| 76 | 11.5 | 10.3 | 6.5 |
| 77 | 13.1 | 8.7 | 7.4 |
| 78 | 11.7 | 9.3 | 8 |
| 79 | 10.5 | 8.8 | 8.5 |
| 80 | 11.3 | 9.1 | 6.7 |

(continued)

| Time (d) | Daily release dose (μg) | | |
|---|---|---|---|
| | 0.2 mm | 0.4 mm | 0.6 mm |
| 81 | 11.8 | 9.5 | 7.6 |
| 82 | 12.6 | 10.6 | 7.9 |
| 83 | 12.3 | 10.5 | 8.2 |
| 84 | 11.4 | 9.7 | 6.9 |
| 85 | 12.2 | 8.3 | 6.8 |
| 86 | 11.5 | 9.4 | 7.6 |
| 87 | 12.7 | 9.6 | 7.1 |
| 88 | 12.5 | 9.2 | 6 |
| 89 | 11.5 | 9.8 | 6.5 |
| 90 | 11.9 | 8.2 | 6.3 |
| Average daily release dose (μg) | 13.08 | 10.34 | 8.25 |

The results showed that, by controlling factors such as outer diameter and release area unchanged, the *in vitro* release amount of the reservoir-type estradiol implants made from silicone tubes with three different wall thicknesses decreased gradually with the increase of wall thickness. To ensure adequate drug release, the wall thickness should be minimized. However, in practical operations, a wall thickness of 0.2 mm is already the limit. Reducing the wall thickness further would compromise the smooth extrusion molding of the silicone tube. Therefore, a wall thickness of 0.2 mm was ultimately adopted for the silicone tube.

3.3.3 Investigation of drug release area of silicone tubes

The effect of the medicinal core length within the silicone tube on *in vitro* release is shown in the table below.

| Release area ($cm^2$) | Average daily release dose (μg/d) |
|---|---|
| 0.69 | $3.63 \pm 1.42$ |
| 1.38 | $6.67 \pm 1.18$ |
| 2.07 | $10.37 \pm 2.08$ |
| 2.76 | $13.08 \pm 1.79$ |
| 3.45 | $17.02 \pm 2.78$ |

The results indicated that as the release area increased, the daily drug release amount of the reservoir-type estradiol implants also increased. To further assess the relationship between the release area and the drug release amount of the implant, data analysis was performed on the release amount and release area of the five groups of preparations, and the results are shown in Fig. 15.
The fitted equations and graphs illustrated that the drug release amount was linearly related to the release area, with the release amount increasing as the release area increased. Based on this relationship, the drug release amount can be regulated by adjusting the release area by fixing the outer diameter and wall thickness of the silicone tube. In practical applications, due to the large individual differences among patients, reservoir-type estradiol implants of different specifications can be produced to meet varying needs, which enables personalized administration, reduces side effects, and maximizes benefits.

3.4 Long-term *in vitro* release experiment of estradiol implants

Under the same conditions as the final preparation (refer to 2.4 Long-term *in vitro* release experiment of reservoir-type estradiol implants described above in this section), three batches of reservoir-type implants were prepared and subjected to long-term *in vitro* release experiments at a temperature of 37°C and a shaking speed of 100 rpm. The results are shown in the table below.

| Time (d) | Daily release dose (μg) | | |
|---|---|---|---|
| | ZJ001-1 | ZJ002-1 | ZJ003-1 |
| 1 | 26.2 | 25.3 | 27.3 |
| 2 | 24.8 | 22.3 | 26.8 |
| 3 | 19.3 | 18.2 | 20.9 |
| 4 | 19.4 | 15.5 | 17.2 |
| 5 | 18.2 | 17.5 | 16.8 |
| 6 | 17.5 | 16.2 | 16.4 |
| 7 | 15.8 | 16.8 | 18.7 |
| 8 | 15.4 | 15.6 | 16.9 |
| 9 | 16.2 | 15.5 | 15.5 |
| 10 | 14.2 | 14.5 | 13.6 |
| 11 | 15.4 | 14.2 | 15.9 |
| 12 | 14.6 | 14.3 | 15.1 |
| 13 | 14.8 | 12.6 | 14.2 |
| 14 | 13.8 | 12.5 | 14.3 |
| 15 | 13.9 | 14.3 | 12.9 |
| 16 | 14.3 | 13.5 | 13.3 |
| 17 | 12.4 | 12.6 | 12.7 |
| 18 | 13.1 | 12.7 | 13.4 |
| 19 | 12.2 | 13.7 | 12.1 |
| 20 | 12.7 | 13.1 | 14.5 |
| 21 | 11.3 | 15.6 | 13.6 |
| 22 | 12.1 | 13.5 | 12.9 |
| 23 | 13.4 | 13.4 | 11.6 |
| 24 | 13.2 | 12.1 | 12.5 |
| 25 | 12.1 | 11.7 | 11.5 |
| 26 | 11.9 | 13.4 | 12.6 |
| 27 | 12.2 | 11.6 | 12.4 |
| 28 | 12.3 | 12.3 | 13.1 |
| 29 | 11.9 | 13.1 | 10.7 |
| 30 | 13.1 | 13.3 | 12.5 |
| 31 | 12.9 | 12.8 | 13.6 |
| 32 | 14.1 | 11.9 | 13.2 |
| 33 | 11.8 | 14.6 | 12.7 |
| 34 | 12.4 | 15.2 | 10.8 |
| 35 | 11.6 | 14.3 | 12.2 |
| 36 | 13.8 | 11.3 | 13.6 |
| 37 | 14.2 | 10.8 | 11.2 |
| 38 | 11.7 | 12.1 | 11 |

(continued)

| Time (d) | Daily release dose ($\mu$g) | | |
|---|---|---|---|
| | ZJ001-1 | ZJ002-1 | ZJ003-1 |
| 39 | 11.2 | 15.4 | 13.4 |
| 40 | 12.6 | 13.5 | 10.2 |
| 41 | 13.1 | 11.5 | 10.9 |
| 42 | 13.5 | 11.9 | 11.4 |
| 43 | 12.8 | 11.7 | 11.6 |
| 44 | 12.2 | 10.2 | 12.7 |
| 45 | 11.7 | 12.9 | 11.3 |
| 46 | 11.3 | 13 | 11.5 |
| 47 | 11.9 | 11.5 | 10.4 |
| 48 | 11.1 | 12.5 | 12.2 |
| 49 | 12.7 | 10.4 | 12.4 |
| 50 | 12.4 | 11.6 | 13.2 |
| 51 | 13 | 10.2 | 11.9 |
| 52 | 11.8 | 9.6 | 13.1 |
| 53 | 12.5 | 11.4 | 11.8 |
| 54 | 12.1 | 11 | 12.4 |
| 55 | 12.4 | 11.6 | 11.5 |
| 56 | 11.9 | 12.3 | 10.2 |
| 57 | 13.7 | 11.5 | 10.6 |
| 58 | 12.9 | 11.2 | 11.4 |
| 59 | 12.6 | 10.3 | 12.6 |
| 60 | 11.8 | 12.7 | 12.1 |
| 61 | 10.4 | 13.2 | 11.8 |
| 62 | 11.5 | 11.3 | 11.6 |
| 63 | 11.2 | 10.8 | 10.9 |
| 64 | 12.2 | 10.6 | 10.7 |
| 65 | 11 | 13.5 | 11.4 |
| 66 | 11.8 | 11 | 12.1 |
| 67 | 11.5 | 11.6 | 11.5 |
| 68 | 10.8 | 11.9 | 13.5 |
| 69 | 12.5 | 11.6 | 12.3 |
| 70 | 12.7 | 11.2 | 11.7 |
| 71 | 11.9 | 13.6 | 12.2 |
| 72 | 11.6 | 12.3 | 12.4 |
| 73 | 12.3 | 11.9 | 11.5 |
| 74 | 13.1 | 10.6 | 11.2 |
| 75 | 12.8 | 12.9 | 10.9 |
| 76 | 11.5 | 11.1 | 11.9 |

(continued)

| Time (d) | Daily release dose ($\mu$g) | | |
|---|---|---|---|
| | ZJ001-1 | ZJ002-1 | ZJ003-1 |
| 77 | 13.1 | 10.8 | 11.3 |
| 78 | 11.7 | 10.4 | 11.7 |
| 79 | 10.5 | 10.7 | 11.6 |
| 80 | 11.3 | 13.2 | 10.8 |
| 81 | 11.8 | 11.5 | 11.2 |
| 82 | 12.6 | 10.3 | 11.7 |
| 83 | 12.3 | 10.7 | 11.6 |
| 84 | 11.4 | 12.5 | 10.2 |
| 85 | 12.2 | 10.9 | 10.8 |
| 86 | 11.5 | 11.4 | 12.1 |
| 87 | 12.7 | 11.3 | 11.3 |
| 88 | 12.5 | 11.6 | 12.1 |
| 89 | 11.5 | 10.5 | 12.6 |
| 90 | 11.9 | 11.2 | 11.5 |
| 91 | 11.2 | 11.5 | 10.9 |
| 92 | 10.6 | 11.7 | 11.4 |
| 93 | 11 | 11.5 | 11.8 |
| 94 | 10.9 | 11.9 | 11.5 |
| 95 | 11.7 | 12.1 | 11.2 |
| 96 | 10.1 | 12.3 | 12.5 |
| 97 | 11.9 | 11.5 | 13.1 |
| 98 | 11.6 | 11.8 | 12.7 |
| 99 | 10.8 | 11.3 | 12.4 |
| 100 | 11.1 | 11.4 | 11.9 |
| 101 | 12.7 | 10.9 | 11.4 |
| 102 | 12.4 | 11.6 | 11.2 |
| 103 | 11.7 | 12.1 | 12.6 |
| 104 | 11.2 | 11.8 | 12 |
| 105 | 11.5 | 11.3 | 11.7 |
| 106 | 11.6 | 10.6 | 11.8 |
| 107 | 10.9 | 11.2 | 10.9 |
| 108 | 11.8 | 11.5 | 10.5 |
| 109 | 11.1 | 11.7 | 10.6 |
| 110 | 10.9 | 11.3 | 11.9 |
| 111 | 10.6 | 12 | 11.2 |
| 112 | 11.5 | 10.7 | 10.6 |
| 113 | 11.2 | 10.9 | 9.2 |
| 114 | 11.5 | 10.7 | 10.1 |

(continued)

| Time (d) | Daily release dose (μg) | | |
|---|---|---|---|
| | ZJ001-1 | ZJ002-1 | ZJ003-1 |
| 115 | 11.3 | 11.4 | 10.3 |
| 116 | 10.9 | 11.2 | 10.7 |
| 117 | 10.6 | 11.6 | 10.5 |
| 118 | 10.7 | 10.4 | 10.6 |
| 119 | 11.3 | 10.6 | 11.3 |
| 120 | 10.9 | 11.1 | 11.4 |
| 121 | 11.1 | 11.8 | 10.8 |
| 122 | 11.4 | 11.6 | 10.7 |
| 123 | 11.3 | 12.1 | 10.8 |
| 124 | 10.6 | 12.3 | 11.4 |
| 125 | 10.7 | 10.3 | 11.1 |
| 126 | 11.5 | 9.7 | 11.6 |
| 127 | 11.2 | 10.6 | 10.9 |
| 128 | 11.6 | 10.5 | 10.8 |
| 129 | 11.3 | 10.7 | 11.6 |
| 130 | 10.8 | 10.9 | 11.3 |
| 131 | 10.4 | 10.3 | 11.4 |
| 132 | 10.2 | 10.8 | 10.6 |
| 133 | 10.7 | 11.4 | 10.7 |
| 134 | 10.5 | 11.2 | 11.5 |
| 135 | 10.3 | 11.3 | 11.6 |
| 136 | 11.6 | 10.8 | 12.4 |
| 137 | 11.3 | 10.5 | 12.2 |
| 138 | 10.4 | 10.9 | 11.8 |
| 139 | 10.2 | 11 | 11.4 |
| 140 | 10.6 | 11.7 | 10.9 |
| 141 | 10.3 | 10.2 | 11 |
| 142 | 10.7 | 10.9 | 11.2 |
| 143 | 11.1 | 10.4 | 10.6 |
| 144 | 11.3 | 10.6 | 10.7 |
| 145 | 10.9 | 11.2 | 10.8 |
| 146 | 10.5 | 11.5 | 10.3 |
| 147 | 10.4 | 10.9 | 11.1 |
| 148 | 11.2 | 11.2 | 11.5 |
| 149 | 10.6 | 10.8 | 11.4 |
| 150 | 10.8 | 10.7 | 11.3 |
| 151 | 10.7 | 10.3 | 10.9 |
| 152 | 10.2 | 10.6 | 10.7 |

(continued)

| Time (d) | Daily release dose ($\mu$g) | | |
|---|---|---|---|
| | ZJ001-1 | ZJ002-1 | ZJ003-1 |
| 153 | 11 | 10.5 | 10.3 |
| 154 | 10.8 | 11.4 | 10.2 |
| 155 | 10.9 | 10.7 | 10.2 |
| 156 | 11.2 | 10.6 | 10.6 |
| 157 | 10.6 | 10.7 | 11.2 |
| 158 | 10.5 | 10.9 | 10.4 |
| 159 | 10.2 | 10.1 | 11.6 |
| 160 | 10.3 | 10.3 | 11.7 |
| 161 | 10.7 | 9.6 | 10.5 |
| 162 | 10.3 | 10.7 | 10.4 |
| 163 | 9.6 | 10.7 | 10.6 |
| 164 | 10.9 | 10.3 | 11.1 |
| 165 | 9.7 | 10.9 | 10.3 |
| 166 | 10.2 | 11.3 | 10.6 |
| 167 | 10.8 | 10.2 | 10.9 |
| 168 | 9.8 | 10.8 | 9.5 |
| 169 | 10.1 | 10.6 | 10.7 |
| 170 | 10.5 | 11.2 | 11.4 |
| 171 | 10.3 | 11.3 | 10.5 |
| 172 | 10.2 | 10.2 | 10.8 |
| 173 | 10.1 | 10.6 | 10.4 |
| 174 | 10.4 | 11.4 | 10 |
| 175 | 10.9 | 9.7 | 10.3 |
| 176 | 11.3 | 10 | 10.2 |
| 177 | 10.6 | 10.9 | 10.5 |
| 178 | 10.7 | 10.6 | 11.2 |
| 179 | 10.3 | 11.3 | 10.1 |
| 180 | 10.8 | 10.5 | 10.2 |
| Average daily release dose ($\mu$g) | 11.97 | 11.87 | 11.94 |

The results indicated that the daily release of the three batches of implants fluctuated in the early stage, but gradually stabilized in the later stage, with the average daily *in vitro* release remaining stable within the range of 10-15 $\mu$g. The trend was similar across the batches, demonstrating good reproducibility. According to the literature, the commonly used supplemental dose for estradiol replacement therapy is 20-40 $\mu$g per day. Therefore, implanting two implants can achieve the desired therapeutic effect, which is in line with the experimental expectations.

**IV. Pharmaceutical composition**

**Example 22**

Preparation of pharmaceutical composition: The components listed in the table below were mixed to obtain a pharmaceutical composition (powder-type medicinal core). The particle size of the gestodene bulk drug was 2.81 $\mu$m.

| No. | Bulk drug | Bulk drug mass (mg) | Insoluble excipient | Insoluble excipient mass (mg) | Mass ratio of bulk drug to insoluble excipient |
|---|---|---|---|---|---|
| Example 22-1 | Gestodene bulk drug | 11.4 | Fumed white carbon black | 11.4 | 1:1 |
| Example 22-2 | Gestodene bulk drug | 11.4 | AL-1FP mesoporous silica | 11.4 | 1:1 |
| Example 22-3 | Gestodene bulk drug | 11.4 | XDP3050 mesoporous silica | 11.4 | 1:1 |

Preparation of silicone tubes:

S1: Methyl vinyl silicone rubber was wrapped with a reinforcing agent and then processed by extruding and passing through an open mill, followed by sheeting and uniform mixing to obtain a mixture A. The mixture A was stored in a desiccator at room temperature for 24-72 hours.

S2: The mixture A was divided into a component A1 and a component A2. The component A1 was mixed with a catalyst to obtain a component B1, and the component A2 was mixed with hydrogen-containing silicone oil and an inhibitor to obtain a component B2.

S3: The component B1 and the component B2 were mixed to obtain a mixture B.

S4: The mixture B was formed into a tubular shape by an extrusion process, followed by catalytic addition.

The catalytic addition involved the following steps: subjecting the mixture B sequentially to a first heat treatment at 270°C for approximately 5 seconds, a second heat treatment at 180°C for approximately 2 minutes, and a third heat treatment at 180°C for 24 hours.

In Examples 22-1, 22-2, and 22-3, the raw material compositions of the silicone tubes included the components listed in the table below. The relative molecular weight of methyl vinyl silicone rubber was 100000-800000 g/mol; the content of vinyl groups in the methyl vinyl silicone rubber was 0.17 mol%; the content of Si-H groups in the hydrogen-containing silicone oil was 0.75 mol%; the molar ratio of Si-H groups in the hydrogen-containing silicone oil to vinyl groups in the methyl vinyl silicone rubber was 1.2:1.

| / | Component | Parts by weight |
|---|---|---|
| Methyl vinyl silicone rubber | Methyl vinyl silicone rubber | 100 parts |
| Reinforcing agent | Fumed white carbon black | 40 parts |
| Hydrogen-containing silicone oil | Hydrogen-containing silicone oil | 1.13 parts |
| Catalyst | Platinum catalyst | 0.00001 parts |
| Inhibitor | 2-Methyl-3-butyn-2-ol | 0.7 parts |

Preparation of implants: The prepared silicone tube was disinfected by soaking in 75% ethanol for 30 minutes and then dried by blowing. One end of the silicone tube was sealed with silicone sealant for later use. The prepared pharmaceutical composition was filled into the silicone tube through a filling funnel, serving as the powder-type medicinal core for the implant with a filling length of 2.0 cm. The other end of the silicone tube was then sealed with silicone sealant and cured for 24 hours to prepare a gestodene implant. The silicone tube had an outer diameter of 2.6 mm and a wall thickness of 0.3 mm.

During the filling process, it was observed that there was no airborne powder or spontaneous aggregation in the pharmaceutical composition.

**Comparative Example 1**

In Comparative Example 1, the medicinal core of the implant was gestodene bulk drug, with all other conditions the same as in Example 22.

During the filling process, it was observed that the gestodene bulk drug powder dispersed into the air, and a significant amount of the bulk drug powder adhered to the outer surface of the silicone tube. Additionally, the powder exhibited spontaneous aggregation.

**Effect Example 1: *In vitro* release testing method for implants**

1. Chromatographic conditions

Chromatographic column: Diamonsil® $C_{18}$ column (250 mm × 4.60 mm, 5 μm);
Mobile phase: methanol-water (80:20, v/v);
Column temperature: 30°C;
Detection wavelength: 239 nm;
Flow rate: 1 mL·min$^{-1}$;
Injection volume: 20 μL.

2. The filled implant was placed in an ethanol solution and subjected to ultrasonication for 30 minutes. After ultrasonication, the implant was dried by blowing and then placed in a stoppered conical flask containing the release medium. The stoppered conical flask was placed in a shaker set at 37°C and shaken at 100 rpm for 24 hours. After 24 hours, it was taken out and dried by blowing.

Both ends of the dried gestodene implant were adhered to the bottom and wall of the stoppered conical flask using silicone rubber sealant, ensuring that the silicone tube filled with gestodene bulk drug powder was suspended in the stoppered conical flask without touching the wall, thus maintaining a stable contact area between the drug-loaded segment and the release medium during shaking. After adhesion, it was left to stand for 24 hours to allow the sealant to fully cure. Once cured, the required amount of release medium was accurately added to the stoppered conical flask, which was then placed in a shaker set at 37°C and shaken at 100 rpm. Samples were taken and the medium was exchanged every 24 hours, with the samples being retained for testing.

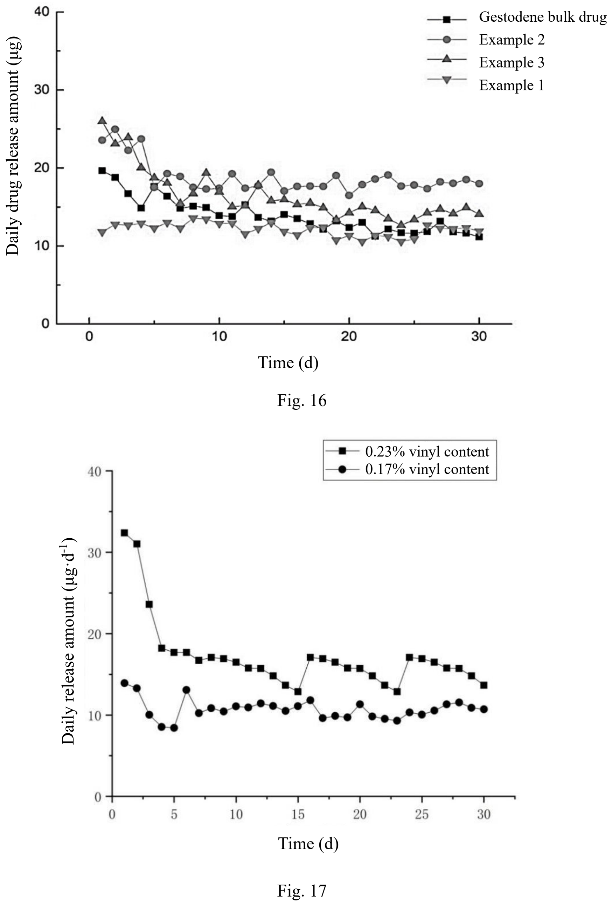

The test results are shown in the table below. The relationship between the *in vitro* daily drug release amount and release time for the implants in Example 22-1, Example 22-2, Example 22-3, and Comparative Example 1 is shown in Fig. 16.

|  | Average release rate (μg/d) | Average drug release (μg/cm/d) | RSD (%) |
|---|---|---|---|
| Comparative Example 1 | 12.67 ± 1.68 | 5.76 ± 0.76 | 13.25 |
| Example 22-1 | 11.78 ± 0.75 | 5.36 ± 0.34 | 6.39 |
| Example 22-2 | 18.02 ± 0.77 | 8.19 ± 0.35 | 4.26 |
| Example 22-3 | 14.71 ± 1.13 | 6.69 ± 0.52 | 7.71 |

As can be seen from the above table, the addition of insoluble excipients in the pharmaceutical composition effectively improved the static electricity of the bulk drug powder during filling, particularly with AL-1FP mesoporous silica and XDP3050 mesoporous silica, which significantly increased the filling efficiency. The *in vitro* release data revealed that the RSD value of the *in vitro* release was significantly lower than that of Comparative Example 1 without adding insoluble excipients in the medicinal core. The lower the RSD value, the smaller the difference in the daily release amounts, indicating less overall release fluctuation, thus ensuring stable drug release. Therefore, the addition of insoluble excipients significantly enhanced the experimental stability of the implants. Among them, the insoluble excipient AL-1FP mesoporous silica showed the best drug release stability, with minimal daily release fluctuations. The release data showed that this excipient greatly improved the daily drug release amount and exhibited excellent stability.

**Effect Example 2: *In vivo* pharmacodynamic study of implants**

The implants from Example 22-1, Example 22-2, Example 22-3, and Comparative Example 1 were implanted in rats. The estrous cycle changes in SD rats post-implantation were observed through vaginal smears, the LH (luteinizing hormone) levels in the rats were measured using the enzyme-linked immunosorbent assay, and the presence or absence of mating behavior in the rats was monitored to study the contraceptive effect of subcutaneous implantation.

1. Instruments and reagents

1.1 Instruments

XS105 Electronic Analytical Balance, Mettler-Toledo Instrument Co., Ltd.
SMD200-2 Electronic Analytical Balance, Ohaus International Trade Co., Ltd.
TG16MW Desktop High-Speed Centrifuge, Hunan Herexi Instrument and Equipment Co., Ltd.
KQ-250DE CNC Ultrasonic Cleaner, Kunshan Ultrasonic Instrument Co., Ltd.
Rat LH Assay Kit, Shanghai Tongwei Biotechnology Co., Ltd.
Electron Microscope, Shenzhen Zhongwei Kechuang Technology Co., Ltd.
Autoclave, Shanghai Shenan Medical Instrument Factory

1.2 Reagents

Gestodene (Purity of 98%, Batch No.: 20190327), Hebei Mokai Technology Development Co., Ltd.
Crystal violet, Tianjin Damao Chemical Reagent Factory
Normal saline, Shandong Yuwang Chemical Reagent Co., Ltd.
4% paraformaldehyde solution, Shanghai Yantuo Biotechnology Co., Ltd.

1.3 Experimental animals

26 healthy male SD rats (weighing 180-200 g) and 93 healthy female SD rats (weighing 180-200 g) were purchased from Benxi Changsheng Biotechnology Co., Ltd., License No. SCXK (Liao) 2020-0001.

2. Experimental methods

2.1 Local irritation test of blank silicone tubes in rats

The silicone tube was prepared using the methods described in Example 22-1, Example 22-2, and Example 22-3, but not filled with drug powder, which was used as a blank silicone tube (specifications: outer diameter of 2.6 mm, wall thickness of 0.3 mm). The blank silicone tube was cut into pieces and soaked in 10 mL of sterile, pyrogen-free purified water. The mixture was heated and extracted at 70°C for 24 hours. The extract was then sterilized by autoclaving for later use. Six experimental rats were randomly divided into two groups: one experimental group and one saline control group. The back of the rats was shaved for the test. The experimental group received an intradermal injection of 0.2 mL of the extract on one side of the rat's back, while the control group received an intradermal injection of 0.2 mL of normal saline on one side of the rat's back. Skin reactions at the injection sites were observed after 24 hours, 48 hours, and 72 hours.

2.2 Histopathological examination after implantation of blank silicone tubes in rats

The prepared blank silicone tubes were sealed, and after the sealant was completely cured, they were sterilized by autoclaving for later use. Nine experimental rats were randomly divided into three groups, with three rats in each group. Each rat had a sterilized homemade silicone tube implanted in the back. The rats were sacrificed 3 days, 10 days, and 30 days post-implantation, respectively. A 2 cm section of tissue around the implanted silicone tube was excised, and the skin tissue was fixed with 4% paraformaldehyde for histopathological examination. The surrounding tissue was observed for inflammation reactions such as redness and swelling. The tissue was then subjected to histological sectioning to examine for any abnormal subcutaneous and muscular lesions at the implantation site.

2.3 *In vivo* pharmacodynamic experiment of gestodene contraceptive implants in rats

Using the aforementioned preparation and process (corresponding to the silicone tubes and medicinal cores of Example 22-1, Example 22-2, Example 22-3, and Comparative Example 1), silicone tubes with an outer diameter of 2.60 mm and a wall thickness of 0.3 mm, along with gestodene implants of the following specifications were prepared. The implants were divided into Experiment groups I, II, III, and IV, with each group further divided into low-dose, medium-dose, and high-dose subgroups, as shown in the table below.

| Group | Low-dose group | Medium-dose group | High-dose group | |
|---|---|---|---|---|
| Experiment group I (Bulk drug only) | 1 cm/10.36 mg | 2.2 cm/22.8 mg | 4 cm/41.45 mg | Drug-loaded segment length/drug loading |
| Experiment group II (Bulk drug: fumed white carbon black = 1:1) | 1 cm/5.18 mg/5.18 mg | 2.2 cm/11.4 mg/11.4 mg | 4 cm/20.725 mg/20.725 mg | Drug-loaded segment length/drug loading/excipient amount |
| Experiment group III (Bulk drug: AL-1FP mesoporous silica = 1:1) | 1 cm/5.18 mg/5.18 mg | 2.2 cm/11.4 mg/11.4 mg | 4 cm/20.725 mg/20.725 mg | Drug-loaded segment length/drug loading/excipient amount |
| Experiment group IV (Bulk drug: XDP3050 mesoporous silica = 1:1) | 1 cm/5.18 mg/5.18 mg | 2.2 cm/11.4 mg/11.4 mg | 4 cm/20.725 mg/20.725 mg | Drug-loaded segment length/drug loading/excipient amount |

Experiment group I: low-dose group: 1 cm/10.36 mg; medium-dose group: 2.2 cm/22.8 mg; high-dose group: 4 cm/41.45 mg (drug-loaded segment length/drug loading).

Experiment groups II, III, and IV (same specifications, different types of excipients added): low-dose group: 1 cm/5.18 mg/5.18 mg; medium-dose group: 2.2 cm/11.4 mg/11.4 mg; high-dose group: 4 cm/20.725 mg/20.725 mg (drug-loaded segment length/drug loading/excipient amount).

The medium-dose groups in Experiments I, II, III, and IV correspond to Comparative Example 1, Example 22-1, Example 22-2, and Example 22-3, respectively. In each experimental group, the low-dose group has a shorter drug-loaded segment length and less drug loading and excipients compared to the medium-dose group, with all other factors (e.g., medicinal core composition and preparation method) remaining the same. The high-dose group has a longer drug-loaded segment length and more drug loading and excipients compared to the medium-dose group, with all other factors (e.g., medicinal core composition and preparation method) remaining the same.

A total of 78 female rats were selected and examined via vaginal smears to check for a normal estrous cycle. The 78 female SD rats, weighing approximately 180-200 g, were randomly divided into one blank group and experimental groups I, II, III, and IV Each of the experimental groups I, II, III, and IV was further divided into low-dose, medium-dose, and high-dose subgroups (the medium-dose groups in Experiments I, II, III, and IV correspond to Comparative Example 1, Example 22-1, Example 22-2, and Example 22-3, respectively). Each group consisted of 6 female rats and 2 male rats.

The female rats were intraperitoneally injected with an appropriate amount of anesthetic. Once the rats were anesthetized, the hair on their neck and back was shaved, and the area was disinfected with 2% iodine tincture. A small incision of approximately 0.5 cm was cut on the local skin of the rat's back using surgical scissors. An implantation needle specifically designed for the implant was inserted subcutaneously to the designated mark on the needle. The sterilized implant was placed inside the implantation needle. The needle was then advanced further into the skin until the implant was fully inserted. The implantation needle was withdrawn, leaving the implant under the skin. The incision was sutured, and the wound was disinfected with 2% iodine tincture. Twenty-four hours after implantation, the female rats were housed with male rats in a ratio of 3: 1.

2.3.1 Observation of rat condition

Refer to section "2.3.1 Observation of rat condition" under "I. Long-acting gestodene contraceptive implants; (IV) *In vivo* experiment of gestodene contraceptive implants in rats".

2.3.2 Vaginal smear test to observe estrous cycle in rats

Refer to section "2.3.2 Vaginal smear test to observe estrous cycle in rats" under "I. Long-acting gestodene contraceptive implants; (IV) *In vivo* experiment of gestodene contraceptive implants in rats".

2.3.3 Observation of vaginal plugs to determine mating and pregnancy in rats

Refer to section "2.3.3 Observation of vaginal plugs to determine mating and pregnancy in rats" under "I. Long-acting gestodene contraceptive implants; (IV) *In vivo* experiment of gestodene contraceptive implants in rats".

2.3.4 Measurement of luteinizing hormone (LH) levels in rats using enzyme-linked immunosorbent assay

Refer to section "2.3.4 Measurement of luteinizing hormone (LH) levels in rats using enzyme-linked immunosorbent assay" under "I. Long-acting gestodene contraceptive implants; (IV) *In vivo* experiment of gestodene contraceptive implants in rats".

3. Experimental results and discussion

3.1 *In vivo* tissue irritation test of silicone tubes in rats

The experimental group received an intradermal injection of 0.2 mL of the extract on one side of the rat's back, while the control group received an intradermal injection of 0.2 mL of normal saline on one side of the rat's back. Skin reactions at the injection sites were observed after 24 hours, 48 hours, and 72 hours. Dissection of the injection sites revealed no signs of redness, swelling, or necrosis, indicating a negative outcome.

3.2 Histopathological examination after implantation of silicone tubes in rats

Muscle tissues from the implantation site were collected on days 3, 10, and 30 post-implantation, and their pathological images (HE, ×2 or HE, ×20) were observed.

The results showed that on day 3 after the implantation of blank silicone tubes, acute inflammatory response was observed, with a slight thickening of the epidermis, a high content of collagen fibers in the dermis, and a large cystic structure formed in the local subcutaneous tissue. Connective tissue proliferation was observed around the cystic structure, accompanied by significant lymphocyte infiltration. On day 10 post-implantation, the acute inflammatory response gradually diminished, with a slight thickening of the epidermis, a high content of collagen fibers in the dermis, and a large cystic structure formed in the local subcutaneous tissue. Connective tissue proliferation was observed around the cystic structure, accompanied by diffuse lymphocyte infiltration. On day 30 post-implantation, the epidermis was structurally intact, and the squamous epithelial cells were normal in structure and tightly arranged, with a high content of collagen fibers in the dermis and a large cystic structure formed in the local subcutaneous tissue. Mild connective tissue proliferation was observed around the cystic structure, accompanied by diffuse lymphocyte and macrophage infiltration.

The implantation sites consistently exhibited large cystic cavities, indicating that the implantation of the silicone tube caused some damage to the subcutaneous tissue, resulting in cystic cavity formation. Mild connective tissue proliferation occurred around these cystic cavities, gradually forming a capsule at the interface between the silicone tube and the tissue. Upon dissection of the silicone tube three days post-implantation, it was observed that the silicone tube could be easily removed. When it was dissected ten days post-implantation, a capsule formed around the silicone tube, making it less movable, indicating that with prolonged implantation, a capsule forms around the silicone tube, preventing it from shifting within the body. Furthermore, the inflammatory response was most pronounced at three days post-implantation, and gradually decreased with prolonged implantation, suggesting that the inflammatory response induced by the silicone tube implantation is capable of subsiding over time.

3.3 *In vivo* pharmacodynamic experiment of gestodene contraceptive implants in rats

3.3.1 Body weight changes in rats before and after implantation

Following the implantation surgery, the rats were observed for their feeding status and activity levels. Normal urination and defecation were noted, and no signs of lethargy were observed.

The body weights of the rats before and after implantation are shown in the table below.

| Group | Weight before implantation (g) | Weight on day 30 post-implantation (g) |
|---|---|---|
| Blank group | 188.00 ± 6.29 | 223.50 ± 6.92 |
| Experiment I low-dose group | 190.00 ± 7.02 | 221.50 ± 4.36 |
| Experiment I medium-dose group (Comparative Example 1) | 182.25 ± 5.56 | 213.25 ± 5.50 |
| Experiment I high-dose group | 183.55 ± 4.56 | 220.25 ± 3.86 |
| Experiment II low-dose group | 193.00 ± 7.67 | 224.50 ± 4.45 |
| Experiment II medium-dose group (Example 22-1) | 189.25 ± 5.65 | 220.25 ± 5.23 |

(continued)

| Group | Weight before implantation (g) | Weight on day 30 post-implantation (g) |
|---|---|---|
| Experiment II high-dose group | 185.55 ± 4.76 | 222.25 ± 3.25 |
| Experiment III low-dose group | 191.02 + 7.76 | 222.54 ± 4.33 |
| Experiment III medium-dose group (Example 22-2) | 186.29 ± 5.50 | 217.23 ± 5.51 |
| Experiment III high-dose group | 182.59 ± 4.86 | 219.95 ± 3.16 |
| Experiment IV low-dose group | 189.00 ± 7.62 | 219.20 ± 4.96 |
| Experiment IV medium-dose group (Example 22-3) | 184.28 ± 5.86 | 215.25 ± 5.80 |
| Experiment IV high-dose group | 184.75 ± 4.59 | 221.95 ± 3.26 |

From the data on the body weight changes observed over a month, it can be concluded that the implantation surgery and the silicone tube did not affect the growth of the rats. The trend in body weight changes in the experimental group was similar to that of the blank group. Overall, the gestodene implant did not have any effect on the growth of the rats.

3.3.2 Observation of estrous cycle in rats via vaginal smears

For the blank control group, vaginal secretions from female rats were collected daily at 24-hour intervals. These secretions were stained with crystal violet for smear observation to monitor the estrous cycle, which was compared to those of the experimental groups I, II, III, and IV. Over a continuous one-month period, it was observed that the rats generally experienced estrus approximately every seven days.
Based on the vaginal smear observation over one month, in the low-dose, medium-dose, and high-dose groups of experiments I, II, III, and IV, a large amount of mucus from the vaginal secretions was observed starting from day 4 post-implantation. The vaginal smears revealed a large number of leukocytes but no keratinocytes, indicating the absence of a normal estrous cycle in these rats.

3.3.3 Observation of vaginal plugs to determine mating and pregnancy in rats

No vaginal plugs were observed in the low-dose, medium-dose, and high-dose groups of experiments I, II, III, and IV, demonstrating that the rats did not mate with male rats and confirming a contraceptive effect.

3.3.4 Measurement of plasma LH concentration in rats using enzyme-linked immunosorbent assay

LH levels in rats were measured using the enzyme-linked immunosorbent assay. The LH levels were determined in six rats in each group 28 days after the implantation of the gestodene contraceptive implants. The results are as follows.
The LH levels in rats are shown in the table below.

| Group | Replicates | LH (IU/L) |
|---|---|---|
| Blank group | 6 | 73.71 ± 4.53 |
| Experiment I low-dose group | 6 | 47.41 ± 3.39 |
| Experiment I medium-dose group (Comparative Example 1) | 6 | 42.31 ± 2.43 |
| Experiment I high-dose group | 6 | 37.46 ± 1.99 |
| Experiment II low-dose group | 6 | 47.97 ± 3.54 |
| Experiment II medium-dose group (Example 22-1) | 6 | 42.89 ± 2.78 |
| Experiment II high-dose group | 6 | 37.95 ± 1.98 |
| Experiment III low-dose group | 6 | 44.35 ± 3.79 |
| Experiment III medium-dose group (Example 22-2) | 6 | 39.46 ± 2.34 |
| Experiment III high-dose group | 6 | 31.43 ± 1.98 |
| Experiment IV low-dose group | 6 | 47.02 ± 3.36 |

(continued)

| Group | Replicates | LH (IU/L) |
|---|---|---|
| Experiment IV medium-dose group (Example 22-3) | 6 | 40.81 ± 2.63 |
| Experiment IV high-dose group | 6 | 33.89 ± 1.93 |

As shown in Fig. 3, the low-dose, medium-dose, and high-dose groups of Experiments I, II, III, and IV all significantly reduced the LH levels in rats compared to the blank group. The higher the dose, the lower the LH levels, indicating a better hormone inhibitory effect. In particular, the experiment group III added the insoluble excipient AL-1FP mesoporous silica exhibited the best *in vitro* drug release stability, and the addition of this excipient greatly improved the daily drug release amount. The *in vivo* data also showed lower LH levels and a better hormone inhibitory effect. It shows that, compared to silicone tubes filled only with gestodene bulk drug powder, the addition of insoluble excipients can significantly improve *in vitro* drug release and enhance *in vivo* efficacy.

## V. Four types of membrane-controlled long-acting implants

## (I) Preparation of membrane-controlled long-acting implants

The membrane-controlled implant consists of an addition-cure silicone tube and a powder-type medicinal core. The silicone tube was produced by adding a reinforcing agent to methyl vinyl silicone rubber raw material, followed by the addition of a rubber compound made from hydrogen-containing silicone oil, an inhibitor, and a catalyst as compounding agents and then high-temperature vulcanization through the extrusion process. The medicinal core was prepared by directly mixing the drug powder with an excipient. Finally, The uniformly mixed drug powder was filled into the silicone tube. The silicone tube filled with the drug-excipient mixture was sealed with end-sealing adhesive to prepare the membrane-controlled long-acting implant.

(1) The preparation for the addition-cure silicone tube is as follows.

The basic preparation for the silicone tube includes a base polymer, a reinforcing agent, a cross-linking agent, a catalyst, and an inhibitor. The selected base polymer was methyl vinyl polysiloxane with a vinyl content of 0.18%-0.23%; the reinforcing agent was fumed white carbon black, added in an amount of 30-50 PHR; the cross-linking agent was hydrogen-containing silicone oil, added in an amount of 0.3-2.0 PHR; the platinum catalyst had a concentration of 3000 ppm and was added in an amount of 0.000002-0.5 PHR; the inhibitor was 2-methyl-3-butyn-2-ol, added in an amount of 0.03-2.0 PHR.

The specific preparation is as follows.

| Material name | Amount |
|---|---|
| Methyl vinyl silicone rubber | 100 PHR |
| Content of vinyl groups in vinyl polysiloxane | 0.17 mol% |
| Fumed white carbon black | 30 PHR |
| Hydrogen-containing silicone oil | 1.01 PHR |
| Molar ratio of Si-H groups in hydrogen-containing silicone oil to vinyl groups in methyl vinyl silicone rubber | 1.2:1 |
| Content of hydrogen in hydrogen-containing silicone oil | 0.75 mol% |
| 2-Methyl-3-butyn-2-ol | 0.7 PHR |
| Platinum catalyst (3000 ppm) | 0.00001 PHR |

(2) The preparation process for the addition-type silicone tube comprises the following steps.

(i) Drying pre-treatment of rubber material: The reinforcing agent, fumed white carbon black, was placed in an oven at 130°C and dried for 12-24 hours for later use. The methyl vinyl silicone rubber raw material was dried at 40°C for 12-24 hours for later use.

(ii) Preparation of rubber compound: The prescribed amount of fumed white carbon black and methyl vinyl silicone rubber raw material were fed into a kneader, ensuring uniform mixing of the fumed white carbon black and raw rubber to obtain the rubber compound. The rubber compound was then transferred to an open mill and processed by extruding and passing through the open mill. The process involved further mixing of the rubber compound by the shearing action of the two rollers of the open mill. The rubber compound was then removed from the open mill in the form of sheets, wrapped in cling film, sealed in a ziplock bag, and stored in a desiccator for 12-24 hours for later use.

(iii) Preparation of components A and B: The rubber compound was evenly divided into components A and B. The prescribed amount of hydrogen-containing silicone oil and inhibitor were added to component A, mixed uniformly in an open mill, and then processed by passing through the open mill several times before being removed in the form of sheets. The prescribed amount of platinum catalyst was added to component B, mixed uniformly in an open mill, and then processed by passing through the open mill several times before being removed in the form of sheets. The prepared components A and B were individually wrapped in cling film, sealed in a ziplock bag, and stored in a desiccator for 12-24 hours for later use.

(iv) Mixing of components A and B: The stored components A and B were fed into an open mill in a 1:1 ratio, and processed by performing triangular wrapping and passing through the open mill 4-6 times, ensuring thorough and uniform mixing of components A and B. The mixture was then removed from the open mill in the form of sheets and cut into strips.

(v) Extrusion of silicone tube: The appropriate-sized mold was installed, and the screw speed of the extruder was set. The cut silicone strips were fed into the extruder, and the rubber material was continuously extruded through the mold opening driven by the rotation of the screw to form a silicone tube.

(vi) First vulcanization treatment: The silicone tube extruded from the mold opening was first passed through a front drying tunnel (vertical hot air vulcanization channel) for rapid high-temperature vulcanization, known as the first vulcanization treatment (at a temperature of 300°C for approximately 5 seconds). This process quickly transformed the silicone tube from a sticky and soft state to an elastic state, achieving preliminary shaping.

(vii) Second vulcanization treatment: After the first vulcanization, the silicone tube entered a rear drying tunnel (horizontal hot air vulcanization channel) for continued vulcanization, known as the second vulcanization treatment (at a temperature of 280°C for approximately 2 minutes). This process ensured a more complete vulcanization reaction of the silicone tube, achieving optimal cross-linking. The finished silicone tube was then continuously transported out by the conveyor belt of the rear drying tunnel. After oven vulcanization at 180°C for 48 hours, a silicone tube with a wall thickness of 0.2 mm was prepared.

Processing of silicone tube: The addition-cure silicone tube (outer diameter of 2.4 mm, inner diameter of 1.6 mm) with qualified appearance and performance was cut into the same length of 44 mm. The cut tubes were placed in a 100 mL beaker with an appropriate amount of distilled water and subjected to ultrasonication for 30 minutes. After ultrasonication, the tubes were rinsed three times with distilled water, then washed with 75% ethanol, and naturally air-dried for later use.

Preparation of implant: One end of the silicone tube was sealed and dried, while the silicone tube was filled with 80 mg of pharmaceutical composition powder through the other end using a tool, and then sealed with end-sealing adhesive. After the end-sealing adhesive was cured, the silicone tube was kneaded and shaken to ensure uniform mixing of the drugs and excipients within the tube. Finally, the outer surface of the silicone tube was cleaned to remove any powder residue.

During the filling process, it was observed that there was no airborne powder or spontaneous aggregation in the pharmaceutical composition.

(3) The preparation for the powder-type medicinal core is as follows.

| Bulk drug | Bulk drug content (%) | Molecular weight | Solubility | Acidity/ Alkalinity | LogP | pKa | Insoluble excipient content (%) | Insoluble pH adjuster content (%) | Stable release amount/ μg |
|---|---|---|---|---|---|---|---|---|---|
| Ibuprofen | 50 | 206 | 60 μg/mL | Acidic | 3.72 | 4.45 | XDP3150 mesoporous silica 50 | Fumaric acid 0 | 260 |
| | 50 | 206 | 60 μg/mL | Acidic | 3.72 | 4.45 | XDP3150 mesoporous silica 45 | Fumaric acid 5 | 102 |
| | 50 | 206 | 60 μg/mL | Acidic | 3.72 | 4.45 | XDP3150 mesoporous silica 30 | Fumaric acid 20 | 88 |
| | 50 | 206 | 60 μg/mL | Acidic | 3.72 | 4.45 | XDP3150 mesoporous silica 15 | Fumaric acid 35 | 72 |
| Paliperidone | 50 | 426 | 50 μg/mL | Alkaline | 3.02 | 7.72 | XDP3050 mesoporous silica 50 | Magnesium hydroxide 0 | 6 |
| | 50 | 426 | 50 μg/mL | Alkaline | 3.02 | 7.72 | XDP3050 mesoporous silica 45 | Magnesium hydroxide 5 | 4 |
| | 50 | 426 | 50 μg/mL | Alkaline | 3.02 | 7.72 | XDP3050 mesoporous silica 30 | Magnesium hydroxide 20 | 3 |
| | 50 | 426 | 50 μg/mL | Alkaline | 3.02 | 7.72 | XDP3050 mesoporous silica 15 | Magnesium hydroxide 35 | 2 |
| Meloxicam | 50 | 351 | 3.4 μg/mL | Acidic | 3.35 | 4 | Fumed white carbon black 50 | Boric acid 0 | 9 |
| | 50 | 351 | 3.4 μg/mL | Acidic | 3.35 | 4 | Fumed white carbon black 45 | Boric acid 5 | 5 |
| | 50 | 351 | 3.4 μg/mL | Acidic | 3.35 | 4 | Fumed white carbon black 30 | Boric acid 20 | 5 |
| | 50 | 351 | 3.4 μg/mL | Acidic | 3.35 | 4 | Fumed white carbon black 15 | Boric acid 35 | 4 |

(continued)

| Bulk drug | Bulk drug content (%) | Molecular weight | Solubility | Acidity/ Alkalinity | LogP | pKa | Insoluble excipient content (%) | Insoluble pH adjuster content (%) | Stable release amount/ $\mu g$ |
|---|---|---|---|---|---|---|---|---|---|
| Puerarin | 50 | 432 | 4 mg/mL | Alkaline | -0.67 | 6.45 | AL-1FP mesoporous silica 50 | Zinc hydroxide 0 | 9 |
| | 50 | 432 | 4 mg/mL | Alkaline | -0.67 | 6.45 | AL-1FP mesoporous silica 45 | Zinc hydroxide 5 | 11 |
| | 50 | 432 | 4 mg/mL | Alkaline | -0.67 | 6.45 | AL-1FP mesoporous silica 30 | Zinc hydroxide 20 | 10 |
| | 50 | 432 | 4 mg/mL | Alkaline | -0.67 | 6.45 | AL-1FP mesoporous silica 15 | Zinc hydroxide 35 | 9 |

Note: The content percentage of the bulk drug, insoluble excipient, and insoluble pH adjuster in the table above refers to the mass percentage in the medicinal core; the total mass of the medicinal core is 80 mg; the particle size $D_{50}$ of ibuprofen is 80 $\mu m$; the particle size $D_{50}$ of paliperidone is 10 $\mu m$; the particle size $D_{50}$ of meloxicam is 80 $\mu m$; the particle size $D_{50}$ of puerarin is 80 $\mu m$.

Preparation of pharmaceutical composition: The components listed in the table above were mixed to obtain a pharmaceutical composition (powder-type medicinal core).

Preparation of pharmaceutical composition: The components listed in Table 8 were mixed to obtain a pharmaceutical composition (powder-type medicinal core).

Herein, the ibuprofen bulk drug had a particle size of 80 $\mu m$; a molecular weight of 206.28; insoluble in water with a solubility of 60 $\mu g/mL$; acidic; a LogP value of 3.72; a pKa of 4.45.

The meloxicam bulk drug had a particle size of 80 $\mu m$; a molecular weight of 351.4; insoluble in water with a solubility of 3.4 $\mu g/mL$; acidic; a LogP value of 3.35; a pKa of 4.

The paliperidone bulk drug had a particle size of 10 $\mu m$; a molecular weight of 426; insoluble in water with a solubility of 50 $\mu g/mL$; alkaline; a LogP value of 3.02; a pKa of 7.72.

The puerarin bulk drug had a particle size of 80 $\mu m$; a molecular weight of 432; sparingly soluble in water with a solubility of 4 mg/mL; alkaline; a LogP value of -0.67; a pKa of 6.45.

| Example No. | Type and mass (mg) of bulk drug | Type and mass (mg) of insoluble excipient | | Mass ratio of bulk drug to insoluble excipient |
|---|---|---|---|---|
| Example 23-1 | Ibuprofen 40 mg | XDP3150 mesoporous silica 36 mg | Fumaric acid 4 mg | 1:1 |
| Example 23-2 | Ibuprofen 40 mg | XDP3150 mesoporous silica 24 mg | Fumaric acid 16 mg | 1:1 |
| Example 23-3 | Ibuprofen 40 mg | XDP3150 mesoporous silica 12 mg | Fumaric acid 28 mg | 1:1 |
| Example 23-4 | Ibuprofen 40 mg | XDP3150 mesoporous silica 40 mg | / | 1:1 |
| Example 24-1 | Meloxicam 40 mg | Fumed white carbon black 36 mg | Boric acid 4 mg | 1:1 |
| Example 24-2 | Meloxicam 40 mg | Fumed white carbon black 24 mg | Boric acid 16 mg | 1:1 |
| Example 24-3 | Meloxicam 40 mg | Fumed white carbon black 12 mg | Boric acid 28 mg | 1:1 |

(continued)

| Example No. | Type and mass (mg) of bulk drug | Type and mass (mg) of insoluble excipient | | Mass ratio of bulk drug to insoluble excipient |
|---|---|---|---|---|
| Example 24-4 | Meloxicam 40 mg | Fumed white carbon black 40 mg | / | 1:1 |
| Example 25-1 | Paliperidone 40 mg | XDP3050 mesoporous silica 36 mg | Magnesium hydroxide 4 mg | 1:1 |
| Example 25-2 | Paliperidone 40 mg | XDP3050 mesoporous silica 24 mg | Magnesium hydroxide 16 mg | 1:1 |
| Example 25-3 | Paliperidone 40 mg | XDP3050 mesoporous silica 12 mg | Magnesium hydroxide 28 mg | 1:1 |
| Example 25-4 | Paliperidone 40 mg | XDP3050 mesoporous silica 40 mg | / | 1:1 |
| Example 26-1 | Puerarin 40 mg | AL-1FP mesoporous silica 36 mg | Zinc hydroxide 4 mg | 1:1 |
| Example 26-2 | Puerarin 40 mg | AL-1FP mesoporous silica 24 mg | Zinc hydroxide 16 mg | 1:1 |
| Example 26-3 | Puerarin 40 mg | AL-1FP mesoporous silica 12 mg | Zinc hydroxide 28 mg | 1:1 |
| Example 26-4 | Puerarin 40 mg | AL-1FP mesoporous silica 40 mg | / | 1:1 |

(4) Processing of silicone tube: The addition-cure silicone tube (outer diameter of 2.4 mm, inner diameter of 1.6 mm) with qualified appearance and performance was cut into the same length of 44 mm. The cut tubes were placed in a 100 mL beaker with an appropriate amount of distilled water and subjected to ultrasonication for 30 minutes. After ultrasonication, the tubes were rinsed three times with distilled water, then washed with 75% ethanol, and naturally air-dried for later use.

(5) Preparation of implant: One end of the silicone tube was sealed and dried, while the silicone tube was filled with 80 mg of pharmaceutical composition powder through the other end using a tool, and then sealed with end-sealing adhesive. After the end-sealing adhesive was cured, the silicone tube was kneaded and shaken to ensure uniform mixing of the drugs and excipients within the tube. Finally, the outer surface of the silicone tube was cleaned to remove any powder residue.

**(II) Content determination and *in vitro* release testing methods for membrane-controlled long-acting implants**

**(1) Ibuprofen**

**Chromatographic conditions**

Chromatographic column: Agilent® C18 column (4.6 mm × 150 mm, 5 μm);
Mobile phase: methanol: 1% sodium acetate buffer solution (70:30, v/v);
Column temperature: 35°C;
Detection wavelength: 273 nm;
Flow rate: 1.0 mL·min$^{-1}$;
Injection volume: 20 μL.

**Release rate determination method**

The release experiment was conducted using the horizontal shaking method. One implant was taken, and both ends of the implant were fixed to the bottom and wall of a 20 mL vial using a silicone adhesive (to prevent the implant from floating on the liquid surface, which could lead to inaccurate release results). After adhesion, the implant was left to stand for 12 hours to allow the silicone adhesive to fully cure. Exactly 15 mL of distilled water was measured as the release medium and injected into a conical flask. The conical flask was then placed in a thermostatic air shaker set at 37°C and shaken at a frequency of 100 rpm. The medium was replaced with an equal volume of fresh medium every 24 hours. The release solution was filtered through a 0.22 $\mu$m microporous filter membrane and detected under the above chromatographic conditions with an injection volume of 20 $\mu$L.

**(2) Paliperidone**

**Chromatographic conditions**

Chromatographic column: Agilent® C8 column (4.6 $\times$ 100 mm, 2.7 $\mu$m);
Mobile phase: methanol: acetonitrile: water (5:25:70, v/v) (pH adjusted to 3 with phosphoric acid and triethylamine);
Column temperature: 35°C;
Detection wavelength: 270 nm;
Flow rate: 0.8 mL·min$^{-1}$;
Injection volume: 20 $\mu$L.

**Release rate determination method**

The release experiment was conducted using the horizontal shaking method. One implant was taken, and both ends of the implant were fixed to the bottom and wall of a 100 mL stoppered conical flask using a silicone adhesive (to prevent the implant from floating on the liquid surface, which could lead to inaccurate release results). After adhesion, the implant was left to stand for 12 hours to allow the silicone adhesive to fully cure. Exactly 100 mL of distilled water was measured as the release medium and injected into a conical flask. The conical flask was then placed in a thermostatic air shaker set at 37°C and shaken at a frequency of 100 rpm. The medium was replaced with an equal volume of fresh medium every 24 hours. The release solution was filtered through a 0.22 $\mu$m microporous filter membrane and detected under the above chromatographic conditions with an injection volume of 20 $\mu$L.

**(3) Meloxicam**

**Chromatographic conditions**

Chromatographic column: Agilent® C18 column (4.6 mm $\times$ 150 mm, 5 $\mu$m);
Mobile phase: methanol: 0.5% phosphoric acid aqueous solution (70:30, v/v);
Column temperature: room temperature;
Detection wavelength: 270 nm;
Flow rate: 1.0 mL·min$^{-1}$;
Injection volume: 20 $\mu$L.

**Release rate determination method**

The release experiment was conducted using the horizontal shaking method. One implant was taken, and both ends of the implant were fixed to the bottom and wall of a 20 mL vial using a silicone adhesive (to prevent the implant from floating on the liquid surface, which could lead to inaccurate release results). After adhesion, the implant was left to stand for 12 hours to allow the silicone adhesive to fully cure. Exactly 15 mL of distilled water was measured as the release medium and injected into a conical flask. The conical flask was then placed in a thermostatic air shaker set at 37°C and shaken at a frequency of 100 rpm. The medium was replaced with an equal volume of fresh medium every 24 hours. The release solution was filtered through a 0.22 $\mu$m microporous filter membrane and detected under the above chromatographic conditions with an injection volume of 20 $\mu$L.

**(4) Puerarin**

**Chromatographic conditions**

Chromatographic column: Diamonsil® C$_{18}$ column (4.6 mm × 250 mm, 5 μm);
Mobile phase: methanol-water (70:30, v/v);
Column temperature: 35°C;
Detection wavelength: 250 nm;
Flow rate: 1.0 mL·min$^{-1}$;
Injection volume: 20 μL.

**Release rate determination method**

The release experiment was conducted using the horizontal shaking method. One implant was taken, and both ends of the implant were fixed to the bottom and wall of a 20 mL vial using a silicone adhesive (to prevent the implant from floating on the liquid surface, which could lead to inaccurate release results). After adhesion, the implant was left to stand for 12 hours to allow the silicone adhesive to fully cure. Exactly 15 mL of distilled water was measured as the release medium and injected into a conical flask. The conical flask was then placed in a thermostatic air shaker set at 37°C and shaken at a frequency of 100 rpm. The medium was replaced with an equal volume of fresh medium every 24 hours. The release solution was filtered through a 0.22 μm microporous filter membrane and detected under the above chromatographic conditions with an injection volume of 20 μL.

**(III) *In vitro* release results of membrane-controlled long-acting implants**

**(1) Ibuprofen**

| Time (d) | Daily release dose (μg) | | | |
|---|---|---|---|---|
| | 36 mg XDP3150 + 4 mg fumaric acid | 24 mg XDP3150 + 16 mg fumaric acid | 12 mg XDP3150 + 28 mg fumaric acid | 40 mg XDP3150 + 0 mg fumaric acid |
| 1 | 120.6 | 116.4 | 114.1 | 307.3 |
| 2 | 144.3 | 153.6 | 134.6 | 363.1 |
| 3 | 156.3 | 133.2 | 154.1 | 272.1 |
| 4 | 156.5 | 123.0 | 162.8 | 310.6 |
| 5 | 141.4 | 142.6 | 91.5 | 339.8 |
| 6 | 131.8 | 111.2 | 88.3 | 290.7 |
| 7 | 120.0 | 103.3 | 84.2 | 300.8 |
| 8 | 108.1 | 96.6 | 120.3 | 310.3 |
| 9 | 112.5 | 100.6 | 114.1 | 296.3 |
| 10 | 106.4 | 84.1 | 97.7 | 289.2 |
| 11 | 106.8 | 100.4 | 71.4 | 290.3 |
| 12 | 107.3 | 97.5 | 74.7 | 268.2 |
| 13 | 110.4 | 88.3 | 71.1 | 274.5 |
| 14 | 109.1 | 93.9 | 70.9 | 265.4 |
| 15 | 101.9 | 90.9 | 70.6 | 265.6 |
| 16 | 100.4 | 90.7 | 71.2 | 263.2 |
| 17 | 105.3 | 90.6 | 71.6 | 270.8 |
| 18 | 105.7 | 90.3 | 72.4 | 278.3 |

(continued)

| Time (d) | Daily release dose (μg) | | | |
| --- | --- | --- | --- | --- |
| | 36 mg XDP3150 + 4 mg fumaric acid | 24 mg XDP3150 + 16 mg fumaric acid | 12 mg XDP3150 + 28 mg fumaric acid | 40 mg XDP3150 + 0 mg fumaric acid |
| 19 | 107.8 | 89.6 | 70.8 | 272.4 |
| 20 | 106.9 | 88.5 | 73.2 | 265.3 |
| 21 | 105.4 | 88.3 | 72.7 | 264.5 |
| 22 | 103.5 | 88.1 | 72.5 | 264.2 |
| 23 | 102.6 | 89.3 | 72.4 | 263.8 |
| 24 | 101.7 | 90.2 | 72.8 | 262.6 |
| 25 | 104.3 | 91.2 | 73.2 | 261.9 |
| 26 | 103.4 | 91.4 | 72.1 | 261.5 |
| 27 | 103.2 | 88.6 | 73.0 | 261.8 |
| 28 | 102.8 | 87.9 | 72.6 | 261.3 |
| 29 | 102.0 | 90.3 | 73.4 | 260.9 |
| 30 | 101.8 | 89.0 | 72.9 | 262.8 |
| 31 | 100.9 | 89.3 | 72.5 | 261.4 |
| 32 | 101.3 | 88.9 | 74.1 | 261.7 |
| 33 | 102.5 | 90.3 | 73.6 | 260.9 |
| 34 | 102.0 | 90.6 | 72.5 | 260.4 |
| 35 | 101.8 | 89.6 | 71.5 | 261.5 |
| 36 | 100.9 | 91.4 | 71.9 | 261.3 |
| 37 | 100.7 | 90.1 | 72.3 | 261.8 |
| 38 | 101.6 | 91.2 | 72.1 | 260.8 |
| 39 | 100.4 | 88.7 | 73.2 | 261.3 |
| 40 | 101.8 | 87.9 | 71.8 | 260.4 |
| 41 | 104.2 | 89.3 | 72.4 | 261.3 |
| 42 | 102.6 | 88.5 | 72.9 | 261.8 |
| 43 | 102.2 | 89.6 | 72.6 | 261.5 |
| 44 | 100.8 | 90.4 | 72.4 | 261.9 |
| 45 | 104.9 | 89.6 | 72.0 | 260.5 |
| 46 | 100.3 | 88.6 | 72.8 | 261.3 |
| 47 | 101.5 | 90.4 | 73.1 | 260.3 |
| 48 | 109.0 | 90.2 | 72.3 | 260.8 |
| 49 | 100.8 | 90.7 | 72.5 | 261.3 |
| 50 | 101.2 | 87.5 | 72.1 | 262.5 |
| 51 | 102.7 | 88.6 | 73.1 | 261.6 |
| 52 | 101.6 | 86.3 | 72.5 | 261.5 |
| 53 | 100.4 | 89.5 | 72.2 | 260.3 |
| 54 | 100.8 | 89.4 | 72.9 | 261.8 |
| 55 | 100.7 | 89.6 | 72.4 | 260.4 |
| 56 | 101.3 | 90.2 | 72.5 | 260.6 |

(continued)

| Time (d) | Daily release dose (µg) | | | |
|---|---|---|---|---|
| | 36 mg XDP3150 + 4 mg fumaric acid | 24 mg XDP3150 + 16 mg fumaric acid | 12 mg XDP3150 + 28 mg fumaric acid | 40 mg XDP3150 + 0 mg fumaric acid |
| 57 | 100.1 | 88.6 | 73.1 | 260.8 |
| 58 | 1029 | 88.9 | 71.0 | 260.3 |
| 59 | 100.5 | 90.3 | 71.1 | 261.2 |
| 60 | 103.5 | 89.2 | 72.3 | 262.4 |
| 61 | 102.2 | 90.3 | 72.5 | 261.1 |
| 62 | 101.8 | 87.5 | 72.4 | 260.9 |
| 63 | 101.5 | 89.7 | 72.6 | 261.5 |
| 64 | 102.3 | 88.8 | 73.2 | 269.8 |
| 65 | 100.7 | 89.8 | 73.0 | 261.5 |
| 66 | 100.4 | 88.7 | 72.0 | 261.3 |
| 67 | 100.8 | 90.5 | 72.2 | 260.3 |
| 68 | 102.4 | 88.6 | 72.5 | 260.8 |
| 69 | 101.5 | 88.9 | 73.0 | 261.3 |
| 70 | 103.5 | 89.6 | 72.0 | 260.1 |
| 71 | 101.2 | 88.2 | 72.4 | 260.5 |
| 72 | 100.8 | 90.3 | 72.2 | 262.5 |
| 73 | 101.2 | 88.5 | 72.1 | 260.3 |
| 74 | 102.7 | 88.2 | 73.5 | 261.8 |
| 75 | 100.6 | 88.9 | 72.7 | 261.9 |
| 76 | 100.6 | 88.7 | 72.3 | 261.3 |
| 77 | 101.8 | 89.4 | 72.4 | 260.6 |
| 78 | 102.4 | 90.2 | 72.1 | 262.5 |
| 79 | 101.7 | 88.9 | 73.2 | 260.5 |
| 80 | 100.3 | 90.9 | 72.0 | 260.8 |
| 81 | 101.3 | 89.6 | 72.0 | 260.9 |
| 82 | 102.8 | 89.3 | 72.4 | 262.3 |
| 83 | 101.2 | 90.2 | 72.8 | 262.4 |
| 84 | 104.7 | 88.4 | 72.0 | 260.4 |
| 85 | 102.1 | 88.8 | 72.5 | 261.9 |
| 86 | 101.7 | 88.2 | 72.4 | 260.4 |
| 87 | 100.5 | 89.3 | 72.3 | 261.4 |
| 88 | 101.4 | 88.2 | 71.8 | 260.9 |
| 89 | 100.2 | 88.0 | 72.9 | 260.4 |
| 90 | 102.6 | 88.5 | 72.3 | 260.4 |

As can be seen from the above table, the addition of insoluble excipients in the pharmaceutical composition effectively improved the static electricity of the bulk drug powder during filling, especially when the insoluble excipient was XDP3150 mesoporous silica, which significantly increased the filling efficiency. Additionally, the addition of insoluble acidic excipient fumaric acid resulted in a considerable adjustment in the release amount of the implant, with a marked improvement in the drug release stability.

**(2) Meloxicam**

| Time (d) | Daily release dose (µg) | | | |
|---|---|---|---|---|
| | 36 mg white carbon black + 4 mg boric acid | 24 mg white carbon black + 16 mg boric acid | 12 mg white carbon black + 28 mg boric acid | 40 mg white carbon black + 0 mg boric acid |
| 1 | 19.7 | 2.2 | 29.7 | 11.9 |
| 2 | 10.0 | 0.9 | 5.4 | 9.4 |
| 3 | 5.3 | 8.6 | 15.8 | 30.4 |
| 4 | 5.2 | 6.7 | 8.7 | 39.0 |
| 5 | 6.6 | 13.7 | 6.0 | 34.3 |
| 6 | 5.5 | 8.3 | 4.5 | 20.4 |
| 7 | 4.1 | 14.7 | 5.8 | 21.4 |
| 8 | 3.8 | 14.7 | 7.0 | 26.7 |
| 9 | 3.3 | 7.5 | 6.2 | 23.3 |
| 10 | 3.5 | 11.1 | 6.3 | 17.8 |
| 11 | 5.3 | 16.9 | 7.4 | 30.7 |
| 12 | 5.1 | 15.0 | 6.2 | 26.9 |
| 13 | 6.0 | 10.0 | 5.8 | 14.0 |
| 14 | 4.0 | 8.4 | 4.5 | 15.0 |
| 15 | 5.5 | 7.0 | 4.6 | 8.5 |
| 16 | 4.9 | 6.1 | 5.2 | 7.5 |
| 17 | 5.0 | 6.1 | 5.2 | 8.1 |
| 18 | 5.4 | 7.2 | 5.8 | 8.3 |
| 19 | 4.3 | 6.5 | 4.8 | 8.3 |
| 20 | 5.3 | 6.2 | 4.5 | 9.9 |
| 21 | 5.1 | 5.8 | 4.7 | 8.4 |
| 22 | 6.0 | 6.3 | 4.2 | 8.8 |
| 23 | 4.0 | 6.6 | 5.1 | 7.9 |
| 24 | 5.5 | 5.4 | 4.6 | 6.0 |
| 25 | 4.9 | 5.8 | 4.6 | 7.8 |
| 26 | 5.0 | 5.2 | 4.0 | 8.4 |
| 27 | 4.6 | 6.3 | 4.2 | 7.2 |
| 28 | 4.3 | 6.2 | 4.9 | 7.3 |
| 29 | 5.3 | 5.3 | 4.3 | 6.8 |
| 30 | 5.3 | 7.2 | 4.1 | 7.0 |
| 31 | 5.1 | 5.4 | 3.7 | 8.3 |
| 32 | 6.0 | 5.3 | 4.7 | 8.5 |
| 33 | 4.0 | 5.2 | 4.4 | 8.4 |
| 34 | 5.5 | 6.7 | 4.7 | 8.8 |

(continued)

| Time (d) | Daily release dose (µg) | | | |
|---|---|---|---|---|
| | 36 mg white carbon black + 4 mg boric acid | 24 mg white carbon black + 16 mg boric acid | 12 mg white carbon black + 28 mg boric acid | 40 mg white carbon black + 0 mg boric acid |
| 35 | 4.0 | 6.1 | 4.2 | 8.0 |
| 36 | 5.5 | 5.6 | 5.4 | 7.4 |
| 37 | 4.9 | 6.2 | 4.8 | 7.9 |
| 38 | 5.0 | 5.8 | 4.6 | 8.4 |
| 39 | 5.4 | 6.3 | 4.4 | 7.0 |
| 40 | 4.3 | 6.6 | 4.2 | 7.3 |
| 41 | 4.5 | 5.4 | 4.0 | 6.5 |
| 42 | 5.2 | 5.7 | 5.2 | 7.6 |
| 43 | 3.4 | 5.2 | 4.5 | 8.8 |
| 44 | 4.3 | 6.1 | 4.3 | 8.3 |
| 45 | 5.5 | 6.2 | 4.6 | 6.9 |
| 46 | 4.2 | 5.3 | 3.7 | 8.6 |
| 47 | 4.7 | 5.4 | 4.2 | 8.8 |
| 48 | 6.0 | 5.8 | 4.3 | 7.4 |
| 49 | 4.0 | 5.2 | 4.9 | 6.8 |
| 50 | 5.5 | 6.2 | 4.2 | 7.6 |
| 51 | 3.4 | 6.1 | 5.3 | 8.4 |
| 52 | 4.4 | 5.9 | 5.1 | 7.9 |
| 53 | 5.6 | 6.3 | 4.6 | 7.1 |
| 54 | 6.2 | 6.3 | 4.6 | 6.9 |
| 55 | 3.3 | 6.6 | 4.0 | 7.0 |
| 56 | 4.8 | 5.8 | 5.3 | 8.5 |
| 57 | 5.5 | 5.4 | 4.7 | 7.3 |
| 58 | 4.9 | 5.2 | 4.2 | 6.8 |
| 59 | 5.0 | 6.6 | 4.1 | 8.0 |
| 60 | 4.6 | 6.2 | 3.9 | 8.3 |
| 61 | 4.3 | 5.8 | 4.2 | 8.1 |
| 62 | 5.3 | 7.2 | 4.4 | 8.2 |
| 63 | 5.3 | 5.6 | 4.8 | 8.8 |
| 64 | 5.1 | 5.1 | 4.4 | 8.5 |
| 65 | 4.1 | 5.7 | 4.2 | 7.4 |
| 66 | 4.0 | 5.1 | 5.1 | 7.6 |
| 67 | 3.8 | 5.2 | 4.6 | 8.8 |
| 68 | 6.1 | 6.3 | 4.4 | 9.0 |
| 69 | 5.2 | 6.6 | 4.0 | 8.4 |
| 70 | 4.5 | 5.9 | 5.3 | 8.8 |
| 71 | 3.9 | 7.3 | 4.0 | 8.0 |
| 72 | 8.6 | 6.5 | 4.1 | 7.3 |

(continued)

| Time (d) | Daily release dose (μg) | | | |
|---|---|---|---|---|
| | 36 mg white carbon black + 4 mg boric acid | 24 mg white carbon black + 16 mg boric acid | 12 mg white carbon black + 28 mg boric acid | 40 mg white carbon black + 0 mg boric acid |
| 73 | 4.5 | 6.2 | 4.1 | 7.9 |
| 74 | 5.3 | 6.2 | 3.7 | 8.8 |
| 75 | 4.6 | 5.6 | 4.7 | 7.9 |
| 76 | 4.9 | 6.1 | 4.4 | 7.3 |
| 77 | 3.8 | 6.6 | 4.7 | 6.6 |
| 78 | 5.5 | 5.2 | 3.7 | 7.6 |
| 79 | 3.8 | 5.7 | 4.6 | 8.9 |
| 80 | 4.2 | 5.2 | 4.3 | 8.1 |
| 81 | 4.4 | 6.4 | 4.9 | 7.3 |
| 82 | 5.2 | 7.8 | 4.5 | 8.5 |
| 83 | 5.6 | 5.4 | 5.0 | 7.3 |
| 84 | 3.9 | 5.8 | 3.9 | 6.6 |
| 85 | 4.2 | 7.2 | 4.2 | 7.0 |
| 86 | 4.0 | 5.6 | 4.8 | 8.7 |
| 87 | 3.5 | 5.1 | 4.0 | 8.3 |
| 88 | 4.7 | 5.7 | 3.9 | 8.0 |
| 89 | 4.3 | 5.1 | 4.7 | 8.1 |
| 90 | 4.6 | 5.2 | 4.2 | 8.8 |

As can be seen from the above table, the addition of insoluble excipients in the pharmaceutical composition effectively improved the static electricity of the bulk drug powder during filling, especially when the insoluble excipient was fumed white carbon black, which significantly increased the filling efficiency. Additionally, the addition of insoluble acidic excipient boric acid resulted in a considerable adjustment in the release amount of the implant, with a marked improvement in the drug release stability.

**(3) Paliperidone**

| Time (d) | Daily dose release (μg) | | | |
|---|---|---|---|---|
| | 36 mg XDP3050 + 4 mg magnesium hydroxide | 24 mg XDP3050 + 16 mg magnesium hydroxide | 12 mg XDP3050 + 28 mg magnesium hydroxide | 40 mg XDP3050 + 0 mg magnesium hydroxide |
| 1 | 3.1 | 2.5 | 5.7 | 2.6 |
| 2 | 1.9 | 3.0 | 2.7 | 2.1 |
| 3 | 1.9 | 4.8 | 3.4 | 1.0 |
| 4 | 3.8 | 3.2 | 3.2 | 0.9 |
| 5 | 2.5 | 0.4 | 2.8 | 0.7 |
| 6 | 3.3 | 1.0 | 3.9 | 1.6 |
| 7 | 2.2 | 1.0 | 2.3 | 1.4 |
| 8 | 0.9 | 2.8 | 2.1 | 4.2 |
| 9 | 1.9 | 1.1 | 4.3 | 3.9 |

(continued)

| Time (d) | Daily dose release (µg) | | | |
|---|---|---|---|---|
| | 36 mg XDP3050 + 4 mg magnesium hydroxide | 24 mg XDP3050 + 16 mg magnesium hydroxide | 12 mg XDP3050 + 28 mg magnesium hydroxide | 40 mg XDP3050 + 0 mg magnesium hydroxide |
| 10 | 4.3 | 0.5 | 2.5 | 2.7 |
| 11 | 2.8 | 0.8 | 2.2 | 1.9 |
| 12 | 3.5 | 3.2 | 3.2 | 2.4 |
| 13 | 3.4 | 1.5 | 2.3 | 2.9 |
| 14 | 5.2 | 3.2 | 2.4 | 1.3 |
| 15 | 4.9 | 2.5 | 2.5 | 4.4 |
| 16 | 3.1 | 2.3 | 2.4 | 5.2 |
| 17 | 5.5 | 2.5 | 2.8 | 4.0 |
| 18 | 4.2 | 2.5 | 2.5 | 5.2 |
| 19 | 3.3 | 2.1 | 2.5 | 5.9 |
| 20 | 3.7 | 2.3 | 2.3 | 5.3 |
| 21 | 3.6 | 2.1 | 2.4 | 5.4 |
| 22 | 3.3 | 2.5 | 2.3 | 5.1 |
| 23 | 4.1 | 2.6 | 2.0 | 5.4 |
| 24 | 4.0 | 2.4 | 2.1 | 5.2 |
| 25 | 3.4 | 2.2 | 2.2 | 5.7 |
| 26 | 3.1 | 2.3 | 2.3 | 5.0 |
| 27 | 3.6 | 2.6 | 2.2 | 5.1 |
| 28 | 4.0 | 2.4 | 2.2 | 5.7 |
| 29 | 3.3 | 2.8 | 2.3 | 5.5 |
| 30 | 3.2 | 2.3 | 2.0 | 5.2 |
| 31 | 4.1 | 2.1 | 2.4 | 5.3 |
| 32 | 3.4 | 2.5 | 2.3 | 5.6 |
| 33 | 3.5 | 2.4 | 2.2 | 5.2 |
| 34 | 3.4 | 2.3 | 2.5 | 5.4 |
| 35 | 4.0 | 2.1 | 2.4 | 5.3 |
| 36 | 3.8 | 2.4 | 2.3 | 5.2 |
| 37 | 3.9 | 2.1 | 2.6 | 5.0 |
| 38 | 3.7 | 2.5 | 2.4 | 5.7 |
| 39 | 3.5 | 2.6 | 2.4 | 5.8 |
| 40 | 3.6 | 2.2 | 2.0 | 6.0 |
| 41 | 3.5 | 2.5 | 2.3 | 5.9 |
| 42 | 3.8 | 2.2 | 2.2 | 5.2 |
| 43 | 4.2 | 2.3 | 2.5 | 6.3 |
| 44 | 3.2 | 2.4 | 2.1 | 5.1 |
| 45 | 3.3 | 2.7 | 2.1 | 5.9 |
| 46 | 3.4 | 2.2 | 2.5 | 7.3 |
| 47 | 3.2 | 2.4 | 2.5 | 4.6 |

(continued)

| Time (d) | Daily dose release (µg) | | | |
|---|---|---|---|---|
| | 36 mg XDP3050 + 4 mg magnesium hydroxide | 24 mg XDP3050 + 16 mg magnesium hydroxide | 12 mg XDP3050 + 28 mg magnesium hydroxide | 40 mg XDP3050 + 0 mg magnesium hydroxide |
| 48 | 3.8 | 2.5 | 2.7 | 5.7 |
| 49 | 3.3 | 2.2 | 2.5 | 7.1 |
| 50 | 4.1 | 2.3 | 2.3 | 5.2 |
| 51 | 3.6 | 2.6 | 2.1 | 5.7 |
| 52 | 3.5 | 2.2 | 2.5 | 5.8 |
| 53 | 3.3 | 2.1 | 2.3 | 5.2 |
| 54 | 3.2 | 2.2 | 2.4 | 6.0 |
| 55 | 5.0 | 2.0 | 2.2 | 5.5 |
| 56 | 3.6 | 4.2 | 2.4 | 5.7 |
| 57 | 3.4 | 2.1 | 2.3 | 5.1 |
| 58 | 4.3 | 2.1 | 2.4 | 5.1 |
| 59 | 3.4 | 2.7 | 2.2 | 5.8 |
| 60 | 3.6 | 2.1 | 2.1 | 7.2 |
| 61 | 3.2 | 2.0 | 2.2 | 5.1 |
| 62 | 3.4 | 4.4 | 2.6 | 5.3 |
| 63 | 3.5 | 2.5 | 2.4 | 6.4 |
| 64 | 4.8 | 4.4 | 2.5 | 5.2 |
| 65 | 3.2 | 2.4 | 2.2 | 5.2 |
| 66 | 3.0 | 2.6 | 2.3 | 5.9 |
| 67 | 3.4 | 2.2 | 2.2 | 5.3 |
| 68 | 3.4 | 2.3 | 2.5 | 5.0 |
| 69 | 3.7 | 2.5 | 2.4 | 5.3 |
| 70 | 5.2 | 2.4 | 2.3 | 5.7 |
| 71 | 3.5 | 2.6 | 2.4 | 5.5 |
| 72 | 3.3 | 4.1 | 2.4 | 6.5 |
| 73 | 4.2 | 2.3 | 2.1 | 5.3 |
| 74 | 3.5 | 2.5 | 2.2 | 5.2 |
| 75 | 3.4 | 4.4 | 2.6 | 5.3 |
| 76 | 3.1 | 2.1 | 2.3 | 6.6 |
| 77 | 4.1 | 2.6 | 2.2 | 5.4 |
| 78 | 5.3 | 2.5 | 2.2 | 5.3 |
| 79 | 3.4 | 3.2 | 2.4 | 5.1 |
| 80 | 3.8 | 2.6 | 2.1 | 5.6 |
| 81 | 4.5 | 2.4 | 2.4 | 7.1 |
| 82 | 3.2 | 3.5 | 2.1 | 6.7 |
| 83 | 3.2 | 4.3 | 2.4 | 5.4 |
| 84 | 6.1 | 2.1 | 2.3 | 5.8 |
| 85 | 3.5 | 2.4 | 2.1 | 6.2 |

(continued)

| Time (d) | Daily dose release (μg) | | | |
|---|---|---|---|---|
| | 36 mg XDP3050 + 4 mg magnesium hydroxide | 24 mg XDP3050 + 16 mg magnesium hydroxide | 12 mg XDP3050 + 28 mg magnesium hydroxide | 40 mg XDP3050 + 0 mg magnesium hydroxide |
| 86 | 3.7 | 2.7 | 2.2 | 5.2 |
| 87 | 3.3 | 2.3 | 2.6 | 5.4 |
| 88 | 3.4 | 2.4 | 2.2 | 5.3 |
| 89 | 3.6 | 2.2 | 2.0 | 5.2 |
| 90 | 3.3 | 2.6 | 2.2 | 5.9 |

As can be seen from the above table, the addition of insoluble excipients in the pharmaceutical composition effectively improved the static electricity of the bulk drug powder during filling, especially when the insoluble excipient was XDP3050 mesoporous silica, which significantly increased the filling efficiency. Additionally, the addition of insoluble basic excipient magnesium hydroxide resulted in a considerable adjustment in the release amount of the implant, with a marked improvement in the drug release stability.

**(4) Puerarin**

| Time (d) | Daily release dose (μg) | | | |
|---|---|---|---|---|
| | 36 mg AL-1FP + 4 mg zinc hydroxide | 24 mg AL-1FP + 16 mg zinc hydroxide | 12 mg AL-1FP + 28 mg zinc hydroxide | **40** mg AL-1FP + 0 mg zinc hydroxide |
| 1 | 11.1 | 7.7 | 21.1 | 11.0 |
| 2 | 7.0 | 11.2 | 23.6 | 22.1 |
| 3 | 15.8 | 11.3 | 10.2 | 11.9 |
| 4 | 29.8 | 10.8 | 10.9 | 9.6 |
| 5 | 22.9 | 11.1 | 11.7 | 21.1 |
| 6 | 23.5 | 29.4 | 27.5 | 21.2 |
| 7 | 12.4 | 33.0 | 8.4 | 11.3 |
| 8 | 21.2 | 39.8 | 10.1 | 8.8 |
| 9 | 20.9 | 6.6 | 9.3 | 8.8 |
| 10 | 6.5 | 12.1 | 8.0 | 6.1 |
| 11 | 13.5 | 26.2 | 8.6 | 10.7 |
| 12 | 25.8 | 13.1 | 11.0 | 31.7 |
| 13 | 16.5 | 16.2 | 7.5 | 5.1 |
| 14 | 8.3 | 15.0 | 9.9 | 15.9 |
| 15 | 10.6 | 23.4 | 9.0 | 9.8 |
| 16 | 8.0 | 19.2 | 10.4 | 19.6 |
| 17 | 16.1 | 8.6 | 13.1 | 11.7 |
| 18 | 12.7 | 14.4 | 8.3 | 10.1 |
| 19 | 8.3 | 18.4 | 10.7 | 15.0 |
| 20 | 9.9 | 10.5 | 10.7 | 8.7 |
| 21 | 9.2 | 12.9 | 7.9 | 10.4 |
| 22 | 10.6 | 20.1 | 9.1 | 12.2 |

(continued)

| Time (d) | Daily release dose (μg) | | | |
|---|---|---|---|---|
| | 36 mg AL-1FP + 4 mg zinc hydroxide | 24 mg AL-1FP + 16 mg zinc hydroxide | 12 mg AL-1FP + 28 mg zinc hydroxide | **40** mg AL-1FP + 0 mg zinc hydroxide |
| 23 | 12.5 | 15.2 | 10.3 | 12.2 |
| 24 | 8.4 | 20.7 | 9.7 | 9.8 |
| 25 | 10.7 | 17.1 | 9.7 | 9.5 |
| 26 | 8.7 | 15.1 | 8.9 | 10.3 |
| 27 | 10.3 | 14.4 | 10.4 | 10.4 |
| 28 | 10.3 | 10.5 | 10.3 | 8.7 |
| 29 | 11.2 | 14.6 | 9.0 | 15.1 |
| 30 | 8.8 | 11.0 | 7.9 | 9.2 |
| 31 | 9.7 | 11.3 | 8.8 | 10.7 |
| 32 | 8.8 | 8.6 | 10.0 | 8.1 |
| 33 | 8.3 | 10.2 | 10.6 | 12.7 |
| 34 | 9.3 | 12.0 | 8.5 | 14.6 |
| 35 | 8.4 | 13.7 | 10.2 | 10.2 |
| 36 | 8.2 | 11.7 | 9.6 | 15.4 |
| 37 | 10.6 | 13.3 | 8.1 | 15.9 |
| 38 | 8.6 | 9.7 | 8.0 | 11.4 |
| 39 | 9.5 | 10.8 | 8.9 | 12.2 |
| 40 | 10.9 | 12.7 | 11.6 | 12.5 |
| 41 | 9.3 | 10.9 | 9.4 | 12.5 |
| 42 | 12.2 | 15.5 | 9.0 | 12.4 |
| 43 | 11.4 | 13.7 | 8.3 | 8.0 |
| 44 | 10.7 | 10.5 | 11.3 | 15.4 |
| 45 | 8.5 | 11.3 | 8.4 | 9.5 |
| 46 | 9.5 | 13.7 | 10.8 | 11.7 |
| 47 | 9.7 | 12.3 | 10.4 | 14.8 |
| 48 | 10.2 | 11.5 | 8.7 | 11.6 |
| 49 | 12.1 | 8.8 | 12.0 | 10.7 |
| 50 | 12.9 | 15.1 | 10.6 | 15.4 |
| 51 | 12.9 | 13.9 | 10.5 | 13.8 |
| 52 | 11.1 | 14.0 | 11.2 | 13.1 |
| 53 | 11.8 | 15.1 | 10.6 | 12.8 |
| 54 | 10.8 | 9.3 | 8.5 | 13.6 |
| 55 | 11.9 | 8.9 | 10.7 | 15.0 |
| 56 | 10.9 | 12.4 | 8.1 | 12.4 |
| 57 | 11.8 | 10.0 | 10.4 | 15.7 |
| 58 | 11.9 | 15.4 | 9.3 | 14.4 |
| 59 | 10.1 | 12.1 | 8.6 | 8.6 |
| 60 | 12.7 | 8.8 | 8.1 | 12.3 |

(continued)

| Time (d) | Daily release dose (µg) | | | |
|---|---|---|---|---|
| | 36 mg AL-1FP + 4 mg zinc hydroxide | 24 mg AL-1FP + 16 mg zinc hydroxide | 12 mg AL-1FP + 28 mg zinc hydroxide | **40** mg AL-1FP + 0 mg zinc hydroxide |
| 61 | 9.0 | 11.7 | 11.2 | 15.1 |
| 62 | 12.1 | 9.7 | 11.0 | 13.7 |
| 63 | 8.2 | 9.8 | 9.7 | 12.3 |
| 64 | 10.0 | 8.9 | 9.0 | 10.1 |
| 65 | 9.4 | 15.1 | 10.6 | 11.5 |
| 66 | 9.7 | 11.6 | 9.6 | 11.4 |
| 67 | 9.6 | 9.7 | 11.8 | 15.7 |
| 68 | 10.2 | 15.4 | 11.4 | 8.7 |
| 69 | 8.4 | 12.9 | 8.1 | 13.0 |
| 70 | 9.5 | 10.3 | 10.5 | 12.2 |
| 71 | 10.1 | 10.3 | 9.1 | 11.2 |
| 72 | 10.4 | 11.4 | 8.9 | 13.2 |
| 73 | 12.6 | 15.3 | 9.2 | 11.7 |
| 74 | 10.8 | 14.1 | 10.8 | 8.9 |
| 75 | 11.2 | 14.1 | 10.2 | 14.3 |
| 76 | 9.6 | 14.9 | 11.2 | 10.9 |
| 77 | 10.8 | 11.2 | 9.9 | 14.3 |
| 78 | 8.4 | 15.5 | 8.0 | 12.4 |
| 79 | 11.5 | 8.6 | 8.6 | 10.2 |
| 80 | 10.7 | 15.1 | 11.0 | 9.5 |
| 81 | 11.5 | 8.4 | 10.4 | 13.9 |
| 82 | 10.6 | 14.0 | 9.4 | 15.9 |
| 83 | 9.8 | 10.2 | 8.5 | 11.1 |
| 84 | 11.3 | 16.0 | 9.4 | 14.5 |
| 85 | 8.0 | 8.1 | 10.7 | 11.7 |
| 86 | 10.0 | 11.8 | 8.6 | 9.9 |
| 87 | 10.7 | 11.7 | 8.1 | 12.3 |
| 88 | 12.7 | 12.0 | 8.8 | 15.1 |
| 89 | 8.3 | 16.7 | 8.7 | 8.4 |
| 90 | 10.9 | 9.9 | 9.5 | 9.0 |

As can be seen from the above table, the addition of insoluble excipients in the pharmaceutical composition effectively improved the static electricity of the bulk drug powder during filling, which significantly increased the filling efficiency. Additionally, the addition of insoluble basic excipient zinc hydroxide resulted in a considerable adjustment in the release amount of the implant, with a marked improvement in the drug release stability.

**VI. Implant**

Example 27 (gestodene implant)

The gestodene implant includes a medicinal core and a silicone tube. The active pharmaceutical ingredient in the medicinal core is gestodene with a particle size $D_{50}$ of 2.81 $\mu$m after pulverization. The filling length (i.e., the length of the medicinal core) is 2.2 cm, and the drug loading is 22.8 mg. The prepared silicone tube was disinfected by soaking in 75% ethanol for 30 minutes and then dried by blowing. One end of the silicone tube was sealed with silicone sealant for later use. Gestodene was loaded into the tube using a filling funnel. The other end was then sealed with silicone sealant and cured for 24 hours to prepare a gestodene implant.

The preparation for the raw material mixture of the silicone tube is the same as that of ZJ001 in "(III) Preparation and *in vitro* release study of gestodene implants; 3.4 Long-term *in vitro* release experiment of gestodene implants" under "I. Long-acting gestodene contraceptive implants".

Preparation of silicone tube: The raw material mixture of the silicone tube was sequentially subjected to extrusion molding and oven vulcanization. Extrusion molding involved sequential heat treatment at 270°C and 180°C, with the extrusion speed controlled at 3.5 rpm. The extrusion molding was carried out in a single-screw extruder. Oven vulcanization involved heat treatment at 180°C for 48 hours. The prepared silicone tube had an inner diameter of 2.0 mm, an outer diameter of 2.6 mm, and a wall thickness of 0.3 mm.

Example 28 (levonorgestrel implant)

The levonorgestrel implant includes a medicinal core and a silicone tube. The active pharmaceutical ingredient in the medicinal core is levonorgestrel (abbreviated as LNG) with a particle size $D_{50}$ of 2.12 $\mu$m after pulverization. The silicone tube (outer diameter of 2.4 mm, inner diameter of 1.6 mm) was cut into the same length of 35 mm. The cut tubes were placed in a 100 mL beaker with an appropriate amount of detergent and distilled water and subjected to ultrasonication for 30 minutes. After ultrasonication, the tubes were rinsed three times with distilled water, then washed with 75% ethanol, and naturally air-dried for later use. One end of the silicone tube was folded, and the tube was precisely filled with 36 mg of LNG powder through the other end using a tool (a medicinal core length of 30 mm). Both ends of the silicone tube were then sealed with adhesive. After the adhesive was cured, the silicone tube was kneaded and shaken to ensure even distribution of the drug within the tube. The drug powder adhered to the surface of the silicone tube was then washed off. The above steps were repeated six times to obtain a set of levonorgestrel implants.

On the basis of Example 27, the preparation for the raw material mixture of the silicone tube (parts by weight) was adjusted as follows, while other aspects remained the same as in Example 27:

white carbon black: 35 parts;
the molar ratio of Si-H groups in hydrogen-containing silicone oil to vinyl groups in methyl vinyl silicone rubber was 1.5:1.

The preparation of the silicone tube was the same as in Example 27.

**Effect Example 3**

1. Establishment of *in vitro* release testing method for gestodene implants

The chromatographic conditions and *in vitro* release testing methods are the same as those in "I. Long-acting gestodene contraceptive implants; (I) Content determination and *in vitro* release testing methods for gestodene contraceptive implants".

The *in vitro* release of gestodene was performed as follows: The release experiment was conducted using the horizontal shaking method. One prepared implant was taken and fixed to the wall of a 50 mL stoppered conical flask using an adhesive to prevent the implant from floating on the liquid surface during release, which could lead to inaccurate release results. Exactly 50 mL of distilled water was measured and injected into the conical flask. The flask was then placed in a thermostatic air shaker set at 37°C and shaken at a frequency of 100 rpm. The medium was replaced with an equal volume of fresh medium every 24 hours. The samples were filtered through a 0.22 $\mu$m microporous filter membrane and injected into the liquid chromatograph for detection.

2. *In vitro* release study of gestodene implants

*In vitro* release experiments were conducted on implants with different silicone tube wall thicknesses, medicinal core side areas, and drug particle sizes. Based on the *in vitro* release results, long-acting contraceptive implants with a daily drug release of 10-30 $\mu$g were prepared. The prepared implant was implanted in the subcutaneous tissue of the human upper arm, where the pH was close to neutral, allowing the release medium to be chosen between water and normal saline. A supersaturated solution of the gestodene bulk drug in water and normal saline, both before and after micronization, was prepared. The solution was placed in a shaker and shaken for 72 hours. After shaking, the mixture was taken out and centrifuged, and the supernatant was filtered. The equilibrium solubility of the gestodene bulk drug in water and normal saline, both before and after micronization, was determined using high performance liquid chromatography. Based on the results, the appropriate release medium was selected. Water was selected as the release medium for the *in vitro* release experiments. In the following experiments to investigate the *in vitro* release amount, the implant from Example 27 was subjected to depowdering pre-treatment, which involved adding 50 mL of anhydrous ethanol to a conical flask containing the implant, followed by ultrasonication for 1 minute. This process was repeated three times, after which 100 mL of distilled water was added and left overnight. The next day, the soaking solution was discarded, and the implant was placed in a shaker for *in vitro* release.

The following experiments to investigate the *in vitro* release amount were conducted in triplicate to examine the long-term stable release and reproducibility of the implant.

The instruments used were as follows:

TS-100B Thermostatic Shaker: Shanghai Jiecheng Experimental Instrument Co., Ltd.
High Performance Liquid Chromatograph: Shimadzu Corporation, Japan
KQ-250DE CNC Ultrasonic Cleaner: Kunshan Ultrasonic Instrument Co., Ltd.
AG245 Ultra-micro Electronic Analytical Balance: Mettler Toledo, Switzerland

(1) Silicone tubes prepared from raw rubber with different vinyl contents

Silicone tubes were prepared using raw rubber with different vinyl contents. The other procedures were the same as in Example 27. The effect of silicone tubes prepared with different vinyl contents on the *in vitro* release of gestodene was investigated. The test results are shown in the table below.

| No. | Variable | Average daily release amount ($\mu$g/d) | RSD (%) |
|---|---|---|---|
| 1 | Vinyl content of 0.17% (Example 27) | 10.57 $\pm$ 0.76 | 6.89 |
| 2 | Vinyl content of 0.23% | 15.33 $\pm$ 1.38 | 10.23 |
| 3 | Vinyl content of 0.1% | 6.21 $\pm$ 1.13 | 18.19 |
| 4 | Vinyl content of 0.4% | 26.66 $\pm$ 6.67 | 25.01 |
| 5 | Vinyl content of 0.5% | 33.32 $\pm$ 8.96 | 26.89 |

As can be seen from the above table, silicone tubes with a vinyl content of 0.1% have too low a vinyl content and too low a release amount. On the other hand, silicone tubes with a vinyl content of 0.4% and 0.5% have too high a vinyl content and too high a release amount. Furthermore, the RSD values of their *in vitro* release are much higher than those with a vinyl content of 0.17% and 0.23%. A higher RSD value indicates greater variability in daily release amounts and larger overall release fluctuation, which cannot achieve stable drug release. Therefore, by comparison, the vinyl content of 0.17% and 0.23% can not only meet the requirements of the release amount but also achieve appropriate RSD values, thus ensuring stable drug release.

As can be seen from Fig. 17, silicone tubes prepared with raw rubber with a vinyl content of 0.17% and 0.23% have a significant effect on the average *in vitro* drug release of the implant. Raw rubber with a vinyl content of 0.23% shows a greater initial burst release compared to that with a vinyl content of 0.17%, and silicone tubes prepared with raw rubber with a vinyl content of 0.17% exhibit more stable *in vitro* release.

The linear relationship between the drug release amount and the medicinal core side area for the implant prepared using sample No. 2 (a vinyl content of 0.23%) in section "Silicone tubes prepared from raw rubber with different vinyl contents" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.2 | 0.50 | 7.04 $\pm$ 2.11 | 14.01 $\pm$ 4.20 |
| 1.4 | 0.2 | 0.88 | 9.52 $\pm$ 1.92 | 10.83 $\pm$ 2.18 |
| 2.2 | 0.2 | 1.38 | 16.84 $\pm$ 2.01 | 12.19 $\pm$ 1.45 |

(continued)

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 2.7 | 0.2 | 1.70 | 20.71 ± 3.82 | 12.21 ± 2.25 |
| 3.3 | 0.2 | 2.07 | 24.51 ± 3.79 | 11.83 ± 1.83 |
| 4.3 | 0.2 | 2.70 | 31.75 ± 3.34 | 11.76 ± 1.24 |

The results indicate that as the side area of the medicinal core increases, the daily release amount of the gestodene contraceptive implant also increases. To determine the relationship between the side area of the medicinal core and the *in vitro* daily release amount, data analysis was conducted on the release amount and medicinal core side area for six sets of preparations.

The relationship between the side area of the medicinal core and the release amount of the implant satisfies the following mathematical equation: y = 11.591x + 0.561, R$^2$ = 0.9947. According to the fitted equation, the release amount shows a linear relationship with the side area of the medicinal core, with the release amount increasing as the side area increases. Based on this relationship, the release amount can be adjusted by fixing the inner and outer diameters and wall thickness of the silicone tube and adjusting the length of the medicinal core. In practical applications of the implant, due to large individual differences and large differences in body weight, it is possible to produce gestodene implants of different specifications to achieve personalized administration, which ensures contraceptive efficacy while reducing side effects to a greater extent.

(2) Silicone tubes prepared with different amounts of hydrogen-containing silicone oil

Silicone tubes were prepared using preparations with different molar ratios of Si-H groups in hydrogen-containing silicone oil to vinyl groups in R-vinyl silicone rubber. The other procedures were the same as in Example 27. The effect of the amount of hydrogen-containing silicone oil on the daily release amount of the implant was investigated. The test results are shown in the table below.

| No. | Variable | Average release rate ($\mu$g/d) | RSD (%) |
|---|---|---|---|
| 1 | Molar ratio of 1.2:1 (Example 27) | 10.57 ± 0.76 | 7.16 |
| 2 | Molar ratio of 1.5:1 | 8.27 ± 0.53 | 6.37 |
| 3 | Molar ratio of 0.3:1 | 2.64 ± 0.55 | 20.83 |
| 4 | Molar ratio of 3.0:1 | 16.54 ± 3.98 | 24.06 |
| 5 | Molar ratio of 1.0:1 | 5.51 ± 1.35 | 24.50 |

As can be seen from the above table, when the molar ratio of Si-H groups in hydrogen-containing silicone oil to vinyl groups in R-vinyl silicone rubber is 0.3:1, the release amount is too low, while a molar ratio of 3:1 results in a too high release amount. Additionally, the RSD values of their *in vitro* release are much higher than those with a molar ratio of 1.2:1 and 1.5:1. A higher RSD value indicates greater variability in daily release amounts and larger overall release fluctuation, which cannot achieve stable drug release. Therefore, by comparison, the molar ratio of 1.2:1 and 1.5:1 can not only meet the requirements of the release amount but also achieve appropriate RSD values, thus ensuring stable drug release.

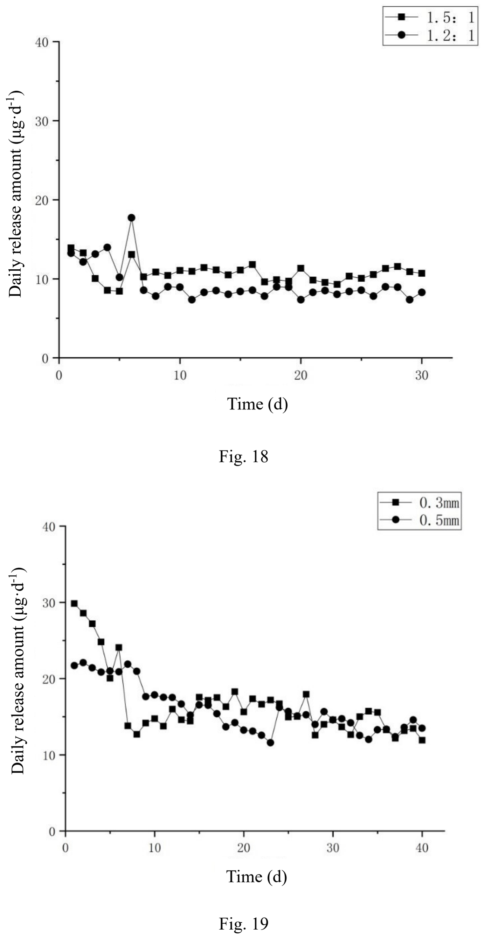

As can be seen from Fig. 18, the daily release amount of contraceptive implants filled with silicone tubes prepared using a preparation with a hydrogen-containing silicone oil molar ratio of 1.2:1 is slightly higher than that of contraceptive implants filled with silicone tubes prepared using a preparation with a hydrogen-containing silicone oil molar ratio of 1.5:1. Additionally, the stability of the release curves does not differ significantly.

The linear relationship between the drug release amount and the drug-loaded segment length for the implant prepared using sample No. 2 (a molar ratio of Si-H groups in hydrogen-containing silicone oil to vinyl groups in R-vinyl silicone rubber of 1.5:1) in section "Silicone tubes prepared with different amounts of hydrogen-containing silicone oil" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.2 | 0.50 | 3.40 ± 0.58 | 6.77 ± 1.16 |

(continued)

| Medicinal core length (cm) | Medicinal core diameter (cm) | Medicinal core side area (cm$^2$) | Average daily release amount (μg/d) | Average drug release (μg/cm$^2$/d) |
|---|---|---|---|---|
| 1.4 | 0.2 | 0.88 | 5.90 ± 0.77 | 6.71 ± 0.88 |
| 2.2 | 0.2 | 1.38 | 9.10 ± 0.85 | 6.59 ± 0.62 |
| 2.7 | 0.2 | 1.70 | 11.21 ± 1.48 | 6.61 ± 0.87 |
| 3.3 | 0.2 | 2.07 | 13.12 ± 1.62 | 6.33 ± 0.78 |
| 4.3 | 0.2 | 2.70 | 16.98 ± 1.76 | 6.29 ± 0.65 |
| 4.3 | 0.14 | 1.89 | 12.99 ± 1.48 | 6.87 ± 0.78 |
| 4.3 | 0.16 | 2.16 | 14.38 ± 1.65 | 6.66 ± 0.76 |
| 4.3 | 0.27 | 3.65 | 23.30 ± 1.73 | 6.39 ± 0.47 |
| 4.3 | 0.33 | 4.46 | 28.32 ± 2.29 | 6.36 ± 0.51 |

The results indicate that when the diameter of the medicinal core is fixed, the daily release amount of the gestodene contraceptive implant increases as the length of the medicinal core increases. When the length of the medicinal core is fixed, the daily release amount of the gestodene contraceptive implant increases as the diameter of the medicinal core increases. To determine the relationship between the side area of the medicinal core and the *in vitro* release, data analysis was conducted on the release amount and side area for different preparations.

The relationship between the side area of the medicinal core and the daily release amount of the implant satisfies the following mathematical equation: $y = 6.257x + 0.498$, $R^2 = 0.9982$. According to the fitted equation, the daily release amount shows a linear relationship with the side area of the medicinal core, with the release amount increasing as the side area increases. Based on this relationship, the release amount can be adjusted by fixing the outer diameter and wall thickness of the silicone tube and adjusting the length of the medicinal core, or fixing the length of the medicinal core and adjusting the outer diameter and wall thickness of the silicone tube. In practical applications of the implant, due to large individual differences and large differences in body weight, it is possible to produce gestodene implants of different specifications to achieve personalized administration, which ensures contraceptive efficacy while reducing side effects to a greater extent.

(3) Silicone tubes with different wall thicknesses

Implants with a silicone tube wall thickness of 0.5 mm were prepared. The other procedures were the same as in Example 27. The *in vitro* release data were measured to investigate the effect of the wall thickness of the homemade silicone tube on the *in vitro* release of the implants. The test results are shown in the table below.

| No. | Variable | Average daily release amount (μg/d) | RSD (%) |
|---|---|---|---|
| 1 | Wall thickness of 0.3 mm (Example 27) | 15.15 ± 1.84 | 12.16 |
| 2 | Wall thickness of 0.5 mm | 14.48 ± 1.61 | 11.11 |

As can be seen from Fig. 19, when controlling the outer diameter and drug-loaded segment length unchanged, the *in vitro* release data for gestodene contraceptive implants filled with silicone tubes with two wall thicknesses are almost identical. It can be concluded that a silicone tube wall thickness between 0.3 mm and 0.5 mm has little effect on the *in vitro* release rate. In the actual drug filling process, the silicone tube with a wall thickness of 0.5 mm has a too small inner diameter, making the drug filling process very difficult. In contrast, the silicone tube with a wall thickness of 0.3 mm has a relatively large inner diameter, making the drug filling process much easier. Therefore, after comprehensive consideration, a wall thickness of 0.3 mm was finally selected for the silicone tube.

The linear relationship between the drug release amount and the drug-loaded segment length for the implant prepared using sample No. 2 (a wall thickness of 0.5 mm) in section "Silicone tubes with different wall thicknesses" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.2 | 0.50 | 4.15 $\pm$ 0.85 | 8.27 $\pm$ 1.69 |
| 1.4 | 0.2 | 0.88 | 7.21 $\pm$ 1.02 | 8.20 $\pm$ 1.16 |
| 2.2 | 0.2 | 1.38 | 11.12 $\pm$ 1.28 | 8.05 $\pm$ 0.93 |
| 2.7 | 0.2 | 1.70 | 13.69 $\pm$ 1.90 | 8.07 $\pm$ 1.12 |
| 3.3 | 0.2 | 2.07 | 16.03 $\pm$ 2.05 | 7.73 $\pm$ 0.99 |
| 4.3 | 0.2 | 2.70 | 20.75 $\pm$ 2.26 | 7.68 + 0.84 |

From the data in the above table, the linear relationship between the medicinal core side area and the daily release amount of the implant is established as: $y = 7.520x + 0.587$, $R^2 = 0.9987$.

(4) Gestodene with different particle sizes in medicinal core

Gestodene with a different filling particle size of 80.3 $\mu$m was prepared, and the other procedures were the same as in Example 27. The test results are shown in the table below.

| No. | Variable | Average daily release amount ($\mu$g/d) | RSD (%) |
|---|---|---|---|
| 1 | Particle size of 2.81 $\mu$m (Example 27) | 14.69 $\pm$ 2.7 | 18.37 |
| 2 | Particle size of 80.3 $\mu$m | 18.79 $\pm$ 3.78 | 20.12 |

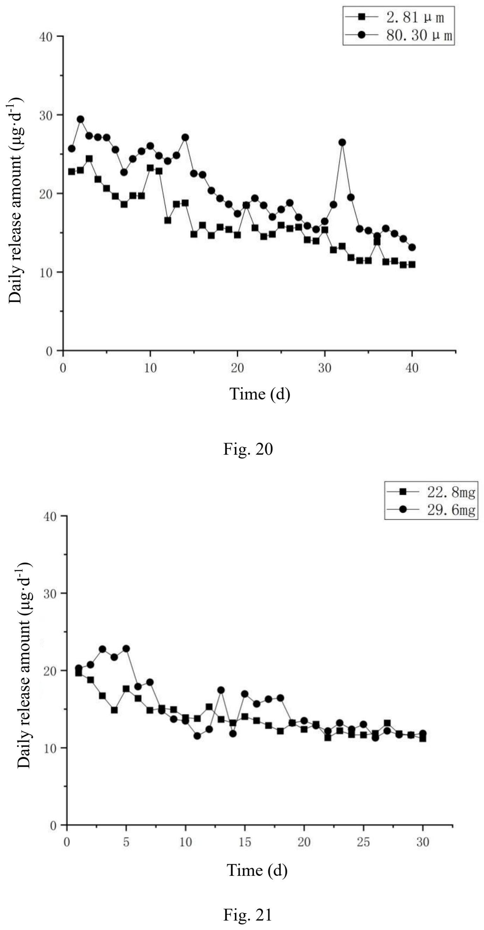

As can be seen from Fig. 20, the average daily release amount of implants filled with micronized gestodene bulk drug is smaller compared to that of implants filled with unmicronized gestodene bulk drug under the same specifications, which is mutually verified with the solubility measurement results. Although the particle size of the bulk drug decreases after micronization, the drug powders aggregate into small clusters due to electrostatic adsorption, reducing their contact area with the release medium and thereby decreasing the *in vitro* release.

After observing the release curves of the *in vitro* release of the two implants, it was found that the implant filled with bulk drug that had not undergone air jet pulverization showed large fluctuations in the *in vitro* release amount around 23 days. It was observed that the release curve of the implant filled with micronized bulk drug stabilized between 10-20 days without large fluctuations around 20 days. Based on these results, it can be concluded that implants filled with bulk drug that has undergone air jet pulverization for *in vitro* release exhibit a more stable release curve. Therefore, micronized drugs are chosen for the preparation of implants to achieve better stability.

The relationship between the drug release amount and the drug-loaded segment length for the implant prepared using sample No. 2 (a particle size of 80.3 $\mu$m) in section "Gestodene with different particle sizes in medicinal core" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.2 | 0.50 | 5.56 $\pm$ 1.41 | 11.06 $\pm$ 2.80 |
| 1.4 | 0.2 | 0.88 | 9.65 $\pm$ 2.13 | 10.97 $\pm$ 2.43 |
| 2.2 | 0.2 | 1.38 | 14.88 $\pm$ 2.82 | 10.77 $\pm$ 2.04 |
| 2.7 | 0.2 | 1.70 | 18.32 $\pm$ 3.08 | 10.80 $\pm$ 1.82 |
| 3.3 | 0.2 | 2.07 | 21.45 $\pm$ 3.28 | 10.35 $\pm$ 1.58 |
| 4.3 | 0.2 | 2.70 | 27.77 $\pm$ 4.50 | 10.28 $\pm$ 1.67 |
| 4.3 | 0.14 | 1.89 | 21.24 $\pm$ 4.98 | 11.23 $\pm$ 2.63 |
| 4.3 | 0.16 | 2.16 | 23.50 $\pm$ 4.91 | 10.88 $\pm$ 2.27 |
| 4.3 | 0.27 | 3.65 | 38.08 $\pm$ 7.03 | 10.45 $\pm$ 1.93 |

(continued)

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 4.3 | 0.33 | 4.46 | 46.30 $\pm$ 7.65 | 10.39 $\pm$ 1.72 |

The results indicate that when the length of the medicinal core is fixed, the daily release amount of the gestodene contraceptive implant increases as the diameter of the medicinal core increases. When the diameter of the medicinal core is fixed, the daily release amount of the gestodene contraceptive implant increases as the length of the medicinal core increases.

To determine the relationship between the release side area of the implant and the *in vitro* release, data analysis was conducted on the release amount and the medicinal core side area for different preparations. The relationship between the side area of the medicinal core and the daily release amount of the implant satisfies the following mathematical equation: $y = 10.229x + 0.802$, $R^2 = 0.9982$. According to the fitted equation, the release amount shows a linear relationship with the side area of the medicinal core, with the release amount increasing as the side area increases.

Based on this relationship, the release amount can be adjusted by fixing the outer diameter and wall thickness of the silicone tube and adjusting the length of the medicinal core, or fixing the length of the medicinal core and adjusting the inner and outer diameters of the silicone tube. In practical applications of the implant, due to large individual differences and large differences in body weight, it is possible to produce gestodene implants of different specifications to achieve personalized administration, which ensures contraceptive efficacy while reducing side effects to a greater extent.

(5) Silicone tubes with different drug loadings

Implants with a drug loading of 29.6 mg were prepared, and the other procedures were the same as in Example 27. The test results are shown in the table below.

| No. | Variable | Average release rate ($\mu$g/d) | RSD (%) |
|---|---|---|---|
| 1 | Drug loading of 22.8 mg (Example 27) | 12.67 $\pm$ 1.07 | 8.47 |
| 2 | Drug loading of 29.6 mg | 13.37 $\pm$ 3.78 | 14.99 |

As can be seen from Fig. 21, when controlling for the same silicone tube wall thickness and filling length, the daily *in vitro* release amounts for implants with different drug loadings are essentially the same, indicating that for the same medicinal core side area, the drug loading has basically no effect on the *in vitro* release of the implant.

When the filling length is 2.2 cm and the drug loading is 22.8 mg, the filling process is relatively smooth. However, when the drug loading is 29.6 mg, the filling process becomes more difficult, which causes the silicone tube to bulge slightly and results in a slightly higher average release amount compared to the preparation with a drug loading of 22.8 mg. The slight increase in release amount is likely due to the bulging of the filling part of the silicone tube and the stretching of the tube wall, which slightly increases the medicinal core side area, rather than being directly related to the drug loading.

The linear relationship between the drug release amount and the drug-loaded segment length for the implant prepared using sample No. 2 (a drug loading of 29.6 mg) in section "Silicone tubes with different drug loadings" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.2 | 0.50 | 5.00 $\pm$ 0.81 | 9.96 $\pm$ 1.61 |
| 1.4 | 0.2 | 0.88 | 8.68 $\pm$ 1.18 | 9.88 $\pm$ 1.34 |
| 2.2 | 0.2 | 1.38 | 13.39 $\pm$ 2.00 | 9.69 $\pm$ 0.45 |
| 2.7 | 0.2 | 1.70 | 16.49 $\pm$ 2.14 | 9.72 $\pm$ 1.26 |
| 3.3 | 0.2 | 2.07 | 19.31 $\pm$ 2.48 | 9.32 $\pm$ 0.20 |
| 4.3 | 0.2 | 2.70 | 24.99 $\pm$ 2.77 | 9.25 $\pm$ 0.02 |

From the data in the above table, the linear relationship between the medicinal core side area and the daily release amount of the implant is established as: y = 9.057x + 0.708, $R^2$ = 0.9987.

3. Establishment of a mathematical model formula between the medicinal core side area and daily release amount of the implant

Based on the investigation of the release amount of the gestodene implant, it can be seen that vulcanization treatment, different vinyl contents, different amounts of hydrogen-containing silicone oil, and the particle size of gestodene can affect the stability of the *in vitro* drug release. These factors further influence whether a stable positive linear correlation can be successfully established between the medicinal core side area and the release amount, so that the release amount of the implant can be determined based on the medicinal core side area. As a result of multi-factor investigation, the release amount was measured for gestodene implants with different medicinal core side areas prepared in Example 27, thus a positive linear correlation between the two variables was successfully established.

Since the implant controls drug release through a cylindrical silicone tube, the release side area of the drug can be expressed by the following formula: S = $\pi$d $\times$ L, where d is the inner diameter of the silicone tube, and L is the length of the drug-loaded segment.

It can be seen from the formula that when the inner diameter of the silicone tube is fixed (0.5-10.0 mm), changing the length of the medicinal core can alter the side area of the medicinal core. When the length of the silicone tube is fixed (0.8-21 cm), changing the diameter of the medicinal core can alter the side area of the medicinal core. In this experiment, silicone tubes with a fixed diameter were selected and filled with gestodene implants with a medicinal core length of 0.8 cm, 1.4 cm, 2.2 cm, 2.7 cm, 3.3 cm, 4.3 cm, 9 cm, 15 cm, 18 cm, and 21 cm for *in vitro* release. Other aspects were the same as in Example 27. The variation pattern of the *in vitro* release amount with the medicinal core side area was investigated. In this experiment, silicone tubes with a fixed length were selected, prepared with a wall thickness of 0.3 mm and an inner diameter of 0.5 mm, 0.8 mm, 1.4 mm, 1.6 mm, 2.0 mm, 2.7 mm, 3.3 mm, 4.3 mm, 6.8 mm, 9.0 mm, or 10.0 mm, and filled with gestodene powder to prepare gestodene implants for *in vitro* release. Other aspects were the same as in Example 27. The variation pattern of the *in vitro* release amount with the medicinal core side area was investigated. The test results are shown in the tables below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.20 | 0.50 | 4.44 $\pm$ 0.91 | 8.85 $\pm$ 1.81 |
| 1.4 | 0.20 | 0.88 | 7.71 $\pm$ 1.56 | 8.77 $\pm$ 1.77 |
| 2.2 | 0.20 | 1.38 | 11.90 $\pm$ 1.42 | 8.61 $\pm$ 1.03 |
| 2.7 | 0.20 | 1.70 | 14.65 $\pm$ 2.69 | 8.64 $\pm$ 1.59 |
| 3.3 | 0.20 | 2.07 | 18.15 $\pm$ 2.81 | 8.76 $\pm$ 1.36 |
| 4.3 | 0.20 | 2.70 | 23.84 $\pm$ 2.52 | 8.83 $\pm$ 0.93 |
| 9.0 | 0.20 | 5.65 | 48.79 $\pm$ 6.58 | 8.63 $\pm$ 1.16 |
| 15.0 | 0.20 | 9.42 | 81.28 $\pm$ 9.84 | 8.63 $\pm$ 1.04 |
| 18.0 | 0.20 | 11.30 | 99.47 $\pm$ 9.82 | 8.80 $\pm$ 0.87 |
| 21.0 | 0.20 | 13.19 | 115.26 $\pm$ 9.72 | 8.74 $\pm$ 0.74 |

| Medicinal core length (cm) | Medicinal core diameter (cm) | Medicinal core side area (cm$^2$) | Average release rate ($\mu$g/d) | Average drug release ($\mu$g/cm$^2$/d) |
|---|---|---|---|---|
| 4.3 | 0.05 | 0.68 | 6.14 $\pm$ 1.32 | 9.09 $\pm$ 1.95 |
| 4.3 | 0.08 | 1.08 | 9.27 $\pm$ 2.09 | 8.58 $\pm$ 1.94 |
| 4.3 | 0.14 | 1.89 | 17.30 $\pm$ 3.36 | 9.15 $\pm$ 1.78 |
| 4.3 | 0.16 | 2.16 | 19.82 $\pm$ 3.64 | 9.17 $\pm$ 1.69 |
| 4.3 | 0.20 | 2.70 | 14.65 $\pm$ 2.69 | 8.64 $\pm$ 1.59 |
| 4.3 | 0.27 | 3.65 | 31.45 $\pm$ 3.82 | 8.63 $\pm$ 1.05 |
| 4.3 | 0.33 | 4.46 | 40.18 $\pm$ 6.30 | 9.02 $\pm$ 1.41 |

(continued)

| Medicinal core length (cm) | Medicinal core diameter (cm) | Medicinal core side area (cm$^2$) | Average release rate ($\mu$g/d) | Average drug release ($\mu$g/cm$^2$/d) |
|---|---|---|---|---|
| 4.3 | 0.43 | 5.81 | 50.73 $\pm$ 5.73 | 8.74 $\pm$ 0.99 |
| 4.3 | 0.68 | 9.18 | 81.13 $\pm$ 8.00 | 8.84 $\pm$ 0.87 |
| 4.3 | 0.90 | 12.15 | 107.96 $\pm$ 11.06 | 8.88 $\pm$ 0.91 |
| 4.3 | 1.00 | 13.50 | 118.71 $\pm$ 11.17 | 8.79 $\pm$ 0.83 |

When the diameter of the medicinal core is fixed, the daily release amount of the gestodene contraceptive implant increases as the length of the medicinal core increases. Based on the data obtained for different medicinal core side areas and daily release amounts, a mathematical model formula is established between the two variables.

The mathematical model established is: y = 8.7363x + 0.0802, R$^2$ = 0.9999.

When the length of the medicinal core is fixed, the daily release amount of the gestodene contraceptive implant increases as the diameter of the medicinal core increases. Based on the data obtained for different medicinal core side areas and daily release amounts, a mathematical model formula is established between the two variables.

The mathematical model established is: y = 8.8140x + 0.1767, R$^2$ = 0.9998.

According to the fitted equations and graphs, it can be seen that the daily release amount shows a linear relationship with the medicinal core side area of the implant. As the medicinal core side area increases, the release amount increases. Based on this relationship, the release amount can be adjusted by fixing the outer diameter and wall thickness of the silicone tube and adjusting the length of the medicinal core, or by fixing the length of the implant and adjusting the inner and outer diameters of the silicone tube, or by simultaneously adjusting the inner and outer diameters of the silicone tube and the length of the medicinal core. In practical applications of the implant, due to large individual differences and large differences in body weight, it is possible to produce gestodene implants of different specifications to achieve personalized administration, which ensures contraceptive efficacy while reducing side effects to a greater extent.

**Effect Example 4**

1. Establishment of *in vitro* release testing method for levonorgestrel implants

The release experiment was conducted using the horizontal shaking method. A set of six implants was fixed to the wall of a 125 mL stoppered conical flask using an adhesive, ensuring an appropriate spacing between each rob (to prevent the implants from floating on the liquid surface, which could lead to inaccurate release results). Exactly 100 mL of distilled water was measured and injected into the conical flask. The flask was then placed in a thermostatic air shaker set at 37°C and shaken at a frequency of 100 rpm. The medium was replaced with an equal volume of fresh medium every 24 hours. The samples were filtered through a 0.22 $\mu$m microporous filter membrane and detected under the following chromatographic conditions with an injection volume of 20 $\mu$L.

Chromatographic column: Diamonsil® C$_{18}$ column (4.6 mm $\times$ 250 mm, 5 $\mu$m);
Mobile phase: methanol-water (80:20, v/v);
Column temperature: 30°C;
Detection wavelength: 240 nm;
Flow rate: 1.0 mL·min$^{-1}$;
Injection volume: 20 $\mu$L.

2. Effect of silicone tube preparation on *in vitro* release rate of levonorgestrel implants

Clean silicone tubes with qualified physical and mechanical properties were selected to prepare implants of the same specifications. After depowdering pre-treatment, the implants were placed in a shaker for release testing, with continuous sampling for 35 days to investigate the effect of the silicone tube preparation on the *in vitro* release rate of the implants.

(1) Effect of vinyl content on *in vitro* release rate of implants

Silicone tubes were extruded using raw rubber with a vinyl content of 0.23% and further prepared into implants. The other conditions were the same as in Example 28. The effect of silicone tubes prepared with different vinyl contents on the *in vitro* release of levonorgestrel was investigated. The test results are shown in the table below.

| No. | Variable | Average release rate (μg/d) | RSD (%) |
|---|---|---|---|
| 1 | Vinyl content of 0.17% (Example 28) | 52.17 ± 2.42 | 4.64 |
| 2 | Vinyl content of 0.23% | 38.79 ± 2.27 | 5.85 |

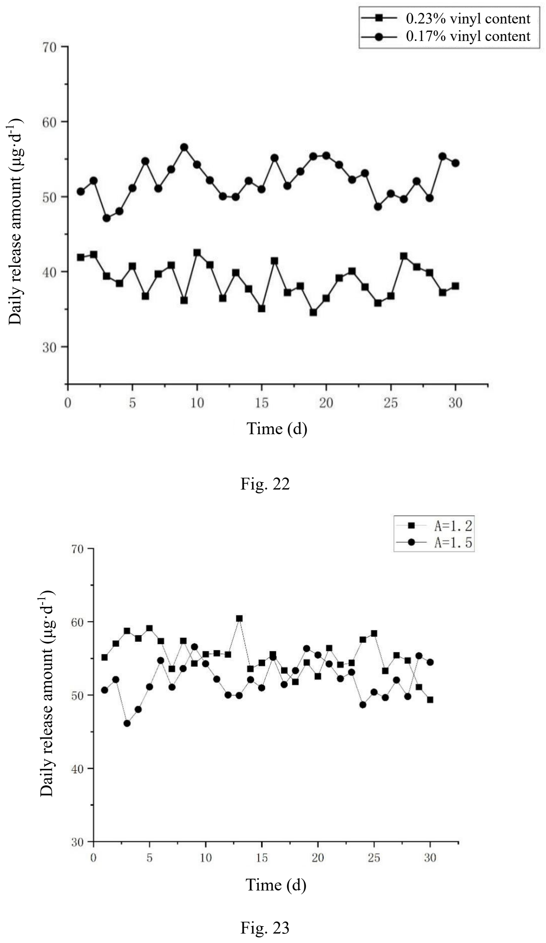

As can be seen from Fig. 22, both groups of implants can release the drug stably. The implant prepared with raw rubber with a vinyl content of 0.23% has a lower daily release amount compared to that prepared with raw rubber with a vinyl content of 0.17%. Moreover, for the implant prepared with raw rubber with a vinyl content of 0.23%, the release amount shows a more linear relationship with the drug-loaded segment length.

The linear relationship between the drug release amount and the medicinal core side area for the implant prepared using sample No. 2 (a vinyl content of 0.23%) in section "Effect of vinyl content on *in vitro* release rate of implants" is shown in the table below.

| Medicinal core length (cm) | Medicinal core diameter (cm) | Medicinal core side area (cm$^2$) | Average release rate (μg/d) | Average drug release (μg/cm$^2$/d) |
|---|---|---|---|---|
| 0.8 | 0.16 | 2.41 | 10.62 ± 0.78 | 4.40 ± 0.32 |
| 1.4 | 0.16 | 4.22 | 16.62 ± 1.13 | 3.94 ± 0.27 |
| 2.2 | 0.16 | 6.63 | 28.10 ± 1.21 | 4.24 ± 0.18 |
| 2.7 | 0.16 | 8.14 | 35.66 ± 1.88 | 4.38 ± 0.23 |
| 3.0 | 0.16 | 9.04 | 39.54 ± 2.23 | 4.37 ± 0.25 |
| 4.3 | 0.16 | 12.96 | 57.10 ± 3.56 | 4.40 ± 0.27 |
| 3.0 | 0.10 | 5.65 | 24.99 ± 1.77 | 4.42 ± 0.31 |
| 3.0 | 0.12 | 6.78 | 28.40 ± 2.01 | 4.19 ± 0.30 |
| 3.0 | 0.20 | 11.30 | 48.65 ± 4.07 | 4.30 ± 0.36 |
| 3.0 | 0.27 | 15.26 | 66.97 ± 5.16 | 4.39 ± 0.34 |
| 3.0 | 0.33 | 18.65 | 76.81 ± 5.63 | 4.12 ± 0.30 |

The results indicate that when the length of the medicinal core is fixed, the daily release amount of the levonorgestrel contraceptive implant increases as the diameter of the medicinal core increases. When the diameter of the medicinal core is fixed, the daily release amount of the levonorgestrel contraceptive implant increases as the length of the medicinal core increases.

To determine the relationship between the side area of the medicinal core and the *in vitro* daily release amount of the implant, data analysis was conducted on the release amount and the side area of the medicinal core for different preparations. The relationship between the side area of the medicinal core and the daily release amount of the implant satisfies the following mathematical equation: $y = 4.2459x + 0.3967$, $R^2 = 0.9957$. According to the fitted equation, the daily release amount shows a linear relationship with the side area of the medicinal core, with the release amount increasing as the side area increases. Based on this relationship, the release amount can be adjusted by fixing the outer diameter and wall thickness of the silicone tube and adjusting the length of the medicinal core, or fixing the length of the medicinal core and adjusting the inner and outer diameters of the silicone tube. In practical applications of the implant, due to large individual differences and large differences in body weight, it is possible to produce levonorgestrel implants of different specifications to achieve personalized administration, which ensures contraceptive efficacy while reducing side effects to a greater extent.

(2) Amount of hydrogen-containing silicone oil

Silicone tubes with a molar ratio of hydrogen-containing silicone oil to methyl vinyl silicone rubber of 1.2:1 were further prepared into implants. The other conditions were the same as in Example 28. The effect of silicone tubes prepared with different molar ratios on the *in vitro* release of levonorgestrel was investigated. The test results are shown in the table below.

| No. | Variable | Average daily release amount (µg/d) | RSD (%) |
|---|---|---|---|
| 1 | Molar ratio of 1.5:1 (Example 28) | 52.17 ± 2.55 | 4.88 |
| 2 | Molar ratio of 1.2:1 | 55.26 ± 2.48 | 4.49 |

Fig. 23 shows the relationship curve between different release times and daily drug release amounts for levonorgestrel implants in Example 28 and the control experiments with molar ratios of 1.2:1 and 1.5:1. In the figure, A = 1.2 indicates a molar ratio of 1.2:1, and A = 1.5 indicates a molar ratio of 1.5:1. As can be seen from Fig. 23, as the amount of hydrogen-containing silicone oil increases, the release rate of the implant gradually decreases. This is mainly due to the varying degrees of cross-linking within the silicone tube, which results in different diffusion rates of the drug in the silicone tube. Implants prepared in Example 28 can achieve stable release, allowing the establishment of a linear relationship between the release amount and the drug-loaded segment length.

The linear relationship between the drug release amount and the medicinal core side area for the implant prepared using sample No. 2 (a molar ratio of hydrogen-containing silicone oil to methyl vinyl silicone rubber of 1.2:1) in section "Effect of vinyl content on *in vitro* release rate of implants" is shown in the table below.

| Medicinal core length (cm) | Medicinal core diameter (cm) | Medicinal core side area (cm$^2$) | Average daily release amount (µg/d) | Average drug release (µg/cm$^2$/d) |
|---|---|---|---|---|
| 0.8 | 0.16 | 2.41 | 15.31 ± 1.02 | 6.35 ± 0.42 |
| 1.4 | 0.16 | 4.22 | 23.76 ± 1.37 | 5.63 ± 0.32 |
| 2.2 | 0.16 | 6.63 | 40.47 ± 1.58 | 6.10 ± 0.24 |
| 2.7 | 0.16 | 8.14 | 51.24 ± 2.34 | 6.30 ± 0.29 |
| 3.0 | 0.16 | 9.04 | 57.33 ± 2.81 | 6.34 ± 0.31 |
| 4.3 | 0.16 | 12.96 | 82.43 ± 3.65 | 6.36 ± 0.28 |

The results indicate that when the diameter of the medicinal core is fixed, the daily release amount of the levonorgestrel contraceptive implant increases as the length of the medicinal core increases.

To determine the relationship between the side area of the medicinal core and the *in vitro* release of the implant, data analysis was conducted on the daily release amount and the side area of the medicinal core for different preparations. The relationship between the side area of the medicinal core and the daily release amount of the implant satisfies the following mathematical equation: $y = 6.4978x - 1.9190$, $R^2 = 0.9979$. According to the fitted equation, the daily release amount shows a linear relationship with the side area of the medicinal core, with the release amount increasing as the side area increases. Based on this relationship, the release amount can be adjusted by fixing the outer diameter and wall thickness of the silicone tube and adjusting the length of the medicinal core. In practical applications of the implant, due to large individual differences and large differences in body weight, it is possible to produce levonorgestrel implants of different specifications to achieve personalized administration, which ensures contraceptive efficacy while reducing side effects to a greater extent.

(3) Amount of white carbon black

Silicone tubes prepared with 30 PHR of white carbon black were further prepared into implants. The other conditions were the same as in Example 28. The effect of silicone tubes prepared with different amounts of white carbon black on the *in vitro* release of levonorgestrel was investigated. The test results are shown in the table below.

| No. | Variable | Average daily release amount (µg/d) | RSD (%) |
|---|---|---|---|
| 1 | 35 PHR of white carbon black (Example 28) | 52.17 ± 2.55 | 4.88 |
| 2 | 30 PHR of white carbon black | 58.15 ± 2.93 | 5.04 |

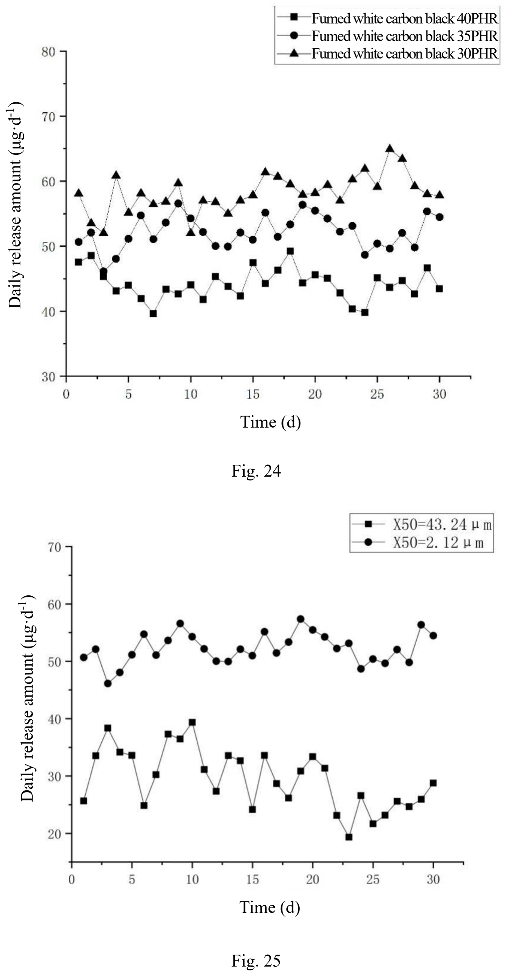

The release test results are shown in Fig. 24, indicating that the amount of white carbon black has a certain effect on the release of the implant. According to the figure, it can be seen that 30-40 PHR of white carbon black can achieve relatively stable release, but the release amount is more stable with 30-35 PHR of white carbon black. Therefore, implants prepared in combination with other necessary features of the present disclosure can establish a more linear relationship between the release amount and the drug-loaded segment length.

The linear relationship between the drug release amount and the medicinal core side area for the implant prepared using sample No. 2 (30 PHR of white carbon black) in section "Amount of white carbon black" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.16 | 2.41 | 15.92 $\pm$ 1.23 | 6.60 $\pm$ 0.51 |
| 1.4 | 0.16 | 4.22 | 24.91 $\pm$ 1.33 | 5.90 $\pm$ 0.32 |
| 2.2 | 0.16 | 6.63 | 41.53 $\pm$ 1.75 | 6.26 $\pm$ 0.26 |
| 2.7 | 0.16 | 8.14 | 53.46 $\pm$ 2.34 | 6.57 $\pm$ 0.29 |
| 3.0 | 0.16 | 9.04 | 59.28 $\pm$ 3.01 | 6.55 $\pm$ 0.33 |
| 4.3 | 0.16 | 12.96 | 85.59 $\pm$ 4.32 | 6.60 $\pm$ 0.33 |
| 3.0 | 0.10 | 5.65 | 37.46 $\pm$ 2.33 | 6.63 $\pm$ 0.41 |
| 3.0 | 0.12 | 6.78 | 42.57 $\pm$ 2.87 | 6.28 $\pm$ 0.42 |
| 3.0 | 0.20 | 11.30 | 71.58 $\pm$ 5.44 | 6.33 $\pm$ 0.48 |
| 3.0 | 0.27 | 15.26 | 100.55 $\pm$ 5.87 | 6.59 $\pm$ 0.38 |
| 3.0 | 0.33 | 18.65 | 117.84 $\pm$ 6.38 | 6.32 $\pm$ 0.34 |

The results indicate that when the length of the medicinal core is fixed, the daily release amount of the levonorgestrel contraceptive implant increases as the diameter of the medicinal core increases. When the diameter of the medicinal core is fixed, the daily release amount of the levonorgestrel contraceptive implant increases as the length of the medicinal core increases.

To determine the relationship between the side area of the medicinal core and the *in vitro* release of the implant, data analysis was conducted on the release amount and release area for different preparations. The relationship between the side area of the medicinal core and the daily release amount of the implant satisfies the following mathematical equation: y = 6.4689x - 0.2767, R$^2$ = 0.9973. According to the fitted equation, the daily release amount shows a linear relationship with the side area of the medicinal core, with the release amount increasing as the side area increases. Based on this relationship, the release amount can be adjusted by fixing the outer diameter and wall thickness of the silicone tube and adjusting the length of the medicinal core, or fixing the length of the medicinal core and adjusting the inner and outer diameters of the silicone tube. In practical applications of the implant, due to large individual differences and large differences in body weight, it is possible to produce levonorgestrel implants of different specifications to achieve personalized administration, which ensures contraceptive efficacy while reducing side effects to a greater extent.

(4) Particle size of LNG

The particle size of the drug powder was measured using a laser particle size analyzer to obtain a D$_{50}$ of 43.24 $\mu$m. The drug powder was then pulverized using an air jet pulverizer to achieve a D$_{50}$ of 2.12 $\mu$m (as in Example 28). The release behavior of implants made from these LNG powders with two particle sizes was then investigated. The experimental results are shown in the table below and Fig. 25.

| No. | Variable | Average daily release amount ($\mu$g/d) | RSD (%) |
|---|---|---|---|
| 1 | Particle size of 2.12 $\mu$m (Example 28) | 52.24 $\pm$ 2.66 | 5.10 |
| 2 | Particle size of 43.24 $\mu$m | 29.51 $\pm$ 5.23 | 17.73 |

Both groups of implants exhibit linear release, but the implant after micronization shows a higher release amount.

According to the Noyes-Whitney dissolution rate equation: $\frac{dc}{dt} = K \cdot S \cdot C$ (where $\frac{dc}{dt}$ is the drug dissolution rate, S is the drug surface area, and C is the solubility). The dissolution rate increases with the increase in the drug surface area. Therefore, as the particle size decreases and the specific surface area increases, the dissolution rate increases, resulting in a higher daily release amount for the implant after micronization. Simultaneously, it can be observed from the figure that the *in vitro* release rate of the drug after micronization is more stable compared to that without micronization. It is hypothesized that the reason is that the solubility of the drug increases after micronization, leading to an increase in the dissolved drug in the silicone tube. This allows the drug to maintain a certain concentration within the inner side of the silicone tube, ensuring relatively stable drug release.

The linear relationship between the drug release amount and the medicinal core side area for the implant prepared using sample No. 2 (a particle size of 43.24 $\mu$m) in section "Particle size of LNG" is shown in the table below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.16 | 2.41 | 8.06 $\pm$ 1.67 | 3.34 $\pm$ 0.69 |
| 1.4 | 0.16 | 4.22 | 12.62 $\pm$ 2.15 | 2.99 $\pm$ 0.51 |
| 2.2 | 0.16 | 6.63 | 21.04 $\pm$ 2.86 | 3.17 $\pm$ 0.43 |
| 2.7 | 0.16 | 8.14 | 27.28 $\pm$ 4.77 | 3.33 $\pm$ 0.59 |
| 3.0 | 0.16 | 9.04 | 30.02 $\pm$ 5.48 | 3.32 $\pm$ 0.61 |
| 4.3 | 0.16 | 12.96 | 43.35 $\pm$ 7.06 | 3.34 $\pm$ 0.54 |

The results indicate that when the diameter of the medicinal core is fixed, the daily release amount of the levonorgestrel contraceptive implant increases as the length of the medicinal core increases.

To determine the relationship between the side area of the medicinal core and the *in vitro* release of the implant, data analysis was conducted on the release amount and the side area of the medicinal core for different preparations. The relationship between the side area of the medicinal core and the daily release amount of the implant satisfies the following mathematical equation: $y = 3.4120x - 0.9893$, $R^2 = 0.9978$. According to the fitted equation, the daily release amount shows a linear relationship with the side area of the medicinal core, with the release amount increasing as the side area increases. In practical applications of the implant, due to large individual differences and large differences in body weight, it is possible to produce levonorgestrel implants of different specifications to achieve personalized administration, which ensures contraceptive efficacy while reducing side effects to a greater extent.

(5) Silicone tube wall thickness

After washing, levonorgestrel implants with the same drug loading were prepared. Following depowdering treatment, they were placed in a shaker for *in vitro* release testing. Samples were continuously taken for 30 days to investigate the effect of the silicone tube wall thickness on the *in vitro* release rate of the implant. Except for the different wall thicknesses, the following experimental scheme was the same as in Example 28. The dimensions of the silicone tube are shown in the table below.

| Silicone tube wall thickness (mm) | Silicone tube specifications | Drug loading (mg) |
|---|---|---|
| 0.5 | 2.4 mm (outer diameter) $\times$ 1.4 mm (inner diameter) $\times$ 35 mm (core length) $\times$ 6 (number of implants per tube) | 216 |
| 0.4 | 2.4 mm (outer diameter) $\times$ 1.6 mm (inner diameter) $\times$ 35 mm (core length) $\times$ 6 (number of implants per tube) | 216 |
| 0.3 | 2.4 mm (outer diameter) $\times$ 1.8 mm (inner diameter) $\times$ 35 mm (core length) $\times$ 6 (number of implants per tube) | 216 |

The *in vitro* release results are shown in the table below.

| Silicone tube wall thickness (mm) | Average daily release amount ($\mu$g) | Relative standard deviation (%) | Relationship between daily release amount and release side area | Linear coefficient R$^2$ |
|---|---|---|---|---|
| 0.3 mm | 59.66 | 5.69 | $y = 6.6085x + 0.3064$ | 0.997 |

(continued)

| Silicone tube wall thickness (mm) | Average daily release amount (μg) | Relative standard deviation (%) | Relationship between daily release amount and release side area | Linear coefficient $R^2$ |
|---|---|---|---|---|
| 0.4 mm | 52.24 | 5.10 | y = 5.8036x - 0.2482 | 0.997 |
| 0.5 mm | 45.42 | 5.71 | y = 5.0172x + 0.3281 | 0.998 |

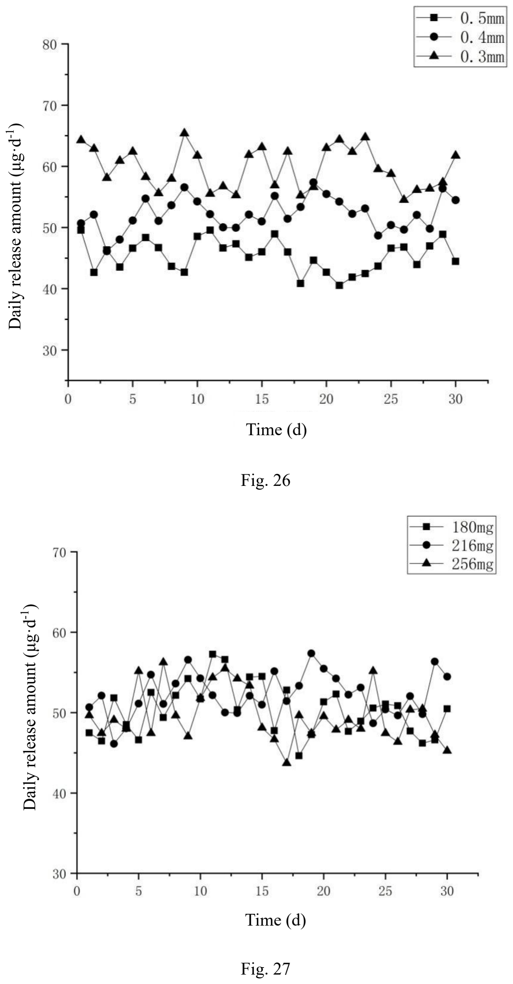

As can be seen from Fig. 26, all groups of preparations exhibit zero-order release. As the wall thickness of the silicone tube increases, the daily release amount of the implant gradually decreases. The implant with a wall thickness of 0.3 mm has a daily release amount of approximately 55-65 μg. The implant with a wall thickness of 0.4 mm has a daily release amount of approximately 45-55 μg. The implant with a wall thickness of 0.5 mm has a daily release amount of approximately 40-50 μg. That is, for every 0.1 mm increase in the wall thickness of the silicone tube, the average daily release amount decreases by approximately 7 μg. Under otherwise constant conditions, the wall thickness of the silicone tube can affect the *in vitro* release rate of the implant.

(6) Drug loading

The levonorgestrel implant prepared in this experiment has a reservoir-type structure, which is made by wrapping levonorgestrel powder with an addition-cure silicone tube as a capsule shell. Therefore, it is necessary to investigate whether the drug loading within the silicone tube has an effect on the *in vitro* release rate of the drug, so as to better control the quality of the levonorgestrel implant. The implant was prepared as shown in the table below. After depowdering treatment, an *in vitro* release test was conducted, with continuous sampling for 35 days to examine the effect of drug loading on the *in vitro* release rate of the implant.

| Drug loading (mg) | Levonorgestrel core length (mm) |
|---|---|
| 180 | 180 |
| 216 | 180 |
| 252 | 180 |

As can be seen from Fig. 27, the daily release amounts among the various preparations do not differ significantly, fluctuating between 45-55 μg. The average daily release amounts are 50.34 μg, 52.24 μg, and 49.70 μg, respectively. Under the premise that the bulk density of the drug powder within the implant tube is sufficient (no emergence of empty tube sections after shaking of the implant), the actual drug loading is several thousand times greater than the daily release amount. Over a prolonged period, the drug concentration within the silicone tube remains supersaturated as the small drug molecules diffuse outward, resulting in minimal variation in the release amount. Therefore, when the bulk density of the drug powder within the implant tube is sufficient and the side area of the medicinal core remains constant, the drug loading of the implant does not have an effect on the release of the implant.

3. Establishment of a mathematical relationship model between drug-loaded segment length and release amount

LNG is released outward through the silicone tube, with the release mechanism primarily driven by diffusion. Therefore, the size of the effective medicinal core side area theoretically directly affects the drug release area of the implant. Silicone tubes of different lengths were used to prepare the implant, as shown in the table below. After depowdering treatment, an *in vitro* release test was conducted, with continuous sampling for 35 days to examine the effect of the medicinal core side area on the *in vitro* release rate of the implant. The test results are shown in the table below.

Based on the investigation of the release amount of the levonorgestrel implant, it can be seen that vulcanization treatment, different vinyl contents, different amounts of hydrogen-containing silicone oil, and the particle size of levonorgestrel can affect the stability of the *in vitro* drug release. These factors further influence whether a stable positive linear correlation can be successfully established between the medicinal core side area and the daily release amount, so that the release amount of the implant can be determined based on the medicinal core side area. As a result of multi-factor investigation, the release amount was measured for levonorgestrel implants with different medicinal core side areas prepared in Example 28, thus a positive linear correlation between the two variables was successfully established.

Since the implant controls drug release through a cylindrical silicone tube, the side area of the medicinal core can be expressed by the following formula: $S = \pi d \times L$, where d is the inner diameter of the silicone tube, and L is the length of the drug-loaded segment.

It can be seen from the formula that when the inner diameter of the silicone tube is fixed (0.5-10.0 mm), changing the length of the medicinal core can alter the side area of the medicinal core. When the length of the silicone tube is fixed (0.8-21 cm), changing the diameter of the medicinal core can alter the side area of the medicinal core. In this experiment, silicone tubes with a fixed diameter were selected and filled with levonorgestrel implants with a medicinal core length of 0.8 cm, 1.5 cm, 2.5 cm, 3.0 cm, 3.5 cm, 4.3 cm, 9 cm, 15 cm, 18 cm, and 21 cm for *in vitro* release. Other aspects were the same as in Example 28. The variation pattern of the *in vitro* release amount with the medicinal core side area was investigated. In this experiment, silicone tubes with a fixed length were selected, prepared with a wall thickness of 0.4 mm and an inner diameter of 0.5 mm, 0.8 mm, 1.4 mm, 1.6 mm, 2.0 mm, 2.7 mm, 3.3 mm, 4.3 mm, 6.8 mm, 9.0 mm, or 10.0 mm, and filled with levonorgestrel powder to prepare levonorgestrel implants for *in vitro* release. Other aspects were the same as in Example 28. The variation pattern of the *in vitro* release amount with the medicinal core side area was investigated. The test results are shown in the tables below.

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 0.8 | 0.16 | 2.41 | 12.36 $\pm$ 0.98 | 5.13 $\pm$ 0.41 |
| 1.5 | 0.16 | 4.52 | 27.48 $\pm$ 1.76 | 6.08 $\pm$ 0.39 |
| 2.5 | 0.16 | 7.54 | 44.15 $\pm$ 1.87 | 5.86 $\pm$ 0.25 |
| 3.0 | 0.16 | 9.04 | 47.94 $\pm$ 2.81 | 5.30 $\pm$ 0.3 1 |
| 3.5 | 0.16 | 10.55 | 58.30 $\pm$ 3.76 | 5.53 $\pm$ 0.36 |
| 4.3 | 0.16 | 12.96 | 72.19 $\pm$ 3.11 | 5.57 $\pm$ 0.24 |
| 9.0 | 0.16 | 27.13 | 141.29 $\pm$ 12.63 | 5.21 $\pm$ 0.47 |
| 15.0 | 0.16 | 45.22 | 268.49 $\pm$ 21.16 | 5.94 $\pm$ 0.47 |
| 18.0 | 0.16 | 54.26 | 289.33 $\pm$ 18.57 | 5.33 $\pm$ 0.34 |
| 21.0 | 0.16 | 63.30 | 368.14 $\pm$ 27.79 | 5.82 $\pm$ 0.44 |

| Medicinal core length (cm) | Medicinal core diameter (cm) | Medicinal core side area (cm$^2$) | Average daily release amount ($\mu$g/d) | Average drug release ($\mu$g/cm$^2$/d) |
|---|---|---|---|---|
| 3.0 | 0.05 | 2.83 | 14.54 $\pm$ 1.60 | 5.15 $\pm$ 0.57 |
| 3.0 | 0.08 | 4.52 | 27.48 $\pm$ 2.35 | 6.08 $\pm$ 0.52 |
| 3.0 | 0.14 | 7.91 | 42.35 $\pm$ 3.90 | 5.35 $\pm$ 0.49 |
| 3.0 | 0.16 | 9.04 | 47.94 $\pm$ 2.81 | 5.30 $\pm$ 0.3 1 |
| 3.0 | 0.20 | 11.30 | 61.76 $\pm$ 4.34 | 5.46 $\pm$ 0.38 |
| 3.0 | 0.27 | 15.26 | 89.33 $\pm$ 5.86 | 5.85 $\pm$ 0.38 |
| 3.0 | 0.33 | 18.65 | 105.82 $\pm$ 6.10 | 5.67 $\pm$ 0.33 |
| 3.0 | 0.43 | 24.30 | 139.81 $\pm$ 6.75 | 5.75 $\pm$ 0.28 |
| 3.0 | 0.68 | 38.43 | 231.28 $\pm$ 15.43 | 6.02 $\pm$ 0.40 |
| 3.0 | 0.90 | 50.87 | 277.97 $\pm$ 17.62 | 5.46 $\pm$ 0.35 |
| 3.0 | 1.00 | 56.52 | 316.27 $\pm$ 17.36 | 5.60 $\pm$ 0.31 |

As can be seen from the above table, when the diameter of the medicinal core is fixed, the daily release amount of the levonorgestrel contraceptive implant increases as the length of the medicinal core increases. Based on the data obtained for different medicinal core side areas and release amounts, a mathematical model formula is established between the two variables.

The mathematical model established is: y = 5.6865x - 1.7641, R$^2$ = 0.9951.

As can be seen from the above table, when the length of the medicinal core is fixed, the daily release amount of the levonorgestrel contraceptive implant increases as the diameter of the medicinal core increases. Based on the data obtained for different medicinal core side areas and release amounts, a mathematical model formula is established between the two variables.

The mathematical model established is: $y = 5.6294x + 0.5000$, $R^2 = 0.9970$.

According to the fitted equations and graphs, it can be seen that the daily release amount shows a linear relationship with the medicinal core side area of the implant. As the medicinal core side area increases, the daily release amount increases. Based on this relationship, the release amount can be adjusted by fixing the outer diameter and wall thickness of the silicone tube and adjusting the length of the medicinal core, or by fixing the length of the implant and adjusting the inner and outer diameters of the silicone tube, or by simultaneously adjusting the inner and outer diameters of the silicone tube and the length of the medicinal core. In practical applications of the implant, due to large individual differences and large differences in body weight, it is possible to produce levonorgestrel implants of different specifications to achieve personalized administration, which ensures contraceptive efficacy while reducing side effects to a greater extent.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection of the present disclosure is defined by the appended claims.

**Claims**

1. A raw material composition of a silicone material, comprising the following components in parts by weight:

    R-vinyl silicone rubber: 100 parts;
    a reinforcing agent: 20-80 parts;
    hydrogen-containing silicone oil: 0.3-3.0 parts;
    a catalyst: $\geq$ 0.000002 parts, preferably 0.000002-0.00005 parts;
    wherein
    in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
    the content of vinyl groups in the R-vinyl silicone rubber is 0.10-0.50 mol%;
    the content of Si-H groups in the hydrogen-containing silicone oil is 0.18-1.6 mol%;
    the molar ratio of Si-H groups in the hydrogen-containing silicone oil to vinyl groups in the R-vinyl silicone rubber is (0.5-4): 1;
    optionally, the raw material composition further comprises an inhibitor, and the inhibitor is an inhibitor capable of inhibiting the addition reaction between the R-vinyl silicone rubber and the hydrogen-containing silicone oil.

2. The raw material composition of the silicone material according to claim 1, wherein the raw material composition of the silicone material satisfies one or more of the following conditions:

    1) the R-vinyl silicone rubber is methyl vinyl silicone rubber; the methyl vinyl silicone rubber may be methyl vinyl silicone rubber with a relative molecular weight of 100000-800000 g/mol;
    2) the content of vinyl groups in the R-vinyl silicone rubber is 0.10-0.23 mol%, such as 0.17 mol% or 0.23 mol%, preferably 0.17-0.23 mol%;
    3) the reinforcing agent is one or more of white carbon black, diatomite, quartz powder, silica powder, calcium carbonate, aluminum hydroxide, magnesium oxide, titanium white powder, magnesium silicate, carbon black, zinc oxide, iron oxide, titanium dioxide, zirconium silicate, and calcium carbonate, such as white carbon black; the white carbon black may be fumed white carbon black, precipitated white carbon black, gel white carbon black, or surface-treated white carbon black;
    4) the amount of the reinforcing agent is 30-80 parts, such as 30 parts, 35 parts, 40 parts, 45 parts, 50 parts, or 60 parts;
    5) the amount of the hydrogen-containing silicone oil is 0.4-2.8 parts, such as 0.42 parts, 0.67 parts, 0.84 parts, 1.01 parts, 1.26 parts, 1.36 parts, 1.51 parts, 1.68 parts, or 2.52 parts;
    6) the content of Si-H groups in the hydrogen-containing silicone oil is 0.36-1.6 mol%, such as 0.36 mol%, 0.5 mol%, 0.75 mol%, 1.0 mol%, or 1.6 mol%;
    7) the molar ratio of Si-H groups in the hydrogen-containing silicone oil to vinyl groups in the methyl vinyl silicone rubber is (0.8-4.0):1, such as 0.8:1, 1.0:1, 1.2:1, 1.5:1, 1.8:1, 2.0:1, or 3.0:1, preferably (1.2-1.8):1 or (1.2-1.5):1;
    8) the inhibitor is an acetylenic alcohol compound, a nitrogen-containing compound, or an organic peroxide, such as methylbutynol, or such as 2-methyl-3-butyn-2-ol;

9) the amount of the inhibitor is 0.03-2.0 parts, such as 0.3-1.0 parts, or such as 0.3 parts, 0.5 parts, 0.7 parts, or 0.9 parts;

10) the catalyst is a rhodium catalyst, a palladium catalyst, or a platinum catalyst, preferably a platinum catalyst; the concentration of platinum in the platinum catalyst may be 3000 ppm, meaning that the mass concentration of platinum in the platinum catalyst is 3000 parts per million; and

11) the amount of the catalyst is 0.000005-0.00005 parts, such as 0.000005 parts, 0.00001 parts, 0.00002 parts, or 0.00003 parts.

3. The raw material composition of the silicone material according to claim 1 or 2, wherein

(1) the raw material composition of the silicone material comprises the following components in parts by weight:

methyl vinyl silicone rubber: 100 parts;
a reinforcing agent: 20-80 parts;
hydrogen-containing silicone oil: 0.3-3.0 parts;
a catalyst: 0.000002-0.00005 parts;
an inhibitor: 0.03-2.0 parts;
wherein
the content of vinyl groups in the methyl vinyl silicone rubber is 0.10-0.50 mol%;
the content of Si-H groups in the hydrogen-containing silicone oil is 0.18-1.6 mol%;
the molar ratio of Si-H groups in the hydrogen-containing silicone oil to vinyl groups in the methyl vinyl silicone rubber is (0.5-4): 1;
alternatively,

(2) the raw material composition of the silicone material comprises the following components in parts by weight:

methyl vinyl silicone rubber: 100 parts;
a reinforcing agent: 30-60 parts;
hydrogen-containing silicone oil: 0.4-2.8 parts;
a catalyst: 0.000002-0.00005 parts;
an inhibitor: 0.3-1.0 parts;
wherein
the content of vinyl groups in the methyl vinyl silicone rubber is 0.17-0.23 mol%;
the content of Si-H groups in the hydrogen-containing silicone oil is 0.18-1.6 mol%;
the molar ratio of Si-H groups in the hydrogen-containing silicone oil to vinyl groups in the methyl vinyl silicone rubber is (0.8-2): 1;
alternatively,

(3) the raw material composition of the silicone material comprises the following components in parts by weight:

methyl vinyl silicone rubber: 100 parts;
a reinforcing agent: 30-45 parts;
hydrogen-containing silicone oil: 0.42-2.52 parts;
a catalyst: 0.000002-0.00005 parts;
an inhibitor: 0.3-0.9 parts;
wherein
the content of vinyl groups in the methyl vinyl silicone rubber is 0.17-0.23 mol%;
the content of Si-H groups in the hydrogen-containing silicone oil is 0.5-1.0 mol%;
the molar ratio of Si-H groups in the hydrogen-containing silicone oil to vinyl groups in the methyl vinyl silicone rubber is (1.2-1.8): 1.

4. A preparation method for a silicone material, wherein the preparation method is any one of the following methods:

(1) method 1: when the raw material composition of the silicone material according to any one of claims 1 to 3 does not comprise an inhibitor, the preparation method for the silicone material comprises the following steps:

S1: mixing the R-vinyl silicone rubber according to any one of claims 1 to 3 and the reinforcing agent according to any one of claims 1 to 3 uniformly to obtain a mixture A;

S2: mixing the mixture A and the hydrogen-containing silicone oil according to any one of claims 1 to 3 in the presence of the catalyst according to any one of claims 1 to 3 to obtain a mixture B, then performing catalytic addition;

(2) method 2: when the raw material composition of the silicone material according to any one of claims 1 to 3 further comprises an inhibitor, the preparation method for the silicone material comprises the following steps:

S1: mixing the R-vinyl silicone rubber according to any one of claims 1 to 3 and the reinforcing agent according to any one of claims 1 to 3 uniformly to obtain a mixture A;
S2: dividing the mixture A into a component A1 and a component A2, mixing the component A1 with the catalyst according to any one of claims 1 to 3 to obtain a component B1, and mixing the component A2 with the hydrogen-containing silicone oil according to any one of claims 1 to 3 and the inhibitor according to any one of claims 1 to 3 to obtain a component B2;
S3: mixing the component B1 and the component B2 to obtain a mixture B, then performing catalytic addition.

5. The preparation method for the silicone material according to claim 4, wherein the preparation method for the silicone material satisfies one or more of the following conditions:

1) in the method 1 and method 2, the reinforcing agent is pretreated using the following method: drying at 100-210°C for 1-24 hours;
2) in the method 1 and method 2, the R-vinyl silicone rubber is pretreated using the following method: drying at 30-60°C for 1-24 hours;
3) in the method 1 and method 2, the step of mixing in the S1 involves: wrapping the R-vinyl silicone rubber with the reinforcing agent, then extruding and passing through an open mill, and sheeting;
alternatively, in the method 1 and method 2, the step of mixing in the S1 involves: kneading the R-vinyl silicone rubber and the reinforcing agent in a kneader at 30°C for 30 minutes, then removing the mixture; performing triangular wrapping and passing through an open mill at a roll spacing of 1-10 mm five times, rolling, sheeting, then being left for 24 hours to obtain the mixture A;
4) in the method 1, prior to the catalytic addition, the mixture B undergoes the following post-treatment: performing triangular wrapping and passing through an open mill 4-6 times, then uniformly cutting and sheeting;
5) in the method 2, the step of mixing the component B1 and the component B2 involves: adding the component B1 and the component B2 to an open mill in a 1: 1 ratio, then performing triangular wrapping and passing through the open mill 4-6 times, and uniformly cutting and sheeting; and
6) in the method 1 and the method 2, the step of the catalytic addition involves: subjecting the mixture B to a first heat treatment and a second heat treatment in sequence; preferably, the second heat treatment is followed by a third heat treatment; wherein the temperature of the first heat treatment may be 250-360°C, such as 270-300°C, or such as 270°C, 280°C, or 300°C; the duration of the first heat treatment may be 5 seconds; the temperature of the second heat treatment may be 120-300°C, such as 180-280°C, or such as 180°C, 260°C, or 280°C; the duration of the second heat treatment may be 2 minutes; the temperature of the third heat treatment may be 180°C; the duration of the third heat treatment may be 24-48 hours, such as 48 hours.

6. A preparation method for a silicone material, comprising the following step: molding a mixture of the raw material composition of the silicone material according to any one of claims 1 to 3 through a catalytic addition process, wherein the catalytic addition process sequentially comprises a first heat treatment and a second heat treatment; the temperature of the first heat treatment is 250-360°C, and the temperature of the second heat treatment is 120-300°C, such as 120-280°C.

7. The preparation method for the silicone material according to claim 6, wherein the mixture of the raw material composition of the silicone material is prepared using any one of the following methods:

(1) method 1: when the raw material composition of the silicone material according to any one of claims 1 to 3 does not comprise an inhibitor, the preparation method for the mixture of the raw material composition of the silicone material comprises the following steps:

S1: mixing the R-vinyl silicone rubber according to any one of claims 1 to 3 and the reinforcing agent according to any one of claims 1 to 3 uniformly to obtain a mixture A;
S2: mixing the mixture A and the hydrogen-containing silicone oil according to any one of claims 1 to 3 in the presence of the catalyst according to any one of claims 1 to 3 to obtain a mixture B;

(2) method 2: when the raw material composition of the silicone material according to any one of claims 1 to 3 further comprises an inhibitor, the preparation method for the mixture of the raw material composition of the silicone material comprises the following steps:

S1: mixing the R-vinyl silicone rubber according to any one of claims 1 to 3 and the reinforcing agent according to any one of claims 1 to 3 uniformly to obtain a mixture A;

S2: dividing the mixture A into a component A1 and a component A2, mixing the component A1 with the catalyst according to any one of claims 1 to 3 to obtain a component B1, and mixing the component A2 with the hydrogen-containing silicone oil according to any one of claims 1 to 3 and the inhibitor according to any one of claims 1 to 3 to obtain a component B2;

S3: mixing the component B1 and the component B2 to obtain a mixture B.

8. The preparation method for the silicone material according to claim 6 or 7, wherein the preparation method for the mixture of the raw material composition of the silicone material satisfies one or more of the following conditions:

1) in the method 1 and method 2, the reinforcing agent is pretreated using the following method: drying at 100-210°C for 1-24 hours;

2) in the method 1 and method 2, the R-vinyl silicone rubber is pretreated using the following method: drying at 30-60°C for 1-24 hours;

3) in the method 1 and method 2, the step of mixing in the S1 involves: wrapping the R-vinyl silicone rubber with the reinforcing agent, then extruding and passing through an open mill, and sheeting; alternatively, in the method 1 and method 2, the step of mixing in the S1 involves: kneading the R-vinyl silicone rubber and the reinforcing agent in a kneader at 30°C for 30 minutes, then removing the mixture; performing triangular wrapping and passing through an open mill at a roll spacing of 1-10 mm five times, rolling, sheeting, then being left for 24 hours to obtain the mixture A;

4) in the method 1, prior to the catalytic addition process, the mixture B undergoes the following post-treatment: processing by performing triangular wrapping and passing through an open mill 4-6 times, then uniformly cutting and sheeting; and

5) in the method 2, the step of mixing the component B1 and the component B2 involves: adding the component B1 and the component B2 to the open mill in a 1:1 ratio, processing by performing triangular wrapping and passing through the open mill 4-6 times, then uniformly cutting and sheeting.

9. The preparation method for the silicone material according to at least one of claims 6 to 8, wherein the temperature of the first heat treatment is 250-330°C, such as 270°C, 280°C, 300°C, or 330°C;

and/or, the temperature of the second heat treatment is 150-300°C, such as 150-280°C, or such as 150°C, 180°C, 210°C, 260°C, or 280°C;

and/or, the second heat treatment is followed by a third heat treatment; the temperature of the third heat treatment may be 100-280°C, such as 180°C; the duration of the third heat treatment is preferably 0-72 hours, but not 0, such as 24 hours, 48 hours, or 72 hours.

10. A silicone material, which is prepared using the preparation method for the silicone material according to at least one of claims 4 to 9.

11. A silicone tube, wherein the silicone tube is prepared using the following methods:

forming the silicone material according to claim 10 into a tubular shape by an extrusion process; for example, in the method 1 and method 2 according to any one of claims 4 to 5 and 7 to 9, forming the mixture B into a tubular shape by an extrusion process, followed by catalytic addition;

alternatively, in the preparation method for the silicone material according to any one of claims 4 to 9, performing the catalytic addition process in a tubular mold to obtain the silicone tube; for example, in the method 1 and method 2 according to any one of claims 4 to 5 and 7 to 9, performing the catalytic addition process in a tubular mold to obtain the silicone tube.

12. An implant, comprising a medicinal core and the silicone tube according to claim 11, and the medicinal core comprises an active pharmaceutical ingredient, wherein

the active pharmaceutical ingredient may comprise an active pharmaceutical ingredient acting on the reproductive system, and the active pharmaceutical ingredient acting on the reproductive system may comprise a contraceptive active pharmaceutical ingredient, including levonorgestrel, gestodene, and gestrinone; the active pharmaceutical ingredient acting on the reproductive system may comprise a steroidal estrogen, such as estradiol;

the active pharmaceutical ingredient may be an active pharmaceutical ingredient with a solubility of ≤ 100 mg/mL and a molecular weight of less than 1000 Da, or such as one or more of levonorgestrel, gestodene, gestrinone, estradiol, ibuprofen, paliperidone, meloxicam, and puerarin;

the active pharmaceutical ingredient may be an active pharmaceutical ingredient with a solubility of ≤ 60 mg/mL and a molecular weight of less than 1000 Da;

the active pharmaceutical ingredient may be an active pharmaceutical ingredient with a solubility of ≤ 50 mg/mL and a molecular weight of less than 1000 Da;

the active pharmaceutical ingredient may be an active pharmaceutical ingredient with a solubility of ≤ 10 mg/mL and a molecular weight of less than 1000 Da;

the active pharmaceutical ingredient may be an active pharmaceutical ingredient with a solubility of ≤ 5 mg/mL and a molecular weight of less than 1000 Da;

the medicinal core may be a powder-type medicinal core;

the outer diameter of the silicone tube is preferably 2.0-5.0 mm, such as 2.4 mm or 2.6 mm;

the length of the silicone tube is preferably 1.5-4.5 cm, such as 1.9 cm or 4.4 cm;

the wall thickness of the silicone tube is preferably 0.2-0.5 mm, such as 0.3 mm, 0.4 mm, or 0.5 mm;

the drug release area of the silicone tube is preferably 0.4-15.0 $cm^2$, such as 0.69 $cm^2$, 1.38 $cm^2$, 2.07 $cm^2$, 2.76 $cm^2$, or 3.45 $cm^2$;

the diameter of the medicinal core is preferably 1.5-4.0 mm, such as 1.6 mm or 2.0 mm;

the length of the medicinal core is preferably 1.0-4.0 cm, such as 1.5 cm or 3.9 cm.

13. A pharmaceutical composition, wherein the pharmaceutical composition comprises a bulk drug and an insoluble excipient;

the insoluble excipient comprises a silicon material.

14. The pharmaceutical composition according to claim 13, wherein the density of the insoluble excipient is 1-10000 g/L, such as 1000 g/L;

and/or, the particle size of the insoluble excipient is 1-200 μm;

and/or, the pore size of the silicon material is less than 1 μm, such as 0 nm, 5 nm, 10 nm, 18 nm, 50 nm, or 100 nm;

and/or, in the silicon material, the content of silicon dioxide is more than 50%, preferably 80%, 90%, 95%, 99%, or 99.8%;

and/or, the insoluble excipient comprises one or more of white carbon black, AL-1FP mesoporous silica, XDP3050 mesoporous silica, and XDP3150 mesoporous silica, wherein the white carbon black is preferably fumed white carbon black, precipitated white carbon black, gel white carbon black, or surface-treated white carbon;

preferably, the insoluble excipient is one or more of white carbon black, AL-1FP mesoporous silica, XDP3050 mesoporous silica, and XDP3150 mesoporous silica;

more preferably, the insoluble excipient is one or more of white carbon black, AL-1FP mesoporous silica, and XDP3050 mesoporous silica;

and/or, the insoluble excipient further comprises an insoluble weak acid and/or an insoluble weak base;

the insoluble weak acid preferably comprises one or more of boric acid, fumaric acid, molybdic acid, silicic acid, tungstic acid, and germanic acid, more preferably including boric acid and/or fumaric acid;

the insoluble weak base preferably comprises one or more of magnesium hydroxide, aluminum hydroxide, zinc hydroxide, ferrous hydroxide, and magnesium oxide, more preferably including one or more of magnesium hydroxide, aluminum hydroxide, and zinc hydroxide.

15. The pharmaceutical composition according to claim 13 or 14, wherein the insoluble excipient is one of the following a to z:

a. white carbon black;

b. white carbon black and magnesium hydroxide;

c. white carbon black and aluminum hydroxide;

d. white carbon black and zinc hydroxide;

e. white carbon black and fumaric acid;

f. white carbon black and boric acid;

g. AL-1FP mesoporous silica;

h. AL-1FP mesoporous silica and XDP3050 mesoporous silica;

i. AL-1FP mesoporous silica and XDP3150 mesoporous silica;

j. AL-1FP mesoporous silica and magnesium hydroxide;

k. AL-1FP mesoporous silica and aluminum hydroxide;

l. AL-1FP mesoporous silica and zinc hydroxide;

m. AL-1FP mesoporous silica and fumaric acid;

n. AL-1FP mesoporous silica and boric acid;

o. XDP3050 mesoporous silica;

p. XDP3050 mesoporous silica and magnesium hydroxide;

q. XDP3050 mesoporous silica and aluminum hydroxide;

r. XDP3050 mesoporous silica and zinc hydroxide;

s. XDP3050 mesoporous silica and fumaric acid;

t. XDP3050 mesoporous silica and boric acid;

u. XDP3150 mesoporous silica;

v. XDP3150 mesoporous silica and magnesium hydroxide;

w. XDP3150 mesoporous silica and aluminum hydroxide;

x. XDP3150 mesoporous silica and zinc hydroxide;

y. XDP3150 mesoporous silica and fumaric acid;

z. XDP3150 mesoporous silica and boric acid.

16. The pharmaceutical composition according to at least one of claims 13 to 15, wherein the content of the bulk drug is 10%-99.9%, such as 50%-99.5%, or such as 95%; the percentage represents the mass percentage of the bulk drug in the pharmaceutical composition;

and/or, the content of the insoluble excipient is 0.1%-90%, such as 0.1%-50%, or such as 0.5%-5%; the percentage represents the mass percentage of the insoluble excipient in the pharmaceutical composition;

and/or, the density of the insoluble excipient is 1-10000 g/L, such as 1000 g/L;

and/or, the particle size of the insoluble excipient is 1-200 $\mu$m;

and/or, the bulk drug is a small molecule drug with a solubility of less than 100 mg/mL;

and/or, the molecular weight of the bulk drug is less than 1000 Da;

and/or, the active pharmaceutical ingredient of the bulk drug comprises an active pharmaceutical ingredient acting on the reproductive system, an active pharmaceutical ingredient acting on the urinary system, an active pharmaceutical ingredient acting on chronic diseases related to metabolism and nutrition, an active pharmaceutical ingredient for treating chronic diseases related to connective tissue and rheumatism, an active pharmaceutical ingredient for treating hyperlipidemia, tumors, neuropsychiatric disorders, chronic dental diseases, simple obesity, chronic low back pain, or leukemia, or an active pharmaceutical ingredient acting on the circulatory system;

wherein the active pharmaceutical ingredient acting on the reproductive system preferably comprises a contraceptive active pharmaceutical ingredient; the contraceptive active pharmaceutical ingredient preferably comprises levonorgestrel, gestodene, or gestrinone;

wherein the active pharmaceutical ingredient acting on the urinary system preferably comprises an active pharmaceutical ingredient for treating chronic nephritis, chronic renal failure, or chronic prostatitis; the active pharmaceutical ingredient for treating chronic prostatitis is preferably a non-steroidal anti-inflammatory drug, more preferably ibuprofen;

wherein the active pharmaceutical ingredient acting on chronic diseases related to metabolism and nutrition preferably comprises an active pharmaceutical ingredient for anti-diabetes, treating nutritional deficiency diseases, anti-gout, or anti-osteoporosis; the active pharmaceutical ingredient for anti-gout is preferably an anti-gout drug, more preferably ibuprofen;

wherein the active pharmaceutical ingredient for treating chronic diseases related to connective tissue and rheumatism preferably comprises an active pharmaceutical ingredient for treating rheumatoid arthritis, systemic lupus erythematosus, ankylosing spondylitis, Sjögren's syndrome, vasculitis, idiopathic inflammatory myopathies, systemic sclerosis, or osteoarthritis; the active pharmaceutical ingredient for treating rheumatoid arthritis is preferably a non-steroidal anti-inflammatory drug, more preferably meloxicam or ibuprofen; the active pharmaceutical ingredient for treating osteoarthritis is preferably an analgesic, more preferably ibuprofen or meloxicam;

wherein the active pharmaceutical ingredient for treating neuropsychiatric disorders is preferably a drug for treating schizophrenia, more preferably a benzisoxazole, such as paliperidone;

wherein the active pharmaceutical ingredient for treating chronic low back pain is preferably a non-steroidal analgesic, more preferably ibuprofen or meloxicam;

wherein the active pharmaceutical ingredient acting on the circulatory system preferably comprises an active pharmaceutical ingredient for treating chronic heart failure, coronary heart disease, congenital heart disease, or chronic infective endocarditis; the active pharmaceutical ingredient for treating coronary heart disease is preferably a drug that improves angina symptoms, more preferably puerarin;

more preferably, the bulk drug is a levonorgestrel bulk drug, a gestodene bulk drug, an ibuprofen bulk drug, a paliperidone bulk drug, a meloxicam bulk drug, or a puerarin bulk drug.

17. The pharmaceutical composition according to at least one of claims 13 to 16, wherein when the bulk drug is a gestodene bulk drug, the gestodene bulk drug is in powder form; the particle size of the gestodene bulk drug is preferably 1-180 $\mu$m, more preferably 2.81 $\mu$m;

and/or, when the bulk drug is a levonorgestrel bulk drug, the levonorgestrel bulk drug is in powder form; the particle size of the levonorgestrel bulk drug is preferably 1-180 $\mu$m, such as 2.12 $\mu$m;

and/or, when the bulk drug is an ibuprofen bulk drug, the ibuprofen bulk drug is in powder form; the particle size of the ibuprofen bulk drug is preferably 1-200 $\mu$m, such as 80 $\mu$m;

and/or, when the bulk drug is a paliperidone bulk drug, the paliperidone bulk drug is in powder form; the particle size of the paliperidone bulk drug is preferably 1-200 $\mu$m, such as 1 $\mu$m, 10 $\mu$m, 50 $\mu$m, 80 $\mu$m, 120 $\mu$m, 150 $\mu$m, or 180 $\mu$m;

and/or, when the bulk drug is a meloxicam bulk drug, the meloxicam bulk drug is in powder form; the particle size of the meloxicam bulk drug is preferably 1-200 $\mu$m, such as 1 $\mu$m, 10 $\mu$m, 50 $\mu$m, 80 $\mu$m, 120 $\mu$m, 150 $\mu$m, or 180 $\mu$m;

and/or, when the bulk drug is a puerarin bulk drug, the puerarin bulk drug is in powder form; the particle size of the puerarin bulk drug is preferably 1-200 $\mu$m, such as 1 $\mu$m, 10 $\mu$m, 50 $\mu$m, 80 $\mu$m, 120 $\mu$m, 150 $\mu$m, or 180 $\mu$m;

and/or the pharmaceutical composition is in powder form.

18. An implant, wherein the implant comprises the pharmaceutical composition according to at least one of claims 13 to 17 and a silicone tube;

preferably, the silicone tube is the silicone tube according to claim 11.

19. An implant comprising a medicinal core and a silicone tube;

the medicinal core comprises an active pharmaceutical ingredient, and the particle size $D_{50}$ of the active pharmaceutical ingredient is 180 $\mu$m or less;

the raw material composition of the silicone tube comprises the following components in parts by weight:

R-vinyl silicone rubber: 100 parts;
hydrogen-containing silicone oil: 0.3-3.0 parts;
a reinforcing agent: 20-80 parts;
a catalyst: $\geq$ 0.000002 parts, preferably $2\times10^{-6}$-$5\times10^{-5}$ parts;
wherein
in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl groups in the R-vinyl silicone rubber is 0.05-0.50 mol%;
the content of Si-H groups in the hydrogen-containing silicone oil is 0.18-1.6 mol%; the molar ratio of Si-H groups in the hydrogen-containing silicone oil to vinyl groups in the R-vinyl silicone rubber is (0.5-4): 1;
the preparation method for the silicone tube comprises the following step: subjecting the raw material mixture of the silicone tube to extrusion molding.

20. The implant according to claim 19, wherein the active pharmaceutical ingredient comprises an active pharmaceutical ingredient acting on the reproductive system; the active pharmaceutical ingredient acting on the reproductive system may comprise a contraceptive active pharmaceutical ingredient; the contraceptive active pharmaceutical ingredient comprises, for example, levonorgestrel, gestodene, and gestrinone; the active pharmaceutical ingredient acting on the reproductive system may comprise a steroidal estrogen, such as estradiol;

and/or, the solubility of the active pharmaceutical ingredient is $\leq$ 100 mg/mL;

and/or, the molecular weight of the active pharmaceutical ingredient is less than 1000 Da;

and/or, the particle size $D_{50}$ of the active pharmaceutical ingredient is 1-180 $\mu$m, such as 2.12 $\mu$m, 2.81 $\mu$m, 10 $\mu$m, 43.24 $\mu$m, 80 $\mu$m, 80.3 $\mu$m, or 100 $\mu$m;

and/or, when the active pharmaceutical ingredient is gestodene, the mass of the active pharmaceutical ingredient is 10-100 mg, such as 12.5 mg, 22.8 mg, 29.6 mg, 42 mg, 60 mg, or 84 mg;

and/or, when the active pharmaceutical ingredient is levonorgestrel, the mass of the active pharmaceutical ingredient is 15-300 mg, such as 15 mg, 36 mg, 72 mg, 75 mg, 100 mg, 150 mg, 180 mg, 216 mg, or 252 mg;

and/or, when the active pharmaceutical ingredient is estradiol, the mass of the active pharmaceutical ingredient is 10-300 mg, such as 75 mg;

and/or, when the active pharmaceutical ingredient is ibuprofen, the mass of the active pharmaceutical ingredient is 10-2000 mg, such as 40 mg;

and/or, when the active pharmaceutical ingredient is meloxicam, the mass of the active pharmaceutical ingredient is 10-2000 mg, such as 40 mg;

and/or, when the active pharmaceutical ingredient is paliperidone, the mass of the active pharmaceutical ingredient is 10-2000 mg, such as 40 mg;

and/or, when the active pharmaceutical ingredient is puerarin, the mass of the active pharmaceutical ingredient is 10-2000 mg, such as 40 mg.

21. The implant according to claim 19 or 20, wherein the R-vinyl silicone rubber is methyl vinyl silicone rubber;

and/or, the content of vinyl groups in the R-vinyl silicone rubber is 0.10-0.5 mol%, such as 0.17-0.23 mol%;

and/or, the parts by weight of the hydrogen-containing silicone oil is 0.5-1 parts, such as 0.75 parts;

and/or, the molar ratio of Si-H groups in the hydrogen-containing silicone oil to vinyl groups in the R-vinyl silicone rubber is (1-1.8):1, such as 1:1, 1.2:1, or 1.5:1;

and/or, the content of Si-H groups in the hydrogen-containing silicone oil is 0.5-1 mol%, such as 0.75 mol%;

and/or, the parts by weight of the reinforcing agent is 30-50 parts, such as 35 parts or 40 parts;

and/or, the reinforcing agent is one or more of white carbon black, diatomite, quartz powder, silica powder, calcium carbonate, aluminum hydroxide, magnesium oxide, titanium white powder, magnesium silicate, carbon black, zinc oxide, iron oxide, titanium dioxide, zirconium silicate, and calcium carbonate, such as white carbon black; the white carbon black is, for example, fumed white carbon black, precipitated white carbon black, gel white carbon black, or surface-treated white carbon black;

and/or, the medicinal core further comprises an excipient, and the excipient comprises a silicon material and a pH adjuster;

wherein the silicon material is preferably white carbon black and/or mesoporous silica; the white carbon black is, for example, fumed white carbon black; the mesoporous silica is, for example, one or more of AL-1FP mesoporous silica, XDP3050 mesoporous silica, and XDP3150 mesoporous silica;

wherein the pH adjuster preferably comprises one or more of boric acid, fumaric acid, molybdic acid, silicic acid, tungstic acid, germanic acid, magnesium hydroxide, aluminum hydroxide, zinc hydroxide, ferrous hydroxide, and magnesium oxide;

wherein the mass ratio of the excipient to the active pharmaceutical ingredient is preferably 1:1;

and/or, the active pharmaceutical ingredient is one or more of gestodene, levonorgestrel, estradiol, ibuprofen, meloxicam, paliperidone, and puerarin.

22. The implant according to at least one of claims 19 to 21, wherein the parts by weight of the catalyst is $5\times10^{-6}$-$3\times10^{-5}$ parts, such as $1\times10^{-5}$ parts;

and/or, the catalyst is a platinum catalyst;

and/or, the raw material mixture of the silicone tube further comprises an inhibitor, and the inhibitor is a hydrocarbon;

preferably, the molecular structure of the hydrocarbon contains at least one alkynyl group and one hydroxyl group, such as 2-methyl-3-butyn-2-ol;

the parts by weight of the inhibitor is 0.03-2 parts, preferably 0.1-1 parts, such as 0.7 parts.

23. The implant according to at least one of claims 19 to 22, wherein the extrusion molding comprises sequentially performing a first heat treatment and a second heat treatment;

wherein the temperature of the first heat treatment is preferably 250-360°C, such as 270°C, 300°C, 330°C, or 360°C; the first heat treatment is preferably carried out in a front drying tunnel, with the length of the front drying tunnel being, for example, 0.8 m;

wherein the temperature of the second heat treatment is preferably 120-280°C, such as 120°C, 150°C, 180°C, or 210°C; the second heat treatment is preferably carried out in a rear drying tunnel, with the length of the rear drying tunnel being, for example, 2.5 m;

and/or, the extrusion molding is followed by a third heat treatment;

the temperature of the third heat treatment is 100-280°C, preferably 120-200°C, more preferably 180°C;

the duration of the third heat treatment is 0-72 hours, but not 0, such as 24 hours or 48 hours.

24. The implant according to at least one of claims 19 to 23, wherein a preparation method for the implant comprises the following step: filling the medicinal core into the silicone tube, and then sequentially sealing and curing to obtain the implant;

wherein the duration of the curing is preferably 0-72 hours, but not 0, such as 24 hours or 48 hours;

and/or, the implant further comprises a depowdering treatment.

25. A test method for the drug release amount of the implant according to at least one of claims 19 to 24, wherein the test method comprises the following step:

simulating with different side surface areas of the medicinal core and daily drug release amounts of the implant to obtain a mathematical model formula: y = kx + b;

y is the daily drug release amount, in $\mu$g/d; x is the side surface area of the medicinal core, in $cm^2$; x = $\pi$DL, where D is the diameter of the medicinal core, and L is the length of the medicinal core.

26. The qualitative test method for the drug release amount of the implant according to claim 25, wherein when the medicinal core is gestodene, k is 4 to 16, such as 6.257, 7.520, 8.7363, 8.8140, 9.057, 10.229, or 11.591; b is -4 to 4, such as 0.0802, 0.1767, 0.498, 0.561, 0.587, 0.708, or 0.802; $R^2 > 0.99$;

when the active pharmaceutical ingredient is gestodene, the mathematical model formula is:

$$y = 11.591x + 0.561, R^2 = 0.9947;$$

or,

$$y = 6.257x + 0.498, R^2 = 0.998;$$

or,

$$y = 7.520x + 0.587, R^2 = 0.9987;$$

or,

$$y = 10.229x + 0.802, R^2 = 0.9982;$$

or,

$$y = 9.057x + 0.708, R^2 = 0.9987;$$

or,

$$y = 8.7363x + 0.0802, R^2 = 0.9999;$$

or,

$$y = 8.8140x + 0.1767, R^2 = 0.9998.$$

**27.** The qualitative test method for the drug release amount of the implant according to claim 26, wherein when the medicinal core is levonorgestrel, k is 1 to 10, such as 3.412, 4.2459, 5.0172, 5.6294, 5.6865, 5.8036, 6.4689, 6.4978, or 6.6085; b is -6 to 6, such as -1.7641, -1.919, -0.2767, -0.2482, -0.9893, -0.3064, 0.3281, 0.3967, or 0.5; when the active pharmaceutical ingredient is levonorgestrel, the mathematical model formula is:

$$y = 4.2459x + 0.3967, R^2 = 0.9957;$$

or,

$$y = 6.4978x - 1.9190, R^2 = 0.9979;$$

or,

$$y = 6.4689x - 0.2767, R^2 = 0.9973;$$

or,

$$y = 5.8036x - 0.2482, R^2 = 0.9973;$$

or,

$$y = 3.4120x - 0.9893, R^2 = 0.9978;$$

or,

$$y = 6.6085x + 0.3064, R^2 = 0.997;$$

or,

$$y = 5.0172x + 0.3281, R^2 = 0.998;$$

or,

$$y = 5.6865x - 1.7641, R^2 = 0.9951;$$

or,

$$y = 5.6294x + 0.5000, R^2 = 0.9970.$$

Fig. 1

Fig. 2

Fig. 3

Fig. 4

Fig. 5

Fig. 6

Fig. 7

Fig. 8

Fig. 9

(A, HE, ×2)　　　　　　　　　　　　　(B, HE, ×20)

(C, HE, ×2)　　　　　　　　　　　　　(D, HE, ×20)

Fig. 10

Fig. 11

Fig. 12

$\ln(K) = -18247.5/T + 62.8$
$R^2 = 0.9996$

Fig. 13

Fig. 14

$$y = 4.8101x + 0.197$$
$$R^2 = 0.9973$$

Drug release area (cm$^2$)

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210273495X
- CN 2022102727087
- CN 2022102727072
- CN 2022102791223
- CN 2023102715686
- CN 2023102715690
- CN 2023102737238
- CN 2023102715718
- CN 1727409 A